(19) Europäisches Patentamt
European Patent Office
Office européen des brevets

(11) EP 4 509 144 A1

(12) **EUROPEAN PATENT APPLICATION**
published in accordance with Art. 153(4) EPC

(43) Date of publication:
**19.02.2025 Bulletin 2025/08**

(21) Application number: **23787843.4**

(22) Date of filing: **14.04.2023**

(51) International Patent Classification (IPC):
***A61K 48/00*** (2006.01) ***A61P 35/00*** (2006.01)
***C12N 15/113*** (2010.01)

(52) Cooperative Patent Classification (CPC):
**A61K 49/0054; A61K 48/00; A61K 49/0032; A61P 35/00; C12N 15/113**

(86) International application number:
**PCT/CN2023/088466**

(87) International publication number:
**WO 2023/198201 (19.10.2023 Gazette 2023/42)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **14.04.2022 CN 202210401035**
**14.04.2022 CN 202210377179**
**14.04.2022 CN 202210386578**
**14.04.2022 CN 202210386579**
**14.04.2022 CN 202210401247**

(71) Applicant: **Suzhou Ribo Life Science Co., Ltd.**
**Suzhou, Jiangsu 215300 (CN)**

(72) Inventors:
- **WANG, Shan**
  **Suzhou, Jiangsu 215300 (CN)**
- **LIANG, Zicai**
  **Suzhou, Jiangsu 215300 (CN)**
- **ZHANG, Hongyan**
  **Suzhou, Jiangsu 215300 (CN)**
- **GAO, Shan**
  **Suzhou, Jiangsu 215300 (CN)**
- **HU, Xin**
  **Suzhou, Jiangsu 215300 (CN)**

(74) Representative: **SSM Sandmair Patentanwälte Rechtsanwalt Partnerschaft mbB**
**Joseph-Wild-Straße 20**
**81829 München (DE)**

Remarks:
The complete document including Reference Table(s) and the Sequence Listing(s) can be downloaded from the EPO website

(54) **APTAMER, CONJUGATE, COMPOSITION, METHOD FOR PREPARING SAME, AND USE THEREOF**

(57) Provided is an aptamer, comprising a continuous nucleotide sequence having a sequence set forth in formula (1). Also provided is a conjugate comprising: a delivery group formed by the aptamer, and a functional group. The aptamer provided herein can specifically target and internalize into a tumor cell. The conjugate provided can deliver the functional group to a tumor tissue in a targeted manner, so as to diagnose and/or treat tumors and tumor-related diseases.

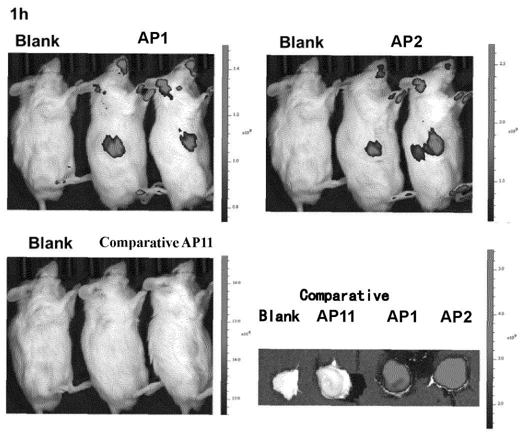

Figure 3A

EP 4 509 144 A1

## Description

### TECHNICAL FIELD

The present disclosure relates to an aptamer, and a conjugate and a pharmaceutical composition comprising a delivery group based on the aptamer. The present disclosure also relates to the preparation methods and uses of the aptamers, conjugates and pharmaceutical compositions.

### BACKGROUND ART

A tumor refers to a neoplasm formed by cell proliferation in local tissues under the action of various tumorigenic factors in the body. Among them, tumor cells that metastasize and invade surrounding tissues are called malignant tumors. According to the classification of the source tissue cells of tumors, the tumors could be generally classified into malignant tumors derived from epithelial cells (cancer), malignant tumors derived from mesenchymal tissue cells (sarcoma), malignant tumors derived from blood stem cells (leukemia, etc.), and malignant tumors derived from glial cells (gliomas). Among them, glioma is the most common primary intracranial malignant tumor, accounting for approximately 40% to 50% of brain tumors, with an annual incidence of 3 to 8 cases per 100,000 persons worldwide. According to the WHO pathological classification standards, gliomas belong to neuroepithelial tumors, which include many pathological types, including but not limited to, pilocytic astrocytoma, diffuse astrocytoma, anaplastic astrocytoma, glioblastoma, oligodendroglioma, anaplastic oligodendroglioma, etc.

Currently, one of the important issues in the art of tumor diagnosis and treatment, especially glioma, is how to specifically deliver diagnostic agents and/or therapeutic agents to tumor tissues and cells and enable these diagnostic agents and/or therapeutic agents to produce corresponding diagnostic and/or therapeutic effects at the appropriate time and in the appropriate manner.

Aptamers, also known as nucleic acid aptamers, are oligonucleotide molecules that can bind to various molecules of interest, such as small molecule compounds, proteins, nucleic acids, and even cells, tissues and organs. The aptamers can provide the important property of "recognizing specific molecules", and thus, similar to antibodies, they are commonly used in biotechnology and therapy. The aptamers can be designed in test tubes and quickly synthesized by chemical methods. They also have the excellent properties of being easy to store and having low or no immunogenicity. Therefore, they have gradually gained attention from researchers in the art in recent years. However, the aptamers suitable for tumor-targeted delivery still need to be further developed and applied in the art.

### SUMMARY OF THE INVENTION

The inventors of the present application have unexpectedly discovered an aptamer that could specifically target tumor cells, especially glioma cells. The aptamer shows high specificity to tumor cells, especially glioma cells, so that it could be effectively enriched in tumor cells, especially glioma cells. Furthermore, by covalently conjugating the aptamer with various functional groups, the resultant conjugates can effectively deliver diagnostic agents and/or therapeutic agents suitable for various types of tumors to tumor cells, especially glioma tissues and/or cells, showing excellent diagnostic and/or therapeutic effects. Thus, the inventors have made the following invention:

In one aspect, the present disclosure provides an aptamer comprising a segment of consecutive nucleotide sequence, wherein the group connecting two adjacent nucleotides is independently a phosphate ester group or a phosphate ester group with modification group(s), each nucleotide is selected from one of modified or unmodified A, U, C or G, and the consecutive nucleotide sequence has a sequence shown in Formula (1) as follows:

$$5'\text{-}T_1\text{-}S_1\text{-}N_a\text{-}S_2\text{-}N_b\text{-}S_3\text{-}N_c\text{-}S_4\text{-}T_2\text{-}3' \qquad \text{Formula (1)}$$

wherein, $T_1$ is a motif consisting of 1-3 nucleotides, $T_2$ is a motif consisting of 0-15 nucleotides, and $T_2$ does not comprise a motif that is completely reverse complementary to $T_1$;

$S_1$ and $S_4$ are each motifs consisting of 3-7 nucleotides, $S_1$ and $S_4$ are of the same length and are completely reverse complementary;

$N_a$ and $N_c$ are each motifs consisting of 1-4 nucleotides, each nucleotide in $N_a$ is not complementary to each nucleotide in Nc, and the total number of U in $N_a$ and $N_c$ accounts for more than 50% of the total number of all nucleotides in $N_a$ and $N_c$;

$S_2$ and $S_3$ are each motifs consisting of 1-4 nucleotides, $S_2$ and $S_3$ are of the same length and are completely reverse complementary;

$N_b$ is a motif consisting of 3-6 nucleotides, and the nucleotides at both ends of $N_b$ do not form AU or GC complementarity.

In another aspect, the present disclosure also provides a conjugate comprising one or more delivery groups and one or more functional groups, , wherein the delivery group is formed by removing one hydrogen atom or functional group from the aptamer provided by the present disclosure, each of the delivery groups is independently linked to the functional group via a covalent bond or via a linking group, each of the functional groups is independently one of a diagnostic agent group, a small molecule therapeutic agent group having a therapeutic effect on tumors, especially gliomas, a functional oligonucleotide group having a therapeutic effect on tumors, especially gliomas, and a delivery auxiliary group.

In yet another aspect, the present disclosure also provides a pharmaceutical composition comprising the conjugate provided by the present disclosure and a pharmaceutically acceptable carrier.

In yet another aspect, the present disclosure also provides use of the aptamer and/or conjugate and/or pharmaceutical composition provided by the present disclosure in the manufacture of a medicament for diagnosing and/or treating tumors and tumor-related diseases or symptoms.

In yet another aspect, the present disclosure also provides a method for diagnosing and/or treating tumors and tumor-related diseases or symptoms, comprising administering an effective amount of the conjugate and/or the pharmaceutical composition provided by the present disclosure to a subject in need thereof.

In yet another aspect, the present disclosure further provides a kit comprising the conjugate and/or the pharmaceutical composition provided by the present disclosure.

## INCORPORATION BY REFERENCE

All publications, patents, and patent applications mentioned in this description are incorporated by reference into the present disclosure to the same extent as if each individual publication, patent, or patent application is specifically and individually incorporated by reference into the present disclosure.

## BENEFICIAL EFFECTS

By using the aptamers provided by the present disclosure, functional groups can be specifically delivered to various tumor cells, especially glioma tissues and cells.

The conjugates and pharmaceutical compositions provided by the present disclosure have excellent ability to target tumors, especially glioma tissues and cells, and can significantly improve diagnostic accuracy of tumor related diseases and/or symptoms, and/or significantly treat or alleviate tumor related diseases and/or symptoms.

In one aspect, the conjugates provided by the present disclosure can efficiently and specifically enter into tumor cells *in vitro.* For example, compared with the comparative conjugates of random sequences, the conjugates provided by the present disclosure all show significantly higher mean fluorescence intensity in U118MG glioma cells, with a R value basically above 2, indicating that the conjugates provided by the present disclosure have excellent ability to enter into U118MG glioma cells. For another example, the conjugates provided by the present disclosure have very strong ability to enter into various cancer cells, such as, U118MG glioma cells, U251 human glioma cells, A549 human non-small cell lung cancer cells, and MCF-7 human breast cancer cells, and basically do not enter into normal cells, such as, SVGp12 normal astrocytes and 293T human renal epithelial cells. For another example, compared with comparative AP10, the conjugates provided by the present disclosure have very strong ability to enter into the interior of U118MG and A549 tumor spheres. For another example, the conjugates with different lengths and sequences provided by the present disclosure all can effectively target U118MG glioma tissue.

In a further aspect, the aptamers provided by the present disclosure can deliver various diagnostic agent groups, such as, different fluorescent groups, to tumor tissue. For another example, the different conjugates comprising fluorescent groups provided by the present disclosure all can exhibit strong fluorescence signals at the site of tumor inoculation in mice, indicating that the aptamers provided by the present disclosure can specifically target U118MG glioma. Furthermore, the conjugates provided by the present disclosure still exhibit strong fluorescence signals from 24h to 48h after administration, indicating that they can stably target glioma tissue for a long period of time. For another example, the conjugates provided by the present disclosure can still reach and target brain gliomas upon tail vein administration, implying that the aptamers provided by the present disclosure also have excellent ability to penetrate the Blood Brain Barrier (BBB) and deliver diagnostic agent groups across the blood brain barrier to brain glioma. For another example, the conjugates with various modifications provided by the present disclosure all can stably target U118MG glioma, A549 human non-small cell lung cancer tumor and PAN02 pancreatic cancer tumor cells, demonstrating broad ability to

specifically target tumors. For another example, by forming a conjugate, the aptamer provided by the present disclosure can efficiently and specifically deliver different diagnostic agent groups (such as different fluorescent groups Cy3 or Cy5) to U118MG glioma, and can remain stable for a long time. For another example, by observing with a laser confocal microscope, the results show that the conjugates provided by the present disclosure can efficiently and specifically deliver diagnostic agent groups into the tumor cells of U118MG glioma, demonstrating excellent targeting effect and potential diagnostic ability.

In a further aspect, the conjugates provided by the present disclosure can specifically deliver various small molecule drug groups, such as small molecule toxin groups, to tumor tissues, and exhibit excellent tumor inhibition effects. For example, the conjugates provided by the present disclosure can effectively deliver MMAE to tumor tissues, demonstrating tumor-targeting ability while reducing the toxicity risk caused by the distribution of MMAE molecule in other tissues. Moreover, various administration manners can effectively inhibit the growth rate of tumor volume and tumor weight, indicating that the conjugates of the present disclosure can effectively inhibit tumor proliferation. In addition, further improving the administration dosage of the conjugates can result in almost no increase in tumor volume during the testing period, demonstrating more excellent anti-tumor effects.

In a further aspect, the conjugates provided by the present disclosure can specifically deliver various functional oligonucleotide groups, such as siRNA groups, to tumor tissues and exhibit excellent tumor cell viability inhibition effects. For example, the aptamers provided by the present disclosure, after conjugating siRNA, not only can efficiently target tumor cells and inhibit the content of target mRNA, but also do not significantly affect the corresponding mRNA levels in normal cells, exhibiting excellent targeting delivery efficiency and high safety. For another example, at different times after administration, various conjugates comprising siRNA groups in the present disclosure all can effectively target and be enriched in U118MG tumor tissues. For another example, the conjugates of the present disclosure can be freely ingested into PANC1 human pancreatic cancer cells in *in vitro* experiments, and show a significant inhibitory effect on hSTAT3 mRNA. For another example, the imaging results in laser confocal imaging and high content imaging systems indicate that the conjugates comprising siRNA groups of the present disclosure can effectively enter into U118MG tumor cells, thereby facilitating the efficient generation of RNAi effect in tumor cells.

Furthermore, the inventors of the present disclosure have unexpectedly discovered that the conjugates and/or pharmaceutical compositions provided by the present disclosure can efficiently pass through the blood brain barrier and target gliomas in the brain upon systemic administration, thereby further improving the delivery efficiency of the functional groups, saving costs, and reducing undesired side effects. For example, the conjugates provided by the present disclosure can pass through the blood brain barrier and effectively target and enter into U118MG glioma in situ upon subcutaneous administration, and significantly inhibit the increase of tumor volume, or even reduce tumor volume to less than 1/10 of the initial volume, or even reduce tumor volume to less than 1/100 as compared with the control group. This indicates that the conjugates of the present disclosure can effectively penetrate the blood brain barrier and efficiently target and enter into glioma, and have a good inhibitory effect on tumor growth, demonstrating good treatment compliance and the ability of efficiently inhibiting tumors.

This indicates that the aptamers provided by the present disclosure have excellent ability of targeting tumors, especially glioma tissues and cells. Conjugates comprising the aptamers provided by the present disclosure and diagnostic agent groups (such as fluorescent developer groups) can significantly improve the success rate of tumor diagnosis, and conjugates comprising the aptamers provided by the present disclosure and therapeutic agent groups can significantly and effectively inhibit tumor growth or reduce the expression level of a cancer-related gene in tumor cells, and have good application prospects.

## BRIEF DESCRIPTION OF THE DRAWINGS

Figures 1A and 1B are respectively the high content imaging images showing AP1 and comparative AP10 entering into U118MG glioma cells and SVGp12 normal astrocytes.

Figure 1C-1G are respectively the high content imaging images showing AP1 and comparative AP10 entering into U251 human glioma cells, A549 human non-small cell lung cancer cells, MCF-7 human breast cancer cells, and 293T human renal epithelial cells.

Figures 2A and 2B are respectively the high content imaging images showing AP1 and comparative AP10 entering into U118MG glioma tumor spheres and A549 human non-small cell lung cancer tumor spheres.

Figures 3A-3D are respectively images showing the *in vivo* imaging and tumor tissue imaging of U118MG subcutaneous tumor model mice treated with different conjugates at 1h, 4h, 24h, and 48h after administration.

Figures 4A and 4B are respectively images showing the fluorescence imaging of brain tissue in U118MG tumor in situ model mice established after administration of different conjugates at 24h and 48h after administration.

Figures 5A-5E are respectively images showing the *in vivo* fluorescence imaging of mice at different time points after administering the conjugates of different sequences provided by the present disclosure and comparative conjugates. Figures 5F-5H are respectively images showing imaging of tumor tissues at different time points.

Figure 6 is an image showing the fluorescence imaging of the organs of one mouse in each group at 1h after administration of the conjugates.

Figure 7 is an image showing the fluorescence imaging of various organ tissues in PAN02 subcutaneous tumor model mice established after administration of the conjugate provided by the present disclosure.

Figures 8A-8E are respectively images showing the fluorescence imaging of the organs in mice administered with the conjugates provided by the present disclosure at 48h or 96h after administration.

Figure 9 is an image showing the fluorescence imaging of tumors in each group of mice on D9 after administration of the conjugates.

Figure 10 is a line graph showing the changes in tumor volume over time in each group of mice after administration of the conjugates provided by the present disclosure or control compounds.

Figures 11A and 11B are respectively figures showing the effects of conjugate AP1 and conjugate 19 at different concentrations on cell viability of U118-MG neuroglioma cells and SVGp12 human astrocytes.

Figure 12 is an image showing the fluorescence imaging results in mice after administration of the conjugates provided by the present disclosure comprising different linking groups.

Figures 13A-13C are respectively images showing the fluorescence imaging results in mice at 1h, 24h, and 48h after administration of different conjugates. Figure 13D is an image showing the fluorescence signal imaging of tumor tissues and kidneys in each group of mice after the mice were euthanized at D5.

Figures 14A-14C are respectively images showing the fluorescence imaging results in mice at 1h, 24h, and 48h after administration of different conjugates. Figure 14D is an image showing the fluorescence signal imaging of tumor tissues and kidneys in each group of mice after the mice were euthanized at D6.

Figure 15 is an image showing the laser confocal imaging results of U118MG glioma at 24h after administration of different conjugates.

Figures 16A and 16B are images showing the fluorescence imaging results in mice after administration of different conjugates.

Figure 17 is a bar graph showing the relative inhibition rate of the conjugates of the present disclosure against hSTAT3 mRNA in PANC-1 human pancreatic cancer cells.

Figure 18 is an image showing the laser confocal imaging results of the conjugates of the present disclosure in U118MG glioma cells.

Figure 19 is a bar graph showing the relative fluorescence intensity results of the conjugates of the present disclosure in U118MG glioma.

Figure 20 is a line graph showing the changes in tumor fluorescence intensity over time in U118MG tumor in situ model mice after administration of the conjugates provided by the present disclosure or a control compound.

Figure 21 is a line graph showing the changes in tumor volume over time in U118MG subcutaneous tumor model mice after administration of the conjugates provided by the present disclosure or a control compound.

Figure 22 is a line graph showing the changes in tumor volume over time in U118MG subcutaneous tumor model mice

after administration of the conjugates provided by the present disclosure at different concentrations or a control compound.

Figure 23 is a line graph showing the changes in tumor volume over time in U118MG subcutaneous tumor model mice after administration of the conjugates provided by the present disclosure at different concentrations or a control compound.

Figure 24 is a line graph showing the changes in tumor volume over time in A549 subcutaneous tumor model mice after administration of the conjugates provided by the present disclosure at different concentrations or a control compound.

## DETAILED DESCRIPTION OF THE INVENTION

The specific embodiments of the present disclosure are described in detail as below. It should be understood that the specific embodiments described herein are only for the purpose of illustration and explanation of the present disclosure and are not intended to limit the present disclosure.

### Definition

In the present disclosure, unless otherwise specified, A, U, C, G and T respectively refer to adenine nucleotide, uracil nucleotide, cytosine nucleotide, guanine nucleotide, and thymine nucleotide, and 2-methylcytosine nucleotide refers to a nucleotide obtained by substituting the 2' hydrogen on the cytosine base of the cytosine nucleotide with a methyl group. The structures of these nucleotides are well known to those skilled in the art. As used herein, a "nucleic acid motif" or a "motif" refers to a nucleic acid sequence fragment in an aptamer, consisting of one or more nucleotides. In some embodiments, a motif is a nucleic acid sequence fragment having a biological function.

As used herein, "alkyl" refers to straight-chain and branched chain saturated hydrocarbyl having the specified number of carbon atoms, typically from 1 to 20 carbon atoms, for example from 1 to 10 carbon atoms, such as from 1 to 8 or from 1 to 6 carbon atoms. For example, $C_1$-$C_6$ alkyl refers to both straight-chain and branched chain alkyl groups encompassing from 1 to 6 carbon atoms. When referring to an alkyl residue with a specific number of carbon atoms, it is intended to encompass all branched and straight-chain forms with that number of carbon atoms; thus, for example, "butyl" is meant to encompass n-butyl, sec-butyl, isobutyl and tert-butyl; "propyl" includes n-propyl and isopropyl. Alkylene is a subset of alkyl, referring to the same residues as alkyl, but having two attachment points.

As used herein, "alkenyl" refers to an unsaturated branched or straight-chain alkyl group having one or more carbon-carbon double bonds obtained by resp3+ectively removing one hydrogen molecule from two adjacent carbon atoms of the parent alkyl. The group can be in either cis or trans configuration of the double bond(s). Typical alkenyl groups include, but are not limited to: ethenyl; propenyl, such as prop-1-en-1-yl, prop-1-en-2-yl, prop-2-en-1-yl (allyl), prop-2-en-2-yl; butenyl, such as, but-1-en-1-yl, but-1-en-2-yl, 2-methyl-prop-1-en-1-yl, but-2-en-1-yl, but-2-en-2-yl, buta-1,3-dien-1-yl, buta-1,3-dien-2-yl; and the like. In certain embodiments, an alkenyl group has from 2 to 20 carbon atoms, while in other embodiments, from 2 to 10, from 2 to 8, or from 2 to 6 carbon atoms. Alkenylene is a subset of alkenyl, referring to the same residues as alkenyl, but having two attachment points.

As used herein, "alkynyl" refers to an unsaturated branched or straight-chain alkyl group having one or more carbon-carbon triple bonds obtained by respectively removing two molecules of hydrogen from adjacent carbon atoms of the parent alkyl. Typical alkynyl groups include, but are not limited to, ethynyl; propynyl, such as, prop-1-yn-1-yl, prop-2-yn-1-yl; butynyl, such as, but-1-yn-1-yl, but-1-yn-3-yl, but-3-yn-1-yl; and the like. In certain embodiments, an alkynyl group has from 2 to 20 carbon atoms, and in other embodiments, from 2 to 10, 2 to 8, or 2 to 6 carbon atoms. Alkynylene is a subset of alkynyl, referring to the same residues as alkynyl, but having two attachment points.

As used herein, "heterocyclyl" refers to a stable 3 to 18 membered non-aromatic ring radical that comprises 2-12 carbon atoms and 1-6 heteroatoms selected from nitrogen, oxygen or sulfur. Unless otherwise stated in the description, the heteroaryl is a monocyclic, bicyclic, tricyclic, or tetracyclic ring system, which may include fused or bridged ring system(s). The heteroatom(s) in the heteroaryl can be oxidized heteroatom(s). One or more nitrogen atoms, if present, can be quatemized nitrogen atoms. The heterocyclyl is partially or fully saturated. The heteroaryl can be linked to the rest of the molecule through any ring atom. Examples of such heterocyclic groups include, but are not limited to, dioxolanyl, thienyl [1,3]dithianyl, decahydroisoquinolyl, imidazolinyl, imidazolidinyl, isothiazolidinyl, isoxazolidinyl, morpholinyl, octahydroindolyl, octahydroisoindolyl, 2-oxapiperazinyl, 2-oxapiperidinyl, 2-oxapyrrolidinyl, oxazolidinyl, piperidinyl, piperazinyl, 4-piperidonyl, pyrrolidinyl, pyrazolidinyl, quinuclidinyl, thiazolidinyl, tetrahydrofuryl, trithianyl, tetrahydropyranyl, thiomorpholinyl, thiamorpholinyl, 1-oxo-thiomorpholinyl, and 1,1-dioxo-thiomorpholinyl. heterocyclylene is a subset of heterocyclyl, referring to the same residues as heterocyclyl , but having two attachment points.

As used herein, "aryl" refers to a radical derived from an aromatic monocyclic or multicyclic hydrocarbyl ring

system by removing hydrogen atom(s) from a ring carbon atom. The aromatic monocyclic or multicyclic hydrocarbyl ring system comprises only hydrogen and carbon, including from 6 to 18 carbon atoms, wherein one or more rings in the ring system is fully unsaturated, i.e., it comprises a cyclic, delocalized (4n+2)$\pi$-electron system in accordance with the Hückel theory. Aryl groups include, but are not limited to, groups such as phenyl, fluorenyl, and naphthyl. Arylene is a subset of aryl, referring to the same residues as aryl, but having two attachment points.

"Heteroaryl" refers to a radical derived from a 3 to 18 membered aromatic ring free radical that comprises from 2 to 17 carbon atoms and 1 to 6 heteroatoms selected from nitrogen, oxygen or sulfur. As used herein, the heteroaryl can be a monocyclic, bicyclic, tricyclic, or tetracyclic ring system, wherein one or more rings in the ring system is fully unsaturated, i.e., it comprises a cyclic, delocalized (4n+2)$\pi$-electron system in accordance with the Hückel theory. Heteroaryl includes fused or bridged ring system(s). The heteroatom(s) in the heteroaryl can be oxidized heteroatom(s). One or more nitrogen atoms, if present, can be quaternized nitrogen atoms. The heteroaryl is linked to the rest of the molecule through any ring atom. Examples of heteroaryl include, but are not limited to, azepinyl, acridinyl, benzimidazolyl, benzindolyl, 1,3-benzodioxazolyl, benzofuranyl, benzoxazolyl, benzo[d]thiazolyl, benzothiadiazolyl, benzo[b][1,4]dioxepinyl, benzo[b][1,4]oxazinyl, 1,4-benzodioxanyl, benzonaphthofuranyl, benzooxazolyl, benzodioxolyl, benzodioxinyl, benzopyranyl, benzopyranonyl, benzofuranyl, benzofuranonyl, benzothienyl, benzothieno[3,2-d]pyrimidinyl, benzotriazolyl, benzo[4,6]imidazo[1,2-a]pyridinyl, carbazolyl, cinnolinyl, cyclopenta[d]pyrimidinyl, 6,7-dihydro-5H-cyclopenta[4,5]thieno[2,3-d]pyrimidinyl, 5,6-dihydrobenzo[h]quinazolinyl, 5,6-dihydrobenzo[h]cinnolinyl, 6,7-dihydro-5H-benzo[6,7]cyclohepta[1,2-c]pyridazinyl, dibenzofuranyl, dibenzothienyl, furanyl, furanonyl, furo[3,2-c]pyridinyl, 5,6,7,8,9,10 hexahydrocycloocta[d]pyrimidinyl, 5,6,7,8,9,10-hexahydrocyclohepta[d]pyridazinyl, 5,6,7,8,9,10- hexahydrocycloocta[d]pyridinyl, isothiazolyl, imidazolyl, indazolyl, indolyl, isoindolyl, indolinyl, isoindolinyl, isoquinolyl, indolizinyl, isoxazolyl, 5,8-methano-5,6,7,8-tetrahydroquinazolinyl, naphthyridinonyl, 1,6-naphthyridinonyl, oxadiazolyl, 2-oxoazepinyl, oxazolyl, oxiranyl, 5,6,6a,7,8,9,10,10a-octahydrobenzo[h]quinazolinyl, 1-phenyl-1H-pyrrolyl, phenazinyl, phenothiazinyl, phenoxazinyl, phthalazinyl, pteridinyl, purinyl, pyrrolyl, pyrazolyl, pyrazolo[3,4-d]pyrimidinyl, pyridinyl, pyrido[3,2-d]pyrimidinyl, pyrido[3,4-d]pyrimidinyl, pyrazinyl, pyrimidinyl, pyridazinyl, pyrrolyl, quinazolinyl, quinoxalinyl, quinolinyl, tetrahydroquinolinyl, 5,6,7,8-tetrahydroquinazolinyl, 5,6,7,8-tetrahydrobenzo[4,5]thieno[2,3-d]pyrimidinyl, 6,7,8,9-tetrahydro-5H-cyclohepta[4,5]thieno[2,3-d]pyrimidinyl, 5,6,7,8-tetrahydropyrido[4,5-c]pyridazinyl, thiazolyl, thiadiazolyl, triazolyl, tetrazolyl, triazinyl, thieno[2,3-d]pyrimidinyl, thieno[3,2-d]pyrimidinyl, thieno[2,3-c]pridinyl and thiophenyl/thienyl. Heteroarylene is a subset of heteroaryl, referring to the same residues as heteroaryl, but having two attachment points.

Aptamers provided by the present disclosure

In one aspect, the present disclosure provides an aptamer, comprising a consecutive nucleotide sequence, wherein the group linking two adjacent nucleotides is independently a phosphate ester group or a phosphate ester group with modification group(s), and each nucleotide is selected from one of modified or unmodified A, U, C or G. The consecutive nucleotide sequence has the sequence shown by Formula (1):

$$5'\text{-}T_1\text{-}S_1\text{-}N_a\text{-}S_2\text{-}N_b\text{-}S_3\text{-}N_c\text{-}S_4\text{-}T_2\text{-}3' \qquad \text{Formula (1)}$$

wherein $T_1$ is a motif consisting of 1-3 nucleotides. The inventors have found that the presence of $T_1$ may facilitate the aptamer provided by the present disclosure to exhibit high tumor-targeting effect. In some embodiments, $T_1$ consists of 2 nucleotides. In this case, the aptamer provided by the present disclosure has more excellent tumor-targeting ability. In some embodiments, $T_1$ consists of 2 nucleotides and comprises at least one C. In some embodiments, $T_1$ is CU, UC or AC in the 5'-3' direction.

$T_2$ is a motif consisting of 0-15 nucleotides. The inventors have found that $T_2$ with these numbers of nucleotides and with various nucleotide sequences does not significantly affect the tumor-targeting ability of the aptamers. In some embodiments, $T_2$ is a motif consisting of 0-10 nucleotides. In some embodiments, in the 5'-3' direction, $T_2$ consists of 1-9 nucleotides starting with U. In this case, the aptamer may have more excellent stability.

$T_2$ does not comprise the motif that is complete reverse complementary to $T_1$. In the context of the present disclosure, "reverse complementary" means that hydrogen bond linkages are formed between two nucleotide sequences or motifs according to the rules of nucleic acid base pairing, and each nucleotide of one nucleotide sequence or motif in the 5'-3' direction can subsequentially form base pairs with each nucleotide of the other end nucleotide sequence or motif in the 3'-5' direction. In some embodiments, "reverse complementary" includes one or more of AU, GC, and UG complementarity.

In the aptamer provided by the present disclosure, $S_1$ and $S_4$ are each motifs consisting of 3-7 nucleotides, while $S_1$ and $S_4$ are of the same length and are completely reverse complementary. The aptamer with the motifs $S_1$ and $S_4$ above has better stability and can target tumor tissues and cells over a long period of time. In some embodiments, $S_1$ and $S_4$ each consist of 3-5 nucleotides and are of the same length. In some embodiments, in the reverse complement formed by $S_1$ and $S_4$, GC complementarity accounts for more than 40% of the total number of complementarity. In this case, the aptamer

provided by the present disclosure has further more excellent stability and tumor-targeting ability. In some embodiments, in the 5'-3' direction, $S_1$ is GCU and $S_4$ is AGC, or $S_1$ is GAGU and $S_4$ is GCUC, or $S_1$ is GGAGU and $S_4$ is GCUCU, or $S_1$ is UAUGG and $S_4$ is CCAUG.

In the aptamer provided by the present disclosure, $N_a$ and $N_c$ are each motifs consisting of 1-4 nucleotides. Each nucleotide in $N_a$ is not complementary to each nucleotide in $N_c$, and the total number of U in $N_a$ and $N_c$ accounts for more than 50% of the total number of all nucleotides in $N_a$ and $N_c$. The aptamer with the motifs $N_a$ and $N_c$ above shows excellent tumor tissue-targeting ability, and when the $N_a$ and $N_c$ motifs are missing or the total number of U in $N_a$ and $N_c$ is insufficient, the aptamer basically shows no tumor tissue-targeting effect. In some embodiments, the sum of the number of nucleotides in $N_a$ and $N_c$ is an integer of 2-4. In some embodiments, the sum of the number of nucleotides in $N_a$ and $N_c$ is 3 or 4, and the sum of the number of U in $N_a$ and $N_c$ is 2 or 3. In some embodiments, in the 5'-3' direction, $N_a$ and/or $N_c$ are U, UU, UC or CU.

In the aptamer provided by the present disclosure, $S_2$ and $S_3$ are each motifs consisting of 1-4 nucleotides, while $S_2$ and $S_3$ are of the same length and are completely reverse complementary. By comprising the motifs $S_2$ and $S_3$, the aptamer provided by the present disclosure shows good stability and excellent tumor-targeting ability. In some embodiments, $S_2$ and $S_3$ each consist of 2-3 nucleotides and are of the same length. In some embodiments, the reverse complement formed by $S_2$ and $S_3$ comprises at least one GC complementarity, and in this case, the reverse complementarity has better stability. In some embodiments, in the 5'-3' direction, $S_2$ is CA and $S_3$ is UG, or $S_2$ is AC and $S_3$ is GU, or $S_2$ is GCC and $S_3$ is GGU.

$N_b$ is a motif consisting of 3-6 nucleotides, and the nucleotides at both ends of $N_b$ do not form AU or GC complementarity. Without being limited by theory, the aptamer with the motif $N_b$ above can maintain a specific configuration in terms of space, thereby enabling the aptamer provided by the present disclosure to stably and efficiently target tumor tissues and cells. In some embodiments, $N_b$ consists of 4-5 nucleotides. In some embodiments, in the 5'-3' direction, $N_b$ is GACG, GACGU, GACCG, UACU, GUUG or GAUCU.

The inventors of the present disclosure have unexpectedly found that the aptamer with the sequence shown in Formula (1) above provided by the present disclosure can effectively target tumors, especially glioma tissues, thereby allowing the aptamer provided by the present disclosure as a delivery vehicle to deliver the functional group with diagnostic/therapeutic effect on tumors to the tumors. Further, the aptamer provided by the present disclosure is able to specifically enter tumor cells and thus deliver diagnostic/therapeutic agent groups more effectively at cellular level, even genetic level.

In some embodiments, in the aptamer provided by the present disclosure, the consecutive nucleotide sequence has a length of 18-50 nucleotides, or 20-40 nucleotides, or 21-36 nucleotides, or 24-32 nucleotides. The aptamers having these lengths of the consecutive nucleotide sequences can more easily target tumors and achieve a good balance between synthesis cost and targeting effect.

In some embodiments, in the aptamer provided by the present disclosure, the consecutive nucleotide sequences have the following sequences as shown in SEQ ID NO: 1, SEQ ID NO: 2, or SEQ ID NO: 3:

5'-CUGCUUCAGACGUGUUUAGCUU-3' (SEQ ID NO: 1)
wherein, in the 5'-3' direction, $T_1$ is CU, $S_1$ is GCU, $N_a$ is U, $S_2$ is CA, $N_b$ is GACG, $S_3$ is UG, $N_c$ is UU, $S_4$ is AGC, $T_2$ is UU;

5'-CUGAGUUCAGACGUGUUGCUCU-3' (SEQ ID NO: 2)
wherein, in the 5'-3' direction, $T_1$ is CU, $S_1$ is GAGU, $N_a$ is U, $S_2$ is CA, $N_b$ is GACG, $S_3$ is UG, $N_c$ is UU, $S_4$ is GCUC, $T_2$ is U;

5'- UCUAUGGCUGCCGAUCUGGUCUCCAUGUACGU -3' (SEQ ID NO: 3),
wherein, in the 5'-3' direction, $T_1$ is CU, $S_1$ is GAGU, $N_a$ is U, $S_2$ is CA, $N_b$ is GACG, $S_3$ is UG, $N_c$ is UU, $S_4$ is GCUC, $T_2$ is U.

In some embodiments, the consecutive nucleotide sequence has the nucleotide sequence as shown in SEQ ID NO: 4:

5'- $N_6$GGAGUUCA$N_1N_2N_3N_4$UG$N_5$GCUC$N_7$-3' (SEQ ID NO: 4),
wherein, $N_1$, $N_2$ and $N_3$ independently of one another are one of A, U, C and G, $N_4$ is U, C or G or a motif consisting of two of U, C or G; $N_5$ is U, CU or UU; $N_6$ is CU, UC or AC; $N_7$ is U, UU or UU$N_8$, and $N_8$ is a motif consisting of 1-15 nucleotides.

In the nucleotide sequence above, in the 5'-3' direction, $T_1$ is the motif as shown by $N_6$, $S_1$ is the motif represented by GGAGU, $N_a$ is U, $S_2$ is CA, $N_b$ is the motif $N_1N_2N_3N_4$ consisting of $N_1$, $N_2$, $N_3$ and $N_4$, $S_3$ is UG, $N_c$ is the motif represented by $N_5$, $S_4$ is the motif consisting of GCUC and the first nucleotide in $N_7$, and $T_2$ is the motif consisting of the remaining nucleotides in $N_7$.

The aptamer comprising the nucleotide sequences as shown in SEQ ID NO: 4 above can more effectively target tumors, especially gliomas, and can be enriched in tumor tissues.

Experimental verification shows that, in the nucleotide sequence as shown in SEQ ID NO: 4, the above selection of $N_1$, $N_2$, $N_3$ and $N_4$ does not significantly affect the tumor-targeting ability of the aptamer provided by the present disclosure. In some embodiments, the motif $N_1N_2N_3N_4$ consisting of $N_1$, $N_2$, $N_3$ and $N_4$ is one of GACG, GACGU, GACCG, UACU, GUUG, or GAUCU, and the aptamers comprising these motifs have higher tumor-specific targeting effects.

In some embodiments, in the nucleotide sequence as shown in SEQ ID NO: 4, $N_5$ is U or UU. In this case, the aptamers provided by the present disclosure all have excellent tumor-targeting effects.

In some embodiments, the consecutive nucleotide sequence has any one of the nucleotide sequences as shown in SEQ ID NOs: 5-11:

5'- CUGGAGUUCAGACGUGUUGCUCUU -3' (SEQ ID NO: 5)
5'- CUGGAGUUCAGACGUUGUGCUCUU -3' (SEQ ID NO: 6)
5'- CUGGAGUUCAGACCGUGUGCUCUU -3' (SEQ ID NO: 7)
5'- CUGGAGUUCAGACGUGUUGCUCU -3' (SEQ ID NO: 8)
5'- ACGGAGUUCAGACGUGUUGCUCUU -3' (SEQ ID NO: 9)
5'- CUGGAGUUCACUACUGUUGCUCUU -3' (SEQ ID NO: 10)
5'- CUGGAGUUCAGUUGUGUUGCUCUU -3' (SEQ ID NO: 11).

The aptamers having the nucleotide sequences above provided by the present disclosure show highly tumor-targeting effects.

In some embodiments, the motif $N_8$ consists of 1-15 nucleotides. In some embodiments, $N_8$ consists of 1-8 nucleotides.

In some embodiments, the presence of the motif $N_8$ makes the aptamer provided by the present disclosure more stable against an *in vivo* exonuclease, thereby enabling it to exert the tumor-targeting effect *in vivo* for a long period of time. In some embodiments, $N_8$ can increase or maintain the tumor-targeting effect of the aptamer provided by the present disclosure. In some embodiments, the motif $N_8$ consists of 8 nucleotides, considering the balance among stability, targeting, and synthesis efficiency. In some embodiments, in the 5'-3' direction, the motif $N_8$ is CCGAUCUC. In some embodiments, the consecutive nucleotide sequence has any one of the nucleotide sequences as shown in SEQ ID NOs: 12-14:

5'-CUGGAGUUCAGACGUGUUGCUCUUCCGAUCUC-3' (SEQ ID NO: 12)
5'-CUGGAGUUCAGACGUUGUGCUCUUCCGAUCUC-3' (SEQ ID NO: 13)
5'-CUGGAGUUCAGACCGUGUGCUCUUCCGAUCUC-3' (SEQ ID NO: 14)

In the aptamer provided by the present disclosure, the terminal groups at the 5' end of the ribose of the 5' terminal nucleotide and at the 3' end of the ribose of the 3' terminal nucleotide are independently hydroxyl or phosphate groups, and the selection of these terminal groups does not change the targeting ability of the aptamer provided by the present disclosure. In some embodiments, the terminal groups at the 5' end of the ribose of the 5' terminal nucleotide and at the 3' end of the ribose of the 3' terminal nucleotide are hydroxyl groups in the aptamer provided by the present disclosure.

In the aptamer provided by the present disclosure, each nucleotide may be a modified or unmodified nucleotide. In general, modifications of nucleotides may alter the stability and/or the tumor-targeting ability of the aptamer provided by the present disclosure. In some embodiments, at least one nucleotide in the aptamer provided by the present disclosure is a modified nucleotide. In some embodiments, at least one group connecting two adjacent nucleotides in the aptamer provided by the present disclosure is a phosphate ester group with modification group(s).

The modification of nucleotide includes, but are not limited to, modification of sugar, modification of base, and/or substitution of a nucleotide with a nucleotide analog. In some embodiments, in the aptamer provided by the present disclosure, each of the modified nucleotides independently is one of 2'-halogen modified nucleotide, 2'-alkoxy modified nucleotide, 2'-alkyl modified nucleotide, 2'-substituted alkyl modified nucleotide, 2'-amino modified nucleotide, 2'-substituted amino modified nucleotide, 2'-deoxy nucleotide, a nucleotide with a modified base, and a nucleotide analog.

In the context of the present disclosure, a "fluoro modified nucleotide" refers to a nucleotide formed by substituting the 2'-hydroxy of the ribose group of the nucleotide with a fluoro group, which has a structure as shown by the following Formula (7). A "non-fluoro modified nucleotide" refers to a nucleotide formed by substituting the 2'-hydroxy of the ribose group of the nucleotide with a non-fluoro group, or a nucleotide analogue. In some embodiments, each non-fluoro modified nucleotide is independently selected from the group consisting of a nucleotide formed by substituting the 2'-hydroxy of the ribose group of the nucleotide with a non-fluoro group, or a nucleotide analogue.

These nucleotides formed by substituting the 2'-hydroxy of the ribose group with a non-fluoro group are well-known to those skilled in the art, and can be selected from one of 2'-alkoxy modified nucleotide, 2'-alkyl modified nucleotide, 2'-substituted alkyl modified nucleotide, 2'-amino modified nucleotide, 2'-substituted amino modified nucleotide, and 2'-deoxy nucleotide.

In some embodiments, the 2'-alkoxy modified nucleotide is a methoxy modified nucleotide (2'-OMe), as shown by Formula (8). In some embodiments, the 2'-amino modified nucleotide (2'-$NH_2$) is as shown by Formula (9). In some embodiments, the 2'-deoxy nucleotide (DNA) is as shown by Formula (10):

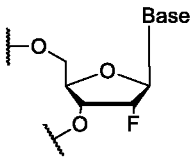 , 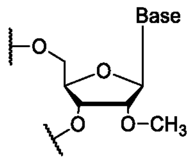 , 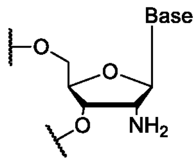 , 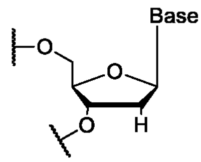 .

Formula (7) Formula (8) Formula (9) Formula (10)

Those skilled in the art know various ways of modifying the bases of nucleotides. In some embodiments, base modification includes, but is not limited to, adding one or more methyl groups to a base. In some embodiments, thymine (T) is considered as one uracil (U) with a modified base. In some embodiments, 2-methylcytosine is considered as one cytosine (C) with a modified base.

A "nucleotide analogue" refers to a group that can replace a nucleotide in the nucleic acid, while structurally differs from an adenine ribonucleotide, a guanine ribonucleotide, a cytosine ribonucleotide, a uracil ribonucleotide or thymine deoxyribonucleotide. In some embodiments, a nucleotide analogue may be an isonucleotide, a bridged nucleic acid (bridged nucleic acid, abbreviated as BNA) nucleotide or an acyclic nucleotide.

A BNA refers to a nucleotide that is constrained or is not accessible. BNA can comprise a 5-, 6- membered or even a 7-membered ring bridged structure with a "fixed" C3'-endo sugar puckering. The bridge is typically incorporated at the 2'- and 4'-position of the ribose to afford a 2', 4'-BNA nucleotide. In some embodiments, BNA may be LNA, ENA and cET BNA, wherein LNA is as shown by Formula (12), ENA is as shown by Formula (13) and cET BNA is as shown by Formula (14).

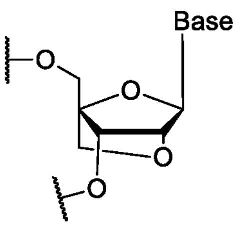 , 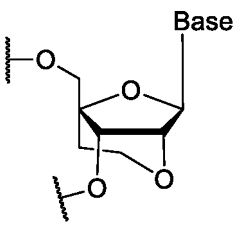 , 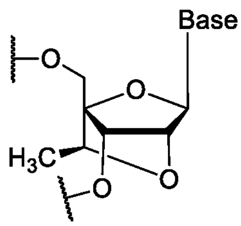 .

Formula (12) Formula (13) Formula (14)

An acyclic nucleotide is a nucleotide in which the ribose ring is opened. In some embodiments, an acyclic nucleotide may be an unlocking nucleic acid (UNA), a glycerol nucleic acid (GNA) or a peptide nucleic acids (PNA), wherein UNA is as shown by Formula (15) and GNA is as shown by Formula (16):

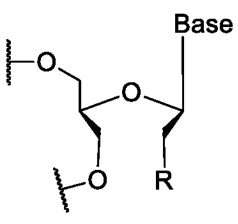 , 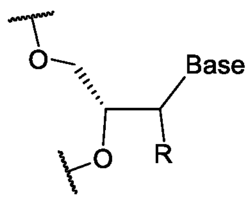 .

Formula (15) Formula (16)

In the above Formula (15) and Formula (16), R is selected from H, OH, or alkoxy (O-alkyl).

A peptide nucleic acid is a nucleotide analog formed by replacing the glycoside-phosphate backbone with a polypeptide backbone. In some embodiments, the peptide nucleic acid may be, for example, a nucleotide analog formed by replacing the glycoside-phosphate unit with a 2-aminoethyl glycine bond.

An isonucleotide is a compound in which the position of the base on the ribose ring in the nucleotide is changed. In some embodiments, an isonucleotide may be a compound in which the base is transposed from position-1' to position-2' or -3' on the ribose ring, as shown by Formula (17) or (18), respectively.

In the above compounds of Formulae (17)-(18), "Base" represents a nucleic acid base, such as, A, U, G, C, or T; R

is selected from H, OH, F, or a non-fluoro group above.

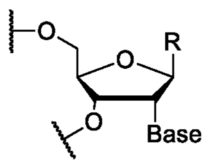
Formula (17)

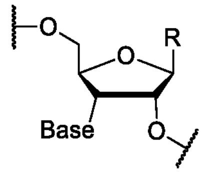
Formula (18)

In some embodiments, a nucleotide analogue is selected from the group consisting of an isonucleotide, LNA, ENA, cET, UNA, and GNA.

In some embodiments, each non-fluoro modified nucleotide is a methoxy modified nucleotide. In the context of the present disclosure, the methoxy modified nucleotide refers to a nucleotide formed by substituting the 2'-hydroxy of the ribose group with a methoxy group.

In the context of the present disclosure, a "fluoro modified nucleotide", a "2'-fluoro modified nucleotide", a "nucleotide in which 2'-hydroxy of the ribose group is substituted with a fluoro group" and a "nucleotide comprising 2'-fluororibosyl" have the same meaning, referring to the nucleotide formed by substituting the 2'-hydroxy of the ribose group with a fluoro group, having the structure as shown by Formula (7). A "methoxy modified nucleotide", a "2'-methoxy modified nucleotide", a "nucleotide in which 2'-hydroxy of a ribose group is substituted with a methoxy" and a "nucleotide comprising 2'-methoxyribosyl" have the same meaning, referring to a compound in which 2'-hydroxy of the ribose group in the nucleotide is substituted with a methoxy, and has a structure as shown by Formula (8).

In some embodiments, each cytosine nucleotide of the consecutive nucleotide sequence in the aptamer provided by the present disclosure is a fluoro modified cytosine nucleotide, and/or each uracil nucleotide of the consecutive nucleotide sequence in the aptamer provided by the present disclosure is a fluoro modified uracil nucleotide. In some embodiments, each nucleotide of the consecutive nucleotide sequence in the aptamer provided by the present disclosure is a 2'-methoxy modified nucleotide. In some embodiments, one or more uracil nucleotides in the aptamer provided by the present disclosure have modified bases.

The group connecting two adjacent nucleotides may be a phosphate ester group or a modified phosphate ester group. The modification of a phosphate ester group may be, for example, substituting at least one non-bridging oxygen atom in a phosphate ester group with a sulfur atom to form a phosphorothioate group or a phosphorodithioate group. In some embodiments, at least one group connecting two adjacent nucleotides in the aptamer provided by the present disclosure is a phosphorothioate group. In some embodiments, at least one of the three groups connecting two adjacent nucleotides in the first four nucleotides at the 5' terminal of the aptamer provided by the present disclosure is a phosphorothioate group. In some embodiments, at least two of the three groups connecting two adjacent nucleotides in the first four nucleotides at the 5' terminal of the aptamer provided by the present disclosure are phosphorothioate groups. In some embodiments, at least one group connecting two adjacent nucleotides in the first four nucleotides at the 3' terminal of the aptamer provided by the present disclosure is a phosphorothioate groups. In some embodiments, at least two of the three groups connecting two adjacent nucleotides in the first four nucleotides at the 5' terminal of the aptamer provided by the present disclosure are phosphorothioate groups. In some embodiments, each group connecting two adjacent nucleotides in the aptamer provided by the present disclosure is a phosphorothioate group.

The aptamer with the modification above not only has low cost, but also can make the aptamer less susceptible to cleavage by *in vivo* ribonuclease, thereby increasing the stability of the aptamer and make it more resistant to nuclease hydrolysis. Meanwhile, the aptamer with the modification above has higher activity of targeting tumor tissues and/or cells.

In some embodiments, the consecutive nucleotide sequence has one of the nucleotide sequences as shown in SEQ ID NOs: 15-39:

5'- CfUfGGAGUfUfCfAGACfGUfGUfUfGCfUfCfUfUfCfCfGAUfCfUfCf -3' (SE Q ID NO: 15)
5'- CfUfGGAGUfUfCfAGACfGUfGUfUfGCfUfCfUfUf -3' (SEQ ID NO: 16)
5'- CfUfGGAGUfUfCfAGACfGUfUfGUfGCfUfCfUfUfCfCfGAUfCfUfCf -3' (SE Q ID NO: 17)
5'- CfUfGGAGUfUfCfAGACfGUfUfGUfGCfUfCfUfUf -3' (SEQ ID NO: 18)
5'- CfUfGGAGUfUfCfAGACfCfGUfGUfGCfUfCfUfUfCfCfGAUfCfUfCf -3' (SE Q ID NO: 19)
5'- CfUfGGAGUfUfCfAGACfCfGUfGUfGCfUfCfUfUf -3' (SEQ ID NO: 20)
5'- CmUmGmGmAmGmUmUmCmAmGmAmCmGmUmGmUmUmGmCmUmCm UmUmCmCmGmAmUmCmUmCm -3' (SEQ ID NO: 21)
5'- CmUmGmGmAmGmUmUmCmAmGmAmCmGmUmGmUmUmGmCmUmCm UmUm -3' (SEQ ID NO: 22)
5'- CmUmGmGmAmGmUmUmCmAmGmAmCmGmUmUmGmUmGmCmUmCm UmUmCmCmGmAmUmCmUmCm -3' (SEQ ID NO: 23)
5'- CmUmGmGmAmGmUmUmCmAmGmAmCmGmUmUmGmUmGmCmUmCm UmUm -3' (SEQ ID NO: 24)
5'- CmUmGmGmAmGmUmUmCmAmGmAmCmCmGmUmGmUmGmCmUmCm UmUmCmCmGmAmUmCmUmCm -3' (SEQ ID NO: 25)
5'- CmUmGmGmAmGmUmUmCmAmGmAmCmCmGmUmGmUmCrmCmUmCm UmUm -3' (SEQ ID NO: 26)
5'- CmUmGmGmAmGmUmUmCmAmGmAmCmGmUmGmUmUmGmCmUmCm Um -3' (SEQ ID NO: 27)
5'- CmUmGmAmGmUmUmCmAmGmAmCmGmUmGmUmUmGmCmUmCmUm -3' (SEQ ID NO: 28)
5'- CmUmGmCmUmUmCmAmGmAmCmGmUmGmUmUmAmGmCmUmUm -3' (SEQ ID NO: 29)
5'- AmCmGmGmAmGmUmUmCmAmGmAmCmGmUmGmUmUmGmCmUmCm UmUm -3' (SEQ ID NO: 30)
5'- CmUmGmGmAmGmUmUmCmAmCmUmAmCmUmGmUmUmGmCmUmCm UmUm -3' (SEQ ID NO: 31)
5'- CmUmGmGmAmGmUmUmCmAmGmUmUmGmUmGmUmUmGmCmUmCm UmUm -3' (SEQ ID NO: 32)
5'- UmCmUmAmUmGmGmCmUmGmCmCmGmAmUmCmUmGmGmUmCmUm CmCmAmUmGmUmAmCmGmUm -3' (SEQ ID NO: 33)
5'- CmsUmsGmGmAmGmUmUmCmAmGmAmCmGmUmGmUmUmGmCmUmC mUmUmCmCmGmAmUmCmsUmsCm -3' (SEQ ID NO: 34)
5'- CmsUmsGmGmAmGmUmUmCmAmGmAmCmGmUmGmUmUmGmCmUmC msUmsUm -3' (SEQ ID NO: 35)
5'- CmsUmsGmGmAmGmUmUmCmAmGmAmCmGmUmUmGmUmGmCmUmC mUmUmCmCmGmAmUmCmsUmsCm -3' (SEQ ID NO: 36)
5'- CmsUmsGmGmAmGmUmUmCmAmGmAmCmGmUmUmGmUmGmCmUmC msUmsUm -3' (SEQ ID NO: 37)
5'- CmsUmsGmGmAmGmUmUmCmAmGmAmCmCmGmUmGmUmGmCmUmC mUmUmCmCmGmAmUmCmsUmsCm -3' (SEQ ID NO: 38)
5'- CmsUmsGmGmAmGmUmUmCmAmGmAmCmCmGmUmGmUmGmCmUmC msUmsUm -3' (SEQ ID NO: 39)

wherein C, G, U, and A represent the base composition of the nucleotide; m represents that the nucleotide adjacent to the left side of the letter m is a methoxy modified nucleotide; f represents that the nucleotide adjacent to the left side of the letter f is a fluoro modified nucleotide; s represents that the two nucleotides adjacent to both sides of the letter s are linked by a phosphorothioate linkage.

The aptamer provided by the present disclosure can be obtained by the conventional preparation methods of oligonucleotides in the art, such as solid phase synthesis and liquid phase synthesis methods, wherein solid phase synthesis has commercial customized services. A modified nucleotide can be introduced into the aptamer provided by the present disclosure by using a nucleoside monomer with a corresponding modification. The methods for preparing a nucleoside monomer with a corresponding modification and the methods for introducing a modified nucleotide into the aptamers are also well known to those skilled in the art. All nucleoside monomers with modifications can be commercially available or prepared by known methods.

Conjugates

In another aspect, the present disclosure provides a conjugate, comprising one or more delivery groups and one or more functional groups, wherein the delivery group is formed by removing one or more hydrogen atoms or one or more functional groups from the aptamer provided by the present disclosure; each of the delivery group is independently linked to the functional group via a covalent bond or via a linking group; each of the functional group is independently one of a diagnostic agent group, a small molecule therapeutic agent group having a therapeutic effect on tumors, a functional oligonucleotide group having a therapeutic effect on tumors, and a delivery auxiliary group. By linking functional groups through a covalent bond or a linking group to form conjugates, the conjugates provided by the present disclosure can deliver functional groups conjugated to tumors.

The delivery group is formed by removing one or more hydrogen atoms or one or more functional groups from the aptamer provided by the present disclosure. In some embodiments, the 5' group of the ribose of the 5' terminal nucleotide and the 3' group of the ribose of the 3' terminal nucleotide of the aptamer provided by the present disclosure are hydroxyl groups, and the delivery group is formed by removing one hydrogen atom in the 5' hydroxyl of the 5' terminal nucleotide from the aptamer provided by the present disclosure; in some embodiments, the delivery group is formed by removing one hydrogen atom in the 3' hydroxyl of the 3' terminal nucleotide from the aptamer provided by the present disclosure; in some embodiments, the delivery group is formed by removing 5' hydroxyl in the 5' terminal nucleotide from the aptamer provided by the present disclosure; in some embodiments, the delivery group is formed by removing 3' hydroxyl in the 3' terminal nucleotide from the aptamer provided by the present disclosure. In some embodiments, the delivery group is formed by removing the 2'-hydroxyl of the ribose in the nucleotide from the aptamer provided by the present disclosure.

In some embodiments, the conjugate provided by the present disclosure has a structure as shown by Formula (101):

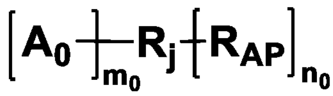

(101)

wherein each $R_{AP}$ group is independently a group having a structure as shown by Formula (102):

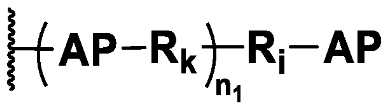

(102)

wherein each AP group is identical or different, and independently represents one of the delivery groups; each $A_0$ group is identical or different, and independently represents one of the functional groups; $R_j$, each $R_k$ or each $R_i$ is identical or different, and independently represents a covalent bond or a linking group respectively and, and $R_i$ and $R_k$ are not covalent bonds at the same time; $m_0$ is an integer of 1 to 6; $n_0$ is an integer of 1 to 6; each $n_1$ independently of one another represents an integer of 0 to 4; - represents the site where the group is covalently linked.

In some embodiments, $m_0$ is an integer of 1 to 6, that is, the conjugate represented by Formula (101) comprises 1 to 6 of the functional groups $A_0$. Considering delivery efficiency and cost, in some embodiments, $m_0$ is an integer of 1 to 4, that is, the conjugate represented by Formula (101) comprises 1 to 4 of the functional groups $A_0$. In some embodiments, $m_0$ is 1, that is, the conjugate represented by Formula (101) comprises one of the functional group $A_0$.

In some embodiments, $n_0$ is an integer of 1 to 6, that is, the conjugate represented by Formula (101) comprises 1 to 6 $R_{AP}$ groups. Considering delivery efficiency and cost, in some embodiments, $n_0$ is an integer of 1 to 3, that is, the conjugate represented by Formula (101) comprises 1 to 3 $R_{AP}$ groups. In some embodiments, $n_0$ is 1, that is, the conjugate represented by Formula (101) comprises one $R_{AP}$ group.

In some embodiments, each $n_1$ independently of each other represents an integer of 0 to 4, and $R_i$ and $R_k$ are not covalent bonds at the same time, so that each $R_{AP}$ group comprises 1 to 5 delivery groups AP obtained from the aptamer provided by the present disclosure. In some embodiments, each $n_1$ independently of each other represents an integer of 0 to 1, so that each $R_{AP}$ group comprises 1-2 delivery groups AP. In some embodiments, $n_0$ is 1 and $n_1$ is 0; in thi case, the conjugate represented by Formula (101) comprises one functional group AP.

In the $R_{AP}$ group, the function of $R_k$ and $R_i$ is to covalently link the delivery group AP to the $R_j$ group, and link to the functional group $A_0$ through the $R_j$ group. Therefore, as long as the above-mentioned linkage can be achieved, any $R_k$ or $R_i$ that has no negative affects on the delivery group AP and the functional group $A_0$ can be used in the present invention. In some embodiments, each of the $R_k$ or each of the $R_i$ is independently a straight-chain alkylene having a length of 1-70 carbon atoms, or one or more carbon atoms in the straight-chain alkylene are replaced by one or more groups selected from the group consisting of: C(O), NH, O, S, CH=N, $S(O)_2$, $OP(O)_2$, OP(O)(S), $C_5$-$C_8$ glycosidylene, $C_2$-$C_{10}$ alkenylene, $C_2$-$C_{10}$ alkynylene, $C_6$-$C_{10}$ arylene, $C_3$-$C_{18}$ heterocyclylene, and $C_5$-$C_{10}$ heteroarylene; and wherein the straight-chain alkylene may have any one or more substituents selected from the group consisting of: $C_1$-$C_{10}$ alkyl, $C_6$-$C_{10}$ aryl, $C_5$-$C_{10}$ heteroaryl, $C_1$-$C_{10}$ haloalkyl, -$OC_1$-$C_{10}$ alkyl, -$OC_1$-$C_{10}$ alkylphenyl, -$C_1$-$C_{10}$ alkyl-OH, -$OC_1$-$C_{10}$ haloalkyl, -$SC_1$-$C_{10}$ alkyl, -$SC_1$-$C_{10}$ alkylphenyl, -$C_1$-$C_{10}$ alkyl-SH, -$SC_1$-$C_{10}$ haloalkyl, halogen substituent, -OH, -SH, -$NH_2$, -$C_1$-$C_{10}$ alkyl-$NH_2$, -N($C_1$-$C_{10}$ alkyl)($C_1$-$C_{10}$ alkyl), -NH($C_1$-$C_{10}$ alkyl), -N($C_1$-$C_{10}$ alkyl)($C_1$-$C_{10}$ alkylphenyl), -NH($C_1$-$C_{10}$ alkylphenyl), cyano, nitro, -$CO_2$H, -C(O)O($C_1$-$C_{10}$ alkyl), -CON($C_1$-$C_{10}$ alkyl)($C_1$-$C_{10}$ alkyl), -CONH($C_1$-$C_{10}$ alkyl), -$CONH_2$, -NHC(O)($C_1$-$C_{10}$ alkyl), -NHC(O)(phenyl), -N($C_1$-$C_{10}$ alkyl)C(O)($C_1$-$C_{10}$ alkyl), -N($C_1$-$C_{10}$ alkyl)C(O)(phenyl), -C(O)$C_1$-$C_{10}$ alkyl, -C(O) $C_1$-$C_{10}$ alkylphenyl, -C(O)$C_1$-$C_{10}$ haloalkyl, -OC(O)$C_1$-$C_{10}$ alkyl, -$SO_2$($C_1$-$C_{10}$ alkyl), - $SO_2$(phenyl), -$SO_2$($C_1$-$C_{10}$ haloalkyl), -$SO_2NH_2$, -$SO_2$NH($C_1$-$C_{10}$ alkyl), -$SO_2$NH(phenyl), -$NHSO_2$($C_1$-$C_{10}$ alkyl), -$NHSO_2$(phenyl), and -$NHSO_2$ ($C_1$-$C_{10}$ haloalkyl). Considering synthesis cost and synthesis difficulty and the tumor-targeting effect of the conjugates, in some embodiments, each $n_1$ is 0, and each $R_i$ is independently a covalent bond, or is one following linking group, or one connection combination of more than one following linking groups: $C_1$-$C_{20}$ alkylene, phosphate ester bond, phosphorothioate bond, amide bond, ester bond, ether bond, thioether bond, disulfide bond, 1,2,3- triazolylene group, a polyethylene glycol subunit, pyrrolidinylene group, 2-oxopyrrolidinylene group, phenylene, cyclohexylene, 2-succinimidylene group, 2-thiosuccinimidylene group, an amino acid subunit and a nucleotide subunit.

In some embodiments, the linking group $R_j$ comprises the linking groups that are well known to those skilled in the art to be useful for antibody-conjugated drugs. The linking group $R_j$ may be cleavable or non-cleavable. In some embodiments, the linking group $R_j$ may be cleavable. In the context, "cleavable" means that after the conjugate of the present disclosure targets a tumor, the covalent bond of the linking group $R_j$ undergoes cleavage in the tumor environment and/or within tumor cells, releasing an individual functional group to produce a therapeutic or diagnostic effect. In some

embodiments, the linking group $R_j$ comprises one or more of an active enzyme linking group, a sulfatase-cleavable linking group, a galactose-cleavable linking group, a lysosomal protease-sensitive linking group, a peptidyl linking group, a glucuronide linking group, an acid-sensitive cleavable linking group, or a glutathione-sensitive disulfide linking group. In some embodiments, the linking group $R_j$ comprises a peptidyl linking group. In some embodiments, the peptidyl linking group is selected from one or more of a valine-citrulline dipeptide linker (Val-Cit), an alanine-alanine dipeptide linker (Ala-Ala), a valine-alanine dipeptide linker (Val-Ala), a glycine-glycine-phenylalanine-glycine tetrapeptide linker (Gly-Gly-Phc-Gly). In some embodiments, the linking group $R_j$ is selected from one of N-succinimidyl 4-(2-pyridyldithio)butyrate (SPDB), N- succinimidyl-4-(2-thiopyridinylene) pentanoate (SPP), (S)-2-((S)-2-amino-3-methylbutanamido)-5-ureidopentanoic acid (Val-Cit-PAB-OH), N-succinimidyl-4-(N-maleimidomethyl)cyclohexane-1-carboxylate (SMCC) or 2-(phosphate-$(CH_2)_6$-S-)-maleimidohexanoyl-valine-citrulline-p-aminobenzylene. In some embodiments, the linking group $R_j$ comprises the linking groups listed in Mckertish CM, Kayser V. Advances and Limitations of Antibody Drug Conjugates for Cancer. Biomedicines. 2021 Jul 23; 9 (8): 872., which are incorporated herein by reference in their entirety.

In some embodiments, the linking group $R_j$ comprises one or more of a valine-citrulline dipeptide linker (Val-Cit), a polyethylene glycol subunit, an iminohexylene, an N-succinimidyl group and a GAU trinucleotide linking group. In some embodiments, each $R_i$ is independently one following group or one connection combination of two following groups: a covalent bond, a disulfide bond, a dodecylene group, a valine-citrulline dipeptide linker (Val-Cit), a polyethylene glycol subunit, an iminohexylene, an N-succinimidyl group or a GAU trinucleotide subunit.

The function of the group $R_j$ is to connect the $R_{AP}$ group and the functional group $A_0$, thereby the functional group $A_0$ is specifically delivered to tumor tissues and/or cells via the tumor-targeting effect of the delivery group AP in the $R_{AP}$ group. Therefore, any $R_j$ group that is capable of achieving the above-mentioned linkage without affecting the tumor-targeting function of the delivery group AP and the effect of the functional group $AP_0$ could achieve the purpose of the present invention and solve the technical problem to be solved by the present invention. In some embodiments, after the conjugate represented by Formula (101) reaches tumor tissues and/or enters tumor cells, the cleavage of $R_j$ takes place, releasing the pharmaceutically active molecule corresponding to the individual functional group $A_0$. In some embodiments, the $R_j$ is not cleaved *in vivo,* while the presence of the $R_j$ group and the $R_{AP}$ group in the conjugate would not affect the diagnostic and/or therapeutic effect of the functional group $A_0$.

In some embodiments, $R_j$ is a covalent bond, $m_0$ is 1; in this case, the conjugate represented by formula (101) comprises one functional group $A_0$ and one $R_{AP}$ group, and each $R_{AP}$ group is directly linked to the functional group $A_0$. In some embodiments, each $R_{AP}$ group is linked to the same atom of the functional group $A_0$. In some embodiments, each $R_{AP}$ group is linked to different atoms of the functional group $A_0$.

In some embodiments, $R_j$ is a linking group, which comprises a main chain moiety, a side chain moiety, and a conjugation linking moiety.

The main chain moiety is respectively linked to the conjugation linking moiety and the side chain moiety. In some embodiments, the main chain moiety is a straight-chain alkylene group having a length of 1-70 carbon atoms, or one or more carbon atoms in the straight-chain alkylene group are replaced by one or more groups selected from the group consisting of: C(O), NH, O, S, CH=N, $S(O)_2$, $OP(O)_2$, $C_5$-$C_8$ glycosidylene, $C_2$-$C_{10}$ alkenylene, $C_2$-$C_{10}$ alkynylene, $C_6$-$C_{10}$ arylene, $C_3$-$C_{18}$ heterocyclylene, and $C_5$-$C_{10}$ heteroarylene; and wherein the straight-chain alkylene may have any one or more substituents selected from the group consisting of: $C_1$-$C_{10}$ alkyl, $C_6$-$C_{10}$ aryl, $C_5$-$C_{10}$ heteroaryl, $C_1$-$C_{10}$ haloalkyl, -$OC_1$-$C_{10}$ alkyl, -$OC_1$-$C_{10}$ alkylphenyl, -$C_1$-$C_{10}$ alkyl-OH, -$OC_1$-$C_{10}$ haloalkyl, -$SC_1$-$C_{10}$ alkyl, -$SC_1$-$C_{10}$ alkylphenyl, -$C_1$-$C_{10}$ alkyl-SH, -$SC_1$-$C_{10}$ haloalkyl, halogen substituent, -OH, -SH, -$NH_2$, -$C_1$-$C_{10}$ alkyl-$NH_2$, -N($C_1$-$C_{10}$ alkyl)($C_1$-$C_{10}$ alkyl), -NH($C_1$-$C_{10}$ alkyl), -N($C_1$-$C_{10}$ alkyl) ($C_1$-$C_{10}$ alkylphenyl), -NH($C_1$-$C_{10}$ alkylphenyl), cyano, nitro, -$CO_2$H, -C(O)O($C_1$-$C_{10}$ alkyl), -CON($C_1$-$C_{10}$ alkyl)($C_1$-$C_{10}$ alkyl), -CONH($C_1$-$C_{10}$ alkyl), -$CONH_2$, -NHC(O)($C_1$-$C_{10}$ alkyl), -NHC(O)(phenyl), -N($C_1$-$C_{10}$ alkyl)C(O)($C_1$-$C_{10}$ alkyl), -N($C_1$-$C_{10}$ alkyl)C(O)(phenyl), -C(O)$C_1$-$C_{10}$ alkyl, -C(O)$C_1$-$C_{10}$ alkylphenyl, -C(O)$C_1$-$C_{10}$ haloalkyl, -OC(O)$C_1$-$C_{10}$ alkyl, -$SO_2$($C_1$-$C_{10}$ alkyl), -$SO_2$(phenyl), - $SO_2$($C_1$-$C_{10}$ haloalkyl), -$SO_2NH_2$, -$SO_2$NH($C_1$-$C_{10}$ alkyl), -$SO_2$NH(phenyl), - $NHSO_2$($C_1$-$C_{10}$ alkyl), -$NHSO_2$(phenyl), and -$NHSO_2$($C_1$-$C_{10}$ haloalkyl).

The side chain moiety is respectively linked to the main chain moiety and the $R_{AP}$ group. In some embodiments, each side chain moiety is independently a covalent bond or a straight-chain alkylene group having a length of 1-70 carbon atoms, or one or more carbon atoms in the straight-chain alkylene group are replaced by one or more groups selected from the group consisting of: C(O), NH, O, S, CH=N, $S(O)_2$, $OP(O)_2$, $C_5$-$C_8$ glycosidylene, $C_2$-$C_{10}$ alkenylene, $C_2$-$C_{10}$ alkynylene, $C_6$-$C_{10}$ arylene, $C_3$-$C_{18}$ heterocyclylene, and $C_5$-$C_{10}$ heteroarylene; and wherein the straight-chain alkylene has any one or more substituents selected from the group consisting of: $C_1$-$C_{10}$ alkyl, $C_6$-$C_{10}$ aryl, $C_5$-$C_{10}$ heteroaryl, $C_1$-$C_{10}$ haloalkyl, -$OC_1$-$C_{10}$ alkyl, -$OC_1$-$C_{10}$ alkylphenyl, -$C_1$-$C_{10}$ alkyl-OH, -$OC_1$-$C_{10}$ haloalkyl, -$SC_1$-$C_{10}$ alkyl, -$SC_1$-$C_{10}$ alkylphenyl, -$C_1$-$C_{10}$ alkyl-SH, -$SC_1$-$C_{10}$ haloalkyl, halogen substituent, -OH, -SH, -$NH_2$, -$C_1$-$C_{10}$ alkyl-$NH_2$, -N($C_1$-$C_{10}$ alkyl)($C_1$-$C_{10}$ alkyl), -NH($C_1$-$C_{10}$ alkyl), -N($C_1$-$C_{10}$ alkyl) ($C_1$-$C_{10}$ alkylphenyl), -NH($C_1$-$C_{10}$ alkylphenyl), cyano, nitro, -$CO_2$H, -C(O)O($C_1$-$C_{10}$ alkyl), -CON($C_1$-$C_{10}$ alkyl)($C_1$-$C_{10}$ alkyl), -CONH($C_1$-$C_{10}$ alkyl), -$CONH_2$, -NHC(O)($C_1$-$C_{10}$ alkyl), -NHC(O)(phenyl), -N($C_1$-$C_{10}$ alkyl)C(O)($C_1$-$C_{10}$ alkyl), -N($C_1$-$C_{10}$ alkyl)C(O)(phenyl), -C(O)$C_1$-$C_{10}$ alkyl, -C(O)$C_1$-$C_{10}$ alkylphenyl, -C(O)$C_1$-$C_{10}$ haloalkyl, -OC(O)$C_1$-$C_{10}$ alkyl, -$SO_2$($C_1$-$C_{10}$ alkyl), -$SO_2$(phenyl), - $SO_2$($C_1$-$C_{10}$ haloalkyl), -$SO_2NH_2$, -$SO_2$NH($C_1$-$C_{10}$ alkyl), -$SO_2$NH(phenyl), - $NHSO_2$($C_1$-$C_{10}$ alkyl), -$NHSO_2$(phenyl), and -$NHSO_2$($C_1$-$C_{10}$

haloalkyl).

The conjugation linking moiety is respectively linked to the main chain moiety and the functional group $A_0$. In some embodiments, each conjugation linking moiety is independently a covalent bond, or one following linking structure, or one connection combination of more than one following linking structures: $C_1$-$C_{10}$ straight-chain alkylene, phosphate ester bond, phosphorothioate bond, amide bond, ester bond, ether bond, disulfide bond, 1,2,3-triazolylene group, a polyethylene glycol subunit, pyrrolidinylene group, 2-oxopyrrolidinylene group, phenylene, cyclohexylene, 2-succinimidylene group, 2-thiosuccinimidylene group, an amino acid subunit and a nucleotide subunit.

In some embodiments, each of the conjugation linking moiety in the linking group $R_j$ is respectively linked to the main chain moiety and one of the functional groups $A_0$; the number of the side chain moieties is $n_0$ and each side chain moiety is respectively linked to the main chain moiety and one of the $R_{AP}$ groups. Thus, each functional group $A_0$ and the $R_{AP}$ group independently of one another are linked to a linking group $R_j$. In some embodiments, all side chain moieties are linked to the same atom in the main chain moiety; alternatively, each side chain moiety is linked to different atoms in the main chain moiety.

In some embodiments, $m_0$ is 1, and the linking group $R_j$ comprises the structure as shown in Formula (301):

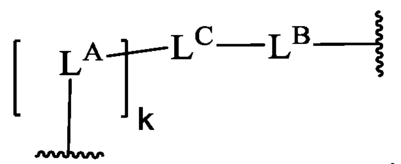
,

Formula (301)

wherein k is an integer of 1 to 3; $L^C$ is the main chain moiety, $L^A$ is the side chain moiety, $L^B$ is the conjugation linking moiety, and - represents the site where the group is covalently linked.

The main chain moiety $L^C$ is a covalent bond or a 2-4 valence, straight-chain or branched $C_1$-$C_{25}$ saturated hydrocarbyl, or one or more carbon atoms in the saturated hydrocarbyl are replaced by one or more groups selected from the group consisting of: C(O), NH, O, S, CH=N, $S(O)_2$, $OP(O)_2$, $C_5$-$C_8$ glycosidylene, $C_2$-$C_5$ alkenylene, $C_2$-$C_5$ alkynylene, $C_6$-$C_{10}$ arylene, $C_3$-$C_8$ heterocyclylene, and $C_5$-$C_{10}$ heteroarylene; and wherein the saturated hydrocarbyl may have any one or more substituents selected from the group consisting of: $C_1$-$C_5$ alkyl, $C_6$-$C_{10}$ aryl, $C_5$-$C_{10}$ heteroaryl, -O-$C_1$-$C_5$ alkyl, -$OC_1$-$C_5$ alkylphenyl, -$C_1$-$C_5$ alkyl-OH, -$SC_1$-$C_5$ alkyl, nitro, -C(O)O($C_1$-$C_5$ alkyl), -CON($C_1$-$C_5$ alkyl)($C_1$-$C_5$ alkyl), -CONH($C_1$-$C_5$ alkyl), -$CONH_2$, -NHC(O)($C_1$-$C_5$ alkyl), -NHC(O)(phenyl), -N($C_1$-$C_5$ alkyl)C(O)($C_1$-$C_5$ alkyl), -N($C_1$-$C_5$ alkyl)C(O)(phenyl), -C(O)$C_1$-$C_5$ alkyl, -C(O)$C_1$-$C_5$ alkylphenyl, -OC(O)$C_1$-$C_5$ alkyl, -$SO_2$($C_1$-$C_5$ alkyl), -$SO_2$(phenyl), -$SO_2NH_2$, -$SO_2$NH($C_1$-$C_5$ alkyl), -$SO_2$NH(phenyl), -$NHSO_2$($C_1$-$C_5$ alkyl), and -$NHSO_2$(phenyl). In some embodiments, $L^C$ is a 2-4 valence, $C_1$-$C_{25}$ saturated hydrocarbyl, or one or more carbon atoms in the saturated hydrocarbyl are replaced by one or more groups selected from the group consisting of: C(O), NH, O, S, CH=N, $S(O)_2$, $OP(O)_2$, $C_5$-$C_8$ glycosidylene, $C_2$-$C_5$ alkenylene, $C_2$-$C_5$ alkynylene, $C_6$-$C_{10}$ arylene, $C_3$-$C_8$ heterocyclylene, and $C_5$-$C_{10}$ heteroarylene; and wherein the saturated hydrocarbyl may have any one or more substituents selected from the group consisting of: $C_1$-$C_5$ alkyl, $C_6$-$C_{10}$ aryl, $C_5$-$C_{10}$ heteroaryl, -O-$C_1$-$C_5$ alkyl, -$OC_1$-$C_5$ alkylphenyl, -$C_1$-$C_5$ alkyl-OH, -$SC_1$-$C_5$ alkyl, nitro, and -$CONH_2$. In some embodiments, $L^C$ is of 5-30 atoms in length, wherein the length of $L^C$ refers to the number of the chain-forming atoms in the longest chain of atoms formed by the atom directly linked to $L^A$ to the atom directly linked to $L^B$ in $L^C$. To simplify the structure, in some embodiments, $L^C$ is of 8-25 atoms in length.

The side chain moiety $L^A$ is a covalent bond, or $C_1$-$C_{20}$ alkylene group, or one or more carbon atoms in the alkylene group are replaced by one or more groups selected from the group consisting of: C(O), NH, O, S, CH=N, $S(O)_2$, $OP(O)_2$, $C_5$-$C_8$ glycosidylene, $C_2$-$C_5$ alkenylene, $C_2$-$C_5$ alkynylene, $C_6$-$C_{10}$ arylene, $C_3$-$C_8$ heterocyclylene, and $C_5$-$C_{10}$ heteroarylene; and wherein the alkylene may have any one or more substituents selected from the group consisting of: $C_1$-$C_5$ alkyl, $C_6$-$C_{10}$ aryl, $C_5$-$C_{10}$ heteroaryl, -O-$C_1$-$C_5$ alkyl, -$OC_1$-$C_5$ alkylphenyl, -$C_1$-$C_5$ alkyl-OH-$SC_1$-$C_5$ alkyl, -$SC_1$-$C_5$ alkylphenyl, -$C_1$-$C_5$ alkyl-SH, -OH, -SH, -$NH_2$, -$C_1$-$C_5$ alkyl-$NH_2$, -N($C_1$-$C_5$ alkyl)($C_1$-$C_5$ alkyl), -NH($C_1$-$C_5$ alkyl), -N($C_1$-$C_5$ alkyl) ($C_1$-$C_5$ alkylphenyl), -NH($C_1$-$C_5$ alkylphenyl), nitro, -C(O)O($C_1$-$C_5$ alkyl), -CON($C_1$-$C_5$ alkyl)($C_1$-$C_5$ alkyl), -CONH($C_1$-$C_5$ alkyl), -$CONH_2$, -NHC(O)($C_1$-$C_5$ alkyl), -NHC(O)(phenyl), -N($C_1$-$C_5$ alkyl)C(O)($C_1$-$C_5$ alkyl), -N($C_1$-$C_5$ alkyl)C(O)(phenyl), -C(O)$C_1$-$C_5$ alkyl, -C(O)$C_1$-$C_5$ alkylphenyl, -OC(O)$C_1$-$C_5$ alkyl, -$SO_2$($C_1$-$C_5$ alkyl), -$SO_2$(phenyl), -$SO_2NH_2$, -$SO_2$NH($C_1$-$C_5$ alkyl), -$SO_2$NH(phenyl), -$NHSO_2$($C_1$-$C_5$ alkyl), and -$NHSO_2$(phenyl).

The conjugation linking moiety $L^B$ is one following linkage, or one connection combination of 2-5 following linkages: phosphate ester bond, phosphorothioate bond, amide bond, ester bond, ether bond, disulfide bond.

In some embodiments, k is an integer of 1 to 3; $L^C$ comprises any one of the groups represented by formula (L1) - (L3), and is linked to $L^A$ moeity through an ether bond in the group represented by formula (L1) - (L3) :

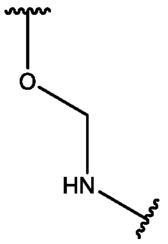

(L1)

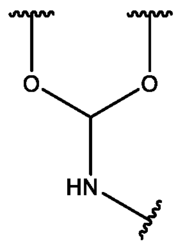

(L2)

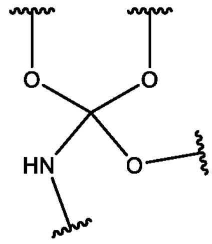

(L3)



represents the site where a group is linked to the rest of the molecule.

In some embodiments, k = 1, $L^C$ comprises a group represented by Formula (L1), and the oxygen atom in the group (L1) is directly linked to $L^A$. In some embodiments, k = 2, $L^C$ comprises a group represented by Formula (L2), and each of two oxygen atoms in the group (L1) is directly linked to one $L^A$. In some embodiments, k = 4, $L^C$ comprises a group represented by Formula (L3), and each of three oxygen atoms in the group (L1) is directly linked to one $L^A$.

$L^B$ is a phosphate ester bond or a disulfide bond.

Each $L^A$ is a covalent bond, or each $L^A$ is selected from the group consisting of the groups (L4)-(L23) and the connection combinations thereof:

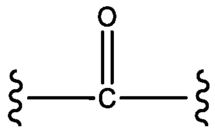, 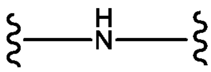, 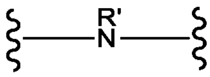, 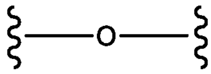,

(L4) (L5) (L6) (L7)

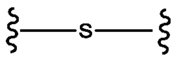, 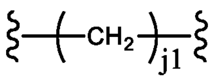, 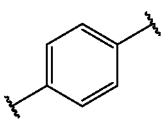, 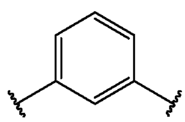,

(L8) (L9) (L10) (L11)

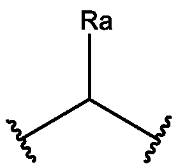, 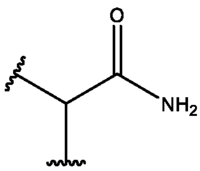, 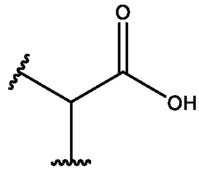, 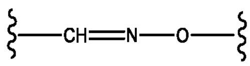,

(L12) (L13) (L14) (L15)

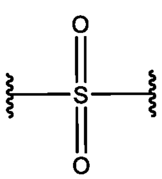, 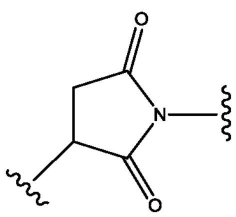, 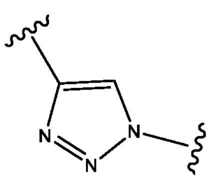, 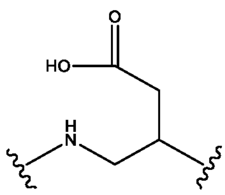,

(L16) (L17) (L18) (L19)

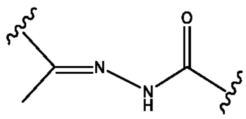, 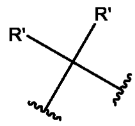, 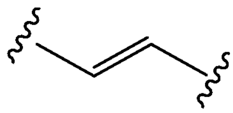 and 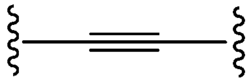;

(L20) (L21) ( L22) (L23)

In the formula, each j 1 is an integer of 1 to 10;

each R' is a $C_1$-$C_{10}$ alkyl;

each Ra is a hydrogen atom, $C_1$-$C_{10}$ alkyl, or selected from the group consisting of the groups (L24)-(L37):

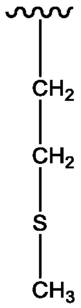, 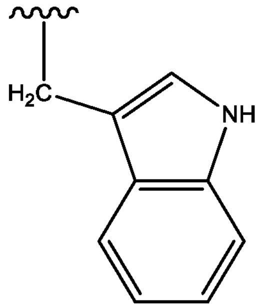, 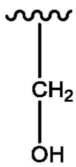, 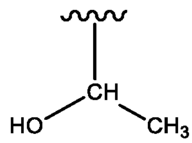, 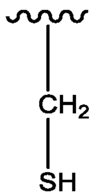, 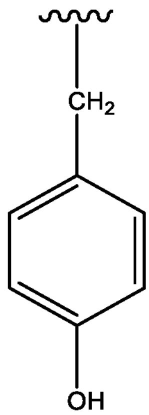,

(L24) (L25) (L26) (L27) (L28) (L29)

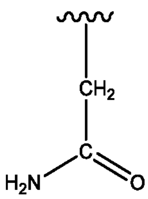, 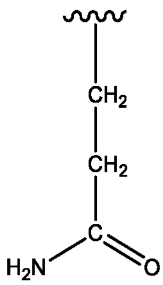, 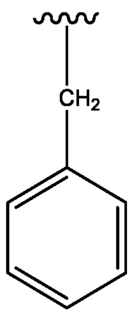, 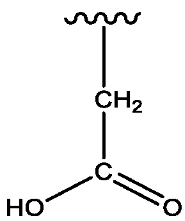, 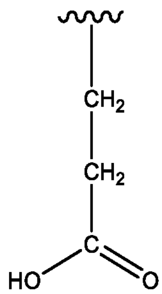,

(L30) (L31) (L32) (L33) (L34)

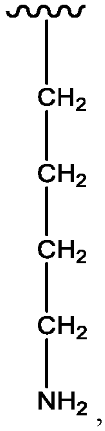,
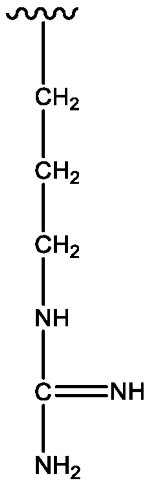
and
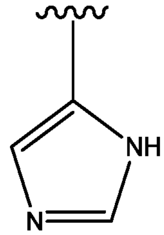.

(L35) (L36) (L37)

In some embodiments, $L^A$ is of 3-35 atoms in length, wherein the length of the $L^A$ refers to the number of the chain-forming atoms in the longest chain of atoms formed by the atom directly linked to $L^C$ to the atom directly linked to $R_{AP}$ in $L^A$. In some embodiments, each $L^A$ is a connection combination of at least two of the groups (L4) to (L9), (L13), (L14), and (L18). In some embodiments, each $L^A$ is a connection combination of at least two of the groups (L4), (L5), (L7), (L9), (L13), (L14) and (L18).

In some embodiments, $L^A$ has an amide bond-containing structure as shown by Formula (302); $L^B$ has a *N*-acylpyrrolidine-containing structure as shown by Formula (303), comprising carbonyl groups and oxygen atoms; $L^C$ is a linking group based on (hydroxymethyl)aminomethane, bis(hydroxymethyl)aminomethane, or tris(hydroxymethyl)aminomethane:

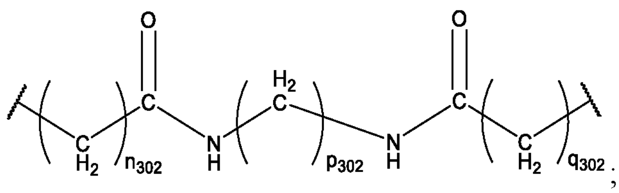;

Formula (302)

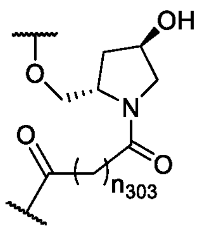;

Formula (303)

wherein $n_{302}$, $q_{302}$, and $p_{302}$ independently of one another are an integer of 2-6 and, optionally, $n_{302}$, $q_{302}$, and $p_{302}$ independently of one another are 2 or 3; $n_{303}$ is an integer of 4-16 and, optionally, $n_{303}$ is an integer of 8-12, and 〰 represents the site where a group is covalently linked.

In some embodiments, each of the side chain moieties $L^A$ is linked to an $R_{AP}$ group via a phosphate ester bond, an ether bond, or an ester bond, and is linked to said main chain moiety $L^C$ by forming an ether bond with the oxygen atom of a hydroxyl group in the main chain moiety $L^C$; the conjugation linking moiety $L^B$ is linked to the main chain moiety $L^C$ by forming an amide bond between the nitrogen atom of the amino group in the main chain moiety $L^C$ and the carbonyl group in Formula (303), and is linked to the functional group $A_0$ by forming a phosphate ester bond, an ether bond, or an ester bond between the oxygen atom in Formula (303) and the functional group $A_0$. In some embodiments, the main chain moiety $L^C$ is a linking group based on (hydroxymethyl)aminomethane, bis(hydroxymethyl)aminomethane, or tris(hydroxymethyl)aminomethane, and the main chain moiety $L^C$ is linked to each of the side chain moieties $L^A$ through an ether bond via the oxygen atom of the hydroxyl group, and is linked to the conjugation linking moiety $L^B$ through an amide bond via the nitrogen atom of the amino group. Therefore, 1-3 side chains in the linking group $R_j$ are linked to the carbon atom of the same aminomethyl group, and are linked to delivery group-containing $R_{AP}$ groups via the conjugation linking moiety $L^B$.

In some embodiments, the conjugate has a structure as shown by Formula (305):

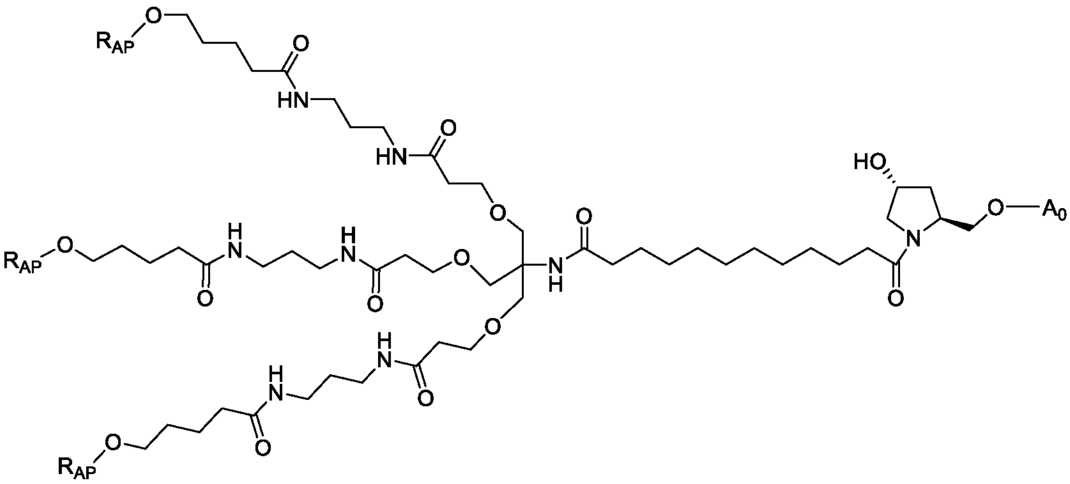

Formula (305).

In some embodiments, the linking group $R_j$ comprises a structure as shown by Formula (306):

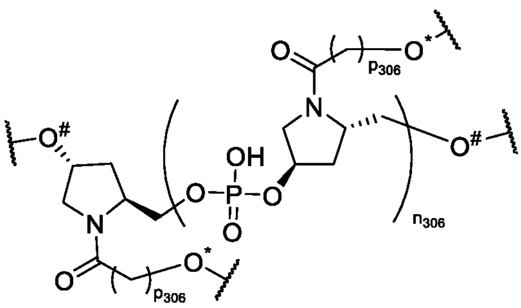

,

Formula (306)

wherein $n_{306}$ is an integer of 0-3, each $p_{306}$ is independently an integer of 1-6, and represents the site where a group is covalently linked; the connection combination formed by all pyrrolidinylene groups and any possible phosphodiester group constitutes the main chain moiety, the atomic chain between the carbonyl group linked to the nitrogen atom on a pyrrolidinylene group and an oxygen atom marked with * constitutes each side chain moiety, and the side chain moiety is linked to an $R_{AP}$ group by forming an ether bond between the oxygen atom marked with * and the $R_{AP}$ group; at least one of the oxygen atoms marked with # is a conjugation linking moiety and is linked to the functional group $A_0$ by forming an ether bond, an ester bond, or a phosphate ester bond with the functional group $A_0$, and the other oxygen atom marked with # is linked to a hydrogen atom to form a hydroxyl group, or is linked to a $C_1$-$C_3$ alkyl group to form a $C_1$-$C_3$ alkoxy group. Therefore, 1-3 side chain moieties in the linking group $R_j$ are linked to different carbon atoms in the main chain moiety, and are linked to delivery-group-containing $R_{AP}$ groups via an oxygen atom.

In some embodiments, the conjugate provided by the present disclosure has a structure as shown by Formula (307a), (307b), or (307c):

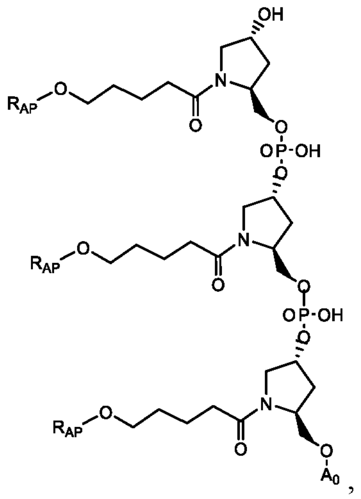,
Formula (307a)

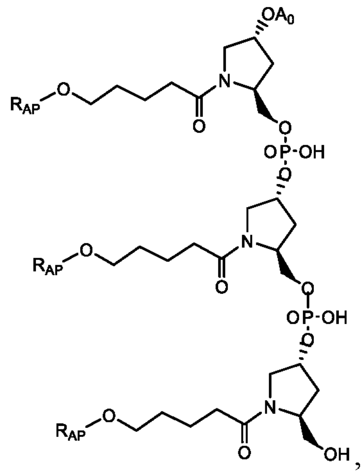,
Formula (307b)

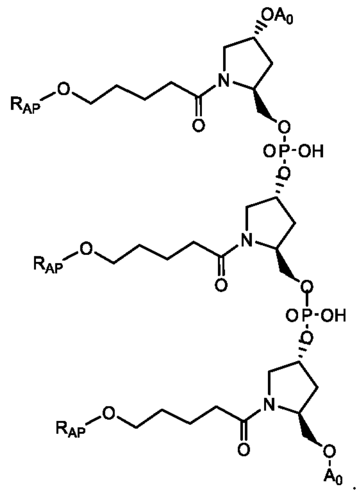.
Formula (307c)

In some embodiments, the conjugate provided by the present disclosure has a structure as shown by Formula (308):

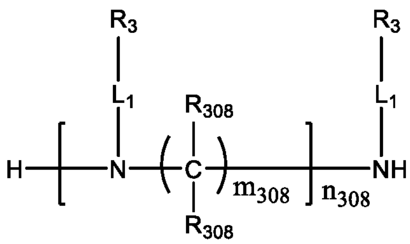,

Formula (308)

wherein $n_{308}$ may be an integer of 1-10; in some embodiments, in view of various factors such as synthesis convenience, structure/process costs, and tumor cell specificity, $n_{308}$ is an integer of 2-6. In some embodiments, $n_{308}$ is 3 or 4.

Each $R_3$ is independently a functional group $A_0$, or a delivery group AP-containing $R_{AP}$ group. In some embodiments, at least one $R_3$ is a functional group $A_0$, and at least one $R_3$ is the $R_{AP}$ group. In some embodiments, one $R_3$ is a functional group $A_0$, and the other $R_3$ are the $R_{AP}$ groups.

In some embodiments, when each $m_{308}$ is independently an integer of 2-10, it is considered that in the conjugate, the spatial position among multiple delivery group APs may be more suitable for interaction with corresponding receptors on the surface of tumor cells. In order to make the compound as shown by Formula (308) have simpler structure, easier synthesis, and/or reduced costs, according to some embodiments of the present disclosure, each $m_{308}$ is independently an integer of 2-5; in some embodiments, each $m_{308}$ is equal.

Those skilled in the art could understand that when each $R_{308}$ is independently selected from H, $C_1$-$C_{10}$ alkyl, $C_1$-$C_{10}$ haloalkyl, and $C_1$-$C_{10}$ alkoxy, they would not change the properties of the conjugate as shown by Formula (308) and could all realize the purpose of the present disclosure. In some embodiments, each $R_{308}$ is independently selected from H, methyl or ethyl. In some embodiments, each $R_{308}$ is H.

Each $L_1$ linked to the functional group $A_0$ represents the conjugation linking moiety, and each $L_1$ linked to the $R_{AP}$ represents the side chain moiety. In some embodiments, one $R_3$ is the functional group $A_0$, and the other $R_3$ are the $R_{AP}$ groups. In some embodiments, one or more $L_1$, as the side chain moieties, link the $R_{AP}$ group to the N atom of the nitrogen-containing backbone; and the other one or more $L_1$, as the conjugation linking moieties, link the functional group $A_0$ to the N atom on the nitrogen-containing backbone. The nitrogen-containing backbones together form the main chain moiety of the linking group $R_j$. In the context of the present disclosure, a "nitrogen-containing backbone" refers to the chain structure in the structure as shown by Formula (308), wherein the N atom is coadjacently linked to the carbon atom where $R_{308}$ is linked.

Considering delivery efficiency and synthesis costs, in some embodiments, each $L_1$ independently has a length of 3-25 atoms. In some embodiments, each $L_1$ independently has a length of 4-15 atoms. Those skilled in the art would understand that, though $L_1$ is defined as a linear alkylene for convenience, it may not be a linear group or may have different names, such as an amine or alkenylene produced by the above replacement and/or substitution. For the purpose of the

present disclosure, the length of $L_1$ is the number of the atoms in the chain linking the two attachment points. For this purpose, for the ring obtained by replacing a carbon atom in the straight-chain alkylene(e.g., a heterocyclylene or heteroarylene), the length of the portion in the chain corresponding to the ring is calculated according to the minimum number of atoms between attachment points on the ring.

In some embodiments, $L_1$ is selected from the group consisting of the groups as shown by Formulae L4-L23 above and any connection combination thereof. In some embodiments, each $L_1$ is independently selected from the group consisting of the connection combinations of at least two of the groups L4-L9, L13, L14, and L18. In some embodiments, each $L_1$ is independently a connection combination of at least two of the groups L4, L5, L7, L9, L13, L14, and L18.

In some embodiments, in the conjugate as shown by Formula (308), each $L_1$ has both a site linking to the N atom on the nitrogen-containing backbone and a site linking to the functional group $A_0$ or the $R_{AP}$ group, and the site linking to the N atom on the nitrogen-containing backbone forms an amide bond with the N atom. In some embodiments, one or more $L_1$ are selected from B5, B6, B5', or B6':

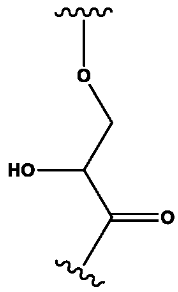
,
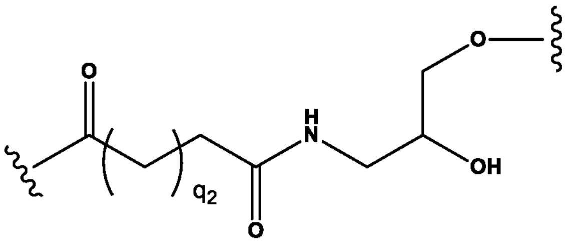
;

(B5) (B6)

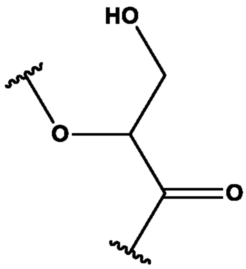
,
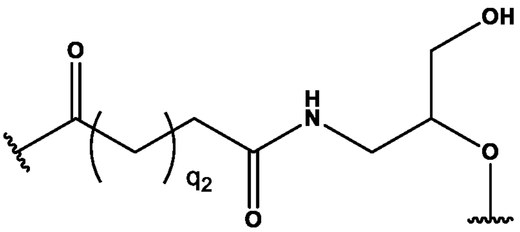
;

(B5') (B6')

wherein represents the site where a group is covalently linked, and $q_2$ is an integer of 1-10. In some embodiments, $q_2$ is an integer of 1-5.

In some embodiments, each $R_{AP}$ group comprises one or more delivery groups. In some embodiments, the compound as shown by Formula (308) comprises multiple functional groups. In some embodiments, each functional group in the compound of Formula (308) is the same functional group. In some embodiments, each functional group in the compound of Formula (308) is a functional group for the same purpose and function. In some embodiments, the compound of Formula (308) comprises different types of functional groups for different purposes and functions.

In some embodiments, the compound as shown by Formula (308) has a structure as shown by Formula (403), (404), (405), (406), (407), (408), (409), (410), (411), (412), (413), (414), (415), (416), (417), (418), (419), (420), (421), (422), (423), (424), (425), (426), or (427):

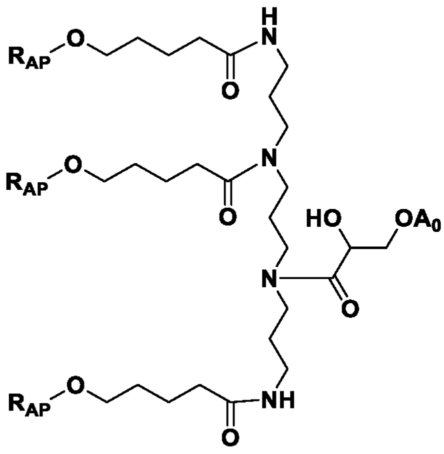

Formula (403)

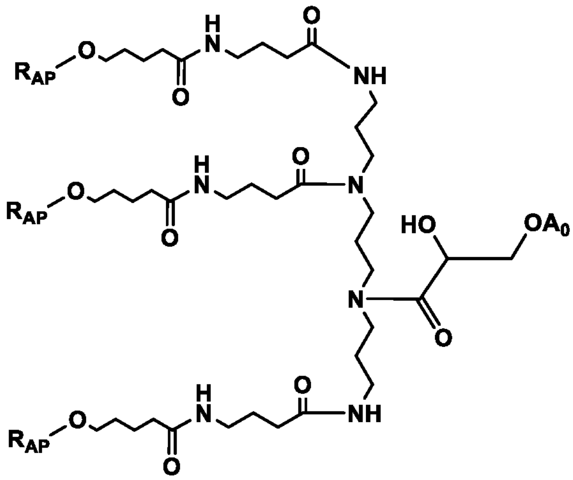

Formula (404)

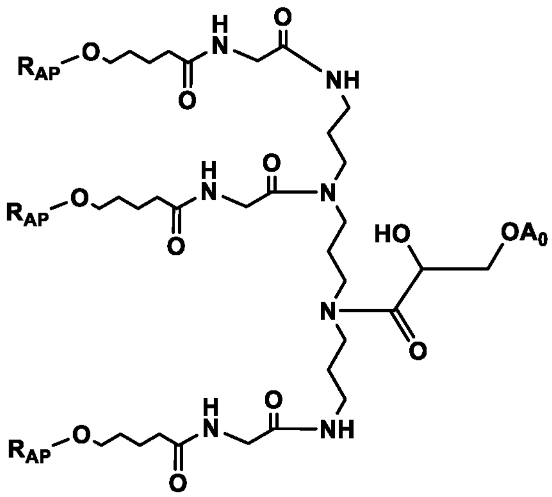

Formula (405)

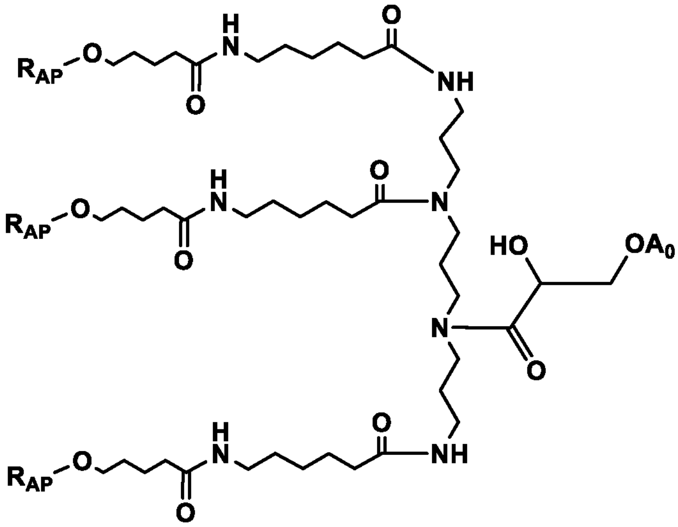

Formula (406)

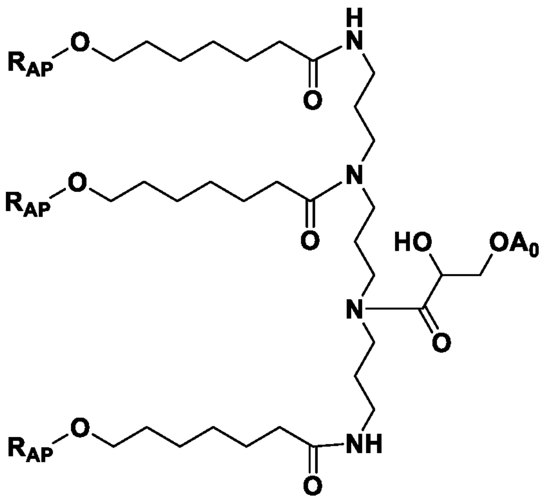

Formula (407)

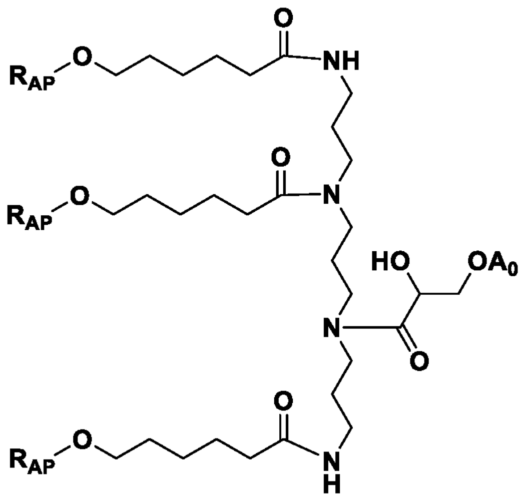

Formula (408)

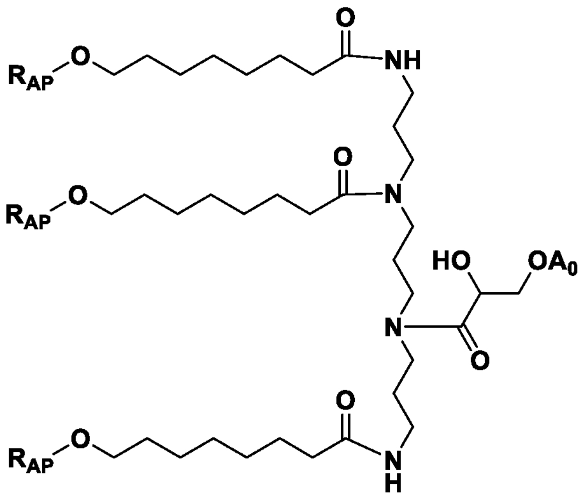

Formula (409)

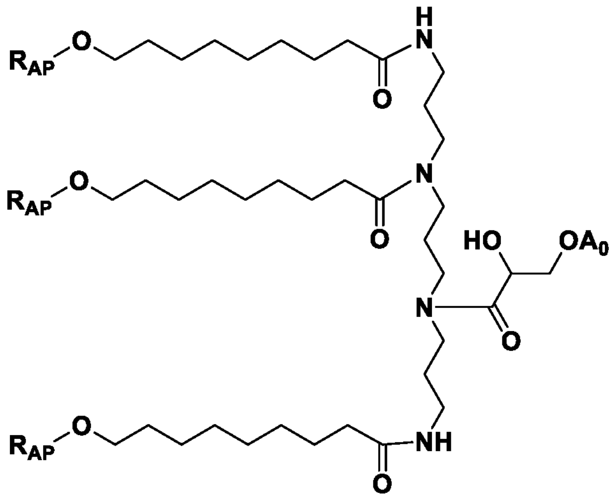

Formula (410)

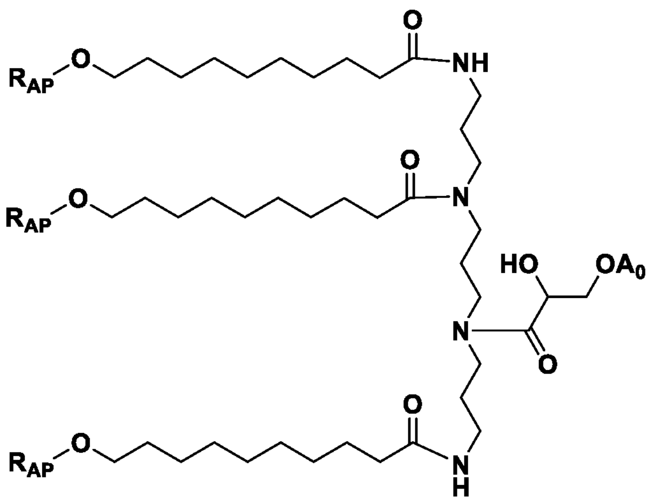

Formula (411)

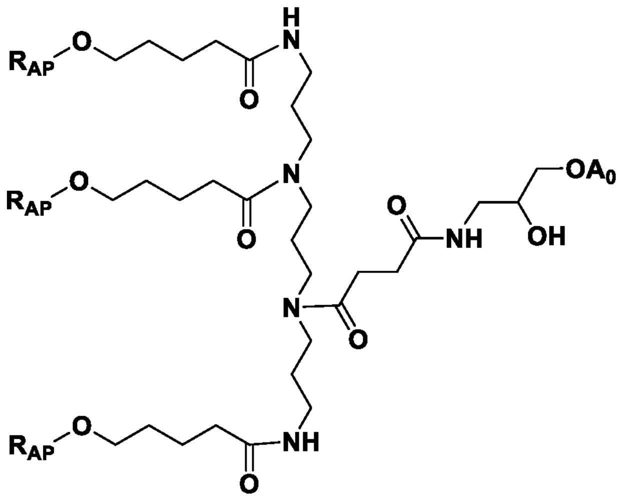

Formula (412)

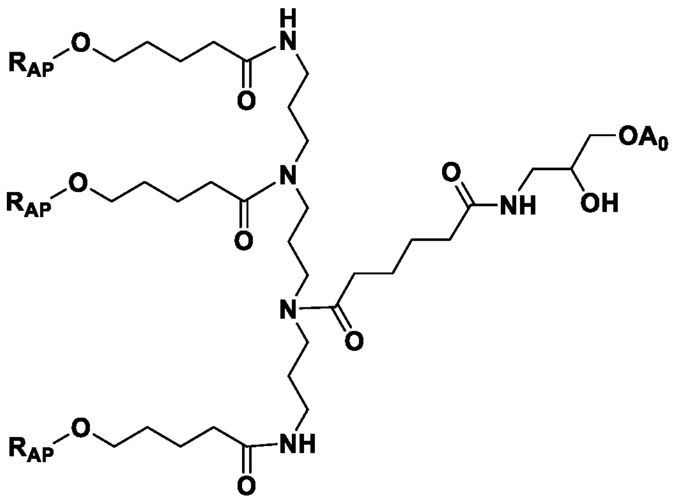

Formula (413)

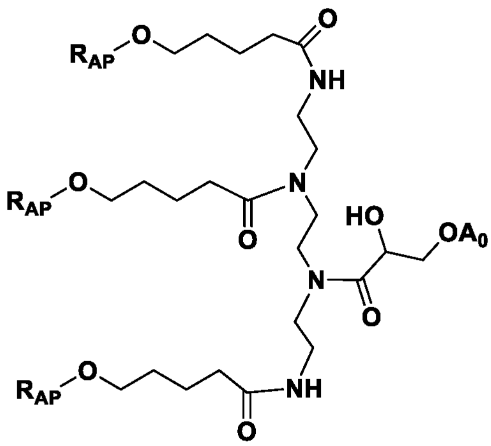

Formula (414)

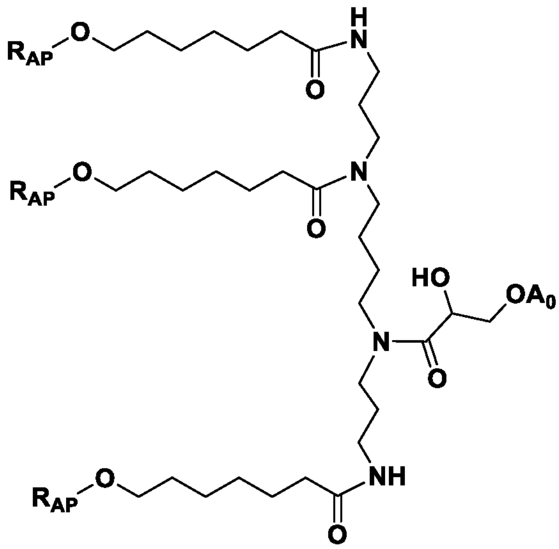

Formula (415)

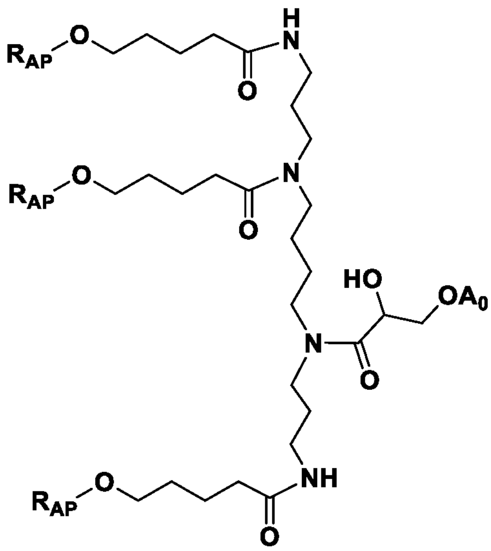

Formula (416)

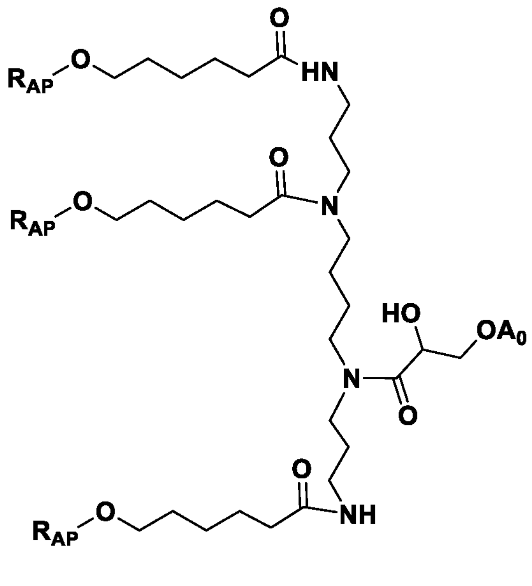

Formula (417)

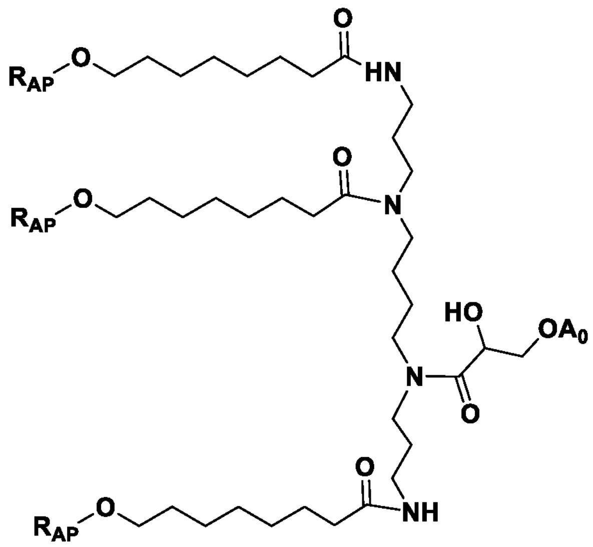

Formula (418)

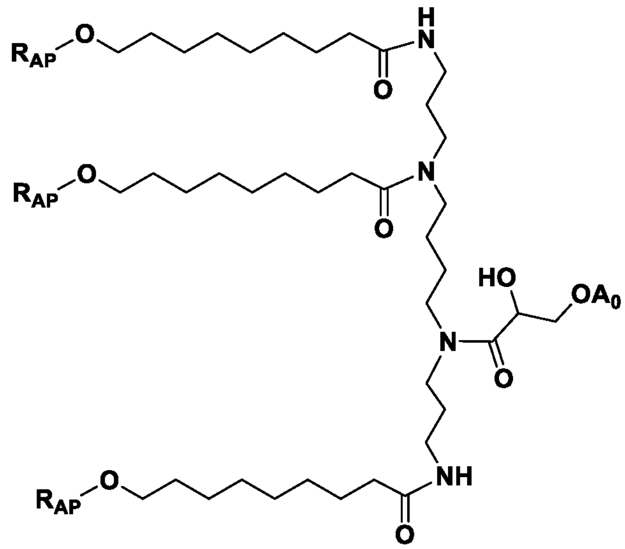

Formula (419)

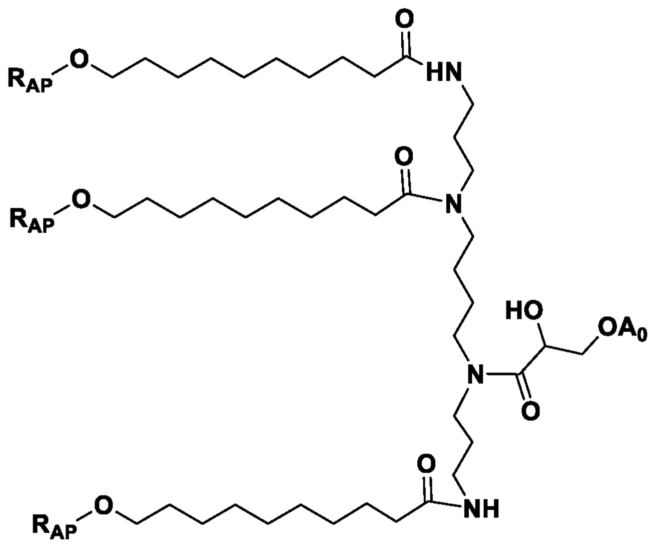

Formula (420)

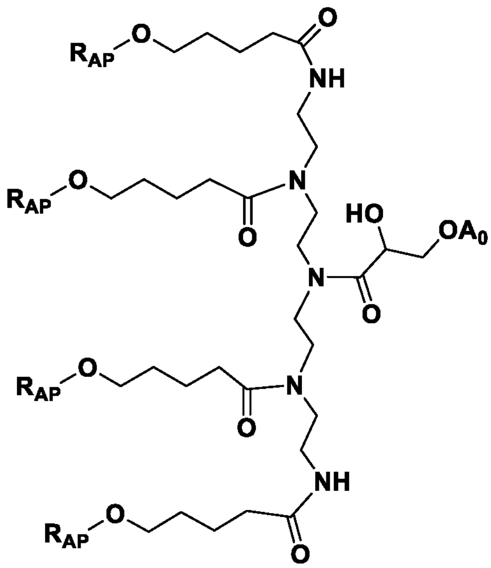

Formula (421)

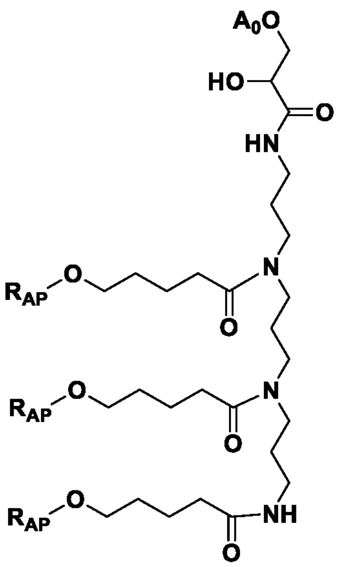

Formula (422)

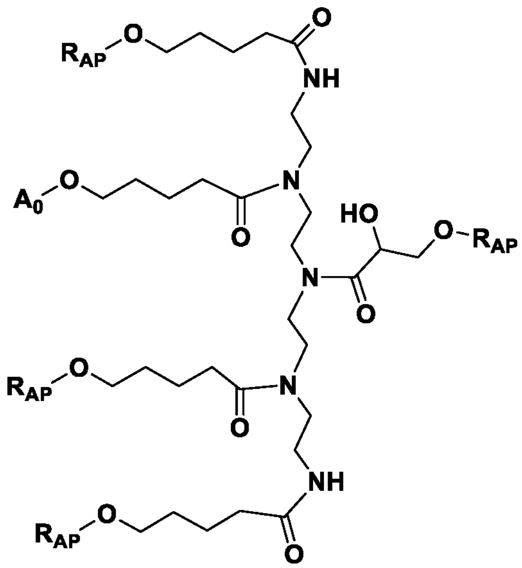

Formula (423)

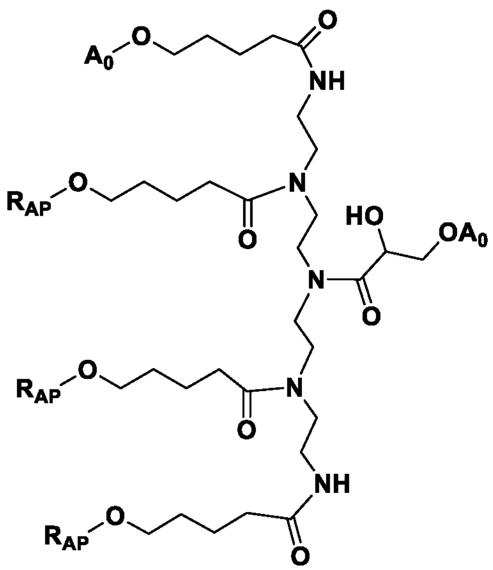

Formula (424)

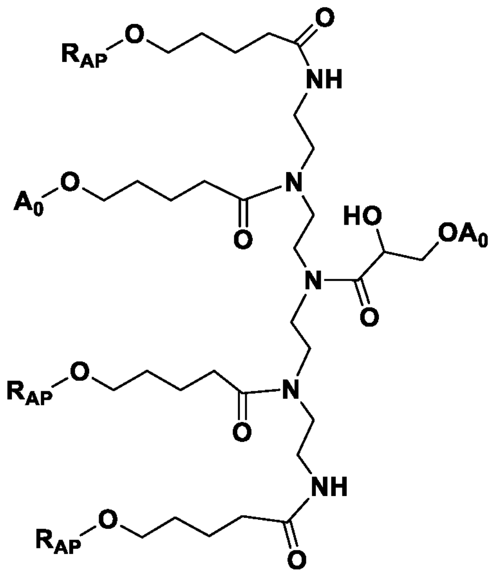

Formula (425)

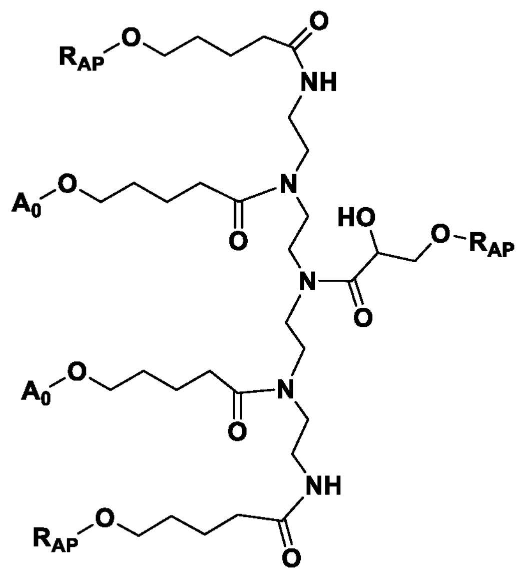

Formula (426)

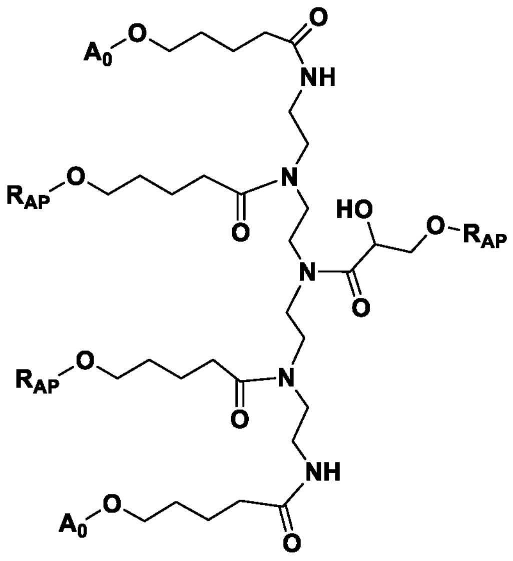

Formula (427)

In some embodiments, the linking group $R_j$ comprises a nucleotide sequence I and a nucleotide sequence II, the nucleotide sequence I and the nucleotide sequence II each comprise 5-25 modified or unmodified nucleotides, and the nucleotide sequence I and the nucleotide sequence II are at least partly reverse complementary, the delivery group is linked to the nucleotide sequence I, the functional group is linked to the nucleotide sequence II, and the nucleotide sequence I and the nucleotide sequence II do not elicit immune response or toxic reaction in a subject. In some embodiments, the nucleotide sequence I and the nucleotide sequence II are substantially reverse complementary or completely reverse complementary; or the nucleotide sequence I and the nucleotide sequence II have the same length and are both 10-20 modified or unmodified nucleotides; or the nucleotide sequence I and the nucleotide sequence II both consist of 17 nucleotides and are completely reverse complementary. In some embodiments, the 3' terminal of the delivery group is linked to the 5' position of the ribose in the 5'-terminal nucleotide of the nucleotide sequence I via a phosphate ester bond, and the functional group is linked to the 5' position of the ribose in the 5'-terminal nucleotide of the nucleotide sequence II; or the functional group comprises a segment of nucleotide sequence, and the 3' terminal is linked to the 5' position of the ribose in the 5'-terminal nucleotide of the nucleotide sequence II via a phosphate ester bond. In some embodiments, the nucleotide sequence I and the nucleotide sequence II respectively have the sequences as shown by SEQ ID NO: 40 and SEQ ID NO: 41:

5'-GUACAUUCUAGAUAGCC-3' (SEQ ID NO: 40)
5'-GGCUAUCUAGAAUGUAC-3' (SEQ ID NO: 41).

In some embodiments, the nucleotide sequence I and the nucleotide sequence II respectively have sequences as shown by SEQ ID NO: 42 and SEQ ID NO: 43:

5'-GmUfAmCfAmUfUfCfUfAmGmAmUfAmGmCfCf-3' (SEQ ID NO: 42)
5'-GmGmCfUfAmUfCfUfAmGmAmAmUfGmUfAmCf-3' (SEQ ID NO: 43)

The conjugate provided by the present disclosure may comprise one or more functional groups. In the context of the present disclosure, a functional group refers to a free radical-containing group formed by removing one or more atoms, functional groups, or some structural groups (e.g., removing one hydrogen atom) from an active agent molecule having corresponding functionality (e.g., a diagnostic agent compound, a small molecule therapeutic agent group compound having a therapeutic effect on tumors, especially gliomas, and/or a functional oligonucleotide compound capable of regulating the expression level of a cancer-related gene in tumor cells), and the functional group is covalently linked to the rest of the molecule of the conjugate provided by the present disclosure via the free radical.

In some embodiments, at least one functional group in the conjugate provided by the present disclosure is a diagnostic agent group, and each of the diagnostic agent group is independently selected from contrast agent groups or fluorescence imaging groups (fluorescent groups). In some embodiments, all functional groups in the conjugate provided by the present disclosure are the above diagnostic agent groups. By comprising a diagnostic agent group, the conjugate provided by the present disclosure can deliver the diagnostic agent group to tumors, and thus specifically, efficiently, and

accurately diagnose relevant diseases and/or symptoms, such as, the presence and progression of tumors. In some embodiments, the diagnostic agent group may be used for diagnosing tumor cells/tissues in a sample to be tested in an *in vitro* experiment. In some embodiments, the diagnostic agent group may be used for diagnosing the presence and/or characteristic of tumor cells/tissues in a subject.

In some embodiments, at least one functional group in the conjugate provided by the present disclosure is a small molecule therapeutic agent group having a therapeutic effect on tumors, especially gliomas, and each of the small molecule therapeutic agent groups is independently selected from cytotoxin groups, antibiotic groups, angiogenesis inhibitors, or antibody drug groups. In some embodiments, all functional groups in the conjugate provided by the present disclosure are the above small molecule therapeutic agent groups. By comprising the above small molecule therapeutic agent group, the conjugate provided by the present disclosure can specifically deliver the small molecule therapeutic agent group to tumors, and thus, through the effect of the small molecule therapeutic agent group, treat and/or alleviate disease progression or symptoms of tumors. For example, a cytotoxin group is specifically delivered to tumors by the conjugate provided by the present disclosure to specifically kill cancer cells in tumors, thereby decreasing the side effects caused by low targeting ability of the cytotoxin *per se* and significantly decreasing the number of cancer cells in tumors, thus treating tumors.

In some embodiments, at least one functional group in the conjugate provided by the present disclosure is a functional oligonucleotide group, which can regulate the expression level of a cancer-related gene in tumor cells. In some embodiments, all functional groups in the conjugate provided by the present disclosure are the above functional oligonucleotide groups. By comprising the functional oligonucleotide group, the conjugate provided by the present disclosure can specifically deliver the functional oligonucleotide group to tumors, and thus, through the effect of the functional oligonucleotide group (e.g., RNAi effect), regulate the expression level of a cancer-related gene in tumor cells (e.g., inhibiting the expression of an oncogene), thereby treating and/or alleviating the progression or symptoms of tumor-related diseases. In some embodiments, the functional oligonucleotide is an siRNA. In some embodiments, the functional oligonucleotide is an siRNA targeting STAT3 mRNA, RRM2 mRNA, or PLK1. In some embodiments, the functional oligonucleotide is one or more of the siRNA with a sense strand as shown by SEQ ID NO: 44 and an antisense strand as shown by SEQ ID NO: 45, the siRNA with a sense strand as shown by SEQ ID NO: 67 and an antisense strand as shown by SEQ ID NO: 68, and the siRNA with a sense strand as shown by SEQ ID NO: 69 and an antisense strand as shown by SEQ ID NO: 70:

5'-CmsUmsAmGmAmAmAfAfCfUmGmGmAmUmAmAmCmGmUm-3' (SEQ ID NO: 44)
5'-AmsCfsGmUmUmAfUmCmCmAmGmUmUmUfUmCfUmAmGmsCmsCm-3' (SEQ ID NO: 45)
5'-CUUCUUAUUGACACUUACAdT-S-dT-3' (SEQ ID NO: 67)
5'-UGUAAGUGUCAAUAAGAAGdT-S-dT-3' (SEQ ID NO: 68)
5'-CAAGAAGAAUGAAUACAGUdT-S-dT-3' (SEQ ID NO: 69)
5'-ACUGUAUUCAUUCUUCUUGdT-S-dT-3' (SEQ ID NO: 70)

The functional group may be comprised in the conjugate provided by the present disclosure in any suitable manner. For example, the functional group $A_0$ may be linked to the main chain moiety via the conjugation linking moiety above.

In some embodiments, at least one functional group in the conjugate provided by the present disclosure is a delivery aid group selected from one or more of $C_{10}$-$C_{30}$ hydrocarbyl, cholesteryl, and phospholipid groups. By comprising the delivery aid group, the conjugate provided by the present disclosure can be more compatible with the *in vivo* environment in the central nervous system, may have better bioavailability, and/or is delivered to tumors more effectively.

Those skilled in the art may prepare the conjugate provided by the present disclosure using any appropriate synthetic route.

In some embodiments, the synthesis method of the conjugate provided by the present disclosure comprises contacting a protected conjugate with a deprotection reagent in a solvent under deprotection reaction condition, and isolating the conjugate provided by the present disclosure. The protected conjugate is a compound formed by protecting all active functional groups in the conjugate provided by the present disclosure with protecting groups. In some embodiments, the active functional groups include, but are not limited to, hydroxyl, amino, and/or phosphate group, and the protecting groups are correspondingly hydroxyl protecting groups, amino protecting groups, and/or phosphate hydroxyl protecting groups (e.g., cyanoethyl protecting groups). The solvent, deprotection reaction condition, and deprotection reagent to be used are selected and determined according to different protecting groups. In some embodiments, the deprotection reaction condition, solvent, and deprotection reagent are the deprotection reaction condition, solvent, and deprotection reagent used in solid phase synthesis of nucleic acids. In some embodiments, the method comprises adding the protected conjugate to a mixed solution of methylamine aqueous solution and aqueous ammonia, and the deprotection reaction condition comprises reacting for 1-5 h under normal temperature and pressure. In some embodiments, the methylamine aqueous solution and saturated concentrated aqueous ammonia are mixed in equal volumes to obtain the mixed solution,

whose amount is 0.1-10 mL/μmol relative to the protected conjugate. In some embodiments, the isolation comprises performing purification through column chromatography separation, collecting product eluate, and removing solvent. Purification condition may be, for example, performing elution by using a preparative ion chromatography purification column with a gradient eluant of sodium chloride solution and sodium phosphate solution. In some embodiments, gradient elution is performed with: eluant A: 20 mM sodium phosphate (pH 8.1), solvent is water/acetonitrile in 9:1 (v/v); eluant B: 1.5 M sodium chloride, 20 mM sodium phosphate (pH 8.1), solvent is water/acetonitrile in 9:1 (v/v); elution gradient: the ratio of eluant A: eluant B = 100:0-50:50.

In some embodiments, the conjugate provided by the present disclosure has a structure as shown by Formula (101). The synthesis method of the protected conjugate comprises: contacting a compound comprising an active group $R_{x1}$ and a delivery group with a compound comprising an active group $R_{x2}$ and a functional group in an organic solvent under coupling reaction condition, and reacting to obtain the protected conjugate, wherein the delivery group is formed by removing one or more hydrogen atoms or one or more functional groups from the aptamer provided by the present disclosure, and each of the functional group is independently one of a diagnostic agent group, a small molecule therapeutic agent group having a therapeutic effect on tumors, and a functional oligonucleotide group having a therapeutic effect on tumors, wherein all active groups in the delivery group and functional group are protected by protecting groups, and the active group $R_{x1}$ and the active group $R_{x2}$ are groups capable of reacting to form a covalent bond or linking group $R_j$. In some embodiments, the molar ratio of the delivery group linked with an active group $R_{x1}$ to the functional group linked with an active group $R_{x2}$ is $m_0$:$n_0$. In some embodiments, active groups in the delivery group and functional group include, but are not limited to, one or more of hydroxyl, amino, and phosphate group.

Those skilled in the art could obtain the compound comprising an active group $R_{x1}$ and a delivery group by various methods. In some embodiments, the compound comprising an active group $R_{x1}$ and a delivery group may be obtained by nucleic acid synthesis methods well-known to those skilled in the art, for example, phosphoramidite solid phase synthesis or liquid phase synthesis by phosphodiester method/phosphotriester method. In some embodiments, the compound comprising an active group $R_{x1}$ and a delivery group is obtained using the phosphoramidite solid phase synthesis method. The method comprises sequentially linking nucleoside monomers according to the sequence of the nucleotides in an oligonucleotide single strand under condition for phosphoramidite solid phase synthesis , wherein at least one nucleoside monomer is a nucleoside monomer; or after linking all nucleoside monomers, linking a phosphoramidite monomer with an active group $R_{x1}$ according to the phosphoramidite solid phase synthesis method, or linking a protected phosphoramidite monomer, and then removing the protecting group to form the active group $R_{x1}$. The phosphoramidite solid phase synthesis method is well-known to those skilled in the art, whose process and conditions are disclosed in detail in Methods in Molecular Biology, vol. 288: Oligonucleotide Synthesis: Methods and Applications, P17-P31, which is incorporated herein by reference in its entirety.

In some embodiments, the coupling reaction condition is the condensation reaction condition or the condition for thiol-disulfide exchange reaction.

In some embodiments, the coupling reaction condition is the condensation reaction condition, which is acylation condensation reaction condition, dehydration condensation reaction condition, or click chemistry reaction condition, and the active group $R_{x1}$ and active group $R_{x2}$ are groups capable of undergoing the condensation reaction above. In some embodiments, the condensation reaction condition is acylation condensation reaction condition, and the active groups $R_{x1}$ and $R_{x2}$ are groups capable of undergoing acylation condensation reaction to form $R_l$. In some embodiments, the condensation reaction condition is dehydration condensation reaction condition, and one of the active groups $R_{x1}$ and $R_{x2}$ is a group comprising an acyl halide or carboxyl group, and the other is a group comprising an amino or hydroxyl group. In some embodiments, the condensation reaction condition is click chemistry reaction condition, and one of the active groups $R_{x1}$ and $R_{x2}$ is a group comprising an alkynyl group, and the other is a group comprising an azide group. In some embodiments, the condensation reaction condition is Michael addition reaction condition, and one of the active groups $R_{x1}$ and $R_{x2}$ is a group comprising a thiol group, and the other is a group comprising a succinimidyl group. In some embodiments, the condensation reaction condition is the condition for N-hydroxysuccinimide-carbodiimide (NHS-EDC) coupling reaction, and one of the active groups $R_{x1}$ and $R_{x2}$ is a group comprising N-hydroxysuccinimide (NHS) , and the other is a group comprising a carbodiimide (EDC).

In some embodiments, the compound comprising an active group $R_{x1}$ and a delivery group is prepared by contacting the aptamer provided by the present disclosure having an active group $R_{x0}$ with a cross-linking agent under coupling reaction condition, and the cross-linking agent comprises a click chemistry active group and an acylation group. The active group $R_{x0}$ and the acylation group are covalently linked through coupling reaction, linking the click chemistry active group to the aptamer provided by the present disclosure.

In some embodiments, an active group $R_{x1}$ is an active group comprising 1-3 click chemistry active groups at its end, wherein the click chemistry active group comprises a terminal alkynyl group. In some embodiments, the acylation group is an active ester group, for example, one of an NHS ester group, an imidate group, and a pentafluorophenyl ester group. Those skilled in the art could obtain the cross-linking agent by various methods. For example, when the acylation group is a pentafluorophenyl ester group and the click chemistry group comprises a terminal alkynyl group, the cross-linking agent may be prepared according to the method as described in Scheme 1a (A) in Østergaard, Michael E., et al. "Efficient synthesis and biological evaluation of 5'-GalNAc conjugated antisense oligonucleotides." Bioconjugate chemistry 26.8 (2015): 1451-1455, which is incorporated herein by reference in its entirety. In some embodiments, the active group $R_{x0}$ is an amino group. In some embodiments, the coupling condition is basic condition. In some embodiments, the basic condition is the condition in which an weak base aqueous solution is present, for example the condition in which sodium bicarbonate aqueous solution is present.

Those skilled in the art could obtain the aptamer with an active group $R_{x0}$ by various methods. In some embodiments, the aptamer with an active group $R_{x0}$ is prepared by using a phosphoramidite monomer comprising an active group at the corresponding position during aptamer synthesis. Those skilled in the art could obtain a phosphoramidite monomer with an active group by various methods. In some embodiments, the active group $R_{x0}$ is an amino group, and a phosphoramidite monomer with an $R_{x0}$ can be commercially available or prepared by the methods well-known to those skilled in the art. For example, the phosphoramidite monomer with an $R_{x0}$ can be readily commercially available 6-(trifluoroacetylamino)-hexyl-(2-cyanoethyl)-(*N*,*N*-diisopropyl)-phosphoramidite monomer, wherein the active group $R_{x0}$ is an amino group. The active group $R_{x0}$ may be obtained by linking the phosphoramidite monomer to an oligonucleotide single strand, and then removing the trifluoroacetyl protecting group through deprotection reaction readily realized by those skilled in the art (e.g., ammonolysis by concentrated aqueous ammonia) according to the phosphoramidite solid phase synthesis method.

In some embodiments, the coupling reaction condition is one of the conditions for thiol-disulfide exchange reaction, and one of the active groups $R_{x1}$ and $R_{x2}$ is a group comprising a thiol group, and the other comprises a leaving group linked by a disulfide bond. To avoid side reactions, in some embodiments, $R_{x1}$ in the above phosphoramidite monomer with an active group $R_{x1}$ is present in a form of protected $R_{x1}'$, and the preparation method further comprises the step of isolating the compound comprising an active group $R_{x1}$ and a delivery group by contacting the prepared compound comprising the protected active group $R_{x1}'$ and a delivery group with a deprotection reagent under deprotection reaction condition. In some embodiments, the $R_{x1}'$ comprises a disulfide bond leaving group, the deprotection reaction condition is the condition for thiol-disulfide exchange reaction, and the deprotection reagent is a disulfide bond activator. In some embodiments, the disulfide bond activator is dithiodipyridine. Those skilled in the art could obtain the above phosphoramidite monomer with an active group $R_{x1}$ or $R_{x1}'$ by various methods. In some embodiments, the phosphoramidite monomer with an active group $R_{x1}$ or $R_{x1}'$ is commercially purchased. For example, the phosphoramidite monomer as shown by Formula (105) is commercially available.

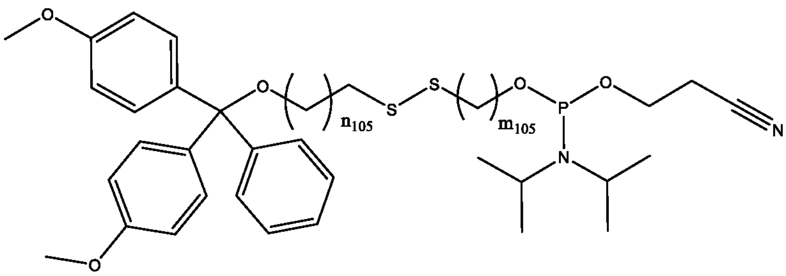
;

Formula (105)

wherein $n_{105}$ and $m_{105}$ independently of one another are an integer of 1-10.

Those skilled in the art could obtain the compound comprising an active group $R_{x2}$ and a functional group by various methods. In some embodiments, the coupling reaction condition is the condition for thiol-disulfide exchange reaction, the active group $R_{x2}$ comprises a thiol group, and the compound comprising an active group $R_{x2}$ and a functional group may be obtained by those skilled in the art via various known methods. For example, it may be prepared by using a phosphoramidite monomer comprising a thiol group according to the phosphoramidite solid phase synthesis method, or may be commercially purchased. In some embodiments, the functional group is a functional oligonucleotide group, the coupling reaction condition is the condition for phosphoramidite solid phase synthesis reaction, the active group $R_{x1}$ is a hydroxyl group, the active group $R_{x2}$ is a phosphoramidite group, and the preparation method comprises sequentially linking nucleoside monomers to a solid phase support linked with a delivery group and an active group $R_{x1}$ according to the nucleic acid sequence of the functional oligonucleotide and the method of phosphoramidite solid phase synthesis reaction. In some embodiments, the functional group is a diagnostic agent group or a small molecule therapeutic agent group, the coupling reaction condition is the condition for phosphoramidite solid phase synthesis reaction, the active group $R_{x2}$ is a phosphoramidite group, and the compound comprising an active group $R_{x2}$ and a functional group may be, for example, a readily commercially available compound comprising a phosphoramidite group and a fluorescent group or a small molecule therapeutic agent group. In some embodiments, the coupling reaction condition is Michael addition reaction

condition, the active group $R_{x2}$ is a *N*-succinimidyl group, and the compound comprising an active group $R_{x2}$ and a functional group may be, for example, a readily commercially available compound comprising a *N*-succinimidyl group and a small molecule therapeutic agent group. In some embodiments, the functional group is a functional oligonucleotide group, the active group $R_{x1}$ and active group $R_{x2}$ are respectively a nucleotide sequence I and a nucleotide sequence II, each of which comprises 5-25 modified or unmodified nucleotides, and the nucleotide sequence I and the nucleotide sequence II are at least partly reverse complementary. The delivery group is linked to the nucleotide sequence I, the functional group is linked to the nucleotide sequence II, and the nucleotide sequence I and the nucleotide sequence II do not elicit immune response or toxic reaction in a subject. The coupling reaction condition is the reaction condition for annealing to form a nucleic acid double strand. In some embodiments, the functional oligonucleotide group is an siRNA, the 5' terminal of the nucleotide sequence I is linked to the delivery group via a phosphodiester bond, and the 3' terminal of the nucleotide sequence II is linked to the 5' terminal of the siRNA via a phosphodiester bond. In some embodiments, the nucleotide sequence I and the nucleotide sequence II both consist of 17 nucleotides and are completely reverse complementary. In some embodiments, the nucleotide sequence I and the nucleotide sequence II respectively have the sequences as shown by SEQ ID NO: 40 and SEQ ID NO: 41. In some embodiments, the nucleotide sequence I and the nucleotide sequence II respectively have the sequences as shown by SEQ ID NO: 42 and SEQ ID NO: 43.

In some embodiments, the conjugate may also be used in the present disclosure in the form of a pharmaceutically acceptable salt or a precursor compound thereof. In the context of the present disclosure, a "pharmaceutically acceptable salt" refers to a corresponding salt formed by a drug to increase the stability, solubility, and/or bioavailability of the drug without pharmaceutically producing additional side effects on the human body, such as potassium salt, sodium salt, or carboxylate. A "precursor compound" refers to a compound that is not identical with the conjugate in terms of structure and function, but could react and form the conjugate provided by the present disclosure after entering into the body or in the body fluid environment, thereby exerting its effect and achieving the purpose of the present disclosure. Under certain circumstances, these precursor compounds can increase the stability, extend the slow-release time, increase the bioavailability, etc. of the drug. In some embodiments, the precursor compound comprises the precursor groups capable of reacting in the human body to form all functional groups $A_0$ in a conjugate. In some embodiments, the precursor compound includes a compound formed by substituting all active hydroxyl groups in a conjugate with acetoxy groups. In some embodiments, the precursor compound comprises a drug precursor group, which is a residue formed by a precursor compound of the diagnostic agent, therapeutic agent, and/or functional oligonucleotide corresponding to the functional group in a conjugate. In some embodiments, the drug precursor group may be, for example, a group formed by substituting active hydrogen in a hydroxyl or amino functional group in the functional group with an acyl, alkyl, or phosphoryl group. Those skilled in the art could understand that use of these pharmaceutically acceptable salts and precursor compounds are also within the scope of the present disclosure.

## Pharmaceutical composition

In one aspect, the present disclosure further provides a pharmaceutical composition, comprising the conjugate provided by the present disclosure and a pharmaceutically acceptable carrier.

The pharmaceutically acceptable carrier may be a carrier conventionally used in the art, for example, but not limited to, one or more of water, normal saline, magnetic nanoparticles (such as $Fe_3O_4$-based or $Fe_2O_3$-based nanoparticles), carbon nanotubes, mesoporous silicon, calcium phosphate nanoparticles, polyethylenimine (PEI), polyamidoamine (PAMAM) dendrimer, poly(L-lysine) (PLL), chitosan, 1,2-dioleoyl-3-trimethylammonium-propane (DOTAP), poly(D&L-lactic/glycolic acid) copolymer (PLGA), poly(2-aminoethyl ethylene phosphate) (PPEEA), poly(2-dimethylaminoethyl methacrylate) (PDMAEMA), and derivatives thereof.

In some embodiments, the pharmaceutically acceptable carrier comprises a physiologically acceptable compound, which exerts the function of, for example, stabilizing the pharmaceutical composition or increasing or decreasing the absorption of the conjugate and/or the pharmaceutical composition. The physiologically acceptable compound is selected from one or more of the following compounds: carbohydrates, such as glucose, sucrose, and/or glucan; antioxidants, such as ascorbic acid and/or glutathione; chelating agents; low molecular weight proteins; compositions that reduce the clearance or hydrolysis of any co-administered substance; excipients; stabilizers and buffers. A detergent may also be used for stabilizing the composition or increasing or decreasing the absorption of the pharmaceutical composition. The physiologically acceptable compound may further include one or more of wetting agents, emulsifiers, dispersants, or preservatives especially used for preventing microbial growth or action. The physiologically acceptable compound is known to those skilled in the art, which is not described here in detail. Those skilled in the art could easily understand that the choices of the pharmaceutically acceptable carrier and physiologically acceptable compound depend on, for example, administration routes and specific physiochemical characteristics of any co-administered substance.

In some embodiments, the pharmaceutically acceptable carrier is sterile and usually does not comprise an undesirable substance. The pharmaceutical composition provided by the present disclosure may further comprise pharmaceutically acceptable auxiliary substances according to requirements to approach physiological conditions, such

as pH regulators and buffers and toxicity regulators, for example sodium acetate, sodium chloride, potassium chloride, calcium chloride, and sodium lactate. The concentration of the conjugate provided by the present disclosure in the pharmaceutical composition may vary within a wide range, and is selected mainly according to fluid volume, viscosity, body weight, etc. and according to the specific administration manner.

In some embodiments, in the pharmaceutical composition, there are no special requirements for the contents of the conjugate and the pharmaceutically acceptable carrier. In some embodiments, the weight ratio of the conjugate to the pharmaceutically acceptable carrier may be 1:(1-500), and in some embodiments, the above weight ratio is 1:(1-50).

In some embodiments, the pharmaceutical composition may also comprise other pharmaceutically acceptable adjuvants, which may be one or more of various formulations and compounds conventionally used in the art. For example, said other pharmaceutically acceptable adjuvants may comprise at least one of a pH buffer, a protection agent, and an osmotic pressure regulator.

The pH buffer may be a tris(hydroxymethyl)aminomethane hydrochloride buffer solution with a pH of 7.5-8.5, and/or a phosphate buffer solution with a pH of 5.5-8.5, such as a phosphate buffer solution with a pH of 5.5-8.5.

The protection agent may be at least one of inositol, sorbitol, sucrose, trehalose, mannose, maltose, lactose, and glucose. The content of the protection agent may be from 0.01 wt% to 30 wt% based on the total weight of the pharmaceutical composition.

The osmotic pressure regulator may be sodium chloride and/or potassium chloride. The content of the osmotic pressure regulator allows the osmotic pressure of the pharmaceutical composition to be 200-700 mOsm/kg. Depending on the desired osmotic pressure, those skilled in the art can readily determine the content of the osmotic pressure regulator. In some embodiments, the dose of the formulation prepared from the pharmaceutical composition will be adjusted according to different administration manners during administration.

In some embodiments, the pharmaceutical composition may be a liquid formulation, for example, an injection solution; or a lyophilized powder for injection, which will be mixed with a liquid adjuvant to form a liquid formulation upon administration. The liquid formulation may be used for, but not limited to, administration by subcutaneous, intramuscular, or intravenous injection, and the pharmaceutical composition may also be delivered by, but not limited to, puncture injection, oropharyngeal inhalation, nasal administration, or other routes. In some embodiments, the pharmaceutical composition is used for administration by subcutaneous, intramuscular, intravenous, or intrathecal injection.

In some embodiments, the pharmaceutical composition may be in the form of a liposome formulation. In some embodiments, the pharmaceutically acceptable carrier used in the liposome formulation comprises an amine-containing transfection compound (hereinafter also referred to as an organic amine), a helper lipid, and/or a PEGylated lipid. Therein, the organic amine, the helper lipid, and the PEGylated lipid may be respectively selected from one or more of the amine-containing transfection compounds or the pharmaceutically acceptable salts or derivatives thereof, the helper lipids, and the PEGylated lipids as described in the Chinese patent application CN103380113A, which is incorporated herein by reference in its entirety.

In some embodiments, the organic amine may be a compound as shown by Formula (201) or a pharmaceutically acceptable salt thereof as described in the Chinese patent application CN103380113A:

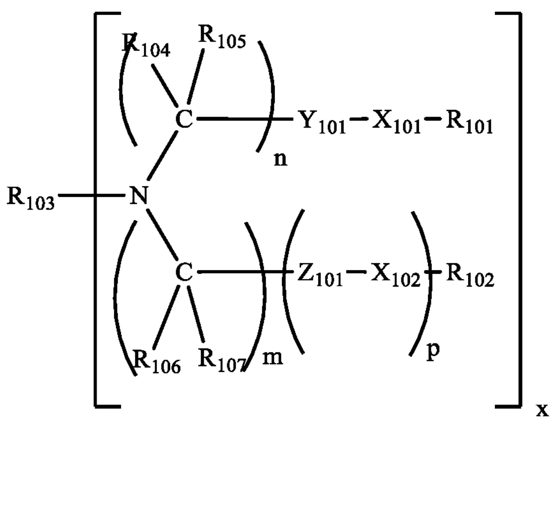

Formula (201),

wherein:

$X_{101}$ and $X_{102}$ independently of one another are O, S, N-A or C-A, wherein A is hydrogen or a $C_1$-$C_{20}$ hydrocarbon chain;

$Y_{101}$ and $Z_{101}$ independently of one another are C=O, C=S, S=O, CH-OH or $SO_2$;

$R_{101}$, $R_{102}$, $R_{103}$, $R_{104}$, $R_{105}$, $R_{106}$, and $R_{107}$ independently of one another are hydrogen; a cyclic or an acyclic,

substituted or unsubstituted, branched or straight-line aliphatic group; a cyclic or an acyclic, substituted or unsubstituted, branched or straight-line heteroaliphatic group; a substituted or unsubstituted, branched or straight-line acyl group; a substituted or unsubstituted, branched or straight-line aryl group; or a substituted or unsubstituted, branched or straight-line heteroaryl group;

x is an integer of 1-10;

n is an integer of 1-3, m is an integer of 0-20, and p is 0 or 1, wherein if m = p = 0, then $R_{102}$ is hydrogen; and if at least one of n and m is 2, then $R_{103}$ and the nitrogen in Formula (201) form a structure as shown by Formula (202) or (203):

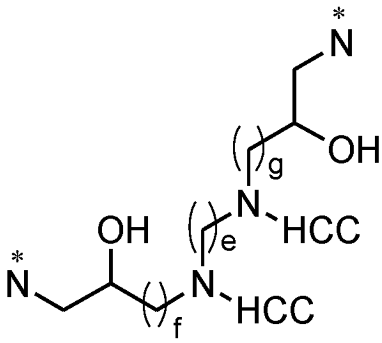

,

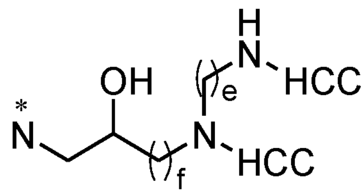

;

Formula (202) Formula (203)

wherein g, e, and f independently of one another are an integer of 1-6, "HCC" represents a hydrocarbon chain, and each *N represents a nitrogen atom in Formula (201).

In some embodiments, $R_{103}$ is a polyamine. In other embodiments, $R_{103}$ is a ketal. In some embodiments, $R_{101}$ and $R_{102}$ in Formula (201) independently of one another are any substituted or unsubstituted, branched or straight-line alkyl or alkenyl, wherein the alkyl or alkenyl has 3 to about 20 carbon atoms (such as 8 to about 18 carbon atoms) and 0-4 double bonds (such as 0-2 double bonds).

In some embodiments, if n and m independently of one another are 1 or 3, $R_{103}$ may be any of the following Formulae (204)-(213):

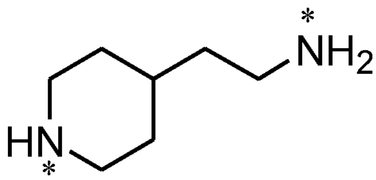

,

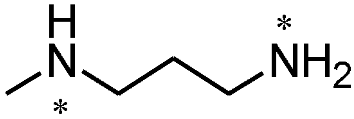

,

Formula (204) Formula (205)

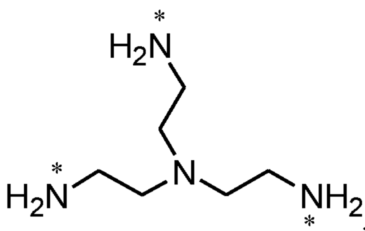

,

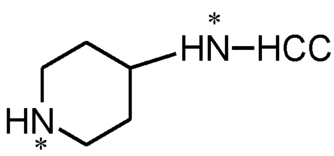

,

Formula (206) Formula (207)

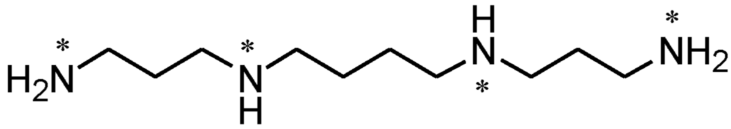,

Formula (208)

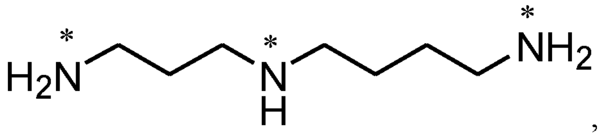,

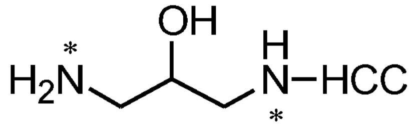,

Formula (209) Formula (210)

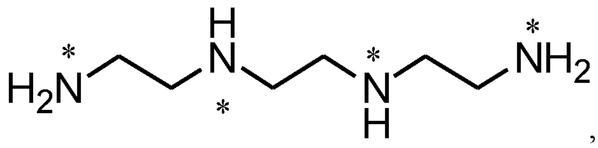,

Formula (211)

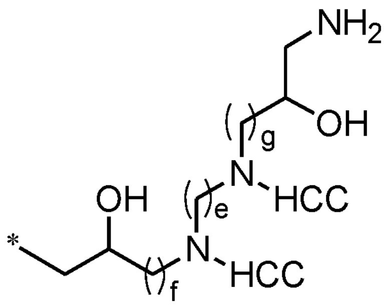, and 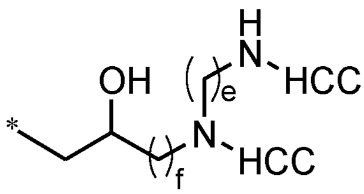;

Formula (212) Formula (213)

wherein, in Formulae (204)-(213), g, e, and f independently of one another are an integer of 1-6, each "HCC" represents a hydrocarbon chain, and each * represents a potential attachment point of $R_{103}$ to the nitrogen atom in Formula (201), wherein each H at any * position may be replaced to achieve the attachment to the nitrogen atom in Formula (201).

Those skilled in the art could obtain the compound as shown by Formula (201) by any appropriate method. In some embodiments, the compound as shown by Formula (201) may be prepared according to the description of the Chinese patent application CN103380113A.

In some embodiments, the organic amine is an organic amine as shown by Formula (214) and/or an organic amine as shown by Formula (215):

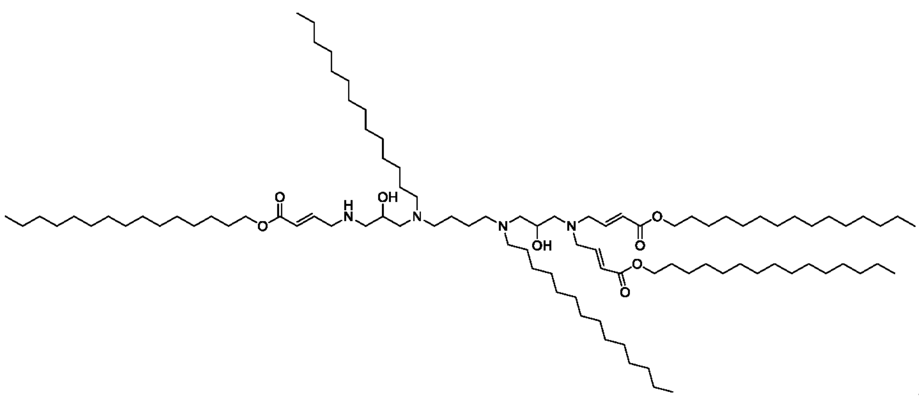,

Formula (214)

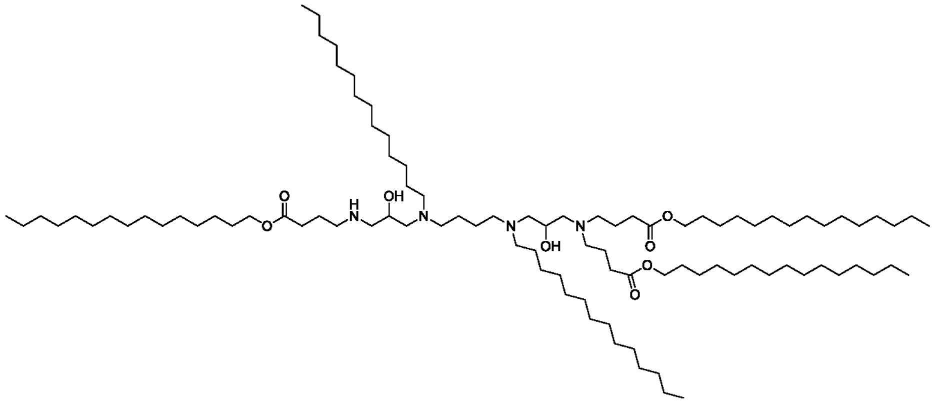

;

Formula (215)

The helper lipid is cholesterol, a cholesterol analog, and/or a cholesterol derivative.

The PEGylated lipid is 1,2-dipalmitoyl-sn-glycero-3-phosphoethanolamine-N-[methoxy(polyethylene glycol)]-2000.

In some embodiments, the molar ratio among the organic amine, the helper lipid, and the PEGylated lipid in the pharmaceutical composition is (19.7-80):(19.7-80):(0.3-50), for example, the molar ratio may be (50-70):(20-40):(3-20).

In some embodiments, the pharmaceutical composition particles formed by the conjugate provided by the present disclosure and the above amine-containing transfection reagents have an average diameter of about 30 nm to about 200 nm, typically about 40 nm to about 135 nm, and more typically, the average diameter of the liposome particles is about 50 nm to about 120 nm, about 50 nm to about 100 nm, about 60 nm to about 90 nm, or about 70 nm to about 90 nm; for example, the average diameter of the liposome particles is about 30, 40, 50, 60, 70, 75, 80, 85, 90, 100, 110, 120, 130, 140, 150, or 160 nm.

In some embodiments, in the pharmaceutical composition formed by the conjugate provided by the present disclosure and the above amine-containing transfection reagents, the weight ratio (weight/weight ratio) of the conjugate to total lipids, e.g., the organic amines, the helper lipids, and/or the PEGylated lipids, ranges from about 1:1 to about 1:50, from about 1:1 to about 1:30, from about 1:3 to about 1:20, from about 1:4 to about 1:18, from about 1:5 to about 1:17, from about 1:5 to about 1:15, from about 1:5 to about 1:12, from about 1:6 to about 1:12, or from about 1:6 to about 1:10. For example, the weight ratio of the conjugate provided by the present disclosure to total lipids is about 1:5, 1:6, 1:7, 1:8, 1:9, 1:10, 1:11, 1:12, 1:13, 1:14, 1:15, 1:16, 1:17, or 1:18.

In some embodiments, the pharmaceutical composition may be marketed with each component being separate, and used in the form of a liquid formulation. In some embodiments, the pharmaceutical composition formed by the conjugate provided by the present disclosure and the above pharmaceutically acceptable carrier may be prepared by various known processes, except for replacing the existing aptamer or conjugate with the conjugate provided by the present disclosure. In some embodiments, the pharmaceutical composition may be prepared according to the following process:

The organic amines, helper lipids, and PEGylated lipids are suspended in alcohol at a molar ratio above and mixed homogeneously to yield a lipid solution; the alcohol is used in an amount such that the resultant lipid solution is present at a total mass concentration of 2 to 25 mg/mL (e.g., 8 to 18 mg/mL). The alcohol is a pharmaceutically acceptable alcohol, such as an alcohol that is in liquid form at about room temperature, for example, one or more of ethanol, propylene glycol, benzyl alcohol, glycerol, polyethylene glycol 200, polyethylene glycol 300, and polyethylene glycol 400, such as ethanol.

The conjugate provided by the present disclosure is dissolved in a buffered salt solution to produce an aqueous solution of the conjugate. The buffered salt solution has a concentration of 0.05 to 0.5 M, such as 0.1 to 0.2 M. The pH of the buffered salt solution is adjusted to 4.0 to 5.5, such as 5.0 to 5.2. The buffered salt solution is used in an amount such that the conjugate is present at a concentration of no more than 0.6 mg/mL, such as 0.2 to 0.4 mg/mL. The buffered salt may be one or more selected from the group consisting of soluble acetates and soluble citrates, such as sodium acetate and/or potassium acetate.

The lipid solution and the aqueous solution of the conjugate are mixed. The product obtained by mixing is incubated at a temperature of 40 to 60°C for at least 2 minutes (e.g., 5 to 30 minutes) to produce an incubated liposome formulation. The volume ratio of the lipid solution to the aqueous solution of the conjugate is 1:(2-5), such as 1:4.

The incubated liposome formulation is concentrated or diluted, subject to impurity removal, and then sterilized to afford the pharmaceutical composition of the present disclosure, which has the following physicochemical parameters: a pH of 6.5 to 8, an encapsulation efficiency of no less than 80%, a particle size of 40 to 200 nm, a polydispersity index of no

more than 0.30, and an osmotic pressure of 250 to 400 mOsm/kg. For example, the physicochemical parameters may be as follows: a pH of 7.2 to 7.6, an encapsulation efficiency of no less than 90%, a particle size of 60 to 100 nm, a polydispersity index of no more than 0.20, and an osmotic pressure of 300 to 400 mOsm/kg.

Therein, the concentration or dilution step may be performed before, after, or simultaneously with removal of the impurities. The method for removing impurities may be any of various existing methods, for example, ultrafiltration using a tangential flow system and a hollow fiber column under 100 kDa, with a phosphate buffer (PBS) at pH 7.4 as ultrafiltration exchange solution. The method for sterilization may be any of various existing methods, such as filtration sterilization on a 0.22 $\mu$m filter.

Use of the aptamer and/or conjugate provided by the present disclosure

In another aspect, the present disclosure further provides the use of the aptamer and/or conjugate and/or pharmaceutical composition provided by the present disclosure in the manufacture of a medicament for diagnosing and/or treating tumors and tumor-related diseases or symptoms.

In another aspect, the present disclosure further provides a method for diagnosing and/or treating tumors and tumor-related diseases or symptoms, comprising administering the conjugate and/or the pharmaceutical composition provided by the present disclosure to a subject in need thereof.

By administering the conjugate and/or the pharmaceutical composition provided by the present disclosure, the method provided by the present disclosure can effectively diagnose and/or treat tumors and tumor-related diseases or symptoms; and with the highly specific targeting effect of the conjugate provided by the present disclosure, the distribution of the diagnostic agent and/or therapeutic agent in other undesired organs/tissues of the body can be decreased, thereby reducing potential side effects, which is of great importance and significant value especially for radiotherapy and/or chemotherapy drugs commonly used in the field of tumor treatment and known for significant side effects. In some embodiments, the medicament may be used for diagnosing tumor cells/tissues in a sample to be tested in an *in vitro* experiment. In some embodiments, the medicament may be used for diagnosing tumor cells/tissues in a subject. In some embodiments, the medicament may be used for treating tumors or tumor-related diseases and symptoms in a subject.

As used herein, the term "administration/administer" refers to placing the conjugate and/or the pharmaceutical composition into a subject by a method or route where the conjugate and/or the pharmaceutical composition is at least partly located at a desired site to achieve a desired effect. The administration routes suitable for the method of the present disclosure include topical administration and systemic administration. In general, topical administration results in the delivery of more of the conjugate and/or the pharmaceutical composition to a particular site as compared with the whole body of the subject; whereas systemic administration results in the delivery of the conjugate and/or the pharmaceutical composition to substantially the whole body of the subject.

Furthermore, the inventors of the present disclosure have unexpectedly found that the conjugate and/or the pharmaceutical composition provided by the present disclosure can efficiently pass through the blood brain barrier and target tumors in the brain upon systemic administration, thereby further improving the delivery efficiency of the functional groups, saving costs, and reducing undesired side effects.

The administration to a subject can be achieved by any suitable route known in the art, including but not limited to, oral or parenteral routes, such as intravenous administration, intramuscular administration, subcutaneous administration, transdermal administration, intratracheal administration (aerosol), pulmonary administration, nasal administration, rectal administration, and topical administration (including buccal administration and sublingual administration). The administration frequency may be once or more times daily, weekly, biweekly, triweekly, monthly, or yearly.

The dose of the conjugate and/or the pharmaceutical composition provided by the present disclosure can be a conventional dose in the art, which may be determined according to various parameters, especially age, weight, and gender of a subject. Toxicity and efficacy may be determined in cell cultures or experimental animals by standard pharmaceutical procedures, for example, by determining LD50 (the lethal dose that causes 50% population death) and ED50 (the dose that can cause 50% of the maximum response intensity in a quantitative response, or that causes 50% of the experimental subjects to have a positive response in a qualitative response). The dose range for human use may be derived based on the data obtained from cell culture analysis and animal studies.

When the conjugate and/or the pharmaceutical composition provided by the present disclosure is administered, for example, to male or female C57BL/6J or C3H/HeNCrlVr mice with an age of 6 to 12 weeks and a body weight of 18 to 25 g (based on the amount of the conjugate and/or the conjugate in the pharmaceutical composition): for the conjugate formed by a conjugate and a pharmaceutically acceptable conjugation molecule, the dosage of the conjugate may be 0.001 to 100 mg/kg body weight, in some embodiments 0.01 to 50 mg/kg body weight, in some further embodiments 0.05 to 20 mg/kg body weight, in some even further embodiments is 0.1 to 15 mg/kg body weight, and in some still further embodiments 0.1 to 10 mg/kg body weight. When the conjugate and/or the pharmaceutical composition provided by the present disclosure is administered, the above dosages may be preferred.

Kit

The present disclosure provides a kit comprising the conjugate and/or the pharmaceutical composition provided by the present disclosure.

In some embodiments, the kit provided by the present disclosure may provide the conjugate and/or the pharmaceutical composition in a container. In some embodiments, the kit provided by the present disclosure may comprise a container for providing pharmaceutically acceptable excipients. In some embodiments, the kit may further comprise additional ingredients, such as stabilizers or preservatives. In some embodiments, the kit provided by the present disclosure may comprise at least one additional therapeutic agent in a container other than the container for providing the conjugate and/or the pharmaceutical composition provided by the present disclosure. In some embodiments, the kit may comprise an instruction for mixing the conjugate and/or the pharmaceutical composition with pharmaceutically acceptable carriers and/or adjuvants or other ingredients (if any).

In the kit provided by the present disclosure, the conjugate and the pharmaceutically acceptable carriers and/or adjuvants, as well as the pharmaceutical composition and/or the pharmaceutically acceptable adjuvants may be provided in any form, e.g., in a liquid form, a dry form, or a lyophilized form. In some embodiments, the conjugate and the pharmaceutically acceptable carriers and/or adjuvants as well as the pharmaceutical composition and the optional pharmaceutically acceptable adjuvants are substantially pure and/or sterile. In some embodiments, the kit provided by the present disclosure may provide sterile water.

Hereinafter, the present disclosure will be further illustrated with reference to the Examples, but is not limited thereto in any respect.

## Example

Unless otherwise specified, the reagents and culture media used in the following Examples are all commercially available, and the procedures used, such as nucleic acid electrophoresis and real-time PCR, are all performed according to the methods described in Molecular Cloning (Cold Spring Harbor LBboratory Press (1989)).

### Preparation Examples 1-9 and 12 Synthesis of the conjugates provided by the present disclosure

The conjugates numbered AP1-AP8 and AP12 in Table 1A were respectively synthesized by the solid phase synthesis methods, comprising sequentially linking all nucleoside monomers in the 3' to 5' direction according to the corresponding nucleotide sequences of AP1-AP8 and AP12 in Table 1A respectively, and then linking the Cy5 phosphoramidite monomer according to the manner of linking nucleoside phosphoramidite monomers in the solid phase synthesis method (pursued from Suzhou GenePharma Inc., Lot No. CY5P21H1B). Subsequently, the nucleotide sequence was added into the mixture solution of methylamine aqueous solution and aqueous ammonia in equal volumes (the amount of the solution relative to the conjugate was 0.5 ml/μmol), the mixture reacted at 25°C for 2h, the solid was removed by filtration, and the supernatant was concentrated in vacuum to dryness.

Purification of the prepared conjugates was achieved by using a preparative ion chromatography purification column (Source 15Q) with a gradient elution of NaCl aqueous solution. Specifically, eluant A was 20 mM sodium phosphate (pH 8.1), solvent was water/acetonitrile in 9:1 (v/v); eluant B was 1.5 M sodium chloride, 20 mM sodium phosphate (pH 8.1), solvent was water/acetonitrile in 9:1 (v/v); elution gradient: the ratio of eluant A: eluant B = 100:0-50:50. The eluate was collected, combined and desalted by using a reverse phase chromatography purification column. The specific condition comprises: using a Sephadex column for desalination (filler: Sephadex G25) and eluting with deionized water. The resultant eluate was concentrated to remove the solvent and lyophilized to respectively obtain conjugates AP1-AP8, in which the 5' position of the ribose of the 5' terminal nucleotide was linked to the fluorescent group Cy5 by a phosphate ester linking group.

Conjugate AP9 was prepared according to the same method, in which the 5' position of the ribose of the 5' terminal nucleotide was linked to the fluorescent group Cy5 by a phosphate ester linking group, with the only difference of sequentially linking nucleoside monomers according to the corresponding nucleic acid sequences of AP9 in Table 1A, and using Cy3 phosphoramidite monomer (purchased from Shanghai Zhaowei Technology Development Co., Ltd., Cat No. OP-038) to replace Cy5 phosphoramidite monomer for preparation.

After completion of the synthesis of the aforementioned conjugates AP1-AP9 and AP12, the resultant conjugates were diluted to a concentration of 0.2 mg/mL by using ultra-pure water (homemade by Milli-Q ultra-pure water instrument, with resistivity of 18.2 MΩ* cm (25°C)). The molecular weight was determined by a LC-MS instrument (LC-MS, liquid chromatography-mass spectrometry, purchased from Waters Crop., model: LCT Premier). As a result, the measured value of the molecular weight was in conformity with the calculated value, indicating that the target conjugates had been obtained.

**Comparative preparation Examples 10, 11, 13 and 14 Synthesis of the**

**comparative conjugates**

The conjugates numbered comparative AP10, comparative AP11, comparative AP13 and comparative AP14 in Table 1A were respectively synthesized according to the method of the preparation Examples 1-9 and 12, with the only difference of sequentially linking nucleoside monomers respectively according to the corresponding sequences of comparative AP10, comparative AP11, comparative AP13 and comparative AP14 in Table 1A. Therefore, the comparative conjugates comparative AP10, comparative AP11, comparative AP13 and comparative AP14 were respectively obtained, in which the 5' position of the ribose of the 5' terminal nucleotide was linked to the fluorescent group Cy5 by a phosphate ester linking group. Therein, comparative AP10 and comparative AP11 were respectively negative control conjugates with different sequences or modification schemes, and their aptamer sequences have certain randomness and almost have no sequence homology with the aptamers provided by the present disclosure. Comparative AP13 and comparative AP14 were respectively comparative conjugates whose sequences have only a few nucleotide differences from the sequences of conjugates AP2 and AP4. Specifically, as compared with AP2, comparative AP13 adds two nucleotides G and A at the 5' terminal, deletes one nucleotide C at the 3' terminal, and deletes one nucleotide U at the position corresponding to the 17th-18the nucleotide from the 5' terminal of AP2 within the strand; as compared with AP4, comparative AP14 adds two nucleotides G and A at the 5' terminal, deletes one nucleotide U at the 3' terminal, and deletes one nucleotide U at the position corresponding to the 17th-18th nucleotide from the 5' terminal of AP2 within the strand.

Table 1A Nucleotide sequences of the conjugates

| Preparation Example | Conjugate | Nucleotide sequence 5'-3' | SEQ ID NO: |
|---|---|---|---|
| Preparation Example 1 | AP1 | CY5-CfUfGGAGUfUfCfAGACfGUfGUfUfGCfUfCfUfUfCfCfGAUfCfUfCf | 15 |
| Preparation Example 2 | AP2 | CY5-CmUmGmGmAmGmUmUmCmAmGmAmCmGmUmGmUmUmGmCmUmCmUmUmCmCmGmAmUmCmUmCm | 21 |
| Preparation Example 3 | AP3 | CY5-CmsUmsGmGmAmGmUmUmCmAmGmAmCmGmUmGmUmUmGmCmUmCmUmUmCmCmGmAmUmCmsUmsCm | 34 |
| Preparation Example 4 | AP4 | CY5-CmUmGmGmAmGmUmUmCmAmGmAmCmGmUmGmUmUmGmCmUmCmUmUm | 22 |
| Preparation Example 5 | AP5 | CY5-CmUmGmGmAmGmUmUmCmAmGmAmCmGmUmUmGmUmGmCmUmCmUmUmCmCmGmAmUmCmUmCm | 23 |
| Preparation Example 6 | AP6 | CY5-CmUmGmGmAmGmUmUmCmAmGmAmCmGmUmUmGmUmGmCmUmCmUmUm | 24 |
| Preparation Example 7 | AP7 | CY5-CmUmGmGmAmGmUmUmCmAmGmAmCmCmGmUmGmUmGmCmUmCmUmUmCmCmGmAmUmCmUmCm | 25 |
| Preparation Example 8 | AP8 | CY5-CmUmGmGmAmGmUmUmCmAmGmAmCmCmGmUmGmUmGmCmUmCmUmUm | 26 |
| Preparation Example 9 | AP9 | CY3-CmUmGmGmAmGmUmUmCmAmGmAmCmGmUmGmUmUmGmCmUmCmUmUmCmCmGmAmUmCmUmCm | 21 |

(continued)

| Preparation Example | Conjugate | Nucleotide sequence 5'-3' | SEQ ID NO: |
|---|---|---|---|
| Comparative preparation Example 10 | Comparative AP10 | CY5-CfCfAGAGUfCfGCfUfGAGAUfUfCfGUfGGCfGCfUfAUfAUf | 46 |
| Comparative preparation Example 11 | Comparative AP11 | CY5-UfCfUfAUfGGCfUfGCfCfGAUfCfUfGGUfCfUfCfCfAUfGUfACfGUf | 47 |
| Preparation Example 12 | AP12 | CY5-UmCmUmAmUmGmGmCmUmGmCmCmGmAmUmCmUmGmGmUmCmUmCmCmAmUmGmUmAmCmGmUm | 33 |
| Comparative preparation Example 13 | Comparative AP13 | CY5-GmAmCmUmGmGmAmGmUmUmCmAmGmAmCmGmUmGmUmGmCmUmCmUmUmCmCmGmAmUmCmUm | 49 |
| Comparative preparation Example 14 | Comparative AP14 | CY5-GmAmCmUmGmGmAmGmUmUmCmAmGmAmCmGmUmGmUmGmCmUmCmUm | 50 |

In Table 1A, C, G, U, and A represent the base composition of a nucleotide; m represents that the nucleotide adjacent to the left side of the letter m is a methoxy modified nucleotide; f represents that the nucleotide adjacent to the left side of the letter f is a fluoro modified nucleotide; s represents that the two nucleotides adjacent to both sides of the letter s are linked by phosphorothioate linkage; CY3 and CY5 respectively represent the attachment sites of fluorescent dye group Cy3 (Cyanine 3) and Cy5 (Cyanine 5) on the aptamer.

**Preparation Examples 15-16 Synthesis of conjugate 15 and conjugate 16**

In preparation Example 15, conjugate 15 was prepared according to the following steps.

(15-1) Preparation of oligonucleotide C1

The oligonucleotide sequence of conjugate 15 in Table 1B was synthesized by the methods of preparation Examples 1-8 and 12, with the only difference that the sequence of the sense strand comprises the sense strand of the siRNA, of which the 3' terminal was linked to delivery group contained in conjugate AP2 by Spacer18-GfAfUf as a linking group, wherein Spacer18 refers to a hexamethylene glycol subunit linking group comprising 6 sequentially linked ethyleneoxy groups and comprising 18 atoms in length. After sequentially linking to the nucleoside monomers GfAfUf in the 3' to 5' direction, the linking group was then linked to hexamethylene glycol (HEG)-phosphoramidite monomer by the solid phase synthesis method. HEG-phosphoramidite monomer has the structure as shown by Formula (15-a).

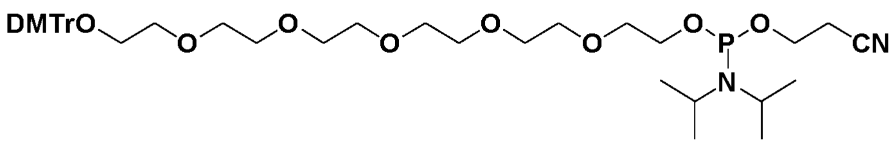

Formula (15-a)

In the solid phase synthesis process, after linking the last nucleoside monomer at the 5' terminal, the phosphoramidite monomer containing -NH-$(CH_2)_6$- group (purchased from Hongene Biotech Pte.Ltd.) was additionally linked according to the method of linking the nucleoside phosphoramidite monomer, the oligonucleotide single strand was cleaved from the solid phase support and subject to ammonolysis concentration to give a crude product, and the crude product was desalted by ultrafiltration and concentrated to dryness to obtain the oligonucleotide single strand C1as shown

in Formula (15-b) (40.00 mg, 5.49 μmol):

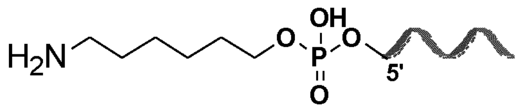

C1

Formula (15-b)

In the Formula,

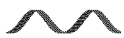

represents the oligonucleotide sequence corresponding to conjugate 15.

(15-2) Preparation of oligonucleotide C2

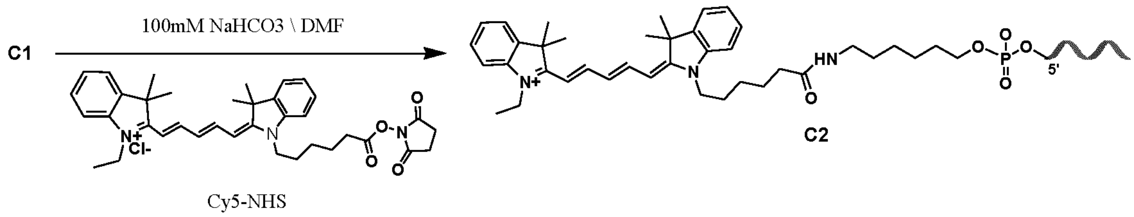


C1 Cy5-NHS C2

346 mg Cy5-NHS was dissolved in 5.4 ml solvent DMF. The concentrated dry oligonucleotide single strand C1 was taken and dissolved in 1.34 mL 100 mM sodium bicarbonate aqueous solution for later use. The solution of Cy5-NHS in DMF was added into the solution of oligonucleotide single strand C1, then triethylamine was added, and the mixture reacted at room temperature overnight in the dark. Methyl tert-butyl ether/ethanol solution (v:v=4: 1) was added into the reaction solution in an amount being 3 times the volume of the reaction solution, the mixture was vortexed for 1 min at room temperature and centrifuged at 3200g for 15 min, then the supernatant was removed, and the pellet was the desired crude product of oligonucleotide single strand C1. The nucleic acid sample was dissolved in pure water and purified by Agilent semi-preparative reversed phase column using the following mobile phase gradient elution: eluant A: 100 mM TEAA-water (pH = 7.2), eluant B: acetonitrile (ACN), gradient: 15-75% (B v/v%). The solvent was removed by concentration to ontain 43.4 mg oligonucleotide single strand C2 linked with the fluorescent group Cy5 by a $-NH-(CH_2)_6-$ group and a phosphate ester linking group.

The sense strand of conjugate 16 was synthesized according to the same method, with the only difference of sequentially linking nucleotides according to the nucleotide sequence of the sense strand of conjugate 16 in Table 1A. Unlike conjugate 3, in which the delivery group was linked to the 3' terminal of the sense strand of the siRNA, the 5' terminal of the sense strand of the siRNA in conjugate 4 was linked to the 3 terminal of the delivery group contained in conjugate AP2 by Spacer18-GfAfUf connection as a linking group, and the 5' position of the ribose of the 5' terminal nucleotide of the delivery group was linked with the fluorescent group Cy5 by a $-NH-(CH_2)_6-$ group and a phosphate ester linking group.

The sequences of the antisense strands of the conjugates numbered conjugate 15 and conjugate 16 in Table 1B were synthesized according to the nucleic acid solid phase synthesis method.

The sense strand and antisense strand of each conjugate were respectively dissolved in water for injection, to give a solution of 40 mg/mL. They were mixed in an equimolar ratio, heated at 50°C for 15 minutes, and cooled to room temperature to form a double-stranded structure by hydrogen bond. Then, the resultant conjugates were purified, desalted, solvent-removed and lyophilized according to the methods described in preparation Examples 1-2, to obtain conjugate 15 and conjugate 16, respectively. After synthesis, for the resultant sense strands and antisense strands of conjugate 3 and conjugate 4, their purities were respectively determined by using Ion Exchange Chromatography (IEX-HPLC) and their molecular weights were analyzed by Liquid Chromatography-Mass Spectrometry (LC-MS). The MS calculated values of the sense strands of conjugates 3 and 4 were 18986.82; the measured value of conjugate 3 was 18984.81, and the measured value of conjugate 4 was 18984.84. The antisense strands of conjugates 3 and 4 were identical; the calculated value was 6881.59 and the measured value of MS molecular ion peak was 1719.2 (4-valence anion). The measured value was in conformity with the calculated value. Conjugates 15 and 16 were respectively the conjugates which comprise a double-stranded siRNA and a delivery group contained in conjugate AP2, wherein the

delivery group was linked to the 5' terminal or the 3' terminal of the sense strand of the siRNA by Spacer18-GfAfUf linking group, and the 5' position of the ribose of the 5' terminal nucleotide of the entire nucleotide sequence comprising the sense strand of the siRNA was conjugately linked to the fluorescent group Cy5 by a -NH-$(CH_2)_6$- group and a phosphate ester linking group.

Table 1B Nucleotide sequences of the conjugates

| Preparation Example | Conjugate | sequence | Nucleotide sequence 5'-3' | SEQ ID NO: |
|---|---|---|---|---|
| Preparation Example 15 | Conjugate 15 | Sense strand | CY5-(NH-C6H12)-CmsUmsAmGmAmAmAfAfCfUmGmGmAmUmAmAmCmGmUm-(Spacer18-GfAfUf)-CmUmGmGmAmGmUmUmCmAmGmAmCmGmUmGmUmUmGmCmUmCmUmUmCmCmGmAmUmCmUmCm | 51 |
| | | Antisense strand | AmsCfsGmUmUmAfUmCmCmAmGmUmUmUfUmCfUmAmGmsCmsCm | 45 |
| Preparation Example 16 | Conjugate 16 | Sense strand | CY5-(NH-C6H12)-CmUmGmGmAmGmUmUmCmAmGmAmCmGmUmGmUmUmGmCmUmCmUmUmCmCmGmAmUmCmUmCm-(Spacer18-GfAfUf)-CmsUmsAmGmAmAmAfAfCfUmGmGmAmUmAmAmCmGmUm | 52 |
| | | Antisense strand | AmsCfsGmUmUmAfUmCmCmAmGmUmUmUfUmCfUmAmGmsCmsCm | 45 |
| Preparation Example 17 | Conjugate 17 | Sense strand | CY5-(NH-C6H12)-CmUmGmGmAmGmUmUmCmAmGmAmCmGmUmGmUmUmGmCmUmCmUmUmCmCmGmAmUmCmUmCmXXXXUUCmsUmsAmGmAmAmAfAfCfUmGmGmAmUmAmAmCmGmUm | 53 |
| | | Antisense strand | AmsCfsGmUmUmAfUmCmCmAmGmUmUmUfUmCfUmAmGmsCmsCm | 45 |
| Preparation Example 18 | Conjugate 18 | Sense strand | CY5-(NH-C6H12)-ChdUhdGmGmAmGmUmUmCmAmGmAmCmGmUmGmUmUmGmCmUmCmUmUmCmCmGmAmUmCmUhdChdXXXXUUCmsUmsAmGmAmAmAfAfCfUmGmGmAmUmAmAmCmGmUm | 54 |
| | | Antisense strand | AmsCfsGmUmUmAfUmCmCmAmGmUmUmUfUmCfUmAmGmsCmsCm | 45 |

(continued)

| Preparation Example | Conjugate | sequence | Nucleotide sequence 5'-3' | SEQ ID NO: |
|---|---|---|---|---|
| Preparation Example 19 | Conjugate 19 | Sense strand A | CY5-(NH-C6H12)-CmUmGmGmAmGmUmUmCmAmGmAmCmGmUmGmUmUmGmCmUmCmUmUmCmCmGmAmUmCmUmCmXXXXGmUfAmCfAmUfUfCfUfAmGmAmUfAmGmCfCf | 55 |
| | | Sense strand B | CmsUmsAmGmAmAmAfAfCfUmGmGmAmUmAmAmCmGmUm | 44 |
| | | Antisense strand | AmsCfsGmUmUmAfUmCmCmAmGmUmUmUfUmCfUmAmGmsCmsCmGmGmCfUfAmUfCfUfAmGfAmAmUfGmUfAmCf | 57 |
| Preparation Example 36 | Conjugate 36 | Sense strand | CY5-CmsUmsAmGmAmAmAfAfCfUmGmGmAmUmAmAmCmGmUm-dTdTdTdTdTdT-CmUmGmGmAmGmUmUmCmAmGmAmCmGmUmGmUmUmGmCmUmCmUmUmCmCmGmAmUmCmUmCm | 56 |
| | | Antisense strand | AmsCfsGmUmUmAfUmCmCmAmGmUmUmUfUmCfUmAmGmsCmsCm | 45 |
| Preparation Example 37 | Conjugate 37 | Sense strand | CmsUmsAmGmAmAmAfAfCfUmGmGmAmUmAmAmCmGmUm-(Spacer18-GfAfUf)-CmUmGmGmAmGmUmUmCmAmGmAmCmGmUmGmUmUmGmCmUmCmUmUmCmCmGmAmUmCmUmCm | 71 |
| | | Antisense strand | CY5-AmsCfsGmUmUmAfUmCmCmAmGmUmUmUfUmCfUmAmGmsCmsCm | 48 |
| Preparation Example 38 | Conjugate 38 | Sense strand | ChdUhdGmGmAmGmUmUmCmAmGmAmCmGmUmGmUmUmGmCmUmCmUmUmCmCmGmAmUmCmUhdChdXXXXUUCmsUmsAmGmAmAmAfAfCfUmGmGmAmUmAmAmCmGmUm | 72 |
| | | Antisense strand | CY5-AmsCfsGmUmUmAfUmCmCmAmGmUmUmUfUmCfUmAmGmsCmsCm | 48 |

In Table 1B, C, G, U, A and T represent the base composition of a nucleotide; m represents that the nucleotide adjacent to the left side of the letter m is a methoxy modified nucleotide; d represents that the nucleotide adjacent to the right side of the letter d is a deoxy nucleotide; f represents that the nucleotide adjacent to the left side of the letter f is a fluoro modified nucleotide; s represents that the two nucleotides adjacent to both sides of the letter s are linked by phosphorothioate linkage; CY3 and CY5 respectively represent the attachment sites of fluorescent dye group Cy3 (Cyanine 3) and

Cy5 (Cyanine 5) on the aptamer; CY5-(NH-C6H12) represents the site in the siRNA that is linked to fluorescent dye group Cy5 by a N-hexyl amino linking group; X represents propylene - phosphate ester group; hd represents that the nucleotide adjacent to the left side of the letter combination hd is a 2'-O-cetyl modified nucleotide; Spacer18 represents hexamethylene glycol subunit linking group above; MMAE represents the attachment site of the small molecule drug group MMAE (Monomethylauristatin E) on the aptamer.

**Preparation Examples 17-18 Synthesis of conjugate 17 and conjugate 18**

The conjugates numbered conjugate 17 and conjugate 18 in Table 1B were synthesized according to the same method as that of preparation Examples 15-16, with the only differences that nucleoside monomers were sequentially linked according to the corresponding nucleic acid sequences of conjugate 17 and conjugate 18 in Table 1B; and, when preparing the sense strand, C3-spacer phosphoramidite monomer (purchased from Shanghai Zhaowei Technology Development Co., Ltd., Cat No. OP-005) was used to replace Spacer18-GfAfUf linking group for linking; the linking was repeated four times and then linked to nucleoside monomers according to the nucleic acid sequences in Table 1B. That is, the resultant conjugate 17 was a conjugate which comprises a double-stranded siRNA and the aptamer provided by the present disclosure, wherein the aptamer was linked to the 5' terminal of the sense strand of the siRNA by four sequentially linked propylene phosphate ester groups as linking groups, and 5' terminal of the aptamer was linked to the fluorescent group Cy5 by -a NH-$(CH_2)_6$- group and a phosphate ester linking group; conjugate 18 was a conjugate in which the first two nucleotides of each terminal of the aptamer in conjugate 17 had 2'-O-cetyl modifications.

**Preparation Example 19 Synthesis of conjugate 19**

The conjugate numbered conjugate 19 in Table 1B was synthesized according to the same method as that of preparation Examples 15-16, with the only difference that nucleoside monomers were sequentially linked according to the corresponding nucleic acid sequence of conjugate 19 in Table 1B, to obtain three nucleotide sequences; and, when annealing, the resultant three nucleotide sequences were dissolved in water for injection and mixed in an equimolar ratio. The resultant conjugate 19 was a conjugate which comprises a double-stranded siRNA and conjugate AP2 provided by the present disclosure, wherein the aptamer was linked to the siRNA by a linking group which was a double strand formed by the complementary nucleotide sequences respectively having the sequences as shown in SEQ ID NO: 28 and SEQ ID NO: 29, the aptamer was linked to the 5' terminal of the sequence as shown in SEQ ID NO: 28, and the siRNA was linked to the 3' terminal of the sequence as shown in SEQ ID NO: 29, and the 5' terminal of the aptamer was linked to the fluorescent group Cy5 by a -NH-$(CH_2)_6$- group and a phosphate ester linking group. After preparation, 12% polyacrylamide gel was prepared; the prepared conjugate 19 was dissolved in nucleic acid sample loading buffer, mixed thoroughly and loaded into the polyacrylamide gel, and vertical electrophoresis was performed under 80V voltage for 60 minutes. After electrophoresis, the gel was stained by Gelstain soaking method comprising immersing the gel in 3x soaking solution and gently agitating at room temperature for 1h, and then imaged on a gel imager. Imaging result showed that the prepared conjugate 19 showed a single band, indicating that a double-stranded conjugate was obtained successfully, i.e. conjugate 19, in which a double-stranded complementary region was formed between each nucleotide sequences.

**Preparation Example 20 Synthesis of conjugate 20**

In this preparation example, conjugate 20 was prepared according to the following steps. Conjugate 20 comprises the aptamer provided by the present disclosure and the small molecule drug group MMAE, wherein the MMAE group was linked to the 5' terminal of the aptamer by linking group, and the linking group was 2-(phosphate-$(CH_2)_6$-S-)-maleimide caproyl -valine-citrulline-p-aminobenzylene.

(20-1) Preparation of aptamer S1

The aptamer sequence of conjugate 20 in Table 1C was synthesized by the solid phase synthesis method, with the only difference that nucleoside monomers were sequentially linked according to the corresponding aptamer sequence of conjugate 20 in Table 1C; in the solid phase synthesis process, after linking the last nucleoside monomer at the 5' terminal, the phosphoramidite monomer containing HO-$(CH_2)_6$-S-S-$(CH_2)_6$-group (purchased from Hongene Biotech Pte.Ltd.) was linked according to the method of linking the nucleoside phosphoramidite monomer, and the aptamer single strand was cleaved from the solid phase support to obtain aptamer single strand S1 as shown in Formula (20-a) (70.00 mg, 6.42 μmol):

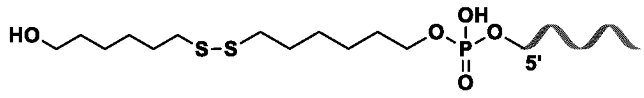

S1

Formula (20-a)

In the Formula,

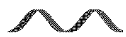

represents the corresponding aptamer sequence of conjugate 20.

(20-2) Synthesis of aptamer S2

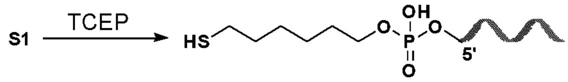


S2

After dissolving 70.0 mg S1 prepared in step (20-1) (6.42 μmol) in 10.0 ml purified water, the TCEP solution of 105 mg TCEP (tri(2-chloroethyl) phosphate ester (0.37 mmol, purchased from Bide Pharmatech Ltd., Lot No. BD155793) in 10.0 ml purified water was added into the resultant solution. The reaction solution was mixed thoroughly, reacted at room temperature for 2h, then diluted with 10 mL purified water, and filtered to obtain a 28 mL reaction solution. The resultant reaction solution was transferred to a 3K ultrafiltration tube and centrifuged at 3900 rpm for 30 min. Then, the steps of ultrafiltration and centrifugation were repeated twice, and the product in the filter membrane was collected to obtain aptamer S2 (67.0 mg, yield: 95.7%).

(20-3) Synthesis of aptamer 20

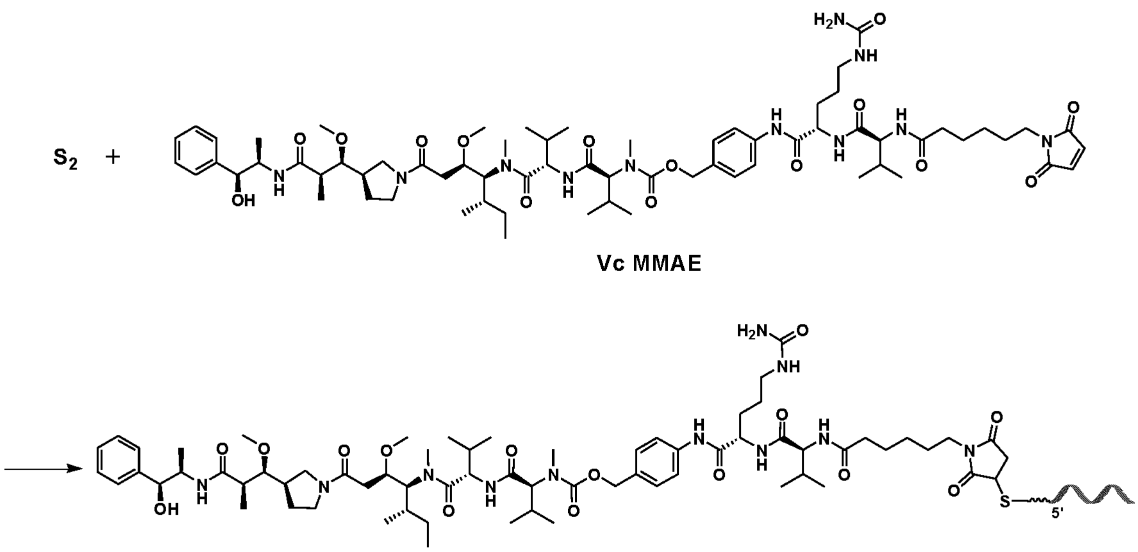


$S_3$

24 mg Vc MMAE (18.56 μmol, 5eq, purchased from CSN Company, Lot No. CSN16143-005) was dissolved in 6.0 ml DMF, and was added with 60 μl triethylamine to obtain a Vc MMAE solution. After dissolving 40.0 mg aptamer S2 prepared in step (20-2) (3.71 μmol, 1eq) in 6.0 ml purified water, the resultant solution was added with the Vc MMAE

solution above, reacted at room temperature for 2h, to obtain the crude product of conjugate 20 (indicated as S3 in process diagram).

The resultant crude product of conjugate 20 was diluted with 0.5 ml purified water and filtered with 0.45 μm filter membrane. The filtrate was purified by Agilent semi-preparative reversed phase column chromatography (the chromatography column used was Kromasil 100-10-C18, 100Å, 10 μ m, 21.2*250 mm), in which gradient elution was performed with the mobile phase: 100 mM triethylamine acetate buffer (TEAA, pH=7.0-7.3) : acetonitrile = 95:5 - 35:65. The eluate of the product peak was collected and evaporated to remove solvent, to obtain conjugate 20 (55 mg, yield 56.7%). The molecular weight was determined by a LC-MS, calculated value: 12092.67, measured value: 12091.68. The fact the measured value was in conformity with the calculated value indicated that conjugate 20 had the structure as shown in S3 and comprised the aptamer provided by the present disclosure and small molecule drug group MMAE, wherein the MMAE group was linked to the 5' terminal of the aptamer by a linking group, and the linking group was 2-(phosphate-$(CH_2)_6$-S-)-maleimide caproyl -valine-citrulline-p-aminobenzylene (2-(phosphate-$(CH_2)_6$-S-) - MC-Val-Cit-PAB).

Table 1C Nucleotide sequences of aptamer single strand

| | | | | |
|---|---|---|---|---|
| Preparation Example 20 | Conjugate 20 | Aptamer single strand | MMAE-CmUmGmGmAmGmUmUmCmAmGmAmCmGmUmGmUmUmGmCmUmCmUmUmCmCmGmAmUmCmUmCm | 21 |
| Preparation Example 39 | Conjugate 39 | Aptamer single strand | MMAE-CmUmGmGmAmGmUmUmCmAmGmAmCmGmUmUmGmUmGmCmUmCmUmUm | 73 |
| Preparation Example 40 | Conjugate 40 | Aptamer single strand | MMAE-CmUmGmGmAmGmUmUmCmAmGmAmCmGmUmGmUmUmGmCmUmCmUmUm | 74 |
| Preparation Example 41 | Conjugate 41 | Aptamer single strand | MMAE-CmUmGmGmAmGmUmUmCmAmGmAmCmCmGmUmGmUmGmCmUmCmUmUm | 75 |
| Preparation Example 42 | Conjugate 42 | Aptamer single strand | MMAE-CmUmGmCmUmUmCmAmGmAmCmGmUmGmUmUmAmGmCmUmUm | 76 |
| Preparation Example 43 | Comparative conjugate 43 | Aptamer single strand | MMAE-GmGmAmCmAmUmGmGmAmUmUmCmUmUmGmUmCmUmGmUmGmUmCmCm | 77 |
| Preparation Example 44 | Comparative conjugate 44 | Aptamer single strand | MMAE-CmGmUmCmUmCmCmAmGmCmAmUmGmUmGmUmAmGmGmUmCmUmCmUmGmUmCmUmCmUmGmAm | 78 |

In Table 1C, C, G, U, and A represent the base composition of a nucleotide; m represents that the nucleotide adjacent to the left side of the letter m is a methoxy modified nucleotide; f represents that the nucleotide adjacent to the left side of the letter f is a fluoro modified nucleotide; MMAE represents the attachment site of small molecule drug group MMAE (Monomethylauristatin E) on the aptamer.

**Preparation Example 21-26 Synthesis of conjugates AP21-AP26 provided by the present disclosure**

The conjugates numbered AP21-AP26 in Table 2 were synthesized according to the method of preparation Examples 1-8 and 12, and the synthesized conjugates were confirmed by determining molecular weights thereof, with the only difference of sequentially linking nucleoside monomers according to the corresponding sequences of AP21-AP26 in Table 2. Therefore, conjugates AP21-AP26 with fluorescent group Cy5 linked at the 5' terminal were obtained respectively. Therein, as compared with AP4 above, AP21 deletes one nucleotide at the 3' terminal; as compared with AP4 above, AP22

has the structure in which the motifs $S_1$ and $S_4$ each delete one nucleotide in the sequence corresponding to Formula (1); AP23 has the structure of AP22 in which the motif $S_1$ deletes one more nucleotide in the sequence corresponding to Formula (1); AP24 is a conjugate obtained by changing the first two nucleotides at the 5' terminal of AP4; as compared with AP4 above, AP25 and AP26 are the conjugates obtained by changing $N_b$ motif in the sequence corresponding to Formula (1).

After completion of the synthesis of the aforementioned conjugates AP11-AP26, the resultant conjugates were diluted to a concentration of 0.2 mg/mL by using ultra-pure water (homemade by Milli-Q ultra-pure water instrument, with resistivity of 18.2 MΩ* cm (25°C)). The molecular weight was determined by a LC-MS instrument (LC-MS, liquid chromatography-mass spectrometry, purchased from Waters Crop., model: LCT Premier). As a result, the measured value of the molecular weight was in conformity with the calculated value, indicating that the target conjugates had been obtained.

The MS calculated values and the MS measured values of conjugates AP11-AP26 are shown in Table 2-a below:

| Number | Calculated MS value | Measured MS value |
|---|---|---|
| AP21 | 8173.61 | 8171.39 |
| AP22 | 7814.38 | 7812.23 |
| AP23 | 7455.14 | 7452.96 |
| AP24 | 8516.85 | 8514.56 |
| AP25 | 8414.74 | Molecular ion peak: 2101.9(4-valence); 1681.4(5-valence) |
| AP26 | 8471.75 | 8469.53 |

**Comparative preparation Examples 27-35 Synthesis of control conjugates comparative AP27 - comparative AP35**

The conjugates numbered comparative AP27- comparative AP35 in Table 2 were synthesized according to the method of preparation Examples 1-9 and 12, and confirmed by determining molecular weight thereof, with the only difference of sequentially linking nucleoside monomers according to the corresponding sequences of comparative AP27- comparative AP35 in Table 2. Therefore, control aptamer conjugates comparative AP27- comparative AP35 with fluorescent group Cy5 linked at the 5' terminal were obtained respectively. Therein, comparative conjugates comparative AP27 and comparative AP28 were control conjugates in which the base composition of the sequences was the same as that of AP4, but the arrangement order of nucleotides was different; conjugates comparative AP29 and comparative AP30 were control conjugates in which the base composition of the sequences was the same as that of AP2, but the arrangement order of nucleotides was different; as compared with comparative AP4, comparative AP31 deletes two nucleotides at the 5' terminal, and deletes one nucleotide at the 3' terminal; as compared with comparative AP4, AP32 deletes $N_b$ motif in the sequence corresponding to Formula (1); as compared with AP4, AP33 deletes $N_a$ and $N_c$ motifs in the sequences corresponding to Formula (1); as compared with comparative AP35, comparative AP34 was a control conjugate which respectively increases multiple nucleotides and changes partial sequence in S2 and S3 motifs.

Table 2 Nucleotide sequences of conjugates

| Preparation Example | Conjugate | Nucleotide sequence 5'-3' | SEQ ID NO: |
|---|---|---|---|
| Preparation Example 21 | AP21 | CY5-CmUmGmGmAmGmUmUmCmAmGmAmCmGmUmGmUmUmGmCmUmCmUm | 27 |
| Preparation Example 22 | AP22 | CY5-CmUmGmAmGmUmUmCmAmGmAmCmGmUmGmUmUmGmCmUmCmUm | 28 |
| Preparation Example 23 | AP23 | CY5-CmUmGmCmUmUmCmAmGmAmCmGmUmGmUmUmAmGmCmUmUm | 29 |
| Preparation Example 24 | AP24 | CY5-AmCmGmGmAmGmUmUmCmAmGmAmCmGmUmGmUmUmGmCmUmCmUmUm | 30 |

(continued)

| Preparation Example | Conjugate | Nucleotide sequence 5'-3' | SEQ ID NO: |
|---|---|---|---|
| Preparation Example 25 | AP25 | CY5-CmUmGmGmAmGmUmUmCmAmCmUmAmCmUmGmUmUmGmCmUmCmUmUm | 31 |
| Preparation Example 26 | AP26 | CY5-CmUmGmGmAmGmUmUmCmAmGmUmUmGmUmGmUmUmGmCmUmCmUmUm | 32 |
| Comparative preparation Example 27 | Comparative AP27 | CY5-GmGmAmCmAmUmGmGmAmUmUmCmUmUmGmUmCmUmGmUmGmUmCmCm | 58 |
| Comparative preparation Example 28 | Comparative AP28 | CY5-GmAmUmCmUmGmUmGmUmCmUmUmAmGmAmCmGmUmGmUmUmGmCmCm | 59 |
| Comparative preparation Example 29 | Comparative AP29 | CY5-GmAmUmCmGmUmGmAmCmGmAmCmUmCmUmUmGmUmCmUmGmCmUmGmUmCmCmUmGmAmUmCm | 60 |
| Comparative preparation Example 30 | Comparative AP30 | CY5-CmGmUmCmUmCmCmAmGmCmAmUmGmUmGmUmAmGmGmUmCmUmCmUmGmUmCmUmCmUmGmAm | 61 |
| Comparative preparation Example 31 | Comparative AP31 | CY5-GmGmAmGmUmUmCmAmGmAmCmGmUmGmUmUmGmCmUmCmUm | 62 |
| Comparative preparation Example 32 | Comparative AP32 | CY5-CmUmGmGmAmGmUmUmCmGmUmUmGmCmUmCmUmUmCmCmGmAmUmCmUmCm | 63 |
| Comparative preparation Example 33 | Comparative AP33 | CY5-CmUmGmGmAmCmAmGmAmCmGmUmGmUmCmUmUmCmCmGmAmUmCmUmCm | 64 |
| Comparative preparation Example 34 | Comparative AP34 | CY5-CmUmGmGmAmGmUmUmCmAmGmAmUmCmAmGmAmCmGmUmGmUmGmUmGmUmUmGmCmUmCmUm | 65 |
| Comparative preparation Example 35 | Comparative AP35 | CY5-CmUmGmGmAmGmUmUmCmAmGmAmCmGmUmGmUmAmGmCmUmCmGmUmGmUmGmAmUmCmUmCm | 66 |

In Table 2, C, G, U, and A represent the base composition of a nucleotide; m represents that the nucleotide adjacent to the left side of the letter m is a methoxy modified nucleotide; f represents that the nucleotide adjacent to the left side of the letter f is a fluoro modified nucleotide; CY5 represent the attachment site of fluorescent dye group Cy5 (Cyanine 5) group on the aptamer.

After completion of the synthesis of the aforementioned comparative conjugates comparative AP27- comparative AP35, the resultant conjugates were diluted to a concentration of 0.2 mg/mL by using ultra-pure water (homemade by Milli-Q ultra-pure water instrument, with resistivity of 18.2 MΩ* cm (25°C)). The molecular weight was determined by a LC-MS instrument (LC-MS, liquid chromatography-mass spectrometry, purchased from Waters Crop., model: LCT Premier). As a result, the measured value of the molecular weight was in conformity with the calculated value, indicating that the target conjugates hd been obtained.

The MS calculated values and the MS measured values of comparative conjugates comparative AP27-comparative AP35 are shown in Table 2-b below:

Table 2-b: The results of mass spectrometry characterization of comparative conjugates

| Number | Calculated MS value | Measured MS value |
|---|---|---|
| comparative AP27 | 8493.81 | 8491.53 |
| comparative AP28 | 8493.81 | 8491.58 |
| comparative AP29 | 11113.50 | 11111.00 |
| comparative AP30 | 11113.50 | 11111.02 |
| comparative AP31 | 7534.23 | 7532.11 |
| comparative AP32 | 9069.14 | 9067.04 |
| comparative AP33 | 8795.08 | 8793.02 |
| comparative AP34 | 11217.59 | 11215.28 |
| comparative AP35 | 11216.60 | 11214.42 |

**Preparation Example 36 Synthesis of conjugate 36**

The conjugate numbered conjugate36 in Table 1B was synthesized according to the same method as that of preparation Examples 1-8 and 12, with the only difference that nucleoside monomers were sequentially linked according to the corresponding nucleic acid sequence of conjugate 36 in Table 1B; and, when preparing the sense strand, the 3' terminal of the sense strand of the siRNA was linked to the delivery group contained in conjugate AP2 by 6 dT phosphate ester groups as linking groups; the 5' terminal of the sense strand of the siRNA was linked to the fluorescent group Cy5 by a phosphate ester linking group.

After completion of synthesis, for the resultant sense strand and antisense strand of conjugate 36, its purity was determined by using Ion Exchange Chromatography (IEX-HPLC) and tits molecular weight was analyzed by Liquid Chromatography-Mass Spectrometry (LC-MS). The calculated value of the sense strand of conjugate 36 was 19660.11, and the measured value was 19658.23. The calculated value was in conformity with the measured value. The calculated value of the antisense strand of conjugate 36 was 6881.59, and the measured value was 6880.83. The calculated value was in conformity with the measured value.

**Preparation Example 37-38 Synthesis of conjugate 37 and conjugate 38**

The conjugates numbered conjugate 37 and conjugate 38 in Table 1B were synthesized according to the same method as that of preparation Examples 1-8 and 12, with the only difference of sequentially linking all nucleoside monomers in the 3' to 5' direction according to the nucleotide sequences of the sense strands or the antisense strands of conjugate 37 and conjugate 38 in Table 1B, to obtain the sense strands and antisense strands of the conjugates, wherein: For conjugate 37, the sense strand comprises the sense strand of the siRNA and the 3' terminal of the sense strand of the siRNA was linked to the 5' terminal of the delivery group contained in conjugate AP2 by Spacer18-GfAfUf as a linking group, and the 5' position of the ribose of the 5' terminal nucleotide of the antisense strand was linked to the fluorescent group Cy5 by a phosphate ester linking group.

For conjugate 38, the sense strand comprises the sense strand of siRNA and the 5' terminal of the sense strand of the siRNA was linked to the 3' terminal of the delivery group contained in conjugate AP2 by four sequentially linked propylene phosphate ester groups, and the 5' position of the ribose of the 5' terminal nucleotide of the antisense strand was linked to the fluorescent group Cy5 by a phosphate ester linking group.

After completion of synthesis, for the resultant sense strands and antisense strands of conjugate 37 and conjugate 38, their purities were determined using Ion Exchange Chromatography (IEX-HPLC) and their molecular weights were analyzed by Liquid Chromatography-Mass Spectrometry (LC-MS). The calculated value of the sense strand of conjugate 37 was 18327.97 and the measured value was 18325.87. The calculated value was in conformity with the measured value. The calculated value of the sense strand of conjugate 38 was 19011.37 and the measured value was 19003.59. The calculated value was in conformity with the measured value. The antisense strands of conjugate 37 and conjugate 38 were identical, and the calculated value was 7415.22, the measured value of was 7413.32. The calculated value was in conformity with the measured value. The results indicated that the resultant conjugate 37 and conjugate 38 had the sequences and structures as shown in Table 1B above.

**Preparation Example 39-44 Synthesis of conjugates 39-42 and comparative conjugates 43-44**

Conjugate 39 was prepared according to the same method as that of preparation Example 20. The molecular weight was determined by LC-MS, the calculated value of conjugate 39 was 9473.03 and the measured value was 9472.06. The measured value was in conformity with the calculated value, indicating that the structures and sequences of conjugate 39 are in conformity with those in Table 1C. The molecular weights of conjugate 40, conjugate 41, conjugate 42, comparative conjugate 43, and comparative conjugate 44 were determined by LC-MS, and the measured values were in conformity with the calculated values. The conjugates numbered conjugate 39, conjugate 40, conjugate 41, conjugate 42, comparative conjugate 43, and comparative conjugate 44 in Table 1C were synthesized according to the method of preparation Example 20, and confirmed by determining molecular weights, with the only difference of sequentially linking nucleoside monomers according to the corresponding sequences of conjugate 39, conjugate 40, conjugate 41, conjugate 42, comparative conjugate 43, and comparative conjugate 44 in Table 1C.

The MS calculated values and the MS measured values of the conjugates are shown in Table 3 below:

Table 3: The results of mass spectrometry characterization of the conjugates

| Number | Calculated MS value | Measured MS value |
|---|---|---|
| Conjugate 20 | 12092.73 | 12091.39 |
| Conjugate 39 | 9473.03 | 9472.07 |
| Conjugate 40 | 9473.03 | 9471.76 |
| Conjugate 41 | 9472.05 | 9472.2 |
| Conjugate 42 | 8434.37 | 8434.09 |
| Comparative conjugate 43 | 9473.03 | 9471.41 |
| Comparative conjugate 44 | 12092.73 | 12091.48 |

**Experimental Example 1 Evaluation and comparison of the ability of the conjugates provided by the present disclosure and control conjugates to enter into U118-MG glioma cells *in vitro***

In this experimental Example, the ability of conjugate AP1 and control conjugate comparative AP10 to enter into U118-MG glioma cells *in vitro* of was evaluated and compared.

U118MG neuroglioma cells (purchased from the Shanghai Cell Bank of Chinese Academy of Sciences) were cultured in DMEM medium (Thermo Fisher) supplemented with 10% fetal bovine serum (FBS, Thermo Fisher) at 37°C in an incubator containing 5% $CO_2$/95% air.

U118MG cells were seeded into a 96-well plate at $5 \times 10^5$ cells/well. Conjugate AP1 prepared above and yeast tRNA were respectively formulated into conjugates solution 1A, 1B, 1C, 1D, 1E, 1F, 1G, 1H, and 1I with DMEM medium, and these conjugate solutions 1A-1I comprised yeast tRNA at a final concentration of 100 μg/mL and respectively comprised conjugate AP1 at final concentrations of 5 nM, 10 nM, 20 nM, 40 nM, 60 nM, 80 nM, 100 nM, 150 nM, 200 nM, and 500 nM.

Control conjugate comparative AP10 prepared above and yeast tRNA were respectively formulated into conjugates solution 1A', 1B', 1C', 1D', 1E', 1F', 1G', 1H', and 1I' with DMEM medium, and these conjugate solutions 1A'-1I' comprised yeast tRNA at a final concentration of 100 μg/mL and respectively comprised control conjugate comparative AP10 at final concentrations of 5 nM, 10 nM, 20 nM, 40 nM, 60 nM, 80 nM, 100 nM, 150 nM, 200 nM, and 500 nM.

The aforementioned culture wells containing U118MG cells were respectively added with the conjugate solutions 1A-1I above in an amount of 200 μL/well and designated as test group 1A- test group 1I. The aforementioned culture wells containing U118MG cells were respectively added with the conjugate solution 1A'-1I' above in an amount of 200 μL/well and designated as control group 1A'- control group 1I'. Another culture well containing U118MG cells was added with 200 μL DMEM medium containing yeast tRNA at a final concentration of 100 μg/mL and designated as blank control group 1J. The 96-well plate containing the above test groups and the blank control group were incubated at 37°C for 1h in the dark; after incubation, the supernatant of the reaction mixture of each test group and the blank control group was aspirated, and DPBS buffer (purchased from Thermo Fisher) was added to wash the plate 3 times (100 μL each time). After the washing solution was aspirated, each culture well was added with 100 μL DMEM medium to obtain a cell suspension. The supernatant was removed by centrifugation and then 500 μL DPBS buffer was added to re-suspend the cells. Flow cytometry analysis was performed with flow cytometer (BD Biosciences): 10,000 cells in each test group or the blank control group were analyzed, the fluorescence intensity distribution was determined and the mean fluorescence intensity

(MFI, a.u.) was calculated. Furthermore, the mean fluorescence intensity corresponding to each final concentration of conjugates is listed in Table 4 below for comparison, and the relative fluorescence difference R is calculated according to the following equation: $R = (MFI_{AP1} - MFI_{CAP10}) / MFI_{CAP10}$.

Therein, $MFI_{AP1}$ represents mean fluorescence intensity of conjugate AP1 in U118-MG glioma cells, and $MFI_{CAP10}$ represents mean fluorescence intensity of comparative AP10 in U118-MG glioma cells. The R value reflects the relative ability of conjugate AP1 to enter into U118-MG glioma cells: when R>2, it indicates that the corresponding conjugate has a very stronger ability to enter into cells (*see* Table 2 of Engineering of Targeted Nanoparticles for Cancer Therapy Using Internalizing Aptamers Isolated by Cell-Uptake Selection. ACS Nano. 2012 January 24; 6(1), which are incorporated herein by reference incorporated herein).

Table 4 The mean fluorescence intensity and R value of conjugates

| Concentration of conjugates (nM) | $MFI_{CAP10}$ (a.u.) | $MFI_{AP1}$ (a.u.) | R |
|---|---|---|---|
| 5 nM | 25.4 | 263.4 | 9.37 |
| 10 nM | 67.4 | 503.4 | 6.47 |
| 20 nM | 176.4 | 1089.4 | 5.18 |
| 40 nM | 443.4 | 1440.4 | 2.25 |
| 60 nM | 666.4 | 2355.4 | 2.53 |
| 80 nM | 787.4 | 2921.4 | 2.71 |
| 100 nM | 1167.4 | 3360.4 | 1.88 |
| 150 nM | 1251.4 | 3969.4 | 2.17 |
| 200 nM | 1868.4 | 7895.4 | 3.23 |
| 500 nM | 4029.4 | 15770.4 | 2.91 |

As can be seen from Table 4, at different concentrations, conjugate AP1 provided by the present disclosure showed significantly higher mean fluorescence intensity in U118MG glioma cells as compared with comparative AP10 with a random sequence, indicating that the conjugate provided by the present disclosure has excellent ability to enter into U118MG glioma cells.

### Experimental Example 2 The entry of conjugates into different cells observed by a fluorescence imaging system

In this experiment, the entry of conjugate AP1 provided by the present disclosure and comparative conjugate comparative AP10 into different cells was observed by a PerkinElmer High Content Imaging system (Operatta).

U118MG glioma cells, SVGp12 normal astrocytes, T98G human brain glioma cells, U251 human glioma cells, A549 human non-small cell lung cancer cells, MCF-7 human breast cancer cells, and 293T human kidney epithelial cells were all purchased from the Shanghai Cell Bank of Chinese Academy of Sciences. Before the experiment, for each cell line, the cells in the logarithmic growth phase were selected and seeded into a 96-well plate at 5000 cells/well (each cell line was seeded into 2 culture wells), and cultured at 37°C in an incubator containing 5% $CO_2$/95% air for 24h. The supernatant was aspirated and DPBS buffer (purchased from Thermo Fisher) was added to wash the plate 2 times (100 μL each time).

Conjugate AP1 prepared above and yeast tRNA were respectively formulated into conjugate solution 2A with DMEM medium, and the conjugate solution 2A comprised yeast tRNA at a final concentration of 100 μg/mL and conjugate AP1 at a final concentration of 100 nM.

Control conjugate comparative AP10 prepared above and yeast tRNA were respectively formulated into control conjugate solution 2B with DMEM medium, and control conjugate solution 2B comprised yeast tRNA at a final concentration of 100 μg/mL and comparative AP10 at a final concentration of 100 nM.

For each cell line, another culture well was added with 200 μL conjugate solution 2A and incubated at 37°C for 30 min in the dark, then the supernatant was aspirated and DPBS buffer was added to wash the well 2 times (100 μL each time) to obtain test group 2X.

For each cell line, another culture well was added with 200 μL control conjugate solution 2B and incubated at 37°C for 30 min in the dark, then the supernatant was aspirated and DPBS buffer was added to wash the well 2 times (100 μL each time) to obtain control group 2Y

Test group 2X and control group 2Y of the above cell lines were respectively added with 100 μL DMEM medium at 37°C, and imaged in a high content imaging system. Normalization was performed according to the mean fluorescence

intensity of control group 2Y, that is, subtracting the mean fluorescence intensity of control group 2Y from the fluorescence intensity of test group 2X before performing imaging. The results are shown in Figure 1A- Figure 1G.

Figures 1A and 1B are respectively the high content imaging images showing the entry of AP1 and comparative AP10 into U118MG glioma cells and SVGp12 normal astrocytes. As can be seen from Figures 1A and 1B, only the U118MG cells treated with AP1 showed strong Cy5 fluorescence signal, while the SVGp12 cells did not show any fluorescence signal in both the test group and the control group, indicating that the conjugate provided by the present disclosure has a very strong ability to enter into U118MG glioma cells as compared with comparative AP10, and basically did not enter into SVGp12 normal astrocytes. The results above also indicate that the conjugate provided by the invention can selectively target and deliver the functional groups to U118MG glioma cells.

Furthermore, Figures 1C-1G are respectively the high content imaging images showing the entry of AP1 and comparative AP10 into U251 human glioma cells, A549 human non-small cell lung cancer cells, MCF-7 human breast cancer cells, and 293T human renal epithelial cells. As can be seen from Figures 1C-1G, the U251 human glioma cells, A549 human non-small cell lung cancer cells and MCF-7 human breast cancer cells treated with AP showed a strong Cy5 fluorescence signal, while neither the tumor cells treated with comparative AP10 nor the 293T human renal epithelial cells showed any fluorescence signal in both the test group and the control group, indicating that the conjugate provided by the present disclosure has a very strong ability to enter into various tumor cells as compared with comparative AP10, and basically did not enter into normal cells. The conjugate provided by the invention can selectively target and deliver the functional groups to various tumor cells.

## Experimental Example 3 The entry of conjugates into tumor spheres observed by a fluorescence imaging system

In this experiment, the entry of conjugate AP1 provided by the present disclosure and comparative conjugate comparative AP10 into U118MG glioma tumor spheres and A549 human non-small cell lung cancer tumor spheres was observed by a PerkinElmer High Content Imaging system (Operatta).

80 μL fresh, sterilized and unsolidified 2% agarose was added into the culture wells of a 96-well plate. After complete solidification, for each cell line, the cells in the logarithmic growth phase were selected and seeded into a 96-well plate at 2000 cells/well (each cell line was seeded into 2 culture wells, 200 μL cell suspension/well), cultured in DMEM medium supplemented with 10% FBS and 1% penicillin-streptomycin, and cultured at 37°C in an incubator containing 5% $CO_2$/95% air. Half of the supernatant was aspirated every 48h and fresh DMEM medium was added along the wall of the culture wells. After 7 days of culture, the cells were observed under the microsope to confirm that complete cell spheres were obtained, indicating successful tumor sphere cultivation.

Each culture well was added with AP1 or comparative AP10 according to the method of experimental Example 2, and imaged in a high content imaging system, with the only difference of directly performing imaging after culture without washing and performing imaging according to the measured fluorescence intensity of Cy5. The results are shown in Figures 2A and 2B.

Figures 2A and 2B are respectively the high content imaging images showing the entry of AP1 and comparative AP10 into U118MG glioma tumor spheres and A549 human non-small cell lung cancer tumor spheres. As can be seen from Figures 2A and 2B, the interiors of AP1-treated U118MG glioma tumor spheres and A549 tumor spheres showed strong Cy5 fluorescence signal, while only the edge of the comparative AP10-treated tumor spheres showed significantly weak fluorescence signal, indicating that the conjugate provided by the present disclosure has a strong ability to enter into the interior of U118MG and A549 tumor spheres as compared with comparative AP10. The results above indicate that the conjugate provided by the invention can effectively deliver the functional groups to the interior of tumors and have high drugability.

## Experimental Example 4 *In vivo* distribution of conjugates in U118MG cell subcutaneous tumor model mice

U118MG human glioma cells (purchased from Guangzhou Jennio Biotech Co., Ltd) were cultured in DMEM complete medium (MACGENE, Cat No. CM15019) supplemented with 10% fetal bovine serum (FBS, RMBIO) at 37°C in an incubator containing 5% $CO_2$/95% air.

U118MG human glioma cells in the logarithmic growth phase were selected and digested (0.25% pancreatic enzyme). The cells were collected, centrifuged to remove the supernatant, and then resuspended in DMEM medium supplemented with 10% FBS to be formulated into a cell culture solution with a concentration of $1\times10^8$ cells/mL.

Experimental animals were 10 NOD-SCID mice (male, 12 weeks old, purchased from SPF (Beijing) Biotechnology Co., LTD.). The cell culture solution above was inoculated at the subcutaneous site of the right forelimb of a NOD-SCID mouse with an inoculation volume of 100 μL per mouse, i.e., each mouse was inoculated with $1\times10^7$ cells. The mice were further fed for 20 days after injection.

AP1, AP2, and comparative AP11 were dissolved into solutions with a concentration of 0.3 mg/mL (based on

aptamer) with 1×DMEM medium (purchased from M&C gene technology (BEIJING) LTD., Lot No. K1902200).

The mice inoculated with U118MG above were randomly divided into 3 groups (4 mice per group). For the 4 mice per group, the administration volume was calculated according to 10 μL/g body weight, and AP1, AP2 and comparative AP11 were respectively administered to different groups of mice by tail vein injection and designated as test group 4A1, test group 4A2 and control group 4A3. Thus, the administration dosage of each mouse was 3 mg/kg (based on aptamer). Another mouse inoculated with U118MG above was administered with 1×DMEM medium by tail vein injection (the administration volume was 10 μL/g body weight), and designated as blank control group 4Y

At 1h after administration, the mice of each group were placed in a small animal *in vivo* optical imaging system IVIS Lumina Series III (PerkinElmer). The mice were anesthetized with isoflurane gas, and the anesthetized mice were placed on their back in the small animal *in vivo* optical imaging system for *in vivo* imaging. At 1h, 4h, 24h and 48h after administration, one mouse of each group was respectively euthanized, and tumor tissues were taken for fluorescence imaging (no blank control group in the 24h and 48h results). The results are respectively shown in Figures 3A, 3B, 3C and 3D.

Figures 3A-3D are respectively images showing the *in vivo* imaging and tumor tissue imaging at 1h, 4h, 24h and 48h after administration, wherein blank represents blank control group 4Y As can be seen from Figures 3A-3D, blank control group 4Y and control group 4A3 administered with comparative AP11 of a random sequence showed substantially no or weak fluorescence signal in mice. In contrast, conjugates AP1 and AP2 provided by the present disclosure showed strong fluorescence signal at the sites of tumor inoculation in mice, indicating that the aptamer provided by the present disclosure can specifically target and deliver the fluorescent group to U118MG glioma. Furthermore, conjugate AP2 still showed strong fluorescence signal at 24h to 48h after administration, indicating that the conjugates provided by the present disclosure could stably target glioma tissue over a long period of time.

**Experimental Example 5 *In vivo* distribution of conjugates in U118MG cell tumor in situ model mice**

U118MG human glioma cells in the logarithmic growth phase cultured as descruved in experimental Example 4 were selected and digested with 0.25% pancreatic enzyme. The cells were collected, the supernatant was aspirated, and then the cells were resuspended in DMEM medium supplemented with 10% FBS to be formulated into a cell culture solution with a cell density of $4\times10^7$ cells/mL.

Experimental animals were 16 NOD-SCID mice (male, 12 weeks old, purchased from SPF (Beijing) Biotechnology Co., LTD.). The cell culture solution above was inoculated into a NOD-SCID mouse: the cell culture solution was injected into the right striatum of a mouse by the manner of lateral ventricle injection in the mouse, and the position was AP (anteroposterior): 1 mm, ML (medial lateral): 1.5 mm, DV (dorsal ventral): 3.5 mm, the injection volume was 10 μL, i.e., each mouse was inoculated with $4\times10^5$ cells. The mice were further fed for 14 days after injection.

AP1, AP2 and comparative AP11 were respectively dissolved into conjugate solutions with a concentration of 2 mg/mL (based on aptamer) with 1×DMEM medium (purchased from M&C gene technology (BEIJING) LTD., Lot No. K1902200). 10 mice above were taken and respectively injected with 50 μL AP1 and AP2 solution by the administration manner of intrathecal injection, with an administration dosage of 100 μg per animal (based on the amount of aptamer). 3 mice per group were administered and respectively designated as test groups 5A and 5B; each of 2 mice was respectively injected with 50 μL comparative AP11 solution with an administration dosage of 100 μg per animal (based on the amount of aptamer) and designated as control group 5C; each of 2 mice was respectively injected with 50 μL DMEM medium and designated as blank control group 5Y.

AP1, AP2 and comparative AP11 were respectively dissolved into conjugate solutions with a concentration of 0.3 mg/mL (based on aptamer) with 1×DMEM medium. The other 6 mice were taken and respectively injected with AP1, AP2 and comparative AP11 solutions by tail vein injection. The administration dosage for all animals was calculated according to body weight, the administration volume was all 10 μL/g , and the administration dosage was 3 mg/kg per animal (based on the amount of aptamer). 2 mice of each group were administered and respectively designated as test groups 5D, 5E and control group 5F.

At 24h after administration, one mouse in each group was respectively euthanized and brain tissue was taken, and the remaining mice were euthanized at 48h after administration and brain tissues were taken. The brain tissues of mice were taken for fluorescence imaging in IVIS Lumina Series III. The results are respectively shown in Figures 4A and 4B.

Figures 4A and 4B are respectively images showing the fluorescence imaging of the brain tissues of the U118MG tumor in situ model mice established after administration of blank control group 5Y, test groups 5A, 5B, 5D and 5E, and control groups 5C and 5F at 24h and 48h after administration. As can be seen from the results in Figures 4A and 4B, the blank control group and the control group of comparative AP11 shows no obvious fluorescence signal at the tumor site, indicating that they do not have significant targeting effect on in situ brain glioma. In contrast, test groups 5A and 5B administered with the conjugates provided by the present disclosure show strong fluorescence signals at the site of tumor inoculation, indicating that the conjugates could effectively target tumor tissues and that the aptamer provided by the present disclosure can effectively deliver different diagnostic agent groups (e.g., fluorescent groups) to tumor tissues.

Furthermore, test group 5E shows obvious fluorescence signals at both 24 and 48h, indicating that conjugate AP2 can still reach and target brain glioma when administered via tail vein, and the conjugates provided by the present disclosure can also penetrate the Blood-Brain Barrier (BBB) and enter into brain glioma.

**Experimental Example 6 *In vivo* distribution of conjugates with different modifications in U118MG subcutaneous tumor model mice**

U118MG subcutaneous tumor model mice were prepared according to the method described in experimental Example 4.

AP2, AP3 and comparative AP12 were respectively dissolved into conjugate soutions with a concentration of 0.3 mg/mL (based on aptamer) with 1 ×DMEM medium. 9 mice were divided into 3 groups (3 mice per group). AP2, AP3 and comparative AP12 solutions were respectively administered to each mouse of each group by tail vein injection. The administration dosage for all animals was calculated according to body weight, the administration volume was all 10 μL/g , and the administration dosage was 3 mg/kg per animal (based on the amount of aptamer). The three groups of mice were respectively designated as test group 6A, test group 6B and test group 6C. The other 2 mice were administered with DMEM medium with an administration volume of 10 μL/g and designated as blank control group 6Y One mouse of each group was placed in a small animal *in vivo* optical imaging system IVIS Lumina Series III (PerkinElmer) at 30 min, 1h, 24h and 48h after administration, respectively. The mice were anesthetized by isoflurane gas, and the anesthetized mice were placed on their back in the small animal *in vivo* optical imaging system for *in vivo* imaging. The results are respectively shown in Figures 5A-5D. On Day 10 after administration, one mouse of blank control group 6Y and one mouse of test group 6A were taken for *in vivo* imaging, and the results are shown in Figure 5E; at 24h and 48h after administration, one mouse of each group was respectively euthanized and tumor tissues were taken for fluorescence imaging, and the results are respectively shown in Figures 5F and 5G. On Day 10 after administration, one mouse of blank control group 6Y and one mouse of test group 6A was respectively euthanized and tumor tissues were taken for fluorescence imaging, and the results are shown in Figure 5H. In Figures 5A-5H, Blank represents blank control group 6Y

Figures 5A-5E are respectively images showing the *in vivo* fluorescence imaging of mice at different time points after administration of the conjugates of different sequences provided by the present disclosure and comparative conjugates; Figures 5F-5H are respectively images showing the tumor tissue imaging at different time points. The results of Figures 5A-5H indicate that fluorescence signals are visible in subcutaneous tumors of all test groups and control group at 30 min after administration. At 4h to 24h after administration, mice in test groups 6A, 6B and 6C administered with AP2, AP3 and AP12 provided by the present disclosure still show high fluorescence intensity; at 48h to Day 10 after administration, mice in test group 6A administered with AP2 still show strong fluorescent signal. These results indicate that the conjugates provided by the present disclosure can stably target tumor tissues over a longer period of time as compared with control conjugates, and the aptamer provided by the present disclosure can stably target and deliver diagnostic agent groups to tumors over a long period of time.

**Experimental Example 7 *In vivo* distribution of conjugates in A549 subcutaneous tumor model mice**

A549 human non-small cell lung cancer cell subcutaneous tumor model mice were prepared according to the method described in experimental Example 4, with the only difference that the cell culture solution was prepared using A549 human non-small cell lung cancer cells instead of U118MG glioma cells.

11 NOD-SCID mice ( male, 6-8 weeks old, purchased from SPF (Beijing) Biotechnology Co., LTD.) were selected and divided randomly: 2 mice in the blank control group and 3 mice in each of the other groups. The culture medium containing A549 tumor cells above was inoculated at the subcutaneous site of the right forelimb of a mouse, and each mouse was inoculated 100 μL A549 tumor cells; thus, through calculation, the number of inoculated cells was $1\times10^7$ cells per mouse.

AP2, AP3 and comparative AP12 were respectively dissolved into conjugate solutions with a concentration of 0.3 mg/mL (based on aptamer) with 1 ×DMEM medium (purchased from M&C gene technology (BEIJING) LTD., Lot No. K1902200). On Day 21 after inoculation of A549 tumor cells, each mouse was respectively adminstered with 1 ×DMEM and AP2, AP3, and AP12 conjugate solutions formulated above by tail vein injection, and the mice injected with 1×DMEM, AP2, AP3, and AP12 conjugate solutions were respectively designated as blank control group, AP2 group, AP3 group, and AP12 group. The administration dosage for all animals was calculated according to body weight, and the administration volume was 10 μL/g per mouse. That is, except the blank control group, the administration dosage of each mouse in the other groups was 3 mg/kg (based on the amount of aptamer); the blank control group was administered with only 10 μL/g DMEM medium.

At 1h and 24h after administration, one mouse of each group was respectively euthanized, and tumor tissues were taken for fluorescence imaging in IVIS Lumina Series III. Two organs of each mouse were arranged horizontally in sequence and placed under the same field of view for taking pictures. The results are shown in Figure 6.

Figure 6 shows the fluorescence imaging of the organs of one mouse in each group at 1h after administration. As can be seen from Figure 6, except the blank control group, strong fluorescence signal intensity was detected in A549 tumors of AP2, AP3 and AP12 groups, and the fluorescence signal intensity of AP3 group was stronger than those of other groups.

As can be seen from the above results, the conjugates with different modifications of the present disclosure can specifically target A549 tumors.

### Experimental Example 8 Ability of the conjugates provided by the present disclosure to enter into PAN02 tumor cells

PAN02 tumor cell model was used in this experimental Example to verify the ability of the conjugates provided by the present disclosure to enter into PAN02 tumor cells.

PAN02 tumor cells (purchased from the Shanghai Cell Bank of Chinese Academy of Sciences) in the logarithmic growth phase were selected, digested and then resuspended in DMEM medium supplemented with 10% FBS until the cell density of PAN02 tumor cells reached $1\times10^8$ cells/mL.

A 6 weeks old C57BL mouse (male) was selected. The medium containing PAN02 tumor cells above was inoculated at the subcutaneous site of the right forelimb of the mouse. The volume of PAN02 tumor cells inoculated in the mouse was 100 $\mu$L; thus, through calculation, the number of the cells inoculated in the mouse was $1\times10^7$ cells. AP2 was dissolved into a conjugate solution with a concentration of 0.3 mg/mL (based on aptamer) with 1 $\times$DMEM medium (purchased from M&C gene technology (BEIJING) LTD., Lot No. K1902200). On Day 21 after inoculation of PAN02 tumor cells, the mouse was administered with AP2 conjugate solution by tail vein injection. The administration dosage was calculated according to mouse body weight, the administration volume was 10 $\mu$L/g , and the administration dosage was 3 mg/kg (based on the amount of aptamer).

At 135 min after administration, the mouse was euthanized. The heart, liver, spleen, lung and tumor tissues of the euthanized mouse were taken for fluorescence imaging in IVIS Lumina Series III, and placed under the same field of view for taking pictures. The results are shown in Figure 7.

Figure 7 is an image showing the fluorescence imaging of various organ tissues in PAN02 subcutaneous tumor model mice established after administration of the conjugates provided by the present disclosure. As can be seen from Figure 7, the fluorescence signal intensity in PAN02 tumor cells is significantly stronger than that in other organs, indicating that the conjugates provided by the present disclosure can specifically target PAN02 pancreatic cancer tumor cells.

### Experimental Example 9 Ability of conjugates conjugated with different fluorescent groups to enter into U118MG glioma cells

U118MG glioma cells model was used in this experimental Example to verify the ability of the conjugates respectively comprising Cy5-fluorescent group and Cy3-fluorescent group provided by the present disclosure to enter into U118MG glioma.

U118MG glioma cells (purchased from Guangzhou Jennio Biotech Co., Ltd) in the logarithmic growth phase were selected, digested and then resuspended in DMEM medium supplemented with 10% FBS until the cell density of U118MG glioma cells reached $1\times10^8$ cells/mL.

11 NOD-SCID mice (male, 6-8 weeks old, purchased from SPF (Beijing) Biotechnology Co., LTD.) was selected and divided randomly: 3 mice in the first group, 2 mice in the second group, 3 mice in the third group, and 3 mice in the fourth group. The culture medium containing U118MG glioma cells above was inoculated at the subcutaneous site of the right forelimb of a mouse, the volume of inoculated cells in each mouse was 100 $\mu$L; thus, through calculation, the number of inoculated cells was $1\times10^7$ cells per mouse.

AP2, AP9 and comparative AP12 were respectively dissolved into conjugate solutions with a concentration of 0.3 mg/mL conjugate solutions (based on aptamer) with 1 $\times$DMEM medium (purchased from M&C gene technology (BEIJING) LTD., Lot No. K1902200). On Day 21 after inoculation of the U118MG glioma above, each mouse was respectively administered with 1$\times$DMEM and AP2, AP9 and AP12 conjugate solutions formulated above by tail vein injection. Therein, 3 mice in the first group were injected with AP2 conjugate solution, 2 mice in the second group were injected with AP9 conjugate solution, 3 mice in the third group were injected with AP12 conjugate solution, and 3 mice in the fourth group were injected with 1$\times$DMEM as the blank control group. The administration dosage for all animals was calculated according to body weight, and the administration volume was 10 $\mu$L/g per mouse. That is, the administration dosage of each mouse in the first to third groups was 3 mg/kg (based on the amount of aptamer).

The day of administration is designated as D1. At 48h, 96h, and 11 days (D12) after administration, one mouse of each group was respectively euthanized. The tumor, lung, liver, and kidney tissues of each euthanized mouse were taken for fluorescence imaging in IVIS Lumina Series III. Four organs of each mouse were arranged vertically in sequence and placed under the same field of view for taking pictures. The results are shown in Figures 8A-8E. Therein, group 1

represents the test group administered with AP2, group 2 represents the test group administered with AP9, group 3 represents the test group administered with AP12, and group 4 represents the blank control group.

Figure 8A shows the fluorescence imaging of the organs of 1 mouse in the first group, 1 mouse in the third group, and 1 mouse in the fourth group at 48h after administration. As can be seen from Figure 8A, no fluorescence signal was detected in the third and fourth groups. At the same time, significant fluorescence signals were detected in tumor tissues of the mice injected with AP2, and no fluorescence signal was detected in tissues other than the metabolic organ kidney, indicating that AP2 can effectively target tumor tissues and has no significant targeted delivery in other tissues as compared with the comparative conjugates.

Figure 8B shows the fluorescence imaging of the organs of 1 mouse in the first group, 1 mouse in the third group, and 1 mouse in the fourth group at 96h after administration. As can be seen from Figure 8B, AP2 still shows significant fluorescence signal in tumor tissues after 96h.

Figure 8C shows the fluorescence imaging of the organs of 1 mouse in the second group and 1 mouse in the fourth group at 48 hours after administration. As can be seen from Figure 8C, significant fluorescence signals can be detected in tumor tissues of the mice injected with Cy3-labeled AP9 as compared with the control group, indicating that the aptamer provided by the present disclosure can efficiently target and deliver different diagnostic agent groups (e.g., fluorescent groups) to tumor tissues, and the conjugates comprising diagnostic agent groups provided by the present disclosure can stably target tumor tissues.

Figure 8D shows the fluorescence imaging of the organs of 1 mouse in the second group and 1 mouse in the fourth group at 96h after administration. As can be seen from Figure 8D, significant fluorescence signals can be detected in tumor tissues of the mice injected with Cy3-labeled AP9, and no fluorescence signal was detected in tissues other than the metabolic organ kidney. It can be seen that the aptamer provided by the present disclosure can efficiently target and deliver different diagnostic agent groups (e.g., different fluorescent groups) to U118MG glioma and can maintain stability over a longer period of time.

Figure 8E shows the fluorescence imaging of the organs of 1 mouse in the first group, 1 mouse in the third group, and 1 mouse in the fourth group on Day 11 after administration. As can be seen from Figure 8E, significant fluorescence signal of AP2 can still be detected in tumor tissues, and no fluorescence signal was detected in tissues other than the metabolic organ kidney on Day 11 after administration. It can be seen that the conjugates provided by the present disclosure can still stably target U118MG glioma tissue with high specificity over a longer period of time.

From above results, it can be seen that the aptamer provided by the present disclosure can not only specifically target glioma tissue, but also deliver different diagnostic agent groups to glioma. Thus, the aptamer provided by the present disclosure shows excellent delivery capability; meanwhile, the conjugates comprising diagnostic agent groups provided by the present disclosure can efficiently and stably target glioma, so that the presence of glioma can be rapidly and effectively diagnosed, and the diagnostic effect can be kept stable over a long period of time.

**Experimental Example 10 Targeting effect of conjugates with different sequences and lengths on U118MG glioma**

U118MG glioma cells model was used in this experimental Example to investigate the targeting effect of conjugates with different sequences and lengths on U118MG glioma.

U118MG glioma cells (purchased from Guangzhou Jennio Biotech Co., Ltd) in the logarithmic growth phase were selected, digested and then resuspended in DMEM medium supplemented with 10% FBS until the cell density of U118MG glioma cells reached $1\times10^8$ cells/mL.

14 NOD-SCID mice (male, 6-8 weeks old, purchased from SPF (Beijing) Biotechnology Co., LTD.) was selected and divided randomly: 2 mice in each group. The culture medium containing U118MG glioma cells above was inoculated at the subcutaneous site of the right forelimb of a mouse, the volume of inoculated U118MG glioma in each mouse was 100 $\mu$L; thus, through calculation, the number of inoculated cells was $1\times10^7$ cells per mouse.

AP2, AP4, AP5, AP6, AP7, AP8, and AP12 were respectively dissolved conjugate solutions with a concentration of 0.3 mg/mL (based on aptamer) with 1×DMEM medium (purchased from M&C gene technology (BEIJING) LTD., Lot No. K1902200). On Day 21 after inoculation of the U118MG glioma above, each mouse was respectively administered with AP2, AP4, AP5, AP6, AP7, AP8, and AP12 conjugate solutions formulated above by tail vein injection. Therein, the groups of mice injected with AP2, AP4, AP5, AP6, AP7, AP8 and AP12 were respectively designated AP2 group, AP4 group, AP5 group, AP6 group, AP7 group, AP8 group and AP12 group. The administration dosage for all animals was calculated according to body weight, and the administration volume was 10 $\mu$L/g per mouse. That is, the administration dosage was 3 mg/kg per mouse (based on the amount of aptamer).

The day of administration is designated as D1. At 1h, 24h (D2), 48h (D3) and 72h (D4) after administration, the mice of each group were placed in a small animal *in vivo* optical imaging system IVIS Lumina Series III. The mice were anesthetized by isoflurane gas, and the anesthetized mice were placed on their back in the small animal *in vivo* optical imaging system for *in vivo* imaging. The fluorescence signal of Cy5 was dynamically detected and the distribution of

different conjugates labeled by Cy5 marker was tracked *in vivo*. Analysis of the observation results showed that fluorescence signals were visible in subcutaneous tumors of all mice at 1h after administration, and there was no significant difference in fluorescence signal intensity among all groups of mice. At 24 and 48h after administration, fluorescence signals could still be detected in each group of mice, but the fluorescence signals in subcutaneous tumors in mice injected with AP6 and AP8 were stronger than those in mice injected with AP2, AP4, AP5, AP7, and AP12.

On D9, one mouse of each group was respectively euthanized. The tumor, lung, liver, and kidney tissues of each euthanized mouse were taken for fluorescence imaging in IVIS Lumina Series III. Tumor tissues of each mouse were arranged vertically and placed under the same field of view for taking pictures. The results are shown in Figure 9.

Figure 9 is an image showing the fluorescence imaging of tumors in each group of mice on D9 after administration. As can be seen from Figure 9, Cy5 fluorescence signal can be detected in tumor tissues of mice in all groups, indicating that the conjugates with different sequences and lengths of the present disclosure can stably and effectively target tumor tissues over a long period of time.

**Experimental Example 11 *In vivo* activity of the conjugates provided by the present disclosure in mice**

In this experimental Example, the *in vivo* anti-tumor activity of conjugate 20 in mice was investigated

The mice in this experiment were purchased from SPF Co., LTD: the germline was NOD-SCID, the grade was SPF, male, 6-8 weeks old; U118MG neuroglioma cells were purchased from Jennio.

U118MG cells in the logarithmic growth phase were selected, digested and then resuspended in DMEM complete medium (MACGENE, No.CM15019) supplemented with 10% fetal bovine serum (FBS, GIBCO., Ltd) for cultivation until the cell density reached $1\times10^8$ cells/mL, to obtain the culture solution containing U118MG cells. The culture solution containing U118MG cells above was inoculated at the subcutaneous site of the right forelimb of a mouse, and the injection volume was 100 μL; thus, each mouse was inoculated with $1\times10^7$ U118MG glioma cells.

The conjugate AP2 above was formulated into a 1.94 mg/mL solution with PBS. Conjugate 20 was respectively formulated into 0.625 mg/mL, 1.25 mg/mL and 2.06 mg/mL solutions with PBS (all calculated based on the amount of aptamer). MMAE (purchased from Shanghai Macklin Inc., Lot No. C12886583) was dissolved into a 0.038 mg/mL solution with a mixed solution of 10%DMSO+90%PBS (volume ratio).

The day of inoculation is designated as D1, and administration is performed once on D8, D12, D16 and D20.

36 mice were randomly divided into the following 6 groups (6 mice per group):

For blank control group 1, PBS was administered by tail vein injection with a single administration volume of 10 μL/g.

For control group 2, conjugate AP2 solution above was administered by tail vein injection with a single administration volume of 10 μL/g and a single administration dosage of 15.5 mg/kg.

For control group 3, MMAE solution above was administered by tail vein injection with a single administration volume of 10 μL/g and a single administration dosage of 0.3 mg/kg.

For test group 4, conjugate 20 solution above with a concentration of 0.625 mg/mL was administered by tail vein injection with a single administration volume of 10 μL/g and a single administration dosage of 5 mg/kg (based on the amount of aptamer), wherein the dosage of MMAE contained was equivalent to 0.3 mg/kg.

For test group 5, conjugate 20 solution above with a concentration of 2.06 mg/mL was administered by tail vein injection with a single administration volume of 10 μL/g and a single administration dosage of 16.5 mg/kg (based on the amount of aptamer), wherein the dosage of MMAE contained was equivalent to 1 mg/kg.

For test group 6, conjugate 20 solution above with a concentration of 1.25 mg/mL was administered by subcutaneous injection with a single administration volume of 5 μL/g and a single administration dosage of 5 mg/kg (based on the amount of aptamer), wherein the dosage of MMAE contained was equivalent to 0.3 mg/kg.

[3] Detection

The long diameters and short diameters of the tumors were measured *in vitro*. The tumor volume was calculated according to the equation 1/2 (long diameter × short diameter$^2$). Prior to the first administration on D8, the tumor volumes in each group were measured and the average tumor volumes were recorded; the tumor volumes in each group were measured and recorded from D16 (twice a week).

Figure 10 is a line graph showing the changes of tumor volume over time in each group of mice. As shown in Figure 10, the tumor volumes increased rapidly in blank control group 1 administered with PBS only and control group 2 administered with AP2. The growth rate of the tumor volume was reduced in control group 3 administered with MMAE only, indicating that MMAE *per se* has an inhibition effect on tumor proliferation. Furthermore, in test groups 4 and 6 with MMAE content comparable to control group 3, the tumor volumes were significantly smaller than control group 3 during the test, showing more excellent anti-tumor activity than control group 3 administered with MMAE only. This result indicates that the conjugates provided by the present disclosure can effectively deliver MMAE to tumor tissues, showing tumor-targeting ability and reducing the risk of toxicity caused by the distribution of MMAE molecules in other tissues, and various

administration manners can all effectively inhibit tumor proliferation. In addition, the tumor volume of test group 5 in which the administration dosage was further improved substantially did not increase during the test, showing more excellent anti-tumor effect, indicating that the conjugates comprising small molecule drug groups provided by the present disclosure can rapidly and steadily produce tumor inhibition effect.

The results above indicate that the conjugates provided by the present disclosure can effectively target and deliver small molecule drug groups with tumor inhibition effects to tumor tissues, and the conjugates comprising small molecule drug groups provided by the present disclosure show good anti-tumor activity and dose-dependent effect.

### Experimental Example 12 Effects of the conjugates provided by the present disclosure on normal cell and tumor cell viability

In this experimental Example, the effects of conjugate 19 on normal cell and tumor cells activity were investigated.

U118MG neuroglioma cells were purchased from Jennio; SVGp12 human astrocytes were purchased from the Shanghai Cell Bank of Chinese Academy of Sciences; CCK8 kit was purchased from DOJINDO Laboratories.

U118MG cells and SVGp12 cells in the logarithmic growth phase were selected and seeded into a 96-well plate of a CCK8 kit at 5000 cells/well, cultured at 37°C with 5%$CO_2$ for 24h. U118-MG cells and SVGp12 cells to be detected were obtained when the cell confluence was observed as 80% under microscope.

AP1, conjugate 19 and yeast tRNA were respectively gradient diluted with DMEM complete medium (MACGENE, No.CM15019) supplemented with 10% fetal bovine serum (FBS, GIBCO) to obtain the following conjugate solutions: AP1 solution 1 (concentration was 0 nM), AP1 solution 2 (concentration was 50 nM), AP1 solution 3 (concentration was 200 nM), AP1 solution 4 (concentration was 800 nM); and the following conjugate solutions: conjugate 19 solution 1 (concentration was 0 nM), conjugate 19 solution 2 (concentration was 25 nM), conjugate 19 solution 3 (concentration was 50 nM), conjugate 19 solution 4 (concentration was 100 nM), conjugate 19 solution 5 (concentration was 200 nM), conjugate 19 solution 6 (concentration was 400 nM), conjugate 19 solution 7 (concentration was 800 nM); and each of the conjugate solutions above also contained yeast tRNA at a final concentration of 100 μg/ml, respectively.

AP1 solution 1, AP1 solution 2, AP1 solution 3, and AP1 solution 4 were respectively added into U118-MG cells to be detected, and incubated at 37°C for 72h in the dark. The experiment was repeated 5 times in each group and designated as blank control group 12A1, test group 12A2, test group 12A3, and test group 12A4, respectively.

AP1 solution 1, AP1 solution 2, AP1 solution 3, and AP1 solution 4 were respectively added into SVGp12 cells to be detected, and incubated at 37°C for 72h in the dark. The experiment was repeated 5 times in each group and designated as blank control group 12B 1, test group 12B2, test group 12B3, and test group 12B4, respectively.

Conjugate 19 solution 1, Conjugate 19 solution 2, Conjugate 19 solution 3, Conjugate 19 solution 4, Conjugate 19 solution 5, Conjugate 19 solution 6, and Conjugate 19 solution 7 were respectively added into U118-MG cells to be detected, and incubated at 37°C for 72h in the dark. The experiment was repeated 5 times in each group and designated as blank control group 12C1, test groups 12C2-12C7, respectively.

Conjugate 19 solution 1, Conjugate 19 solution 2, Conjugate 19 solution 3, Conjugate 19 solution 4, Conjugate 19 solution 5, Conjugate 19 solution 6, and Conjugate 19 solution 7 were respectively added into SVGp12 cells to be detected, and incubated at 37°C for 72h in the dark. The experiment was repeated 5 times in each group and designated as blank control group 12D1, test groups 12D2-12D7, respectively.

CC8 kit was used to detect UV absorbance of test groups and control groups above at the detection wavelength of 450nm in UV absorption spectrum according to the instructions, and the average value of the repeated experiments was taken for each group. Cell viability was calculated according to the following equation:

$$\text{Cell viability \%} = (\text{test group OD450} - \text{blank group OD450}) / (\text{control group OD450} - \text{blank group OD450}) \times 100\%$$

Therein, test group OD450 refers to the absorbance value of each test group at 450nm; control group OD450 refers to the absorbance value of the corresponding blank control group of each test group at 450nm; blank group OA450 refers to the absorbance value of blank colorimetric pool used in UV detection at 450nm. The results are shown in figures 11A and 11B.

Figure 11A shows the effects of conjugate AP1 at different concentrations on cell viability of U118-MG neuroglioma cells and SVGp12 human astrocytes. Figure 11B shows the effects of conjugate 19 at different concentrations on cell viability of U118-MG neuroglioma cells and SVGp12 human astrocytes.

As shown in Figure 11A, only conjugate AP1 comprising diagnostic agent group CY5 at different concentrations has no effect on cell viability of normal cells and tumor cells. As shown in Figure 11B, conjugate 19 comprising siRNA can significantly reduce cell viability of tumor cells, and the inhibitory efficacy was improved as the administration dose increases. Meanwhile, for normal cells, cell viability was only reduced slightly and remained stable basically, indicating that

after conjugation of siRNA, the aptamer provided by the present disclosure can efficiently target tumor cells and inhibit the content of target mRNA, but cannot significantly affect the corresponding mRNA level in normal cells, with excellent targeted delivery efficiency and high safety. In summary, these results indicate that the conjugates comprising functional oligonucleotides provided by the present disclosure can effectively target and deliver functional oligonucleotides with tumor inhibition effects (such as siRNA) to tumor tissues, showing good anti-tumor activity and dose-dependent effect.

**Experimental Example 13 *In vivo* targeting activity of the conjugates provided by the present disclosure in mice**

In this experimental Example, the *in vivo* targeting activity of conjugate 15, conjugate 16, conjugate 17, and conjugate 18 in mice were investigated.

Conjugates 15-18 comprise the same aptamer sequence. For conjugate 15, the sense strand comprises the sense strand of the siRNA, of which the 5' terminal was linked to the fluorescent group Cy5 and the 3' terminal was linked to the aptamer provided by the present disclosure by GAU trinucleotide subunit as a linking group.

For conjugate 16, the sense strand comprises the sense strand of the siRNA of which the 5' terminal was linked to the aptamer provided by the present disclosure by GAU trinucleotide subunit as a linking group, and the 5' terminal of the aptamer was linked to the fluorescent group Cy5.

Conjugate 17 was a conjugate which comprises the double-stranded siRNA and the aptamer provided by the present disclosure, wherein the aptamer was linked to the 5' terminal of the sense strand of the siRNA by four sequentially linked propylene phosphate ester groups as linking groups, and the 5' terminal of the aptamer was linked to the fluorescent group Cy5.

The structure of conjugate 18 was similar to conjugate 17, wherein the structure of the aptamer was slightly different from the aptamer of conjugate AP2 (see Table 1B and preparation Example 18).

U118MG cells were cultured and inoculated subcutaneously into mice according to the method of experimental Example 4.

Conjugate 15, conjugate 16, conjugate 17, and conjugate 18 were respectively formulated into 0.3 mg/mL solutions with PBS.

The administration began 30 days after inoculation of U118MG cells, and the day of administration was designated as D1. The administration manner of tail vein injection was adopted in this experiment: administration was performed once a day, three times in total.

12 mice (body weight was about 25 g) were randomly divided into 4 groups (3 mice per group).

For test group 1, conjugate 15 was administered with a single administration volume of 250 μL and a single administration dosage of 3 mg/kg.

For test group 2, conjugate 16 was administered with a single administration volume of 250 μL and a single administration dosage of 3 mg/kg.

For test group 3, conjugate 17 was administered with a single administration volume of 250 μL and a single administration dosage of 3 mg/kg.

For test group 4, conjugate 18 was administered with a single administration volume of 250 μL and a single administration dosage of 3 mg/kg.

[3] Detection

24 days after first administration, a small animal *in vivo* optical imaging system IVIS Lumina Series III was used for *in vivo* imaging of the mice.

Figure 12 is an image showing the fluorescence imaging results in mice after administrating different conjugates. As can be seen from Figure 12, all conjugates with different binding groups, different binding modes, and different aptamers are enriched in tumor cells. These results indicate that various conjugates provided by the present disclosure can effectively target tumor tissues, despite the aptamers included are linked to the siRNA in different ways or the sequences of the aptamers are changed. Furthermore, the fluorescence signal of conjugate 18 is stronger and shows a diffuse distribution *in vivo* as compared with other conjugates, indicating that conjugate 18 lasts longer in the circulatory system without being excreted by metabolism rapidly, and may therefore have a more durable targeting effect.

**Experimental Example 14 *In vivo* targeting activity of the conjugates in mice**

In this experimental Example, the *in vivo* targeting activity of conjugates AP2, AP21-26 and comparative AP27-comparative AP30 in mice were investigated.

U118MG cells were cultured and inoculated subcutaneously into 24 mice (all female) according to the method of experimental Example 4 to obtain the mice inoculated with U118MG subcutaneous tumors.

Conjugates AP2, AP21-26 and comparative AP27-comparative AP30 were respectively formulated into 0.3 mg/mL solutions with DMEM medium.

The administration began 14 days after inoculation of U118MG cells, and the day of administration was designated as D1. The administration manner of tail vein injection was adopted in this experiment: administration was performed once a day, three times in total.

24 mice inoculated with U118MG subcutaneous tumors were randomly divided into 12 groups (2 mice per group).

For 7 groups of mice, each mouse in each group was administered with AP2, AP21, AP22, AP23, AP24, AP25, or AP26 with a single administration volume of 10 μL/g mouse body weight (through calculation, the single administration dosage was 3 mg/kg), and these groups were designated as test groups 14A-14G, respectively.

For other 4 groups of mice, each mouse in each group was administered with comparative AP27, comparative AP28, comparative AP29, or comparative AP30 with a single dose volume of 10 μL/g mouse body weight (through calculation, the single administration dosage was 3 mg/kg), and these groups were designated as control groups 14H-14K, respectively.

For 2 mice of the last group, each mouse was administered with DMEM medium with an administration volume of 10 μL/g mouse body weight, and this group was designated as blank control group 15Y

At 1h, 24h and 48h after first administration, a small animal *in vivo* optical imaging system IVIS Lumina Series III was used for *in vivo* imaging of the mice. On D5, mice of each group were euthanized, and the tumor tissues and kidneys were taken for fluorescence imaging.

Figures 13A-13C are respectively images showing the fluorescence imaging results in mice at 1h, 24h and 48h after administrating different conjugates, in which the leftmost mouse of the three mice in each panel is a mouse in blank control group 15Y As can be seen from Figure 13A, the blank control group does not show any fluorescence signal; in contrast, the mice of all test groups and control groups show fluorescence signals at subcutaneous tumor sites at 1h after administration; as can be seen from Figures 13B and 13C, only the mice of test groups 14A-14G show strong fluorescence signals at subcutaneous tumor sites at 24h and 48h after administration, while the mice of control groups 14H-14K substantially do not show fluorescence signals or show only weak fluorescence signals. Furthermore, Figure 13D is an image showing the fluorescence signal imaging of tumor tissues and kidneys in each group of mice after the mice were euthanized at D5, wherein blank represents blank control group 14Y As can be seen from Figure 13D, the mice in blank control group 14Y and control group 14H-14K substantially do not show fluorescence signals or show only weak fluorescence signals at tumor tissues; in contrast, the mice in test group 14A-14G which administered with the conjugates provided by the present disclosure show strong fluorescence signals at tumor tissues, while showing only weak fluorescence signals at the metabolic organ kidney, indicating that various aptamer provided by the present disclosure can stably and efficiently target and deliver the fluorescent groups to tumor tissues as compared with the control conjugates. Various conjugates comprising diagnostic agent groups provided by the present disclosure can stably and efficiently target tumor tissues, thereby facilitating the successful diagnosis and monitoring of the presence of tumors.

### Experimental Example 15 *In vivo* targeting activity of conjugates in mice

In this experimental Example, the *in vivo* targeting activity of conjugates AP2, AP12 and comparative AP31-comparative AP35 in mice were investigated.

U118MG cells were cultured and inoculated subcutaneously into 16 mice (all male) according to the method of experimental Example 4 to obtain the mice inoculated with U118MG subcutaneous tumors.

Conjugates AP2, AP12 and comparative AP31-comparative AP35 were respectively formulated into 0.3 mg/mL solutions with DMEM medium.

The administration began 21 days after inoculation of U118MG cells, and the day of administration was designated as D1. The administration manner of tail vein injection was adopted in this experiment: administration was performed once a day, three times in total.

16 mice inoculated with U118MG subcutaneous tumors were randomly divided into 8 groups (2 mice per group).

For 2 groups of mice, each mouse in each group was administered with AP2 or AP12 with a single administration volume of 10 μL/g mouse body weight (through calculation, the single administration dosage was 3 mg/kg), and these groups were designated as test groups 15A-15B, respectively.

For other 5 groups of mice, each mouse in each group was administered with comparative AP31, comparative AP32, comparative AP33, comparative AP34, or comparative AP35 with a single dose volume of 10 μL/g mouse body weight (through calculation, the single administration dosage was 3 mg/kg), and these groups were designated as control groups 15C-15G, respectively.

For 2 mice of the last group, each mouse was administered with DMEM medium with an administration volume of 10 μL/g mouse body weight, and this group was designated as blank control group 15Y

At 1h, 24h and 48h after first administration, a small animal *in vivo* optical imaging system IVIS Lumina Series III was used for *in vivo* imaging of the mice. On D6, mice of each group were euthanized, the tumor tissues and kidneys were

taken for fluorescence imaging.

Figures 14A-14C are respectively images showing the fluorescence imaging results in mice at 1h, 24h and 48h after administrating different conjugates, in which the leftmost mouse of the three mice in each panel is a mouse in blank control group 15Y As can be seen from Figure 14A, the blank control group does not show any fluorescence signal; in contrast, the mice of all test groups and control groups show fluorescence signals at subcutaneous tumor sites at 1h after administration; as can be seen from Figures 14B and 14C, only the mice of test groups 15A and 15B show strong fluorescence signals at subcutaneous tumor sites at 24h and 48h after administration, while the mice of blank control group 15Y and control groups 15C-15G do not show fluorescence signal. Furthermore, Figure 14D is an image showing the fluorescence signal imaging of tumor tissues and kidneys in each group of mice after the mice were euthanized at D6, wherein blank represents blank control group 15Y As can be seen from Figure 14D, the mice in blank control group 15Y and control group 15C-15G do not show fluorescence signal at tumor tissues; in contrast, the mice in test group 15A or 15B which administered with the conjugates provided by the present disclosure show strong fluorescence signals at tumor tissues, while showing only weak fluorescence signals at the metabolic organ kidney, indicating that the conjugates provided by the present disclosure can stably and efficiently target tumor tissues as compared with the control conjugates, and various conjugates without the sequence shown in formula (1) do not show targeting effect on tumor tissues.

**Experimental Example 16 Distribution of conjugates in U118MG human glioma cells**

U118MG human glioma cells (purchased from Guangzhou Jennio Biotech Co., Ltd) were cultured according to the method of experimental Example 4. U118MG human glioma cells in the logarithmic growth phase were selected and digested (0.25% pancreatic enzyme). The cells were collected, centrifuged to remove the supernatant, and then resuspended in serum-free DMEM medium to be formulated into a cell culture solution with a concentration of $1\times10^8$ cells/mL.

Experimental animals were 8 NOD-SCID mice (male, 12 weeks old, purchased from SPF (Beijing) Biotechnology Co., LTD.). The cell culture solution above was inoculated at the subcutaneous site of the right back of a NOD-SCID mouse with an inoculation volume of 100 μL per mouse, i.e., each mouse was inoculated with $1\times10^7$ cells. The mice were further fed for 21 days after injection.

Conjugates AP2, AP4 and comparative AP13 were dissolved into conjugate solutions with a concentration of 0.3 mg/mL (based on aptamer) with serum-free DMEM medium.

21 days after inoculation of U118MG cells, 8 mice were randomly divided into 4 groups (2 mice per group). The administration was performed for each group of mice, and the day of administration was designated as D1. The administration manner of tail vein injection was adopted in this experiment (single administration).

For 3 groups of mice, each mouse in different groups was administered with conjugates AP2, AP4 or comparative conjugate comparative AP13 with a single administration volume of 10 μL/g mouse body weight (through calculation, the single administration dosage was 3 mg/kg), and these groups were designated as test groups 16a, 16b and 16c, respectively.

For the last group, each mouse was administered with DMEM with an administration volume of 10 μL/g mouse body weight, and this group was designated as blank control group 16Y.

At 24h after first administration, the mice were euthanized. The tumor tissues were collected and fixed in 4% paraformaldehyde solution, dehydrated with 15% sucrose for 24h and then dehydrated with 30% sucrose for 24h, infiltrated with OCT embedding agent, and frozen in liquid nitrogen. Then, the frozen tissues were sliced using a freezing microtome (Type POLAR-D-JC, purchased from Sakura Finetek Japan Co., Ltd.) with a thickness of 10 μm, to obtain the tumor tissue sections.

The tumor tissue sections were stood still at room temperature for 5 min, fixed with 4% paraformaldehyde for 15 min, and washed with PBS for 3 times (5 mL each time). Then, the sections were stained with DAPI for 3 minutes in the dark, washed with above steps of washing for 3 times, and sealed, to obtain DAPI stained sections.

Imaging analysis of the resultant DAPI stained sections above was performed by using a laser confocal imager (Type LSM 900 Basic Operation, purchased from Carl Zeiss (Shanghai) Managing Co., LTD.), with the select parameters: Cy5 laser intensity of 20% (em 650nm, ex 670nm), DAPI laser intensity of 1.5% (em 360nm, ex 460nm), taking pictures in Best signal mode. The results are shown in Figure 15.

Figure 15 is an image showing the laser confocal imaging results of U118MG glioma cells 24h after administration of different conjugates to the mice subcutaneously inoculated with U118MG glioma. The experimental results show that the conjugates comprising delivery groups of the present disclosure can efficiently and specifically deliver the diagnostic agent groups to U118MG glioma as compared with blank control group DMEM and comparative conjugate AP13, showing excellent targeting effect and potential diagnostic capability.

**Experimental Example 17 Delivery effect of conjugate 15 and conjugate 36 on U118MG subcutaneous tumor**

U118MG human glioma cells (purchased from Guangzhou Jennio Biotech Co., Ltd) were cultured according to the method of experimental Example 4. U118MG human glioma cells in the logarithmic growth phase were selected and digested (0.25% pancreatic enzyme). The cells were collected, centrifuged to remove the supernatant, and then resuspended in serum-free DMEM medium to be formulated into cell culture solutions with a concentration of $1\times10^8$ cells/mL.

Experimental animals were 9 NOD-SCID mice (male, 12 weeks old, purchased from SPF (Beijing) Biotechnology Co., LTD.). The cell culture solution above was inoculated at the subcutaneous site of the right back of a NOD-SCID mouse with a inoculation volume of 100 μL per mouse, i.e., each mouse was inoculated with $1\times10^7$ cells. The mice were further fed for 21 days after injection.

Conjugate AP2, conjugate 15 and conjugate 36 were dissolved into conjugate solutions with a concentration of 0.5 mg/mL (based on aptamer) with serum-free DMEM medium. The administration began 21 days after inoculation of U118MG cells, and the day of administration was designated as D1. The administration manner of tail vein injection was adopted in this experiment (single administration).

For control group 17a, each mouse was administered with conjugate AP2 with a single administration volume of 10 μL/g mouse body weight (through calculation, the single administration dosage was 5 mg/kg).

For control group 17b, each mouse was administered with conjugate 15 with a single administration volume of 10 μL/g mouse body weight (through calculation, the single administration dosage was 5 mg/kg).

For control group 17c, each mouse was administered with conjugate 36 with a single administration volume of 10 μL/g mouse body weight (through calculation, the single administration dosage was 5 mg/kg).

At 24h (D2) and 48h (D3) after administration, the mice were anesthetized by isoflurane gas (0.41 ml/min isoflurane contained in 4L/min fresh air stream), a small animal *in vivo* optical imaging system IVIS Lumina Series III was used for *in vivo* imaging of the mice.

Figure 16A and figure 16B are images showing the fluorescence imaging results in the mice after administrating different conjugates. The results indicate that at different time after administration, various conjugates comprising the siRNA groups and the delivery groups formed by the aptamers of the present disclosure can effectively target and be enriched into tumor tissues, showing excellent drugability.

**Experimental Example 18 Evaluation of activity of conjugate 37 and conjugate 38 to enter into PANC-1 (human pancreatic cancer cell)**

PANC-1 human pancreatic cancer cells (purchased from Chinese national intrastructure of cell line resource) were cultured in DMEM complete medium (MACGENE, Cat No. CM15019) containing 10% FBS (Gibco, Cat No. 10099-141) at 37°C in an incubator containing 5% $CO_2$/95% air.

PANC-1 human pancreatic cancer cells in the logarithmic growth phase were selected and digested (0.25% pancreatic enzyme). The cells were collected, centrifuged to remove the supernatant, and then resuspended in DMEM medium containing 10% FBS to be formulated into cell culture solution with a concentration of $1\times10^5$ cells/mL. The solutions were mixed thoroughly, seeded into a 24-well plate (with 1 mL/well, i.e. $1\times10^5$ cells/well).

Conjugate 37, conjugate 38, and conjugate AP2 were respectively dissolved into conjugate solutions with a concentration of 20 μM with DMEM.

After respectively culturing the cell solutions in different culture wells of a 24-well plate for 24h, the supernatant was aspirated and 900 μL DMEM medium containing 10% FBS was added into each well. 100 μL blank control group DMEM (hereinafter referred to as control group), conjugate 37, conjugate 38, or conjugate AP2 were added into the corresponding culture medium and freely ingested at a concentration of 2 μM. RNAs were extracted after 7 days and designated as control group and conjugate 9 group, conjugate 10 group and conjugate AP2 group (the latter 3 groups were collectively referred to as test groups).

Subsequently, the cellular total RNA in each well was extracted by using a magnetic bead tissue RNA extraction kit (purchased from NanoMagBio Co., Ltd., Cat. No. NMR0211-20) according to the operation method as described in the instruction, to obtain solutions containing total RNA.

For the cells of each well, a solution containing 1 μg total RNA was taken and the total RNA of the cells of each cell was reverse transcribed by using a reverse transcription kit (purchased from Promega Corporation, Cat. No. A3500) according to the procedures for reverse transcription in the instruction of the kit, in which Oligo $(dT)_{18}$ was used as the primer. After the reaction was completed, 80 μL of DEPC water was added into the reverse transcription reaction system to obtain a 100 μL cDNA-containing solution.

For each reverse transcription reaction system, 5 μL of the above cDNA-containing solution was taken as the template. By using a SYBR Select Master Mix Kit (purchased from Thermo Fisher Scientific, Cat. No.4472908), the target gene hSTAT3 and the internal control gene hGAPDH were amplified in a SteponePlus™ Real-Time fluorescence

quantitative PCR Thermal Cycler (purchased from Thermo Fisher Scientific) according to the operation method the instruction. The amplification procedure included: pre-denaturation at 95°C for 10 minutes, denaturation at 95°C for 30 s, annealing at 60°C for 30 s, and extension at 72°C for 30 s, wherein the process of denaturation, annealing and extension was repeated for 40 times. The melting program is the default program of the instrument.

Table 5 Information of the primers

| Gene name | Primer type | Nucleotide sequence (5'→3') | SEQ ID NO |
|---|---|---|---|
| Human hSTAT3 | Upstream primer | CCTTTGGAACGAAGGGTACA | 79 |
| | Downstream primer | CGGACTGGATCTGGGTCTTA | 80 |
| Human GAPDH | Upstream primer | GGTCGGAGTCAACGGATTT | 81 |
| | Downstream primer | CCAGCATCGCCCCACTTGA | 82 |

For each test group and control group, the above quantitative PCR detection was performed twice.

The fluorescence quantitative data was analyzed by relative quantitative $2^{-\Delta\Delta Ct}$ method, and the calculation method was as follows:

$$\Delta Ct\ (\text{test group}) = Ct\ (\text{target gene of test group}) - Ct\ (\text{internal control gene of test group})$$

$\Delta$Ct (control group) = Ct (target gene of control group) - Ct (internal control gene of control group)

$$\Delta\Delta Ct\ (\text{test group}) = \Delta Ct\ (\text{test group}) - \Delta Ct\ (\text{average of control group})$$

$$\Delta\Delta Ct\ (\text{control group}) = \Delta Ct\ (\text{control group}) - \Delta Ct\ (\text{average of control group})$$

Inhibition rate (%) = (control group $2^{-\Delta\Delta Ct}$ mean value - test group $2^{-\Delta\Delta Ct}$) / control group $2^{-\Delta\Delta Ct}$ mean value $\times$100

The experimental results are visually represented as a bar graph with Mean ± SD values by Graph Prism software. The results are shown in Figure 17, in which blank represents control group.

Figure 17 is a bar graph showing the inhibition rates of conjugate 37, conjugate 38 and conjugate AP2 of the present disclosure against hSTAT3 mRNA in PANC-1 human cancer cells, respectively. According to the results in Figure 17, after administration of conjugate 37, the inhibition rate against hSTAT3 mRNA in PANC1 human pancreatic cancer cells was 33%; after administration of conjugate 38, the inhibition rate against hSTAT3 mRNA in PANC1 human pancreatic cancer cells reached 44%. These results indicate that the conjugates of the present disclosure with different binding modes, different binding groups, and different modified nucleotide sequences can be freely ingested into tumor cells and show the effect of inhibiting the target mRNA.

**Experimental Example 19 Evaluation of the internalization of conjugates 15-19 in U118MG cells**

U118MG human glioma cells (purchased from Guangzhou Jennio Biotech Co., Ltd) were cultured according to the method of experimental Example 4. U118MG cells were digested (0.25% pancreatic enzyme) and cultured in a 12-well plate at $2\times10^5$ cells/well in a cell incubator at 37°C with 5%$CO_2$ for 24h.

Conjugate 15, conjugate 16, conjugate 17, conjugate 18, and conjugate 19 were dissolved into a concentration of 200 μM with serum-free DMEM medium. 10 μL of the compound was added into a 990 μL cell culture well, that is, the final concentration of the compound was 2 μM. After incubating the wells at 37°C for 2h, the supernatant was aspirated, and the wells were washed with PBS for 3 times, fixed with 100 μL 4% paraformaldehyde solution for 10 min, washed with PBS for 3 times, stained with 100 μL DAPI staining solution added for 10 min, washed with PBS for 3 times, and subject to laser confocal imaging.

Laser confocal imaging: Cy5 laser intensity of 2% (em 650nm, ex 670nm), DAPI laser intensity of 1.5% (em 360nm, ex 460nm), taking pictures in Best signal mode. The results are shown in Figure 18.

Figure 18 is an image showing the laser confocal imaging results of the conjugates of the present disclosure in a U118MG glioma.

The results of Figure 18 indicate that all conjugates of the present disclosure with different binding modes, different binding groups, and different modified nucleotide sequences can be freely ingested into tumor cells, and

conjugate 18 with 2'-O-cetyl modification has better ability to enter into tumor cells.

**Experimental Example 20 Evaluation of the internalization of conjugate 15 and conjugate 18 in U118MG cells**

In this experimental Example, the ability of conjugate 15, conjugate 18 and conjugate AP2 to be freely ingested into U118-MG glioma cells *in vitro* was evaluated and compared. U118MG human glioma cells in the logarithmic growth phase cultured according to experimental Example 4 were selected and digested with 0.25% pancreatic enzyme. The cells were collected, centrifuged to remove the supernatant, and then resuspended in DMEM medium supplemented with 10% FBS to be formulated into cell culture solutions with a concentration of $1\times10^5$ cells/mL. The solutions were mixed thoroughly and seeded into a 24-well plate (1000 μL/ well, i.e. $1\times10^5$ cells/well).

Conjugate 15, conjugate 18, and conjugate AP2 were respectively dissolved into conjugate solutions with a concentration of 40 μM with DMEM, and designated as test group 20a, test group 20b and control group 20c.

After culturing the cell solutions for 24h, the culture medium was aspirated and 900 μL serum-free DMEM medium was added into each well. 100 μL blank control group DMEM, conjugate 15, conjugate 18, or conjugate AP2 were added into the corresponding culture medium and freely ingested at a concentration of 4 μM. The cell fluorescence value was measured after 48 hours.

For test group 20a, test group 20b and control group 20c, 100 μL DMEM medium was taken and imaged in a high content imaging system. Normalization was performed according to the mean fluorescence intensity of blank control group DMEM, that is, subtracting the mean fluorescence intensity of the blank control group from the fluorescence intensity of test group 20a, test group 20b and control group 20c before performing imaging. The results are shown in Figure 19.

Figure 19 is a bar graph showing the fluorescence intensity results of the conjugates of the present disclosure in U118MG glioma, wherein DMEM represents the blank control group. The results in Figure 19 show that the conjugates provided by the present disclosure could effectively deliver the siRNA groups into tumor cells, thereby facilitating the efficient generation of RNAi effect in tumor cells.

**Experimental Example 21 *In vivo* activity of the conjugate in U118MG tumor in situ model mice**

According to the method of experimental Example 4, U118MG human glioma cells expressing Luciferase (*Photinus pyralis*) reporter gene were cultured (hereinafter referred to as U118MG-luc human glioma cells, purchased from Nanjing Kebai Biotechnology Co., Ltd.). U118MG-luc human glioma cells in the logarithmic growth phase were taken and digested with 0.25wt% trypsin. The cells were collected and centrifuged, the supernatant was aspirated, and then the cells were resuspended in serum-free DMEM medium to be formulated into a cell culture solution with a cell density of $4\times10^7$ cells/mL.

Experimental animals were 24 Balb/C-nude mice (male, 12 weeks old, purchased from SPF (Beijing) Biotechnology Co., Ltd.). The above cell culture solution was inoculated into the striatum of a Balb/C-nude mouse: the cell culture solution was injected into the right striatum of a mouse by the manner of striatum injection in the mouse, and the position was AP (anteroposterior): 1 mm, ML (medial lateral): 1.5 mm, DV (dorsal ventral): 3.5 mm, and the injection volume was 10 μL, that is, each mouse was inoculated with $4\times10^5$ cells. The mice were further fed for 14 days after in situ tumor inoculation.

Conjugate 20 was dissolved into a conjugate solution with a concentration of 1 mg/mL (based on aptamer) with 1×DMEM medium (purchased from M&C gene technology (BEIJING) LTD., Lot No. K1902200). Conjugate 39 and comparative conjugate 43 were respectively dissolved into conjugate solutions with a concentration of 0.8 mg/mL (based on aptamer).

On Day 15 after inoculation of the in situ tumor, *in vivo* imaging of the mice were performed by a small animal *in vivo* optical imaging system IVIS Lumina Series III. The mice were grouped according to the brain fluorescence intensity (6 mice per group). The day of administration was designated as D1 (i.e., the first day of the experiment, and hereinafter D4, D8, etc., respectively represent Day 4, Day 8 of the experiment, and so on).

*In vivo* imaging method: each mouse was intraperitoneally injected with 10 μL/g body weight of a 15 mg/mL D-luciferin potassium salt working solution (purchased from Yeasen Biotechnology (Shanghai) Co., Ltd.). *In vivo* imaging was performed 10 minutes after injection (IVIS® Lumina III Small Animal *In vivo* Imaging System). After imaging, the fluorescent region (ROI) in the mouse brain was circled and selected, and the fluorescence intensity (Radiance) was measured by software. Under this condition, the Luciferase (*Photinus pyralis*) reporter gene expressed in U118MG-luc human glioma cells can produce a fluorescent response, so the fluorescence intensity can reflect the proliferation number of glioma cells. The higher the fluorescence intensity, the greater the number of glioma cells.

The administration manner of subcutaneous injection was adopted in this experiment. Each group of mice was administered on D1, D4, D8, and D12, respectively. The mice were weighed before administration and were administered based on weight.

For test group 21a, conjugate 20 was administered to each mouse, and the single administration volume was 5

μL/g mouse body weight. Through calculation, the single administration dosage was 5 mg/kg, in which the dose of MMAE contained was equivalent to 0.3 mg/kg.

For test group 21b, conjugate 39 was administered to each mouse, and the single administration volume was 5 μL/g mouse body weight. Through calculation, the single administration dosage was 4 mg/kg, in which the dose of MMAE contained was equivalent to 0.3 mg/kg.

For control group 21c, comparative conjugate 43 was administered to each mouse, and the single administration volume was 5 μL/g mouse body weight. Through calculation, the single administration dosage was 4 mg/kg, in which the dose of MMAE contained was equivalent to 0.3 mg/kg.

For the blank control group, DMEM medium was administered to each mouse, and the single administration volume was 5 μL/g mouse body weight.

According to the above method, *in vivo* imaging analysis of each group of mice was performed on D1, D22, D31, and D39, respectively, and the fluorescence intensity was measured. The results are shown in Figure 20.

Figure 20 is a line graph showing the changes in tumor fluorescence intensity over time of U118MG tumor in situ model mice after administration of the conjugates provided by the present disclosure or the control compound.

As shown in the results in Figure 20, as compared with D1 after administration, the tumor fluorescence intensity (Radiance) of the blank control group and the control group increased significantly as the observation time extends, indicating that the number of U118MG human glioma cells increased significantly; in contrast, the tumor fluorescence intensity of test groups 21a and 21b administered with the conjugates provided by the present disclosure was reduced significantly, and the reduction range could be up to one order of magnitude and could be more than 2 orders of magnitude as compared with the control group, indicating that the number of U118MG human glioma cells was significantly decreased, which may be reduced to 1/10 of the starting number at the beginning of the experiment, or even decreased to less than 1% of that of the control group. It can be seen that even if only by subcutaneous administration, the conjugates comprising tumor therapeutic agent groups and the delivery groups formed by the targeting aptamers provided by the present disclosure can effectively penetrate the blood brain barrier and efficiently target and enter into brain gliomas, and have a good inhibitory effect on tumor growth, showing good treatment compliance and high drugability for efficient tumor inhibition.

**Experimental Example 22 *In vivo* inhibitory activity of the conjugate against U118MG subcutaneous tumors in mice**

U118MG human glioma cells (purchased from GuangZhou Jennio Biotech Co., Ltd) were cultured according to the method of Experimental Example 4. U118MG human glioma cells in the logarithmic growth phase were taken and digested (with 0.25% trypsin). The cells were collected, centrifuged to remove the supernatant, and then resuspended in serum-free DMEM medium to prepare a cell culture solution with a concentration of $1\times10^8$ cells/mL.

Experimental animals were 36 NOD-SCID mice (male, 12 weeks old, purchased from SPF (Beijing) Biotechnology Co.,Ltd.). The above cell culture solution was inoculated into the subcutaneous site of the right back of a NOD-SCID mouse with an inoculation volume of 100 μL per mouse, that is, each mouse was inoculated with $1\times10^7$ cells. The mice were further fed for 7 days after injection.

Conjugate 20 was dissolved into a conjugate solutions with a concentration of 1 mg/mL (based on aptamer) with serum-free DMEM medium. Conjugate 39, conjugate 40, and conjugate 41 were respectively dissolved into conjugate solutions with a concentration of 0.8 mg/mL (based on aptamer). MMAE was dissolved into a solution with a concentration of 0.06 mg/mL with 10% DMSO + 90% serum-free DMEM medium (volume ratio).

Administration began 7 days after U118MG cell inoculation, and the day of administration was designated as D8. The administration manner of subcutaneous administration in the abdomen was adopted in this experiment: administration was performed once on D8, D12, D16, and D20, four times in total.

For blank control group 22a, DMEM was administered to each group of mice, and the single administration volume was 5 μL/g mouse body weight.

For control group 22b, MMAE was administered to each group of mice, and the single administration volume was 5 μL/g mouse body weight. Through calculation, the single administration dosage was 0.3 mg/kg.

For test group 22c, conjugate 20 was administered to each mouse, and the single administration volume was 5 μL/g mouse body weight. Through calculation, the single administration dosage was 5 mg/kg, in which the dose of MMAE contained was equivalent to 0.3 mg/kg.

For the three groups of mice, conjugate 39, conjugate 40 or conjugate 41 was respectively administered to each group of mice, and the single administration volume was 5 μL/g mouse body weight. Through calculation, the single administration dosage was 4 mg/kg, in which the dose of MMAE contained was equivalent to 0.3 mg/kg. The groups were respectively designated as test groups 22d, 22e and 22f.

The tumor volumes were measured on D16, D20, D25, D29, D33, D36, D41, D48, and D60. The long diameter and short diameter of the tumor was measured *in vitro*. The tumor volume was calculated according to the equation 1/2

(long diameter × short diameter$^2$). At the end of the experiment, the tumor tissues were taken on D60 and weighed. Figure 21 is a line graph showing the changes in tumor volume over time in mice on different days after administration of different conjugates. As can be seen from Figure 21, as compared with control group 22b or the blank control group, the tumor volumes and tumor weights of the mice in test groups 22c to 22f administered with the conjugates of the present disclosure were significantly reduced. The above results indicate that the conjugates comprising the delivery groups formed by these aptamers can effectively reach tumor tissues and exhibit good anti-tumor activity.

**Experimental Example 23: *In vivo* activity of long-interval administration of the conjugates in U118MG subcutaneous tumor model mice**

According to the method of Experimental Example 4, 42 mice inoculated with U118MG subcutaneous tumor were obtained, and were further fed after injection.

MMAE was dissolved into solutions with concentrations of 0.03 mg/mL and 0.01 mg/mL with 10% DMSO + 90% serum-free DMEM medium (volume ratio). Conjugate 39 was dissolved into solutions with concentrations of 0.5 mg/mL and 0.165 mg/mL (based on aptamer). Comparative conjugate 44 was dissolved into solutions with concentrations of 0.5 mg/mL and 0.165 mg/mL (based on aptamer).

7 days after inoculation of U118MG cells, all mice were randomly divided into seven groups (6 mice per group), and each group of mice was administered. The day of administration was designated as D8. The administration manner of subcutaneous administration in the abdomen was adopted in this experiment: administration was performed once on D8, D11, D15, D29, D32, and D36, six times in total. The mice were weighed before administration and the administration volume was calculated according to weight.

For the blank control group, DMEM medium was administered to each mouse, and the single administration volume was 10 μL/g mouse body weight.

For test group 23a, MMAE at a concentration of 0.01 mg/mL was administered to each mouse, and the single administration volume was 10 μL/g mouse body weight. Through calculation, the single administration dosage was 0.1 mg/kg.

For test group 23b, conjugate 39 at a concentration of 0.165 mg/mL was administered to each mouse, and the single administration volume was 10 μL/g mouse body weight. Through calculation, the single administration dosage was 1.65 mg/kg (the corresponding dose of MMAE was 0.1 mg/kg).

For control group 23c, comparative conjugate 44 at a concentration of 0.165 mg/mL was administered to each mouse, and the single administration volume was 10 μL/g mouse body weight. Through calculation, the single administration dosage was 1.65 mg/kg (the corresponding dose of MMAE was 0.1 mg/kg).

For test group 23d, MMAE at a concentration of 0.03 mg/mL was administered to each mouse, and the single administration volume was 10 μL/g mouse body weight. Through calculation, the single administration dosage was 0.3 mg/kg.

For test group 23e, conjugate 39 at a concentration of 0.5 mg/mL was administered to each mouse, and the single administration volume was 10 μL/g mouse body weight. Through calculation, the single administration dosage was 5 mg/kg (the corresponding dose of MMAE was 0.3 mg/kg).

For control group 23f, comparative conjugate 44 at a concentration of 0.5 mg/mL was administered to each mouse, and the single administration volume was 10 μL/g mouse body weight. Through calculation, the single administration dosage was 5 mg/kg (the corresponding dose of MMAE was 0.3 mg/kg).

The tumor volumes were measured on D1, D9, D16, D19, D21, D24, D26, D29, D32, D36, D39, D43, D47, D53, D57, D60, D64, D67, D71, D74, D78, D81, D84, D88, D92, D95, and D99, wherein the blank control group was measured on D53, and test groups 7a, 7b and control group 7c were measured on D60, and then the experiment was terminated.

The long diameters and short diameters of the tumors were measured *in vitro.* The tumor volume was calculated according to the equation 1/2 (long diameter × short diameter$^2$). After the experiment was terminated, the tumor tissues were taken and weighed, and the average value was calculated. The results are shown in Figure 22.

Figure 22 is a line graph showing the changes in tumor volume over time in U118MG subcutaneous tumor model mice after administration of the conjugates provided by the present disclosure at different concentrations or a control compound.

As shown in the results of Figure 22, in the blank control group, the tumor volume increased rapidly; in the test groups administered with only MMAE, the growth rate of tumor volume was reduced, indicating that MMAE *per se* showed an inhibitory effect on tumor proliferation.

Furthermore, the tumor volume of test group 23b during the test was significantly smaller than those of test group 23a and control group 23c administered with the equivalent content of MMAE; the tumor volume of test group 23e during the test was significantly smaller than those of test group 23d and control group 23f administered with the equivalent content of MMAE, showing more superior anti-tumor activity than those of test groups 23a and 23d administered with only MMAE, and than that of comparative conjugate 44. After the experiment was terminated, the tumor weights of the mice

administered with the conjugates provided by the present disclosure were also significantly lower than those in the MMAE group and the control group. The above results show that the conjugates provided by the present disclosure can effectively deliver MMAE to tumor tissues, showing tumor-targeting ability, while reducing the toxicity risk caused by the distribution of MMAE molecule in other tissues, and showing dose-dependency and excellent anti-tumor effects.

The above results indicate that the conjugates provided by the present disclosure could effectively target and deliver small molecule drug groups having tumor inhibition effects to tumor tissues, showing good anti-tumor activity and dose-dependent effects.

**Experimental Example 24 *In vivo* activity of the conjugates in U118MG subcutaneous tumor model mice**

According to the method of Experimental Example 4, 42 mice inoculated with U118MG subcutaneous tumor were obtained, and were further fed after injection.

MMAE was dissolved into a solution with a concentration of 0.02 mg/mL with 10% DMSO + 90% serum-free DMEM medium (volume ratio). Conjugate 20 and comparative conjugate 44 were dissolved into solutions with a concentration of 0.33 mg/mL (based on aptamer). Conjugate 39 and conjugate 41 were dissolved into solutions with a concentration of 0.26 mg/mL (based on aptamer). Conjugate 42 was dissolved into a solution with a concentration of 0.23 mg/mL (based on aptamer). 7 days after inoculation of U118MG cells, the inoculated mice above were divided into groups (6 mice per group), and each group of mice was administered. The day of administration was designated as D8. The mice were weighed before administration, and the administration volume was calculated according to the average weight of mice (20g per mouse).

The administration manner of subcutaneous administration in the abdomen was adopted in this experiment: administration was performed once on D8, D12, D15, and D19, four times in total.

For the blank control group, DMEM medium was administered to each mouse, and the single administration volume was 100 μL/g.

For test group 24a, MMAE was administered to each mouse, and the single administration volume was 100 μL. Through calculation, the single administration dosage was 0.1 mg/kg.

For test group 24b, conjugate 20 was administered to each mouse, and the single administration volume was 100 μL. Through calculation, the single administration dosage was 1.65 mg/kg.

For control group 24c, comparative conjugate 44 was administered to each mouse, and the single administration volume was 100 μL. Through calculation, the single administration dosage was 1.65 mg/kg.

For test group 24d, conjugate 39 was administered to each mouse, and the single administration volume was 100 μL. Through calculation, the single administration dosage was 1.32 mg/kg.

For test group 24e, conjugate 41 was administered to each mouse, and the single administration volume was 100 μL. Through calculation, that the single administration dosage was 1.32 mg/kg.

For test group 24f, conjugate 42 was administered to each mouse, and the single administration volume was 100 μL. Through calculation, the single administration dosage was 1.17 mg/kg.

In the above test groups and control groups 24a-24f, the corresponding dose of MMAE of the single administration dosage was 0.1 mg/kg.

The tumor volumes were measured on D1, D9, D16, D19, D22, D26, D30, D36, D40, D43, D47, D50, D54, D57, D61, D64, D68, and D71, wherein the blank control group was measured on D54, and group 8a (administered with only MMAE) was measured on D64, and then the experiment was terminated.

The long diameters and short diameters of the tumors were measured *in vitro.* The tumor volume was calculated according to the equation 1/2 (long diameter × short diameter$^2$). After the experiment was terminated, the tumor tissues from each group were taken and weighed, and the average value was calculated. The results are shown in Figure 23.

Figure 23 is a line graph showing the changes in tumor volume over time and the tumor weight on D72 in each group of mice. As shown in the results in Figure 23, the tumor volume of the blank control group increased rapidly, while the growth rate of the tumor volume of the other groups was reduced; and as compared with test group 24a and control group 24c administered with only MMAE, the growth rate of tumor volume of the test groups administered with the conjugates of the present disclosure at a dose equivalent to a single administration of 0.1 mg/kg MMAE was significantly further reduced. Furthermore, at the experimental endpoint D72, the tumor weights for conjugate 20, conjugate 39, conjugate 41, and conjugate 42 were decreased by at least 58% as compared with that of test group 24a, showing more excellent anti-tumor effects.

**Experimental Example 25 *In vivo* activity of the conjugate in A549 subcutaneous tumor model mice**

A549 human lung adenocarcinoma cells (purchased from GuangZhou Jennio Biotech Co., Ltd) were cultured in DMEM complete medium (MACGENE, Cat. No. CM15019) containing 10% FBS (Gibco, Cat.No. 10099-141) at 37°C in an incubator with 5% $CO_2$/95% air. The cells were digested with 0.25 wt% trypsin and collected, the supernatant was

aspirated, and then the cells were resuspended in serum-free DMEM medium to be formulated into a cell culture solution with a cell density of $1 \times 10^8$ cells/mL.

According to the method of Experimental Example 4, 30 mice inoculated with A549 subcutaneous tumors were obtained, and were further fed after injection.

Conjugate 20 and conjugate 39 were dissolved into conjugate solutions with a concentration of 1 mg/mL (based on aptamer) with serum-free DMEM medium. Comparative conjugate 43 was dissolved into a conjugate solution with a concentration of 0.8 mg/mL (based on aptamer). MMAE was dissolved into a solution with a concentration of 0.06 mg/mL with 10% DMSO + 90% serum-free DMEM medium (volume ratio).

7 days after the inoculation of A549 human lung cancer cells, all mice were divided into groups (6 mice each group), and each mouse was administered. The day of administration was designated as D8. The mice were weighed before administration, and the administration volume was calculated according to mouse body weight.

Each group of mice was administered once on D8, D12, D15, and D19, four times in total.

For blank control group 25a, DMEM was administered to each group of mice, and the single administration volume was 5 μL/g mouse body weight.

For control group 25b, MMAE was administered to each group of mice, and the single administration volume was 5 μL/g mouse body weight. Through calculation, the single administration dosage was 0.3 mg/kg.

For test group 25c, conjugate 20 was administered to each mouse, and the single administration volume was 5 μL/g mouse body weight. Through calculation, the single administration dosage was 5 mg/kg, in which the dose of MMAE contained was equivalent to 0.3 mg/kg.

For test group 25d, conjugate 39 was administered to each mouse, and the single administration volume was 5 μL/g mouse body weight. Through calculation, the single administration dosage was 5 mg/kg, in which the dose of MMAE contained was equivalent to 0.3 mg/kg.

For test group 25e, comparative conjugate 43 was administered to each mouse, and the single administration volume was 5 μL/g mouse body weight. Through calculation, the single administration dosage was 5 mg/kg, in which the dose of MMAE contained was equivalent to 0.3 mg/kg.

Each group of mice was administered once on D46, D50 and D54, with a single administration volume of 10 μL/g mouse body weight. Through calculation, the dose of MMAE contained in the single administration dosage was equivalent to 0.6 mg/kg.

Tumor volumes were measured on D 1, D9, D16, D19, D22, D26, D30, D36, D40, D43, D47, D50, D54, and D57, wherein the blank control group was measured on D54, and then the experiment was terminated.

The long diameters and short diameters of the tumors were measured *in vitro.* The tumor volume was calculated according to the equation 1/2 (long diameter $\times$ short diameter$^2$). The results are shown in Figure 24.

Figure 24 is a line graph showing the changes in tumor volume over time in mice A549 subcutaneous tumor model mice after administration of the conjugates provided by the present disclosure at different concentrations or a control compound. As shown in the results of Figure 24, the tumor volumes of the mice in the blank control group increased rapidly, while the growth rate of the tumor volume in the other groups was reduced; at each period of time, the tumor volumes of the mice administered with conjugate 20 and conjugate 39 were smaller than those of control groups 25b and 25e. The above results indicate that the conjugates comprising the delivery group formed by the aptamers of the present disclosure can effectively target and reach tumor tissues and exhibit good anti-tumor activity.

Some embodiments of the present disclosure are described in detail above, but the present disclosure is not limited to the specific details of the above embodiments. Various simple variations of the technical solutions of the present disclosure can be made within the scope of the technical concept of the present disclosure, and these simple variations are within the scope of the present disclosure.

It is to be noted that each of the specific technical features described in the above embodiments can be combined in any suitable manner as long as no contradiction is caused. In order to avoid unnecessary repetition, various possible combinations are no longer described in the present disclosure.

In addition, various different embodiments of the present disclosure may also be arbitrarily combined as long as it does not deviate from the idea of the present disclosure, which should also be regarded as the disclosure of the present disclosure.

## Claims

1. An aptamer comprising a segment of consecutive nucleotide sequence, wherein the group connecting two adjacent nucleotides is independently a phosphate ester group or a phosphate ester group with modification group(s), each nucleotide is selected from one of modified or unmodified A, U, C or G, and the consecutive nucleotide sequence has a sequence shown in Formula (1):

$$5'\text{-}T_1\text{-}S_1\text{-}N_a\text{-}S_2\text{-}N_b\text{-}S_3\text{-}N_c\text{-}S_4\text{-}T_2\text{-}3' \quad \text{Formula (1)}$$

wherein $T_1$ is a motif consisting of 1-3 nucleotides, $T_2$ is a motif consisting of 0-15 nucleotides, and $T_2$ does not comprise a motif that is completely reverse complementary to $T_1$;
$S_1$ and $S_4$ are each motifs consisting of 3-7 nucleotides, $S_1$ and $S_4$ are of the same length and are completely reverse complementary;
$N_a$ and $N_c$ are each motifs consisting of 1-4 nucleotides, each nucleotide in $N_a$ is not complementary to each nucleotide in $N_c$, and the total number of U in $N_a$ and $N_c$ accounts for more than 50% of the total number of all nucleotides in $N_a$ and $N_c$;
$S_2$ and $S_3$ are each motifs consisting of 1-4 nucleotides, $S_2$ and $S_3$ are of the same length and are completely reverse complementary;
$N_b$ is a motif consisting of 3-6 nucleotides, and the nucleotides at both ends of $N_b$ do not form AU or GC complementarity.

**2.** The aptamer according to claim 1, wherein the length of the consecutive nucleotide sequence is 18-50 nucleotides, or 20-40 nucleotides, or 21-36 nucleotides, or 24-32 nucleotides.

**3.** The aptamer according to claim 1 or 2, wherein $T_1$ consists of 2 nucleotides; or, $T_1$ consists of 2 nucleotides and comprises at least one C; or, in the 5'-3' direction, $T_1$ is CU, UC or AC.

**4.** The aptamer according to claim 1 or 2, wherein $T_2$ consists of 0-10 nucleotides; or, in the 5'-3' direction, $T_2$ consists of 1-9 nucleotides starting with U.

**5.** The aptamer according to claim 1, wherein $S_1$ and $S_4$ each consist of 3-5 nucleotides and are of the same length; or, in the reverse complement formed by $S_1$ and $S_4$, GC complementarity accounts for at least 40% of the total number of complementarity; or, in the 5'-3' direction, $S_1$ is GCU and $S_4$ is AGC, or $S_1$ is GAGU and $S_4$ is GCUC, or $S_1$ is GGAGU and $S_4$ is GCUCU, or $S_1$ is UAUGG and $S_4$ is CCAUG.

**6.** The aptamer according to claim 1, wherein the sum of the number of nucleotides in $N_a$ and $N_c$ is an integer of 2 to 4; or, the sum of the number of nucleotides in $N_a$ and $N_c$ is 3 or 4, and the sum of the number of U in $N_a$ and $N_c$ is 2 or 3; or, in the 5'-3' direction, $N_a$ or $N_c$ is independently U, UU, UC or CU.

**7.** The aptamer according to claim 1, wherein $S_2$ and $S_3$ each consist of 2-3 nucleotides and are of the same length; or, the reverse complement formed by $S_2$ and $S_3$ comprises at least one GC complementarity; or, in the 5'-3' direction, $S_2$ is CA and $S_3$ is UG, or $S_2$ is AC and $S_3$ is GU, or $S_2$ is GCC and $S_3$ is GGU.

**8.** The aptamer according to claim 1, wherein $N_b$ consists of 4 or 5 nucleotides; or, in the 5'-3' direction, $N_b$ is GACG, GACGU, GACCG, UACU, GUUG or GAUCU.

**9.** The aptamer according to claim 1, wherein the consecutive nucleotide sequence has the sequence shown in SEQ ID NO: 1, SEQ ID NO: 2 or SEQ ID NO: 3.

**10.** The aptamer according to claim 1, wherein the consecutive nucleotide sequence has the nucleotide sequence shown in SEQ ID NO: 4:
$5'\text{-}N_6GGAGUUCAN_1N_2N_3N_4UGN_5GCUCN_7\text{-}3'$ (SEQ ID NO: 4),
wherein, $N_1$, $N_2$, $N_3$ independently of one another are one of A, U, C and G; $N_4$ is U, C or G or a motif consisting of two of U, C or G; $N_5$ is U, CU or UU; $N_6$ is CU, UC or AC; $N_7$ is U, UU or $UUN_8$; $N_8$ is a motif consisting of 1-15 nucleotides.

**11.** The aptamer according to claim 10, wherein the motif $N_1N_2N_3N_4$ consisting of $N_1$, $N_2$, $N_3$ and $N_4$ is one of GACG, GACGU, GACCG, UACU, GUUG or GAUCU.

**12.** The aptamer according to claim 10 or 11, wherein the consecutive nucleotide sequence has the nucleotide sequence shown in any one of SEQ ID NOs: 5-11.

**13.** The aptamer according to claim 10, wherein $N_8$ is a motif consisting of 1-8 nucleotides; or, in the 5'-3' direction, the nucleotide sequence of $N_8$ is CCGAUCUC; or, the consecutive nucleotide sequence has a sequence shown in one of

SEQ ID NOs: 12-14.

**14.** The aptamer according to claim 1, wherein each cytosine nucleotide in the consecutive nucleotide sequence is a fluoro modified cytosine nucleotide, and/or each uracil nucleotide in the consecutive nucleotide sequence is a fluoro modified uracil nucleotide; or, each nucleotide in the consecutive nucleotide sequence is a 2'-methoxy modified nucleotide; or, one or more uracil nucleotides in the motifs $N_b$ and $S_3$ in the consecutive nucleotide sequence have a modified base.

**15.** The aptamer according to claim 14, wherein the consecutive nucleotide sequence has the nucleotide sequence shown in one of SEQ ID NOs: 15-33.

**16.** The aptamer according to claim 1, wherein at least one group connecting two adjacent nucleotides in the aptamer is a phosphorothioate group, or each group connecting two adjacent nucleotides is a phosphorothioate group.

**17.** The aptamer according to claim 16, wherein the consecutive nucleotide sequence has the nucleotide sequence shown in one of SEQ ID NOs: 34-39.

**18.** A conjugate comprising one or more delivery groups and one or more functional groups, wherein the delivery group is formed by removing one or more hydrogen atoms or one or more functional groups from the aptamer according to any one of claims 1 to 17; each of the delivery groups is independently linked to the functional group via a covalent bond or via a linking group, each of the functional groups is independently one of a diagnostic agent group, a small molecule therapeutic agent group having a therapeutic effect on tumors, a functional oligonucleotide group having a therapeutic effect on tumors, and a delivery auxiliary group.

**19.** The conjugate according to claim 18, wherein the conjugate has a structure as shown by Formula (101):

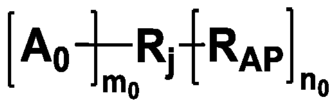

(101)

wherein, each $R_{AP}$ group is independently a group having a structure as shown by Formula (102):

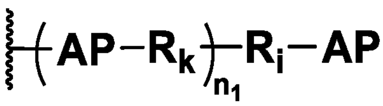

(102)

wherein, each AP group is identical or different and independently represents one of the delivery groups; $R_j$, each $R_k$ or each $R_i$ are identical or different and independently represent a covalent bond or a linking group respectively, and $R_i$ and $R_k$ are not both a covalent bond at the same time; each $n_1$ independently of one another represents an integer of 0 to 4;

each $A_0$ group is the same or different and independently represents one functional group; $m_0$ is an integer of 1 to 6; $n_0$ is an integer of 1 to 6,  represents the site where a group is covalently linked.

**20.** The conjugate according to claim 19, wherein $m_0$ is an integer of 1 to 4, and/or $n_0$ is an integer of 1 to 3, and/or each $n_1$ is independently an integer of 0 to 1;

or, $m_0$ is 1, and/or $n_0$ is 1, and/or at least one or each $n_1$ is 0.

**21.** The conjugate according to claim 19 or 20, wherein each of the $R_k$ or each of the $R_i$ is independently a covalent bond or a straight-chain alkylene group having a length of 1 to 70 carbon atoms, or one or more carbon atoms in the straight-chain alkylene group are replaced by one or more groups selected from the group consisting of: C(O), NH, O, S, CH=N, $S(O)_2$, $OP(O)_2$, OP(O)(S), $C_5$-$C_8$ glycosidylene group, $C_2$-$C_{10}$ alkenylene group, $C_2$-$C_{10}$ alkynylene group, $C_6$-$C_{10}$ arylene group, $C_3$-$C_{18}$ heterocyclylene group, and $C_5$-$C_{10}$ heteroarylene group; and wherein the straight-chain alkylene group may have any one or more substituents selected from the group consisting of: $C_1$-$C_{10}$ alkyl, $C_6$-$C_{10}$

aryl, $C_5$-$C_{10}$ heteroaryl, $C_1$-$C_{10}$ haloalkyl, -$OC_1$-$C_{10}$ alkyl, -$OC_1$-$C_{10}$ alkylphenyl, -$C_1$-$C_{10}$ alkyl-OH, -$OC_1$-$C_{10}$ haloalkyl, -$SC_1$-$C_{10}$ alkyl, - $SC_1$-$C_{10}$ alkylphenyl, -$C_1$-$C_{10}$ alkyl-SH, -$SC_1$-$C_{10}$ haloalkyl, halogen substituent, -OH, -SH, -$NH_2$, -$C_1$-$C_{10}$ alkyl-$NH_2$, -N($C_1$-$C_{10}$ alkyl)($C_1$-$C_{10}$ alkyl), -NH($C_1$-$C_{10}$ alkyl), - N($C_1$-$C_{10}$ alkyl)($C_1$-$C_{10}$ alkylphenyl), -NH($C_1$-$C_{10}$ alkylphenyl), cyano, nitro, -$CO_2$H, - C(O)O($C_1$-$C_{10}$ alkyl), -CON($C_1$-$C_{10}$ alkyl)($C_1$-$C_{10}$ alkyl), -CONH($C_1$-$C_{10}$ alkyl), - $CONH_2$, -NHC(O)($C_1$-$C_{10}$ alkyl), -NHC(O)(phenyl), -N($C_1$-$C_{10}$ alkyl)C(O)($C_1$-$C_{10}$ alkyl), -N($C_1$-$C_{10}$ alkyl)C(O) (phenyl), -C(O)$C_1$-$C_{10}$ alkyl, -C(O)$C_1$-$C_{10}$ alkylphenyl, - C(O)$C_1$-$C_{10}$ haloalkyl, -OC(O) $C_1$-$C_{10}$ alkyl, -$SO_2$($C_1$-$C_{10}$ alkyl), -$SO_2$(phenyl), - $SO_2$($C_1$-$C_{10}$ haloalkyl), -$SO_2NH_2$, -$SO_2$NH($C_1$-$C_{10}$ alkyl), -$SO_2$NH(phenyl), - $NHSO_2$($C_1$-$C_{10}$ alkyl), -$NHSO_2$(phenyl), and -$NHSO_2$($C_1$-$C_{10}$ haloalkyl).

**22.** The conjugate according to claim 21, wherein each $n_1$ is 0, and each $R_j$ is independently a covalent bond, or any one following linking group, or a connection combination of more than one following linking groups: $C_1$-$C_{20}$ alkylene, phosphate ester bond, phosphorothioate bond, amide bond, ester bond, ether bond, thioether bond, disulfide bond, 1,2,3-triazolylene group, a polyethylene glycol subunit, pyrrolidinylene group, 2-oxopyrrolidinylene group, phenylene, cyclohexylene, 2-succinimidylene group, 2-thiosuccinimidylene group, an amino acid subunit, a nucleotide subunit.

**23.** The conjugate according to claim 22, wherein each $R_i$ is independently any one following group or a connection combination of two following groups: a covalent bond, a disulfide bond, propylene phosphate ester group, 2-thiosuccinimidylene group, an amino acid subunit, or a GAU trinucleotide subunit.

**24.** The conjugate according to any one of claims 19 to 23, wherein $R_j$ is a covalent bond and $m_0$ is 1.

**25.** The conjugate according to any one of claims 19 to 23, wherein $R_j$ is a linking group, the linking group $R_j$ comprises a main chain moiety, a side chain moiety and a conjugation linking moiety, wherein the main chain moiety is respectively linked to the conjugation linking moiety and the side chain moiety, each of the side chain moieties is respectively linked to the main chain moiety and the $R_{AP}$ group, each of the conjugation linking moieties is respectively linked to the main chain moiety and the functional group $A_0$, wherein,

the main chain moiety is a straight-chain alkylene group having a length of 1 to 70 carbon atoms, or one or more carbon atoms in the straight-chain alkylene group are replaced by one or more groups selected from the group consisting of: C(O), NH, O, S, CH=N, $S(O)_2$, $OP(O)_2$, $C_5$-$C_8$ glycosidylene group, $C_2$-$C_{10}$ alkenylene group, $C_2$-$C_{10}$ alkynylene group, $C_6$-$C_{10}$ arylene group, $C_3$-$C_{18}$ heterocyclylene group and $C_5$-$C_{10}$ heteroarylene group; and wherein the straight-chain alkylene group may have any one or more substituents selected from the group consisting of: $C_1$-$C_{10}$ alkyl, $C_6$-$C_{10}$ aryl, $C_5$-$C_{10}$ heteroaryl, $C_1$-$C_{10}$ haloalkyl, -$OC_1$-$C_{10}$ alkyl, -$OC_1$-$C_{10}$ alkylphenyl, -$C_1$-$C_{10}$ alkyl-OH, -$OC_1$-$C_{10}$ haloalkyl, -$SC_1$-$C_{10}$ alkyl, -$SC_1$-$C_{10}$ alkylphenyl, -$C_1$-$C_{10}$ alkyl-SH, -$SC_1$-$C_{10}$ haloalkyl, halogen substituent, -OH, -SH, -$NH_2$, -$C_1$-$C_{10}$ alkyl-$NH_2$, -N($C_1$-$C_{10}$ alkyl)($C_1$-$C_{10}$ alkyl ), -NH($C_1$-$C_{10}$ alkyl), -N($C_1$-$C_{10}$ alkyl)($C_1$-$C_{10}$ alkylphenyl), - NH($C_1$-$C_{10}$ alkylphenyl), cyano, nitro, -$CO_2$H, -C(O)O($C_1$-$C_{10}$ alkyl), -CON($C_1$-$C_{10}$ alkyl)($C_1$-$C_{10}$ alkyl), -CONH($C_1$-$C_{10}$ alkyl), -$CONH_2$, -NHC(O)($C_1$-$C_{10}$ alkyl), - NHC(O) (phenyl), -N($C_1$-$C_{10}$ alkyl)C(O)($C_1$-$C_{10}$ alkyl), -N($C_1$-$C_{10}$ alkyl)C(O)(phenyl), -C(O)$C_1$-$C_{10}$ alkyl, -C(O)$C_1$-$C_{10}$ alkylphenyl, -C(O)$C_1$-$C_{10}$ haloalkyl, -OC(O)$C_1$-$C_{10}$ alkyl, -$SO_2$($C_1$-$C_{10}$ alkyl), -$SO_2$(phenyl), -$SO_2$($C_1$-$C_{10}$ haloalkyl), -$SO_2NH_2$, - $SO_2$NH($C_1$-$C_{10}$ alkyl), -$SO_2$NH(phenyl), -$NHSO_2$($C_1$-$C_{10}$ alkyl), -$NHSO_2$(phenyl) and -$NHSO_2$($C_1$-$C_{10}$ haloalkyl);

each of the side chain moieties is independently a covalent bond, or a straight-chain alkylene group having a length of 1 to 70 carbon atoms, or one or more carbon atoms in the straight-chain alkylene group are replaced by one or more groups selected from the group consisting of: C(O), NH, O, S, CH=N, $S(O)_2$, $OP(O)_2$, $C_5$-$C_8$ glycosidylene group, $C_2$-$C_{10}$ alkenylene group, $C_2$-$C_{10}$ alkynylene group, $C_6$-$C_{10}$ arylene group, $C_3$-$C_{18}$ heterocyclylene group and $C_5$-$C_{10}$ heteroarylene group; and the straight-chain alkylene group may have any one or more substituents selected from the group consisting of: $C_1$-$C_{10}$ alkyl, $C_6$-$C_{10}$ aryl, $C_5$-$C_{10}$ heteroaryl, $C_1$-$C_{10}$ haloalkyl, -$OC_1$-$C_{10}$ alkyl, -$OC_1$-$C_{10}$ alkylphenyl, -$C_1$-$C_{10}$ alkyl-OH, -$OC_1$-$C_{10}$ haloalkyl, -$SC_1$-$C_{10}$ alkyl, -$SC_1$-$C_{10}$ alkylphenyl, -$C_1$-$C_{10}$ alkyl-SH, -$SC_1$-$C_{10}$ haloalkyl, halogen substituent, -OH, -SH, -$NH_2$, -$C_1$-$C_{10}$ alkyl-$NH_2$, -N($C_1$-$C_{10}$ alkyl)($C_1$-$C_{10}$ alkyl ), -NH($C_1$-$C_{10}$ alkyl), - N($C_1$-$C_{10}$ alkyl)($C_1$-$C_{10}$ alkylphenyl), -NH($C_1$-$C_{10}$ alkylphenyl), cyano, nitro, -$CO_2$H, - C(O)O($C_1$-$C_{10}$ alkyl), -CON($C_1$-$C_{10}$ alkyl)($C_1$-$C_{10}$ alkyl), -CONH($C_1$-$C_{10}$ alkyl), - $CONH_2$, -NHC(O)($C_1$-$C_{10}$ alkyl), -NHC(O)(phenyl), -N($C_1$-$C_{10}$ alkyl)C(O)($C_1$-$C_{10}$ alkyl), -N($C_1$-$C_{10}$ alkyl) C(O)(phenyl), -C(O)$C_1$-$C_{10}$ alkyl, -C(O)$C_1$-$C_{10}$ alkylphenyl, - C(O)$C_1$-$C_{10}$ haloalkyl, -OC(O)$C_1$-$C_{10}$ alkyl, -$SO_2$ ($C_1$-$C_{10}$ alkyl), -$SO_2$(phenyl), - $SO_2$($C_1$-$C_{10}$ haloalkyl), -$SO_2NH_2$, -$SO_2$NH($C_1$-$C_{10}$ alkyl), -$SO_2$NH(phenyl), - $NHSO_2$($C_1$-$C_{10}$ alkyl), -$NHSO_2$(phenyl) and -$NHSO_2$($C_1$-$C_{10}$ haloalkyl);

each of the conjugation linking moieties is independently a covalent bond, or any one following linking structure, or a connection combination of more than one following linking structures: $C_1$-$C_{10}$ straight-chain alkylene, phosphate ester bond, phosphorothioate bond, amide bond, ester bond, ether bond, disulfide bond, 1,2,3-triazolylene

group, a polyethylene glycol subunit, pyrrolidinylene group, 2-oxopyrrolidinylene group, phenylene, cyclohexylene, 2-succinimidylene group, 2-thiosuccinimidylene group, an amino acid subunit, a nucleotide subunit.

**26.** The conjugate according to claim 25, wherein each of the conjugation linking moieties in the linking group $R_j$ is respectively linked to the main chain moiety and one of the functional groups $A_0$; the number of the side chain moieties is $n_0$, and each of the side chain moieties is respectively linked to the main chain moiety and one of the $R_{AP}$ groups.

**27.** The conjugate according to claim 25 or 26, wherein all of the side chain moieties are linked to the same atom in the main chain moiety; or, each of the side chain moieties is linked to different atoms in the main chain moiety.

**28.** The conjugate according to claim 27, wherein $m_0$ is 1, and the linking group $R_j$ comprises a structure as shown in Formula (301):

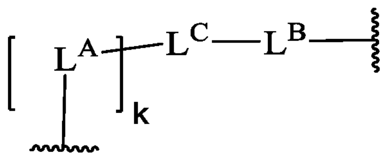

Formula (301),

wherein, k is an integer of 1 to 3; $L^C$ is the main chain moiety, $L^A$ is the side chain moiety, $L^B$ is the conjugation linking moiety, and - represents the site where a group is covalently linked;

the main chain moiety $L^C$ is a covalent bond or a 2- to 4-valence, straight-line or branched $C_1$-$C_{25}$ saturated hydrocarbyl, or one or more carbon atoms in the saturated hydrocarbyl are replaced by one or more gourps selected from the group consisting of: C(O), NH, O, S, CH=N, $S(O)_2$, $OP(O)_2$, $C_5$-$C_8$ glycosidylene group, $C_2$-$C_5$ alkenylene group, $C_2$-$C_5$ alkynylene group, $C_6$-$C_{10}$ arylene group, $C_3$-$C_8$ heterocyclylene group and $C_5$-$C_{10}$ heteroarylene group; wherein the saturated hydrocarbyl may have any one or more substituents selected from the group consisting of: $C_1$-$C_5$ alkyl, $C_6$-$C_{10}$ aryl, $C_5$-$C_{10}$ heteroaryl, -$OC_1$-$C_5$ alkyl, -$OC_1$-$C_5$ alkylphenyl, -$C_1$-$C_5$ alkyl-OH, -$SC_1$-$C_5$ alkyl, nitro, -C(O)O($C_1$-$C_5$ alkyl), -CON($C_1$-$C_5$ alkyl)($C_1$-$C_5$ alkyl), -CONH($C_1$-$C_5$ alkyl), -$CONH_2$, -NHC(O)($C_1$-$C_5$ alkyl), - NHC(O)(phenyl), -N($C_1$-$C_5$ alkyl)C(O)($C_1$-$C_5$ alkyl), -N($C_1$-$C_5$ alkyl)C(O)(phenyl), - C(O)$C_1$-$C_5$ alkyl, -C(O)$C_1$-$C_5$ alkylphenyl, -OC(O)$C_1$-$C_5$ alkyl, -$SO_2$($C_1$-$C_5$ alkyl), - $SO_2$(phenyl), -$SO_2NH_2$, -$SO_2NH$($C_1$-$C_5$ alkyl), -$SO_2NH$(phenyl), -$NHSO_2$($C_1$-$C_5$ alkyl) and -$NHSO_2$(phenyl);

each of the side chain moieties is independently a covalent bond, or a straight-chain alkylene group having a length of 1 to 70 carbon atoms, or one or more carbon atoms in the straight-chain alkylene group are replaced by one or more groups selected from the group consisting of: C(O), NH, O, S, CH=N, $S(O)_2$, $OP(O)_2$, $C_5$-$C_8$ glycosidylene group, $C_2$-$C_{10}$ alkenylene group, $C_2$-$C_{10}$ alkynylene group, $C_6$-$C_{10}$ arylene group, $C_3$-$C_{18}$ heterocyclylene group and $C_5$-$C_{10}$ heteroarylene group; and the straight-chain alkylene group may have any one or more substituents selected from the group consisting of: $C_1$-$C_{10}$ alkyl, $C_6$-$C_{10}$ aryl, $C_5$-$C_{10}$ heteroaryl, $C_1$-$C_{10}$ haloalkyl, -$OC_1$-$C_{10}$ alkyl, -$OC_1$-$C_{10}$ alkylphenyl, -$C_1$-$C_{10}$ alkyl-OH, -$OC_1$-$C_{10}$ haloalkyl, -$SC_1$-$C_{10}$ alkyl, -$SC_1$-$C_{10}$ alkylphenyl, -$C_1$-$C_{10}$ alkyl-SH, -$SC_1$-$C_{10}$ haloalkyl, halogen substituent, -OH, -SH, -$NH_2$, -$C_1$-$C_{10}$ alkyl-$NH_2$, -N($C_1$-$C_{10}$ alkyl)($C_1$-$C_{10}$ alkyl ), -NH($C_1$-$C_{10}$ alkyl), - N($C_1$-$C_{10}$ alkyl)($C_1$-$C_{10}$ alkylphenyl), -NH($C_1$-$C_{10}$ alkylphenyl), cyano, nitro, -$CO_2H$, - C(O)O($C_1$-$C_{10}$ alkyl), -CON($C_1$-$C_{10}$ alkyl)($C_1$-$C_{10}$ alkyl), -CONH($C_1$-$C_{10}$ alkyl), - $CONH_2$, -NHC(O)($C_1$-$C_{10}$ alkyl), -NHC(O)(phenyl), -N($C_1$-$C_{10}$ alkyl)C(O)($C_1$-$C_{10}$ alkyl), -N($C_1$-$C_{10}$ alkyl)C(O)(phenyl), -C(O)$C_1$-$C_{10}$ alkyl, -C(O)$C_1$-$C_{10}$ alkylphenyl, - C(O)$C_1$-$C_{10}$ haloalkyl, -OC(O)$C_1$-$C_{10}$ alkyl, -$SO_2$($C_1$-$C_{10}$ alkyl), -$SO_2$(phenyl), - $SO_2$($C_1$-$C_{10}$ haloalkyl), -$SO_2NH_2$, -$SO_2NH$($C_1$-$C_{10}$ alkyl), -$SO_2NH$(phenyl), -$NHSO_2$($C_1$-$C_{10}$ alkyl), -$NHSO_2$(phenyl) and -$NHSO_2$($C_1$-$C_{10}$ haloalkyl);

each of the conjugation linking moieties is independently a covalent bond, or any one following linking structure, or a connection combination of more than one following linking structures: $C_1$-$C_{10}$ straight-chain alkylene, phosphate ester bond, phosphorothioate bond, amide bond, ester bond, ether bond, disulfide bond, 1,2,3-triazolylene group, a polyethylene glycol subunit, pyrrolidinylene group, 2-oxopyrrolidinylene group, phenylene, cyclohexylene, 2-succinimidylene group, 2-thiosuccinimidylene group, an amino acid subunit, a nucleotide subunit.

**29.** The conjugate according to claim 28, wherein the conjugate has a structure as shown in Formula (305):

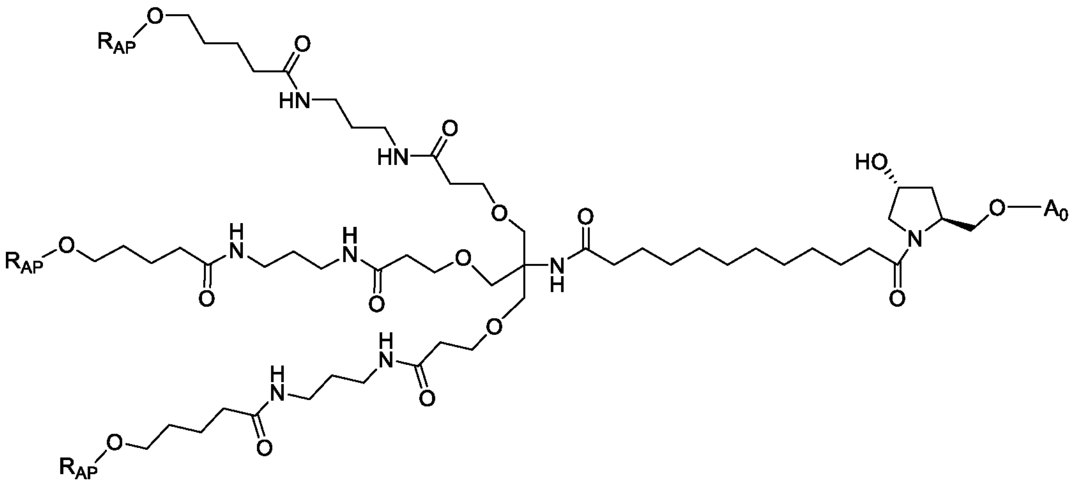

Formula (305).

**30.** The conjugate according to claim 28, wherein the linking group $R_j$ has a structure shown in Formula (306):

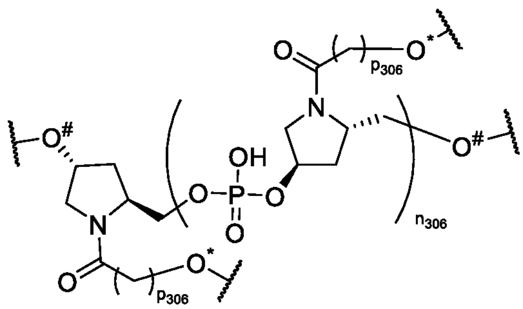

Formula (306),

wherein, $n_{306}$ is an integer of 0-3, each $p_{306}$ is independently an integer of 1 to 6, ∿∿∿ represents the site where a group is covalently linked; the oxygen atom marked by * forms a phosphate ester bond, an ether bond or an ester bond linkage with the $R_{AP}$ group; at least one of the oxygen atoms marked by # forms an ether bond, an ester bond or a phosphate ester bond linkage with the functional group $A_0$, and the remaining oxygen atom marked by # is linked to a hydrogen atom to form a hydroxyl group, or is linked to a $C_1$-$C_3$ alkyl groups to form a $C_1$-$C_3$ alkoxy groups.

**31.** The conjugate according to claim 30, wherein the conjugate has a structure as shown in Formula (307a), (307b) or (307c):

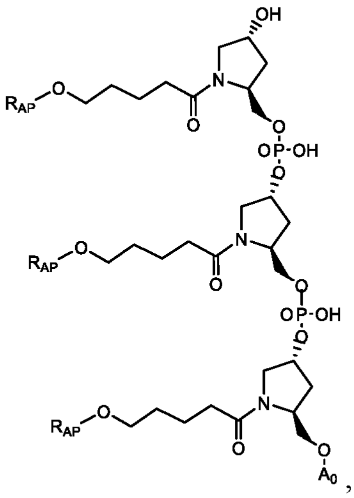

Formula (307a)

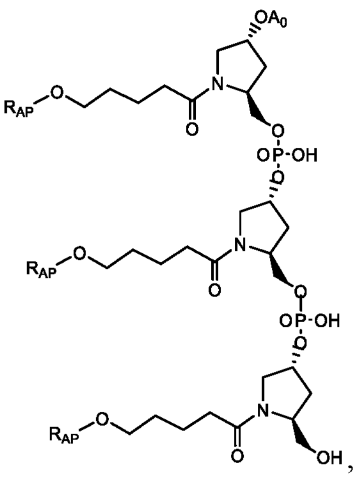

Formula (307b)

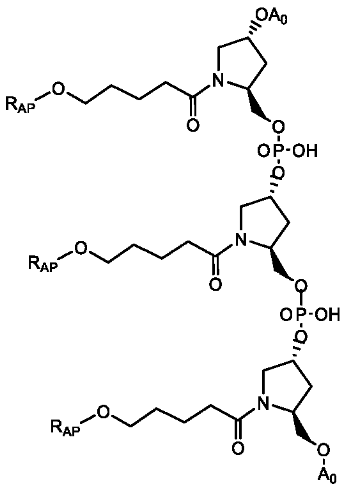

Formula (307c).

**32.** The conjugate according to claim 28, wherein the conjugate has a structure as shown in Formula (308):

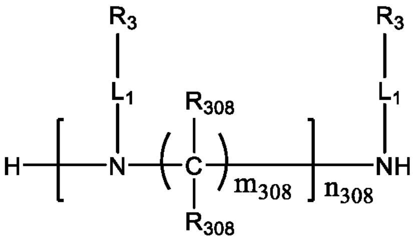

Formula (308),

wherein, $n_{308}$ is an integer selected from 1 to 10;
each $m_{308}$ is independently an integer selected from 2 to 10;
each $R_{308}$ is independently H, $C_1$-$C_{10}$ alkyl, $C_1$-$C_{10}$ haloalkyl or $C_1$-$C_{10}$ alkoxy;
each $R_3$ is independently the functional group $A_0$, or is the $R_{AP}$ group, and at least one $R_3$ is the functional group $A_0$, and at least one $R_3$ is the $R_{AP}$ group; or, one $R_3$ is the functional group $A_0$, and the remaining $R_3$ are the $R_{AP}$ groups;
each $L_1$ linked to the functional group $A_0$ represents the conjugation linking moiety, and each $L_1$ linked to the $R_{AP}$ represents the side chain moiety.

**33.** The conjugate according to claim 32, wherein each $L_1$ is independently selected from the group consisting of groups L4-L23 and any connection combination thereof.

**34.** The conjugate according to claim 33, wherein each $L_1$ is independently selected from the group consisting of connection combinations of at least two of the groups L4-L9, L13, L14, and L18; or, each $L_1$ is independently a connection combination of at least two of the groups L4, L5, L7, L9, L13, L14, and L18.

**35.** The conjugate according to any one of claims 32 to 34, wherein the length of each $L_1$ is independently 3-25 atoms; or, the length of each $L_1$ is independently 4-15 atoms.

**36.** The conjugate according to any one of claims 32 to 35, wherein $n_{308}$ is an integer of 2 to 6, 2 to 4 $R_3$ are the $R_{AP}$ groups, and the remaining $R_3$ are the functional groups.

**37.** The conjugate according to any one of claims 32 to 36, wherein each $m_{308}$ is independently of one another an integer of 2 to 5, and/or each $m_{308}$ is equal.

**38.** The conjugate according to any one of claims 32-37, wherein $n_{308}$ is an integer selected 2 to 4; each $m_{308}$ is independently an integer selected from 2 to 4; and each $R_{308}$ is H.

**39.** The conjugate according to claim 38, wherein one $R_3$ is the functional group $A_0$ and the remaining $R_3$ are the $R_{AP}$ groups.

**40.** The conjugate according to claim 38 or 39, wherein each $L_1$ comprises both an attachment site linked to the N atom of the nitrogen-containing backbone and an attachment site linked to the functional group $A_0$ or the $R_{AP}$ group, and the site linked to the N atom of the nitrogen-containing backbone forms an amide bond to the N atom; or, one or more $L_1$ are selected from B5, B6, B5' or B6':

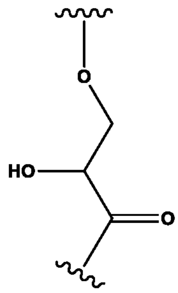
,
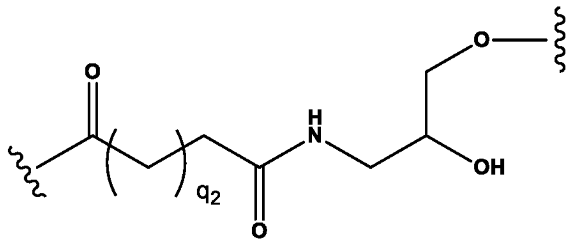
;

(B5) (B6)

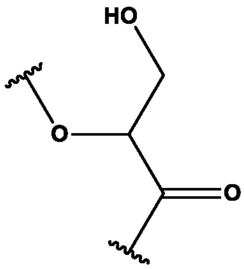
,
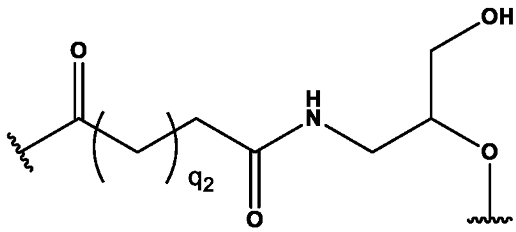
;

(B5') (B6')

wherein,  represents the site where a group is covalently linked, $q_2$ is an integer of 1 to 10; or, $q_2$ is an integer of 1 to 5.

**41.** The conjugate according to any one of claims 32-40, wherein the conjugate has a structure as shown in Formula (403), (404), (405), (406), (407), (408), (409), (410), (411), (412), (413), (414), (415), (416), (417), (418), (419), (420), (421), (422), (423), (424), (425), (426) or (427):

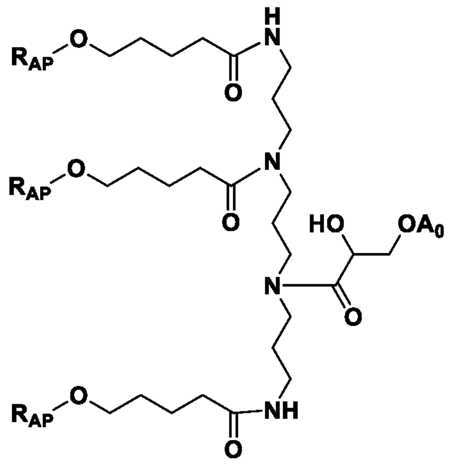

Formula (403)

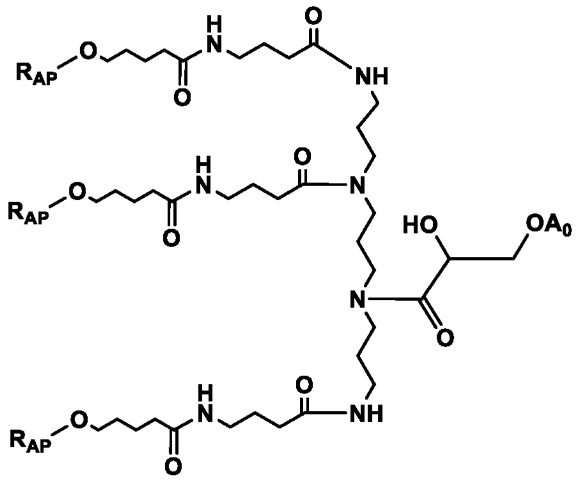

Formula (404)

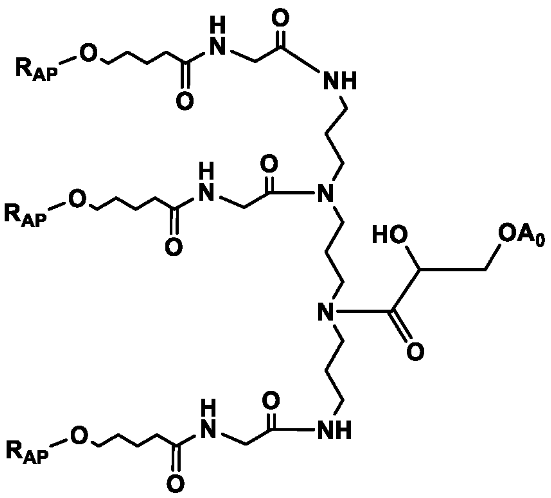

Formula (405)

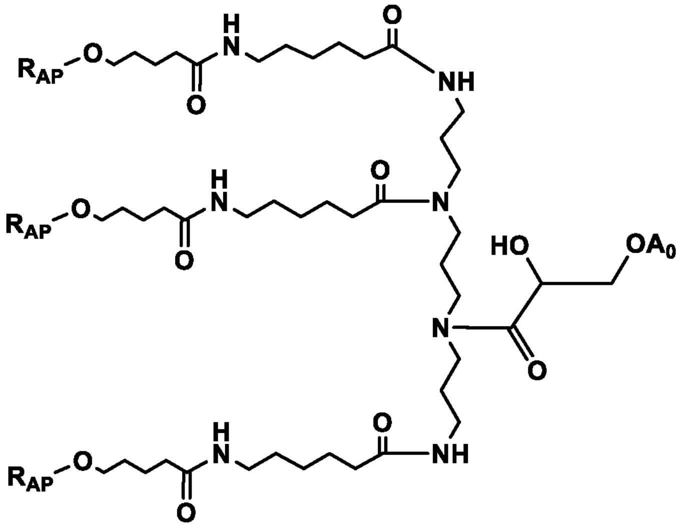

Formula (406)

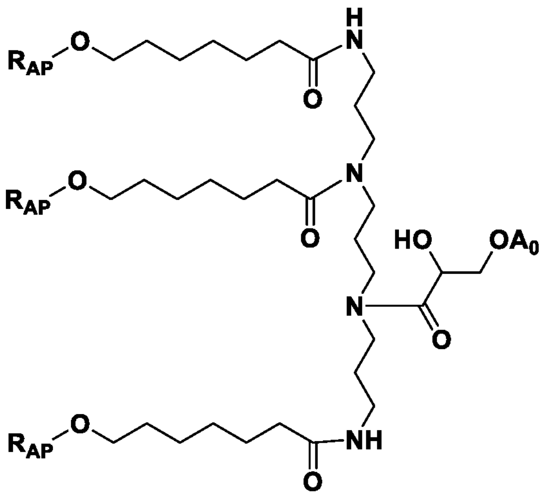

Formula (407)

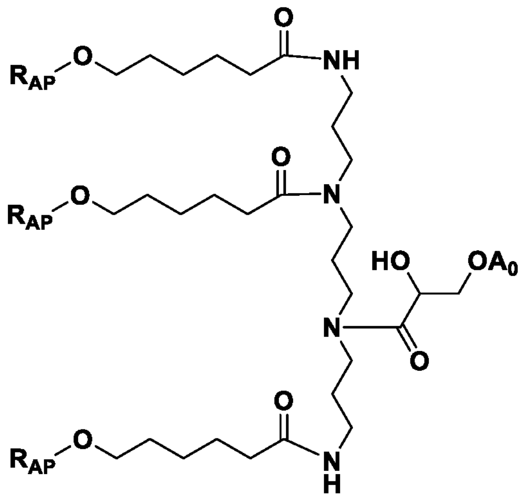

Formula (408)

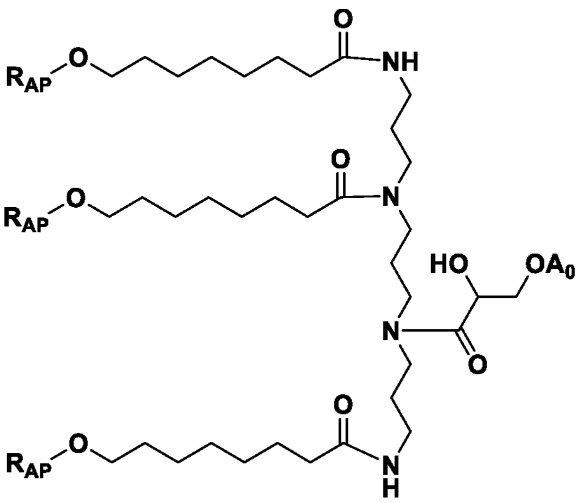

Formula (409)

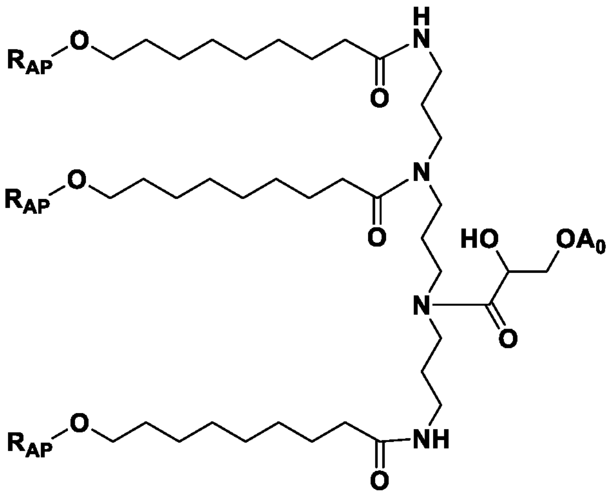

Formula (410)

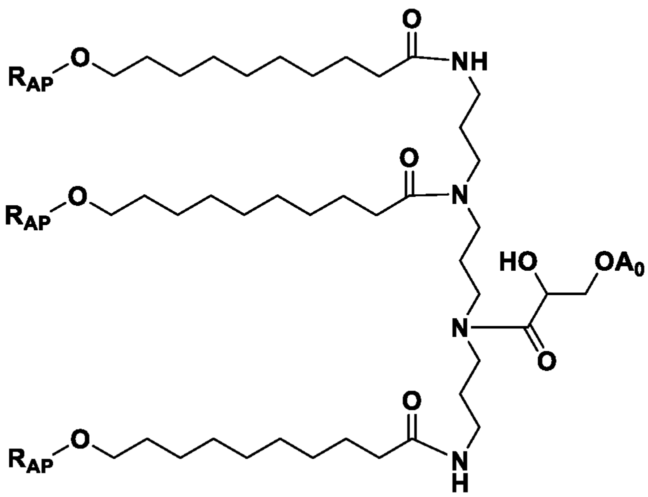

Formula (411)

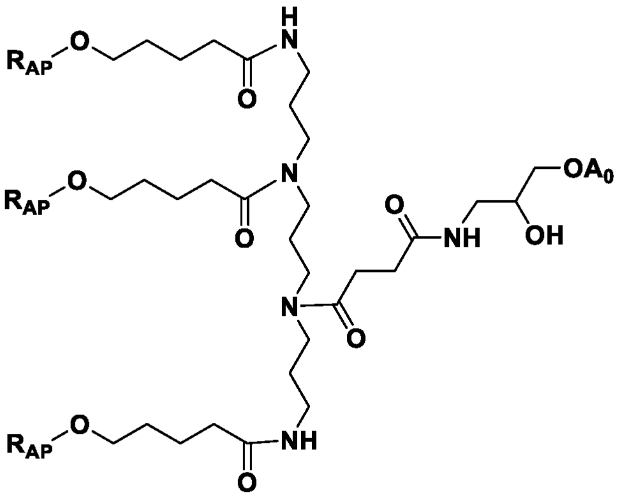

Formula (412)

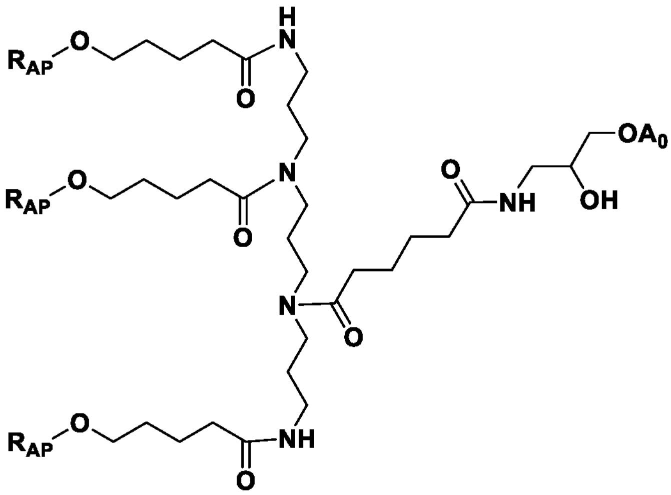

Formula (413)

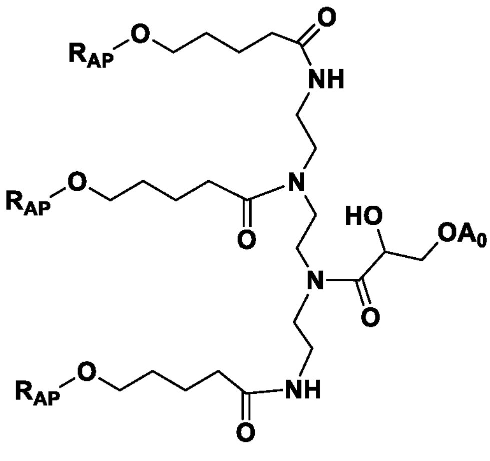

Formula (414)

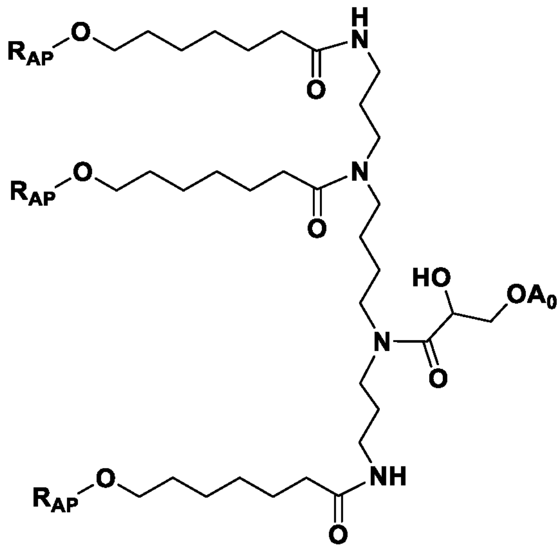

Formula (415)

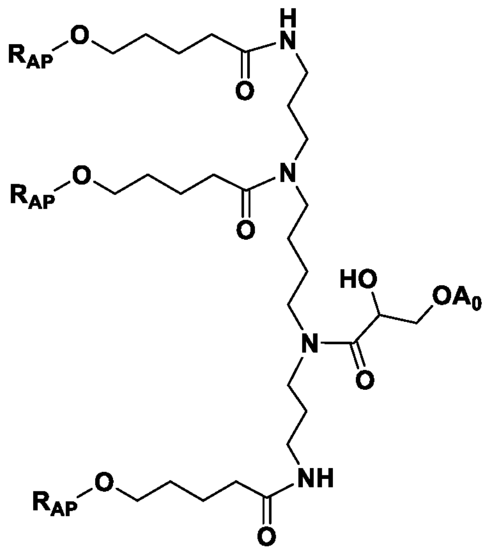

Formula (416)

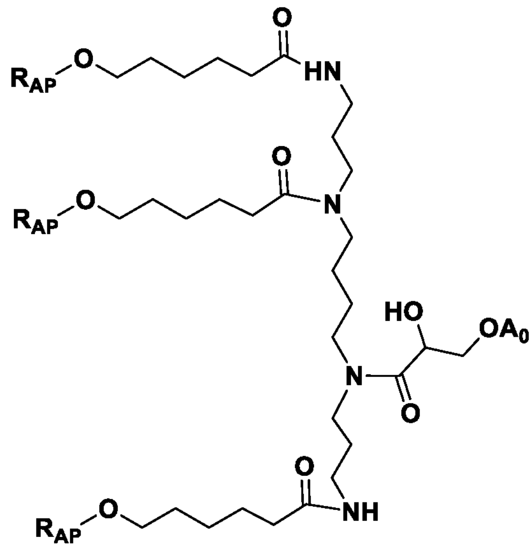

Formula (417)

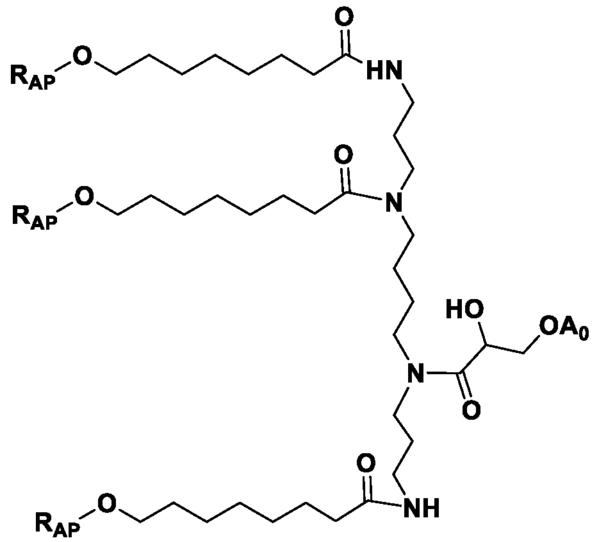

Formula (418)

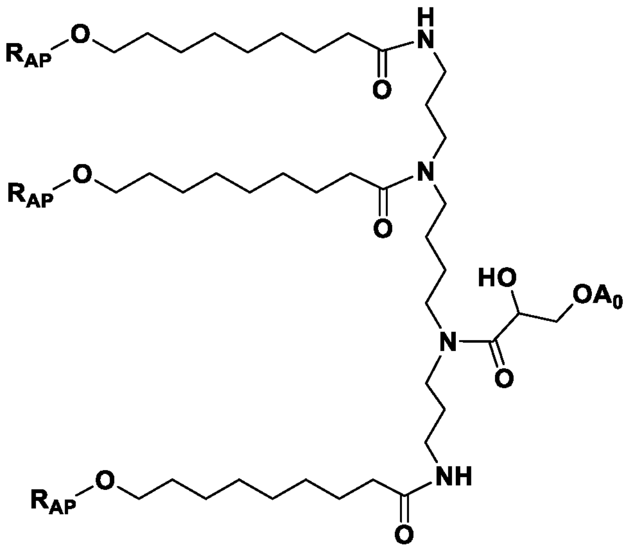

Formula (419)

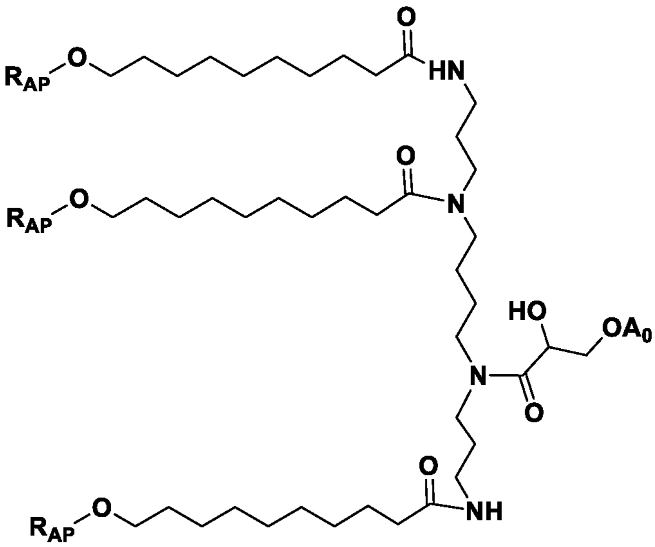

Formula (420)

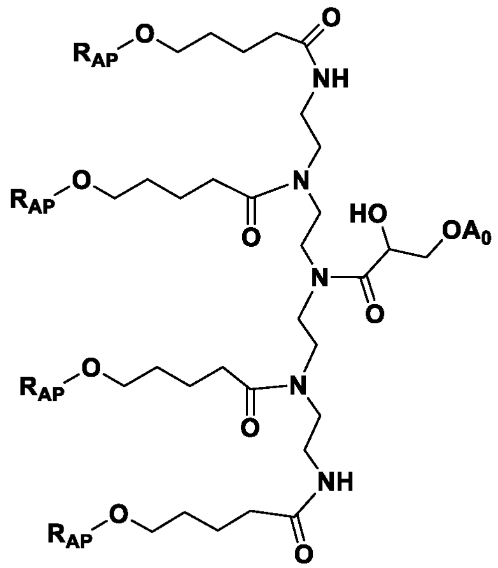

Formula (421)

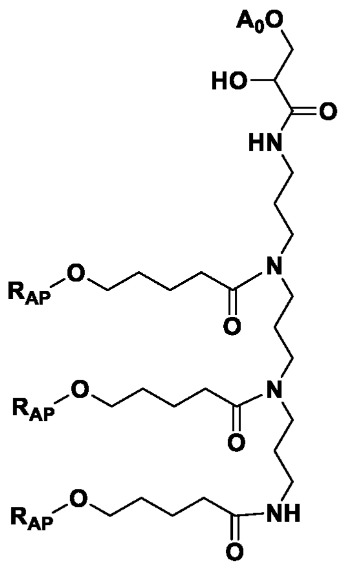

Formula (422)

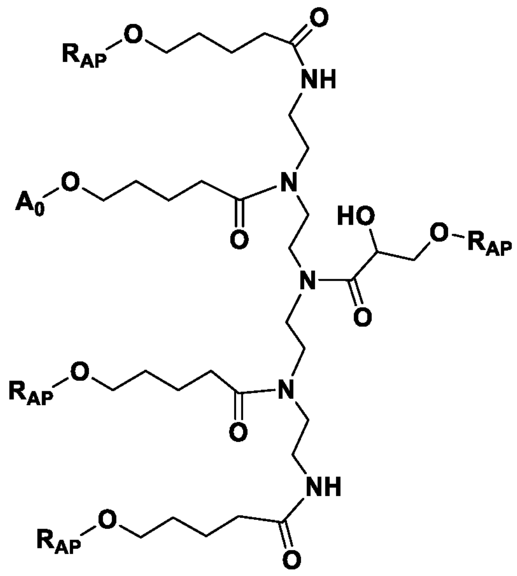

Formula (423)

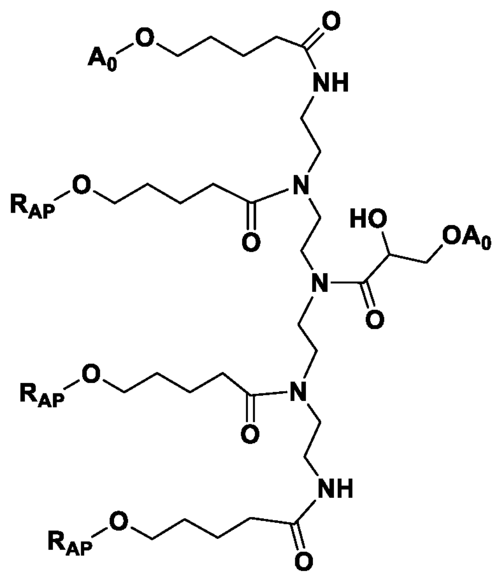

Formula (424)

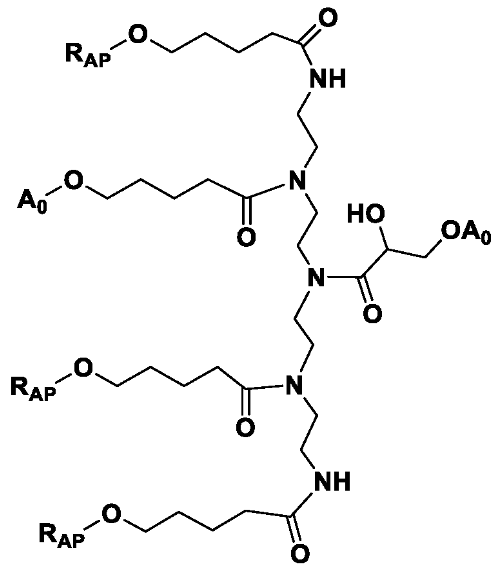

Formula (425)

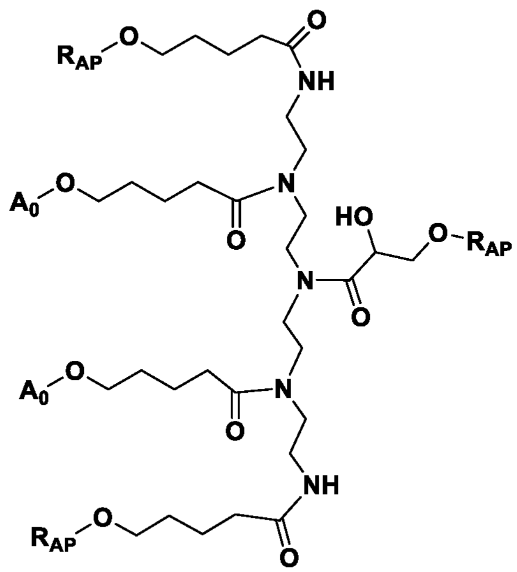

Formula (426)

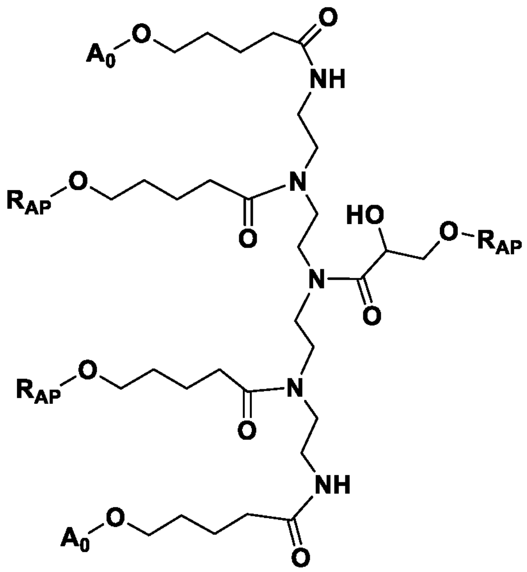

Formula (427)

**42.** The conjugate according to any one of claims 19 to 23, wherein $R_j$ comprises a nucleotide sequence I and a nucleotide sequence II, the nucleotide sequence I and the nucleotide sequence II each comprise 5 to 25 modified or unmodified nucleotides, the nucleotide sequence I and the nucleotide sequence II are at least partially reverse complementary, the delivery group is linked to the nucleotide sequence I, the functional group is linked to the nucleotide sequence II, and the nucleotide sequence I and the nucleotide sequence II do not elicit an immune response or a toxic reaction in a subject.

**43.** The conjugate according to claim 42, wherein the 3' terminal of the delivery group is linked to the 5' position of the ribose of the 5' terminal nucleotide of the nucleotide sequence I via a phosphate ester bond, and the functional group is linked to the 5' position of the ribose of the 5' terminal nucleotide of the nucleotide sequence II; or, the functional group comprises a segment of nucleotide sequence, and the 3' terminal of the nucleotide sequence is linked to the 5' position of the ribose of the 5' terminal nucleotide of the nucleotide sequence II via a phosphate ester bond.

**44.** The conjugate according to claim 43, wherein the nucleotide sequence I and the nucleotide sequence II are substantially reverse complementary or completely reverse complementary; or, the nucleotide sequence I and the nucleotide sequence II are equal in length and are both 10-20 modified or unmodified nucleotides; or, the nucleotide sequence I and the nucleotide sequence II both consist of 17 nucleotides and are completely reverse complementary; or, the nucleotide sequence I and the nucleotide sequence II have the sequences shown in SEQ ID NO: 40 and SEQ ID NO: 41, respectively:

5'-GUACAUUCUAGUAAGCC-3' (SEQ ID NO: 40)
5'-GGCUAUCUAGAAUGUAC-3' (SEQ ID NO: 41),

or, the nucleotide sequence I and the nucleotide sequence II have the sequences shown in SEQ ID NO: 42 and SEQ ID NO: 43, respectively:

5'- GmUfAmCfAmUfUfCfUfAmGmAmUfAmGmCfCf -3' (SEQ ID NO: 42)
5'- GmGmCfUfAmUfCfUfAmGmAmAmUfGmUfAmCf -3' (SEQ ID NO: 43).

**45.** The conjugate according to any one of claims 19-44, wherein $R_j$ is cleavable.

**46.** The conjugate according to any one of claims 18 to 45, wherein at least one or all of the functional groups are diagnostic agent groups, and each of the diagnostic agent groups is independently selected from a contrast agent group or a fluorescent imaging group.

**47.** The conjugate according to any one of claims 18 to 45, wherein at least one or all of the functional groups are small molecule therapeutic agent groups having a therapeutic effect on tumors, and each of the small molecule therapeutic

agent groups is independently selected from a cytotoxin group, an antibiotic group, an angiogenesis inhibitor or an antibody drug group.

**48.** The conjugate according to any one of claims 18 to 45, wherein at least one or all of the functional groups are functional oligonucleotide groups, and the functional oligonucleotide groups are capable of regulating the expression level of a cancer-related gene in tumor cells.

**49.** The conjugate according to any one of claims 18 to 48, wherein at least one of the functional groups is a delivery auxiliary group, and the delivery auxiliary group is selected from one or more of $C_{10}$-$C_{30}$ hydrocarbyl, cholesteryl group, and phospholipid group.

**50.** A pharmaceutical composition comprising the conjugate according to any one of claims 18 to 49 and a pharmaceutically acceptable carrier.

**51.** Use of the aptamer according to any one of claims 1 to 17 and/or the conjugate according to any one of claims 18 to 49 and/or the pharmaceutical composition according to claim 50 in the manufacture of a medicament for diagnosing and/or treating tumors and tumor-related diseases or symptoms.

**52.** A method for diagnosing and/or treating tumors and tumor-related diseases or symptoms, comprising administering an effective amount of the conjugate according to any one of claims 18 to 49 and/or the pharmaceutical composition according to claim 50 to a subject in need thereof.

**53.** A kit comprising the conjugate according to any one of claims 18 to 49 and/or the pharmaceutical composition according to claim 50.

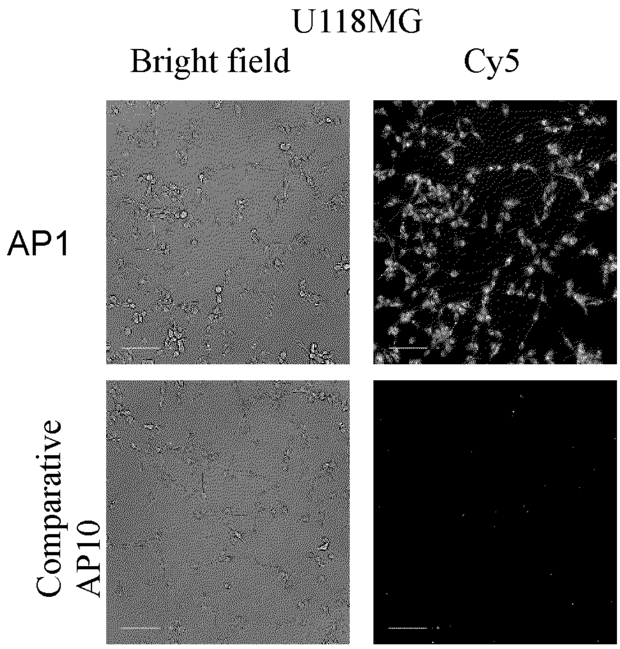


Figure 1A

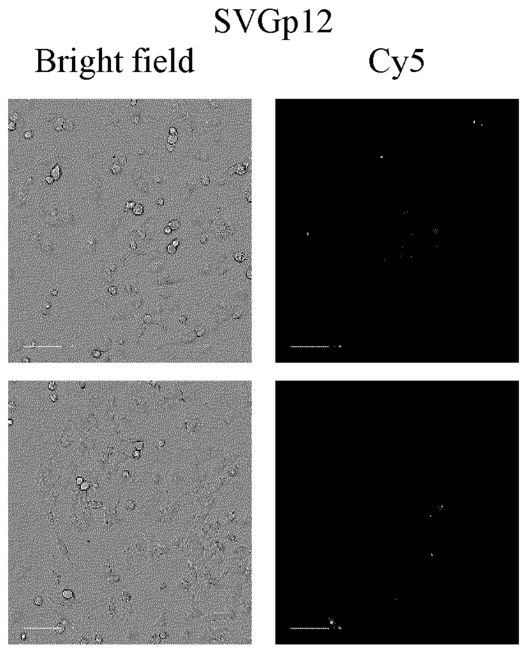


Figure 1B

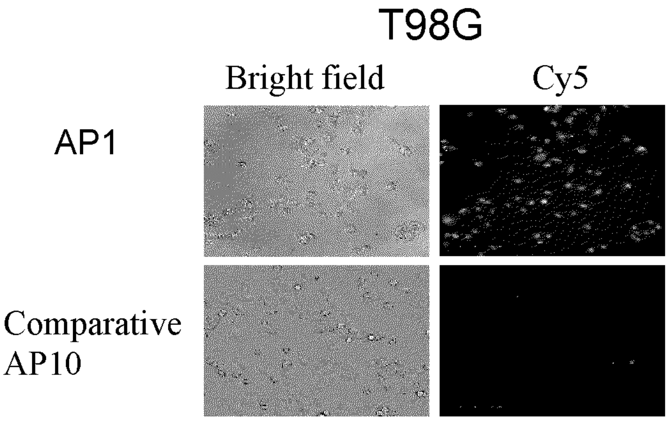


Figure 1C

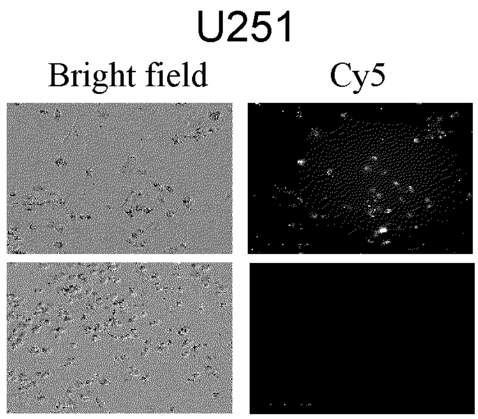


Figure 1D

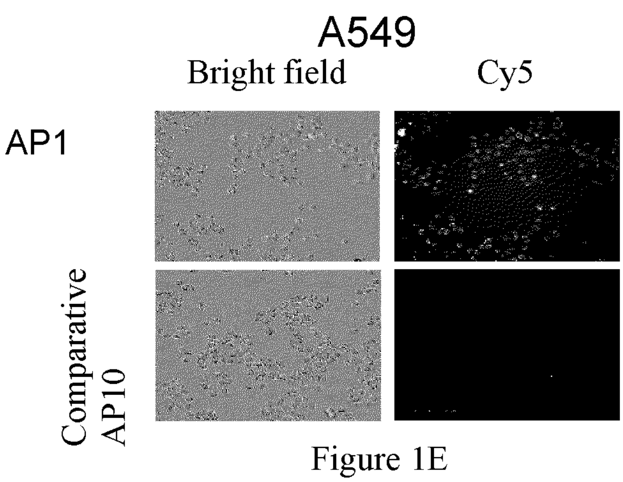


Figure 1E

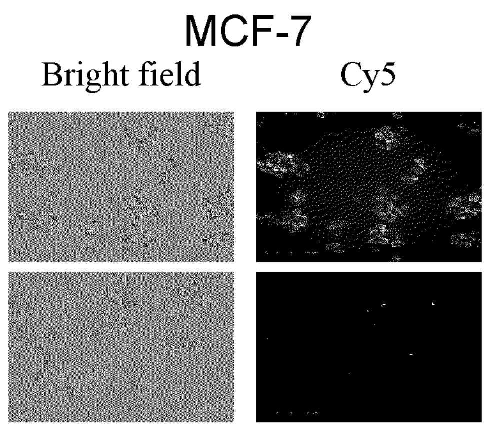


Figure 1F

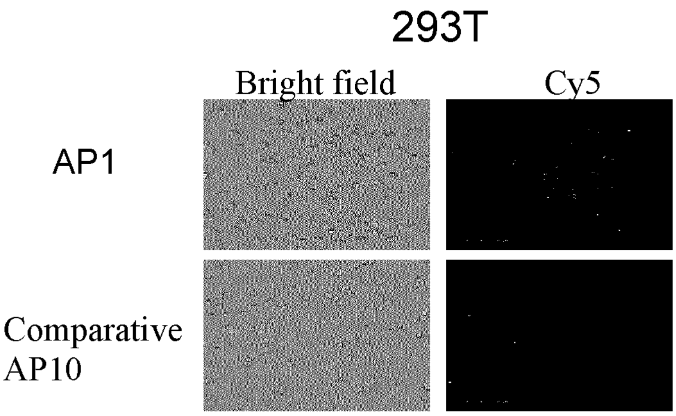


Figure 1G

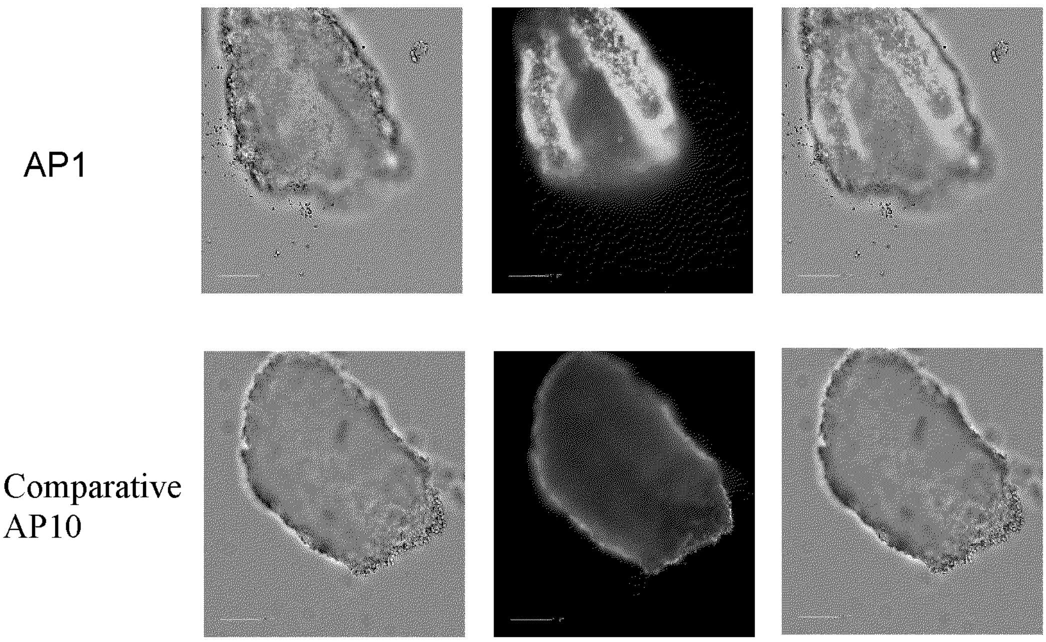


Figure 2A

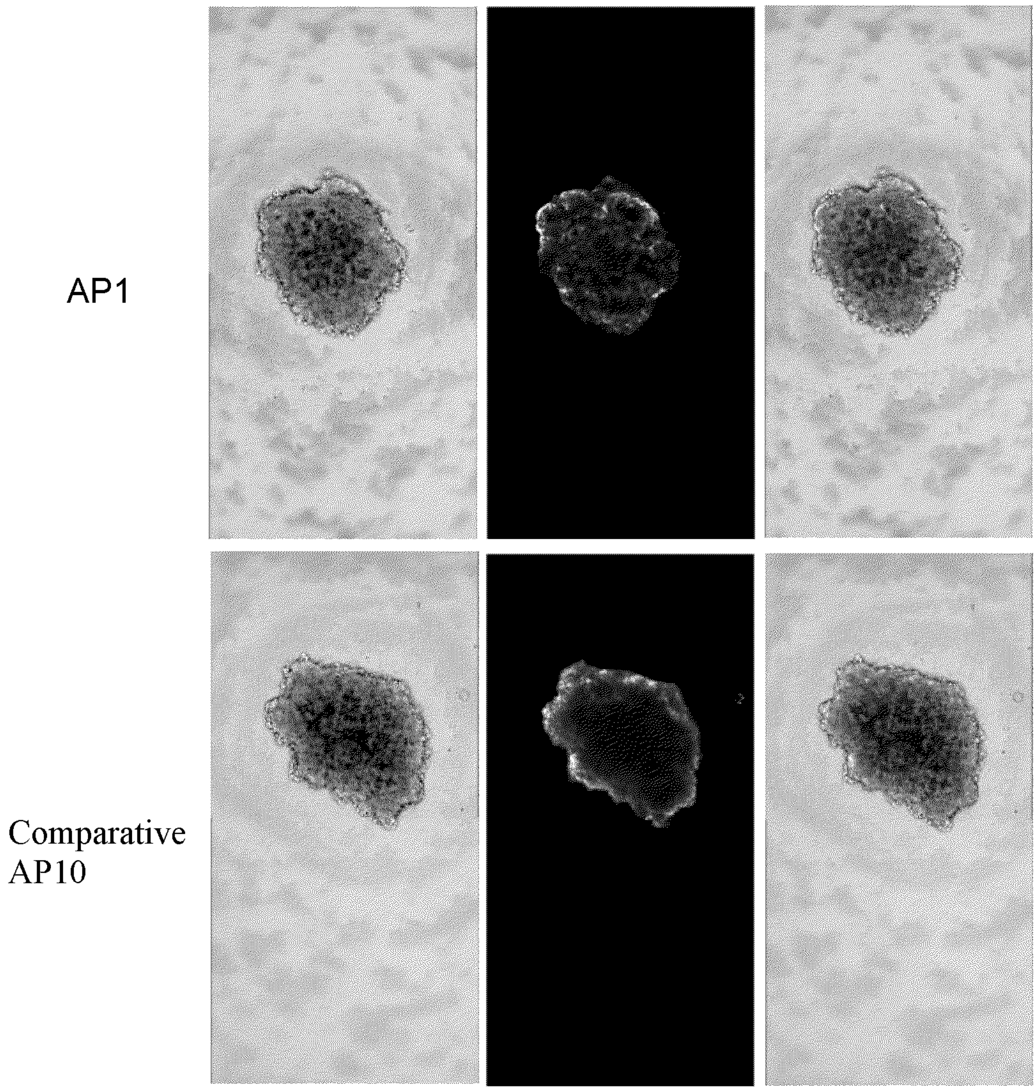


Figure 2B

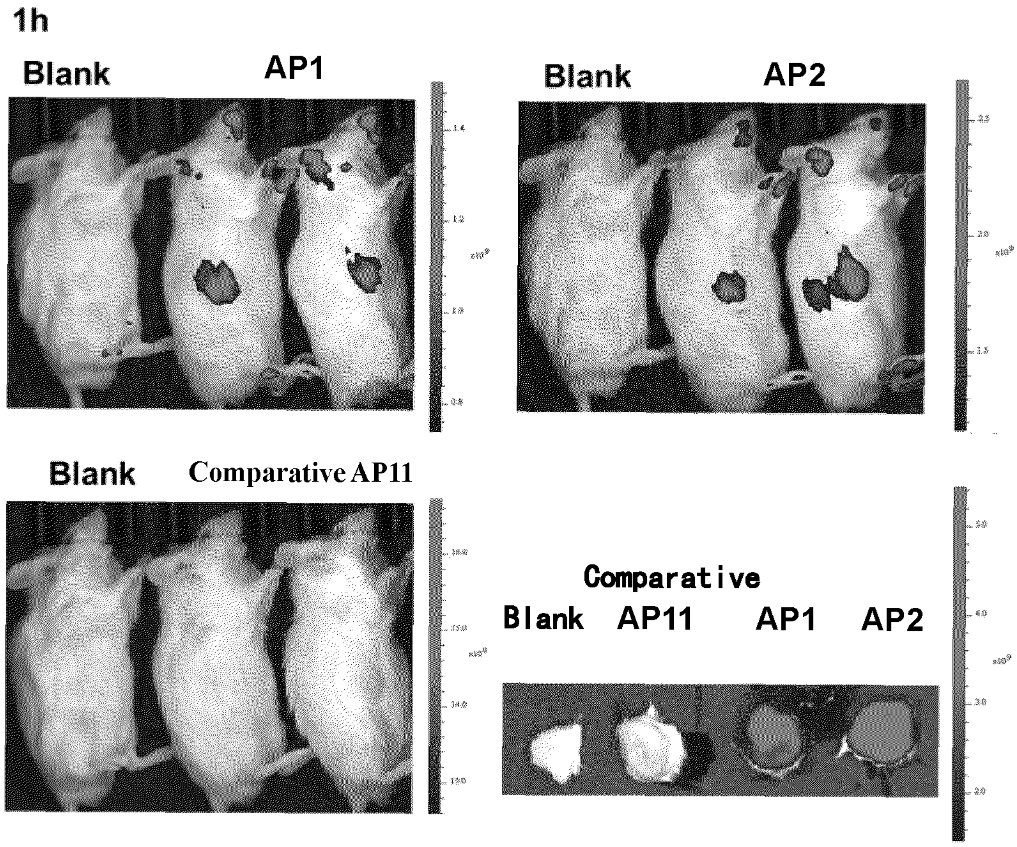


Figure 3A

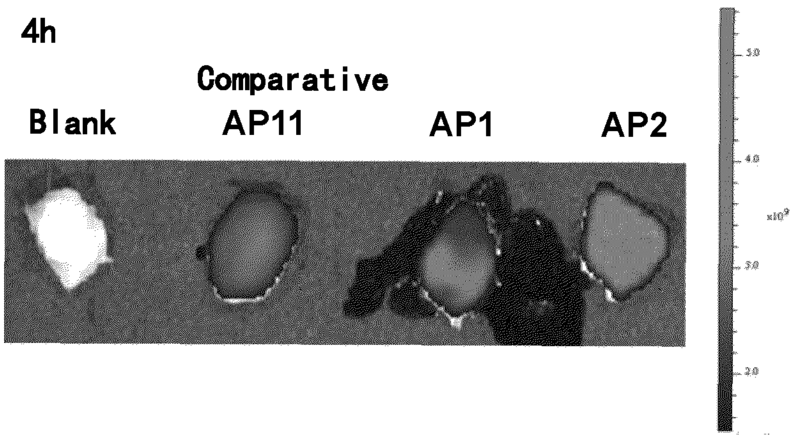


Figure 3B

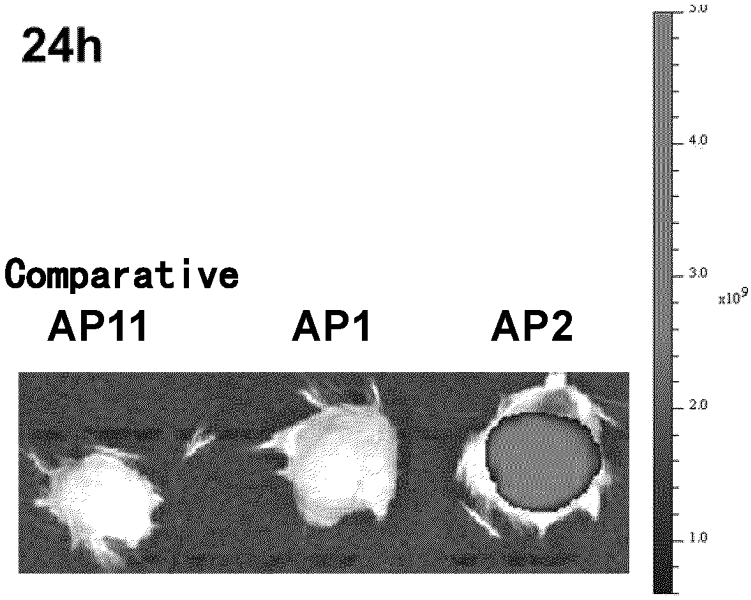


Figure 3C

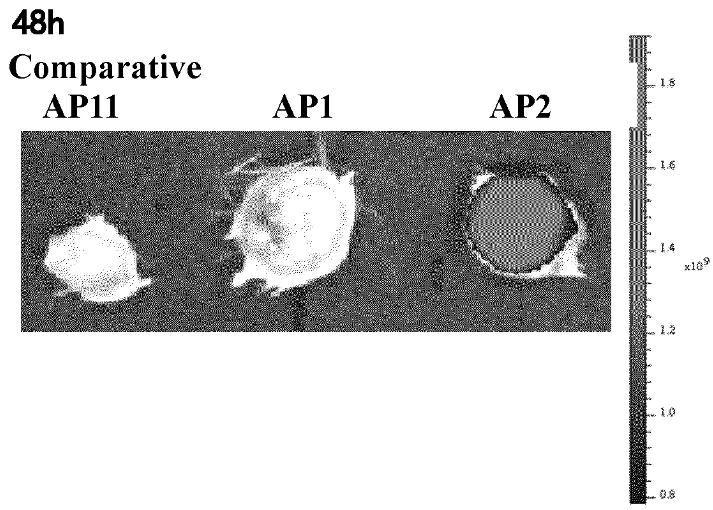


Figure 3D

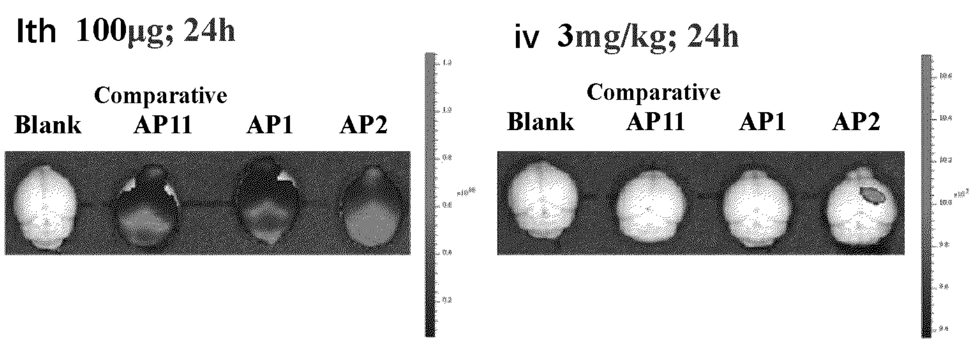


Figure 4A

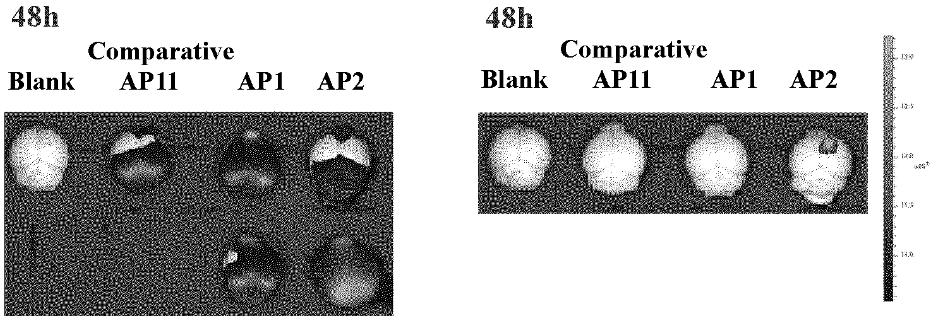


Figure 4B

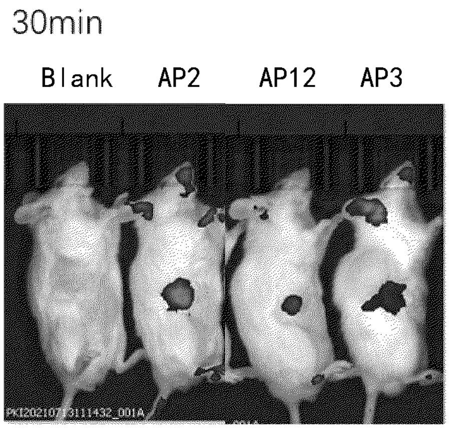


Figure 5A

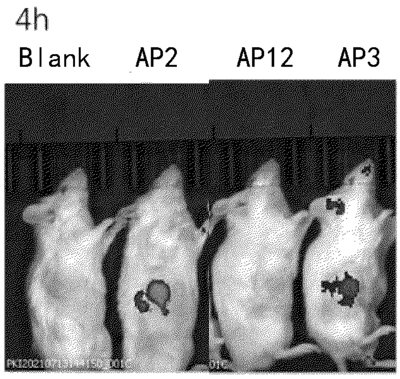


Figure 5B

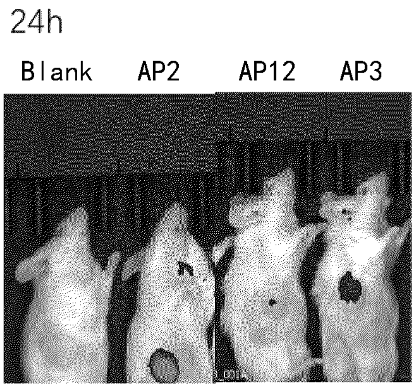


Figure 5C

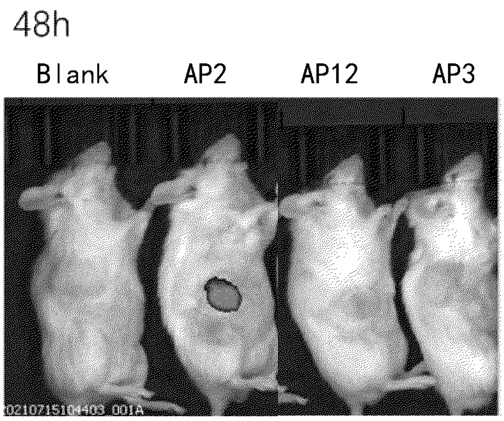


Figure 5D

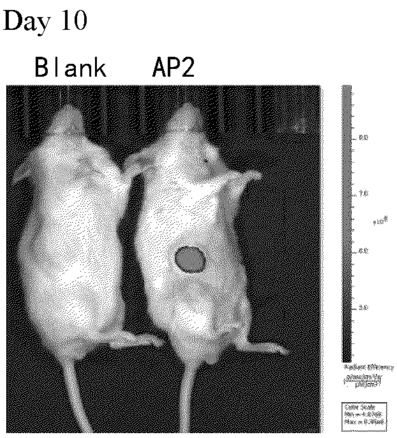


Figure 5E

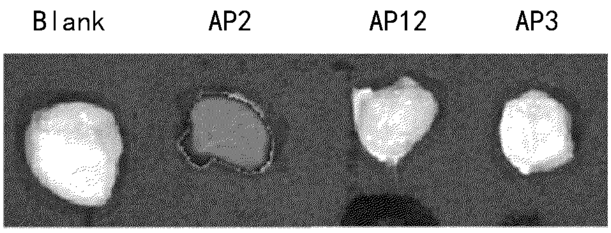


Figure 5F

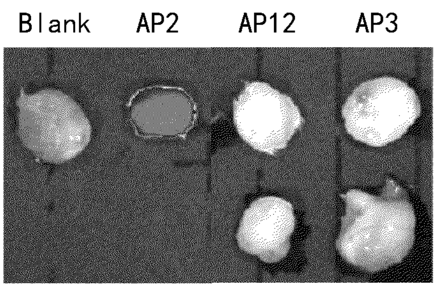


Figure 5G

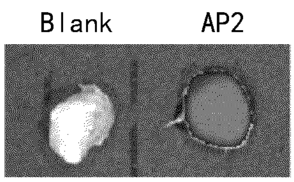


Figure 5H

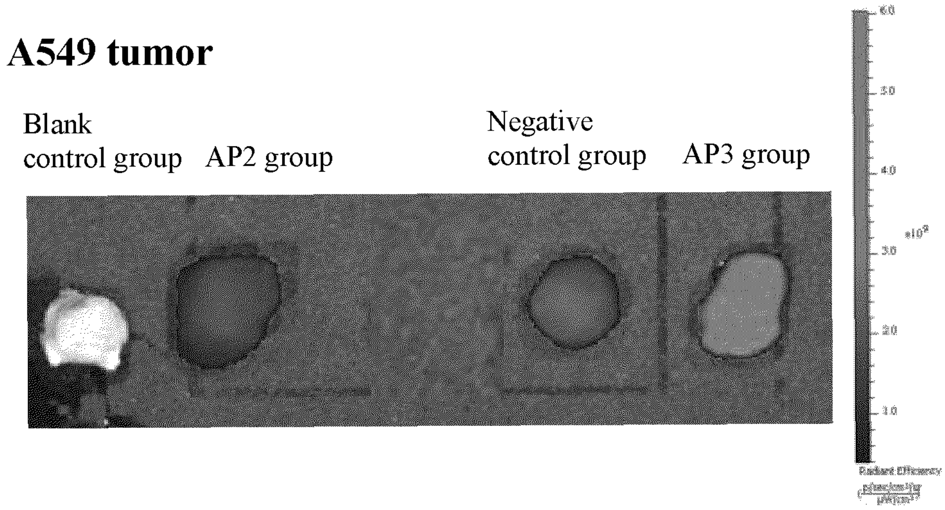


Figure 6

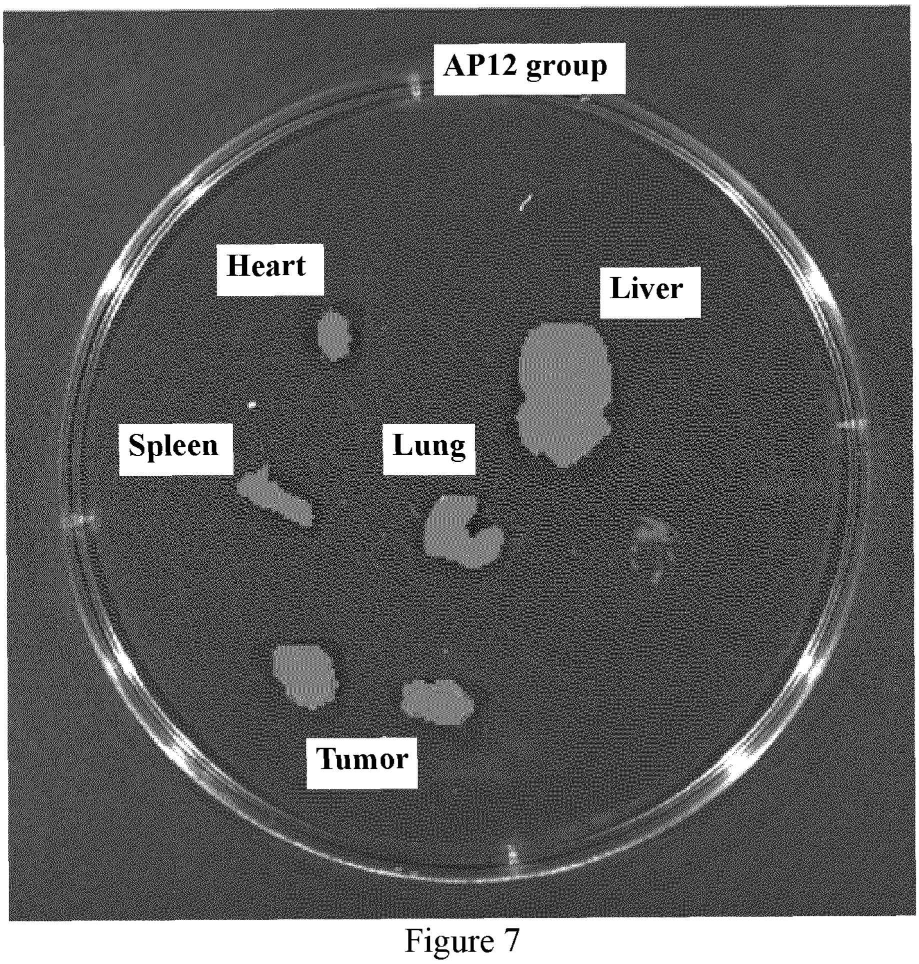


Figure 7

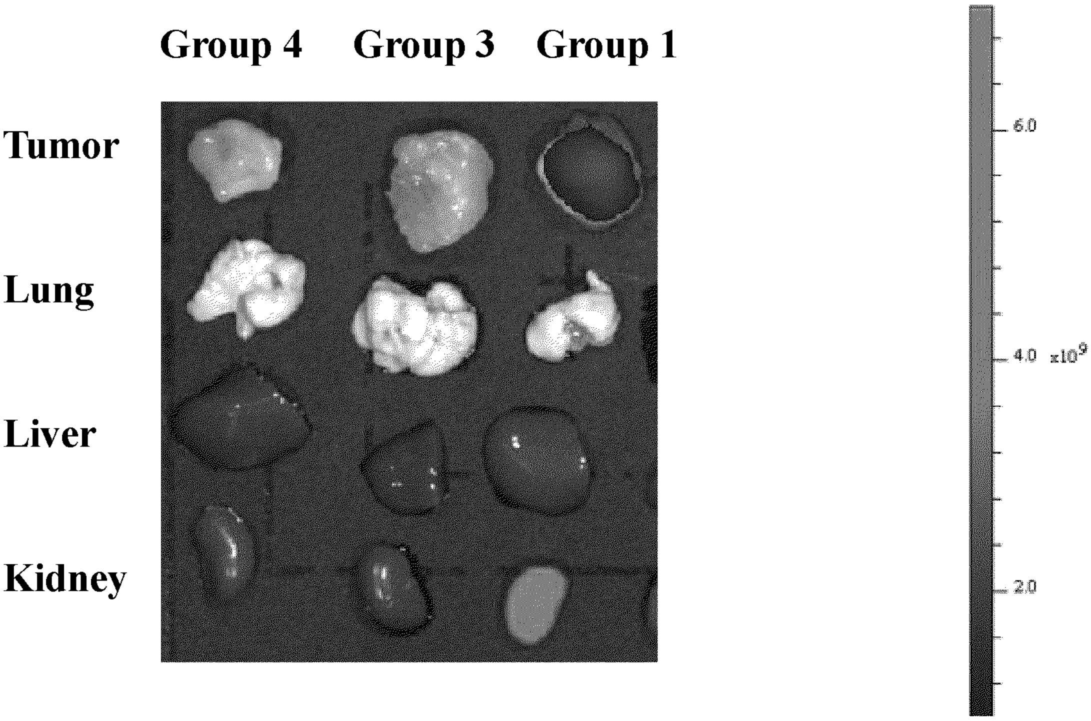


Figure 8A

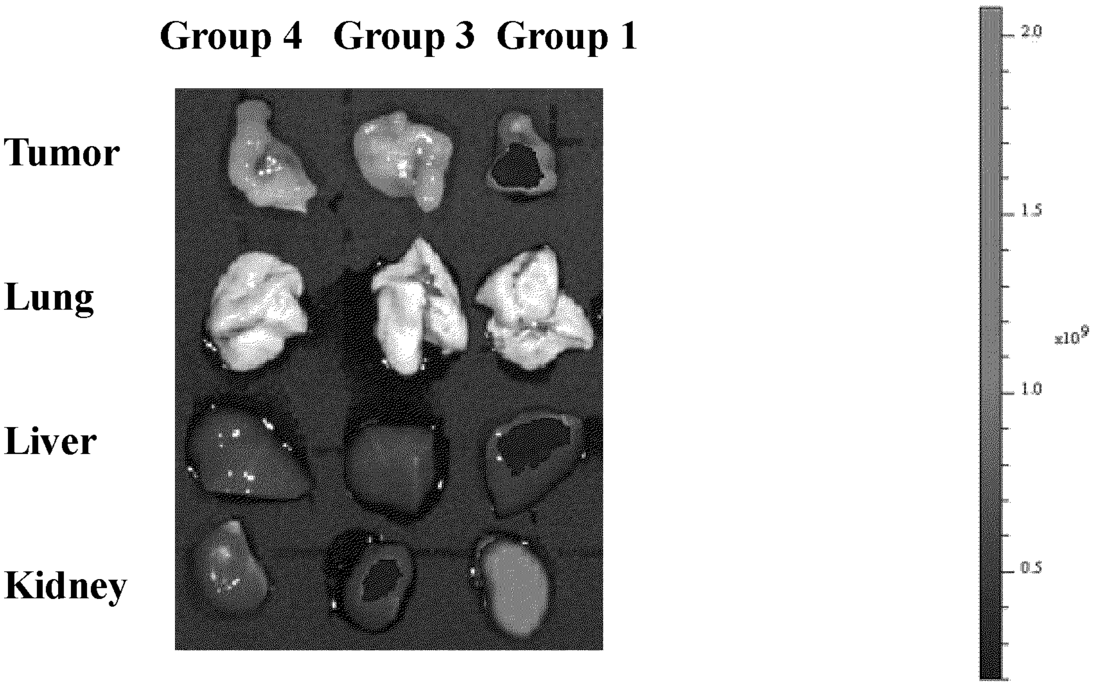


Figure 8B

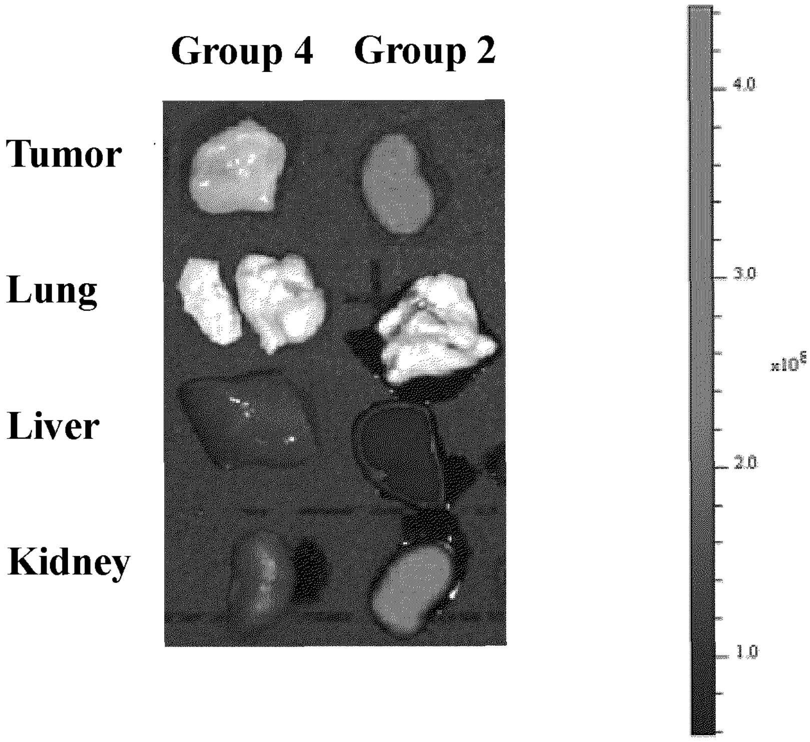


Figure 8C

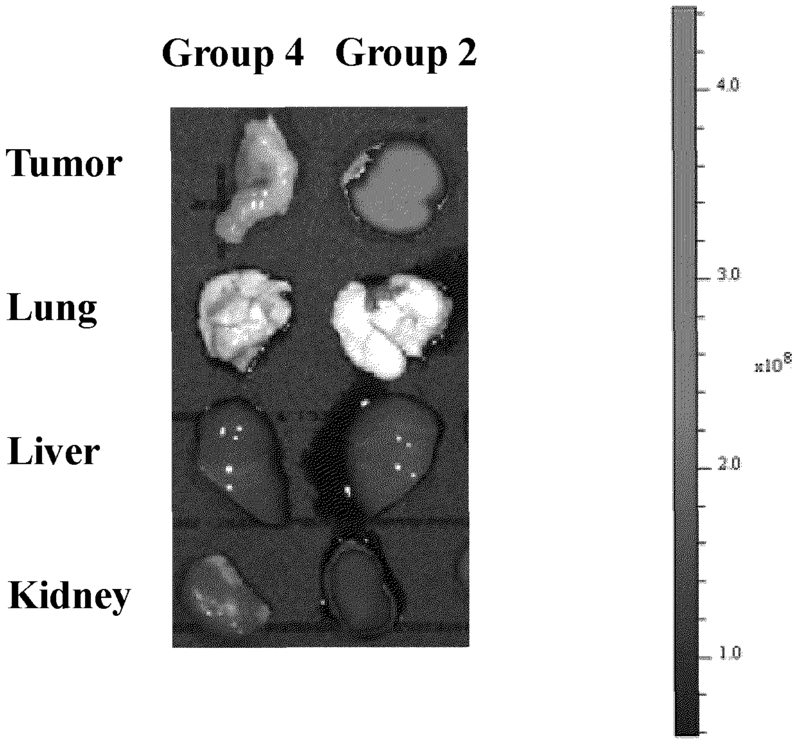


Figure 8D

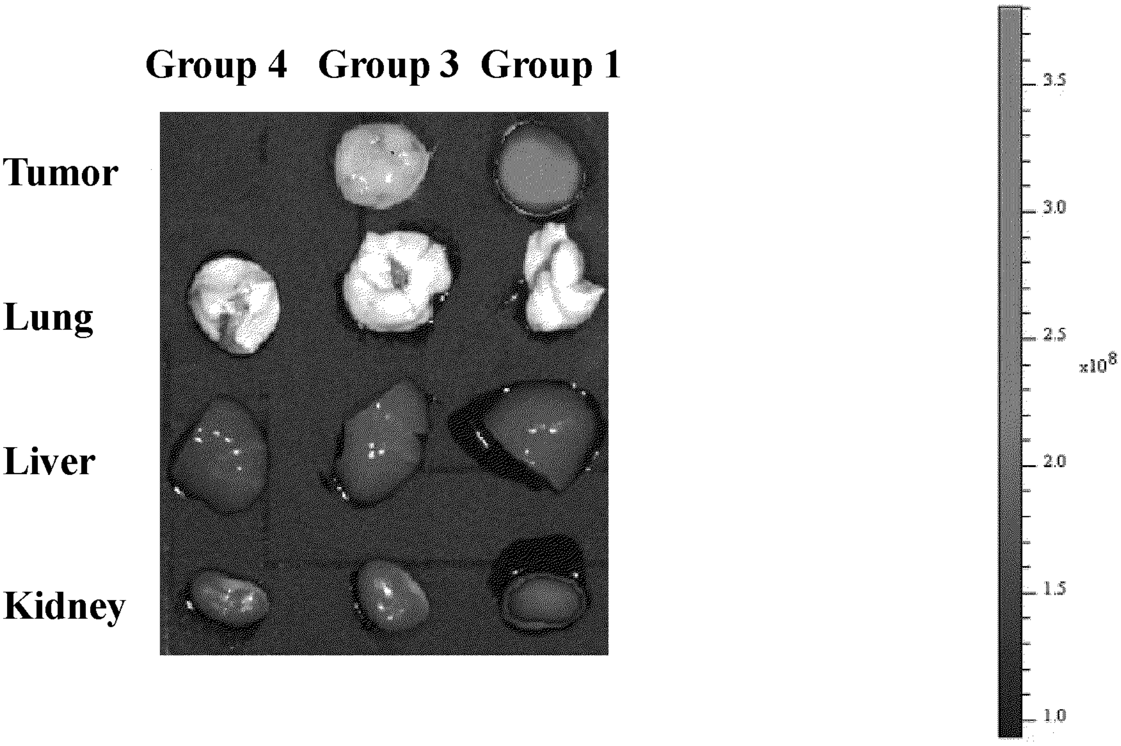


Figure 8E

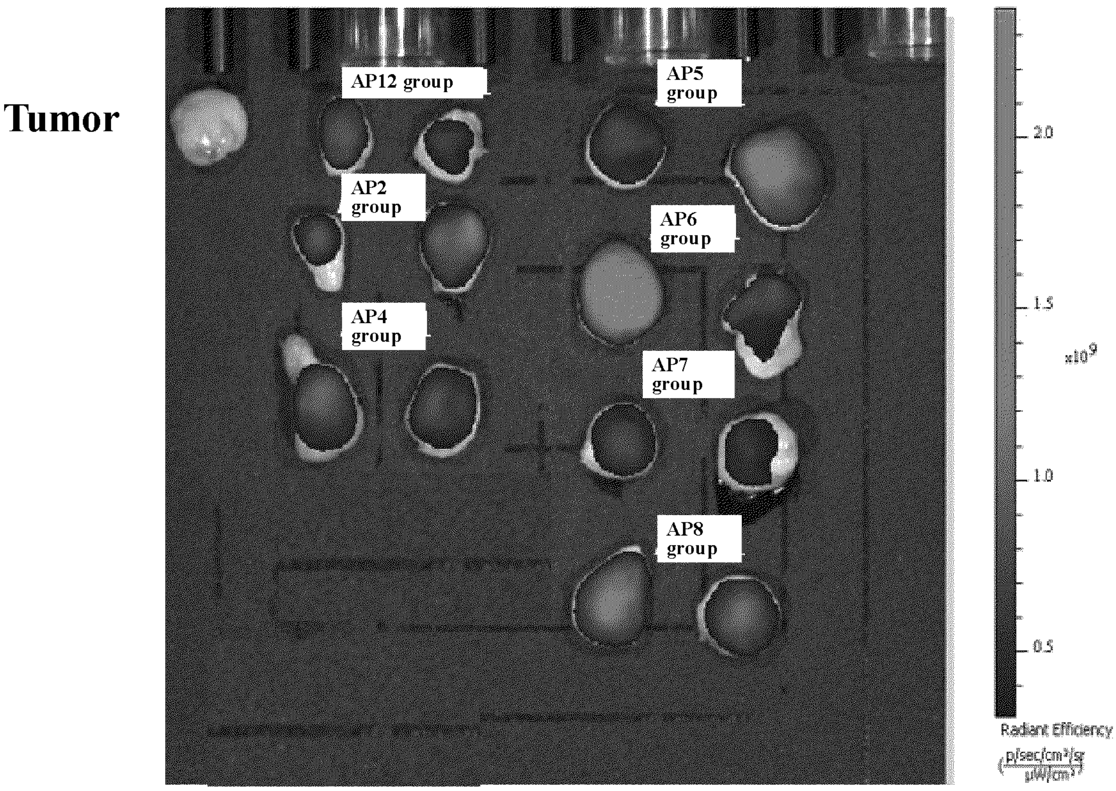


Figure 9

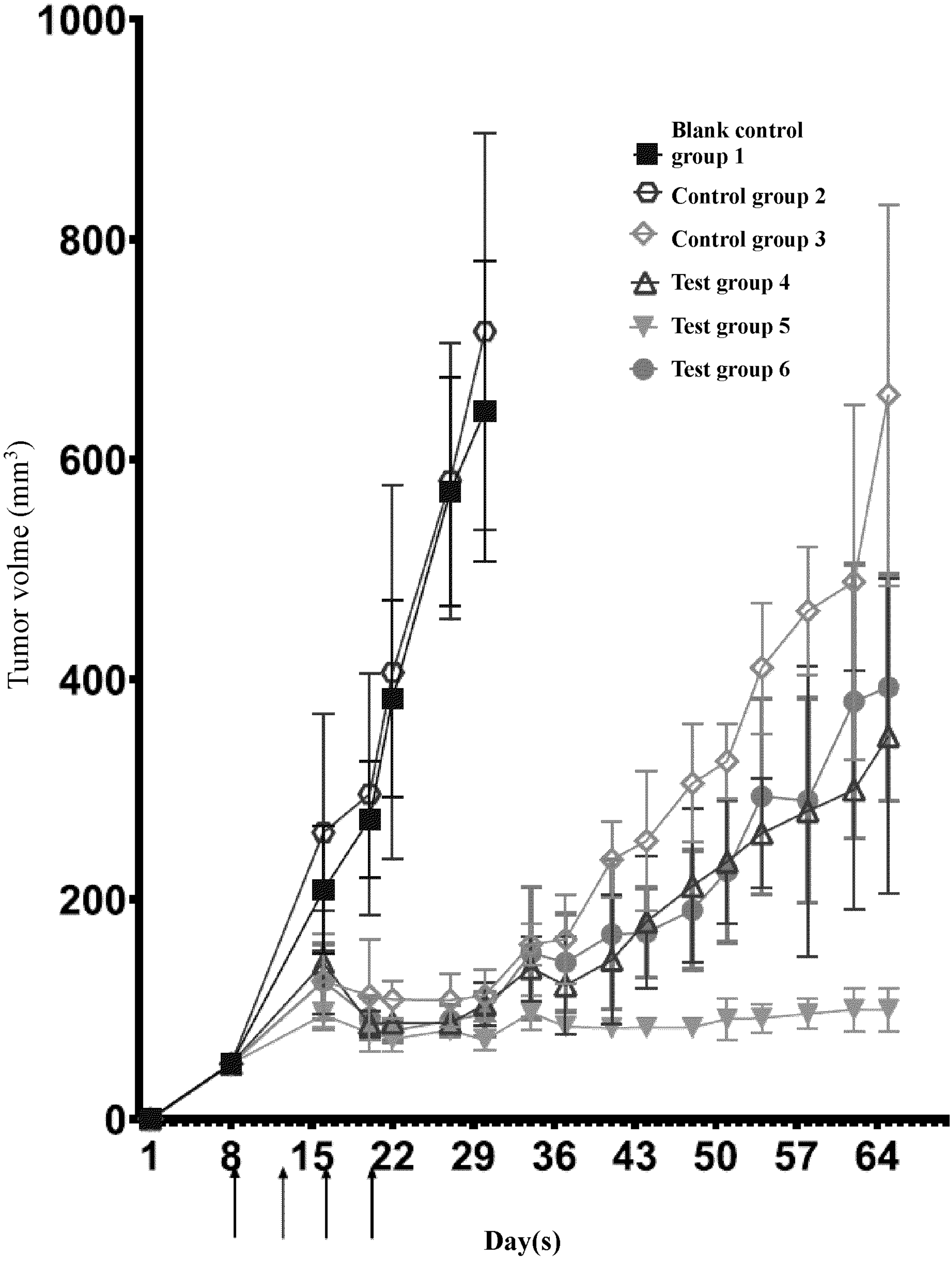
1000
800
600
400
200
0
Tumor volme (mm3)
1
8
15
22
29
36
43
50
57
64
Day(s)
Blank control group 1
Control group 2
Control group 3
Test group 4
Test group 5
Test group 6

Figure 10

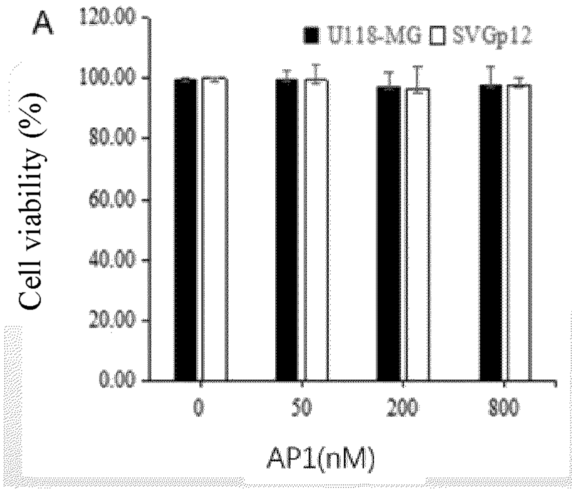


Figure 11A

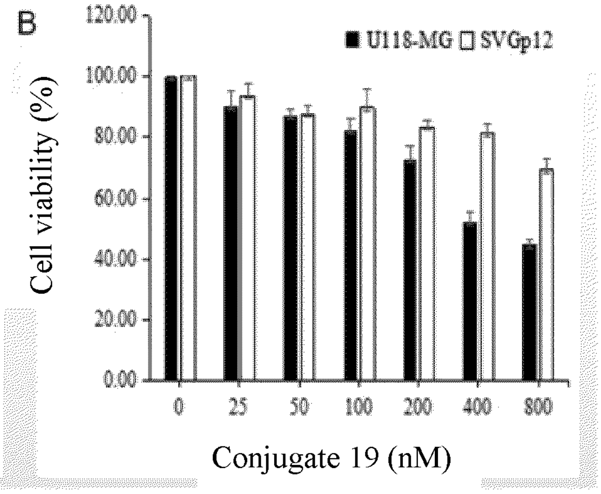


Figure 11B

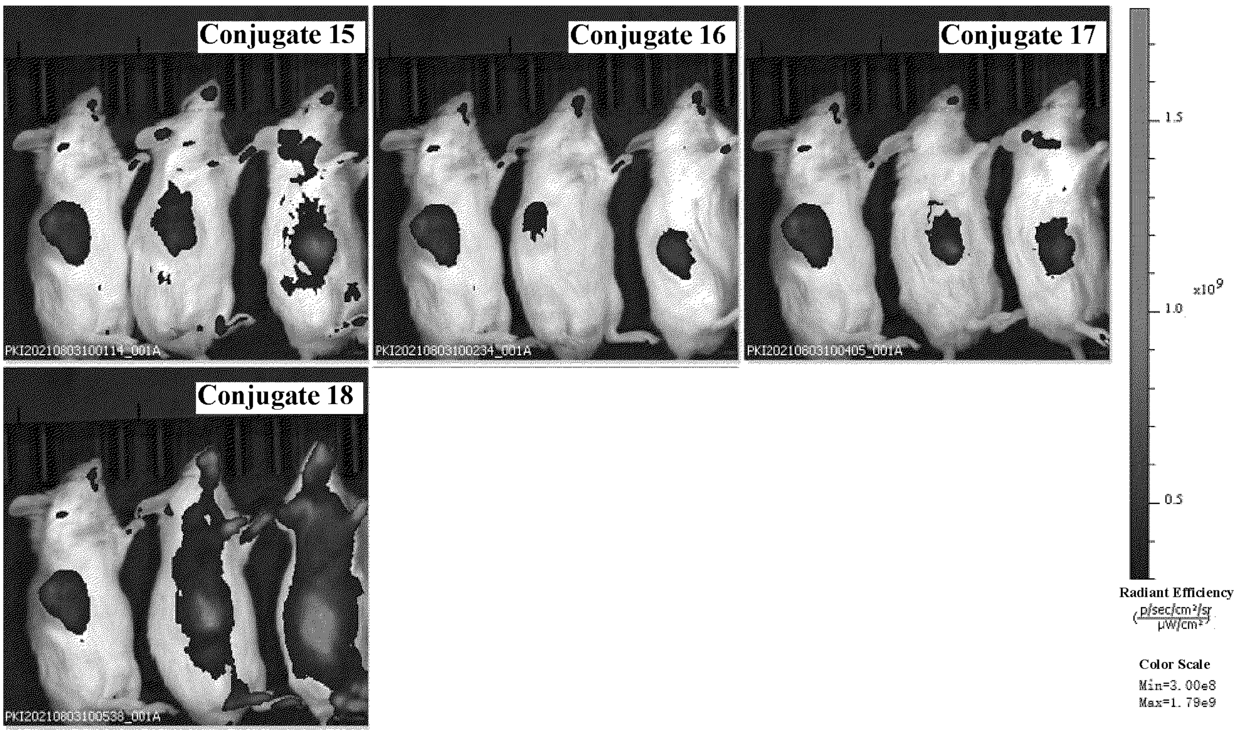


Figure 12

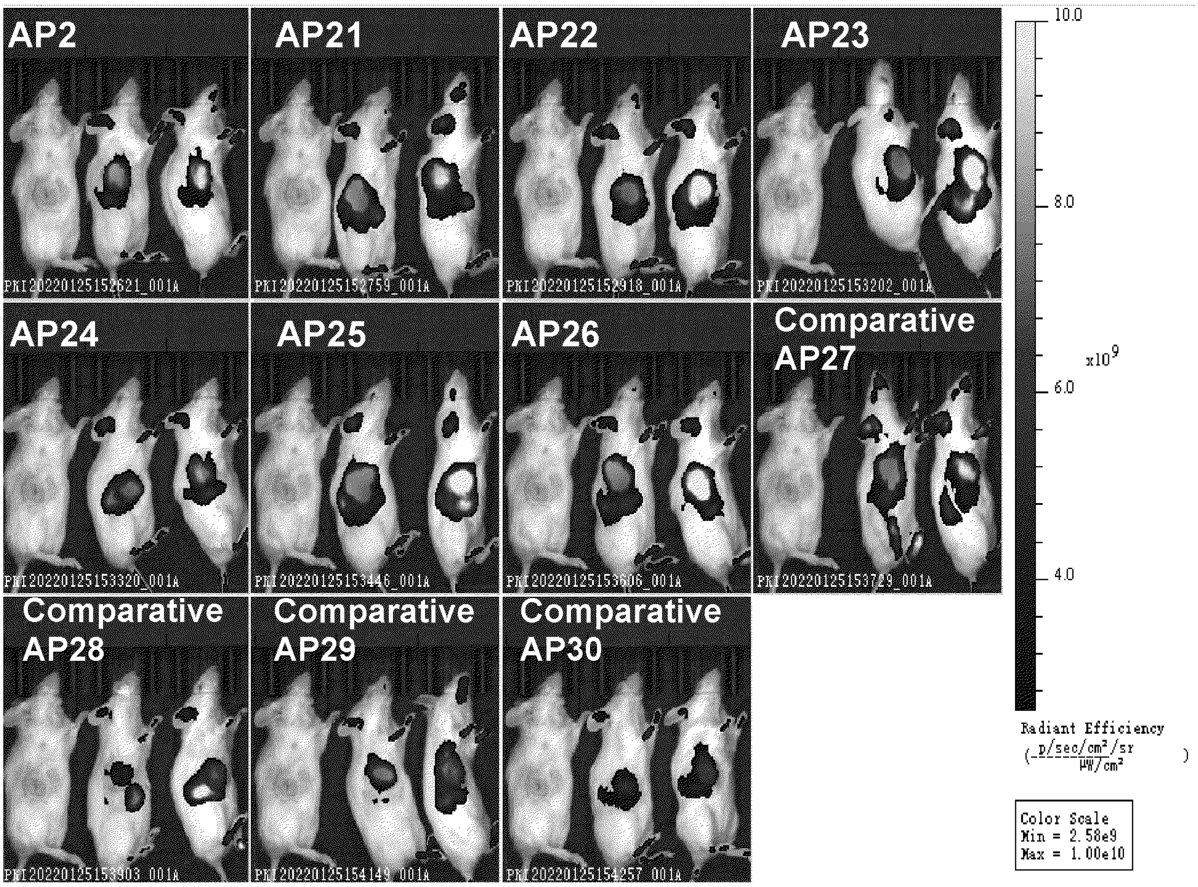


Figure 13A

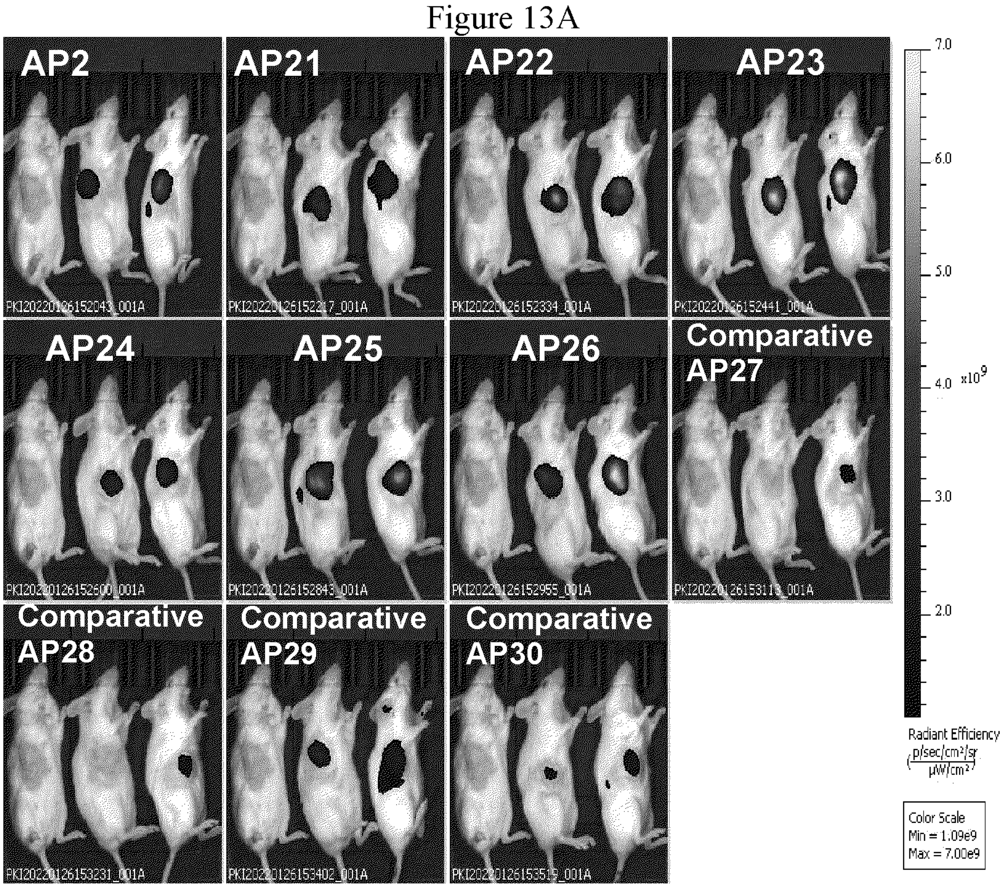


Figure 13B

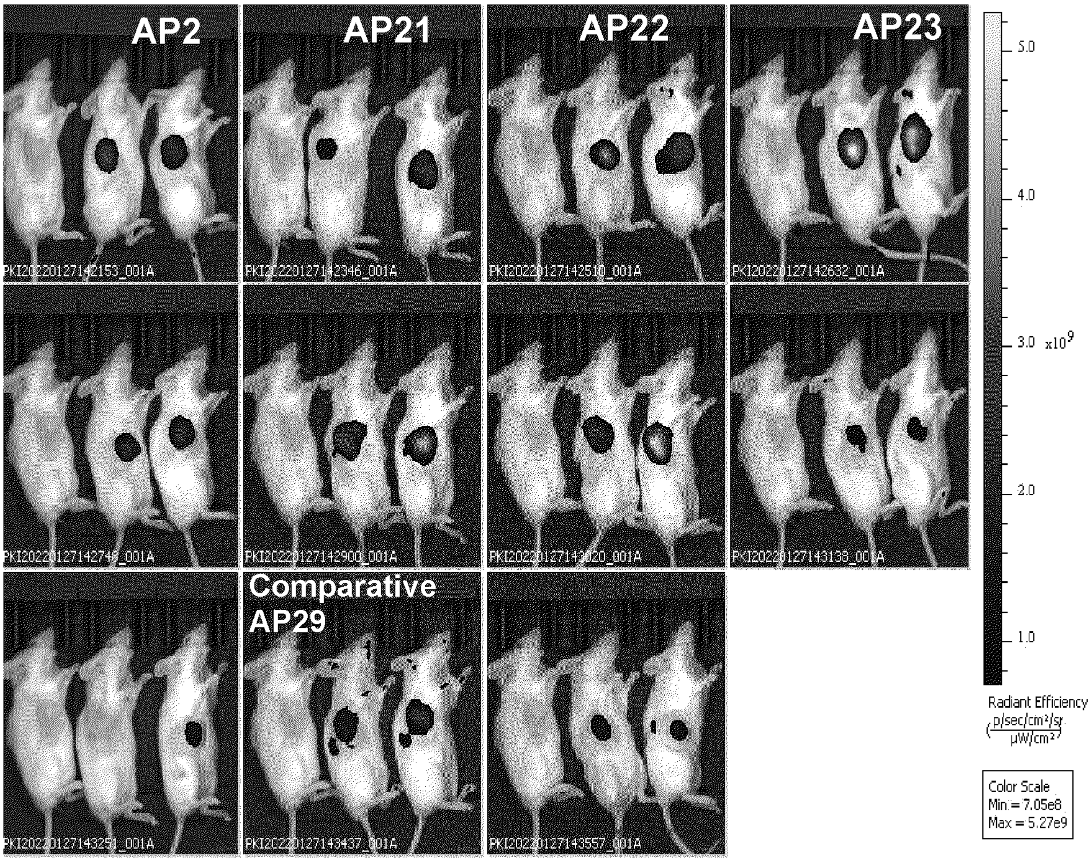


Figure 13C

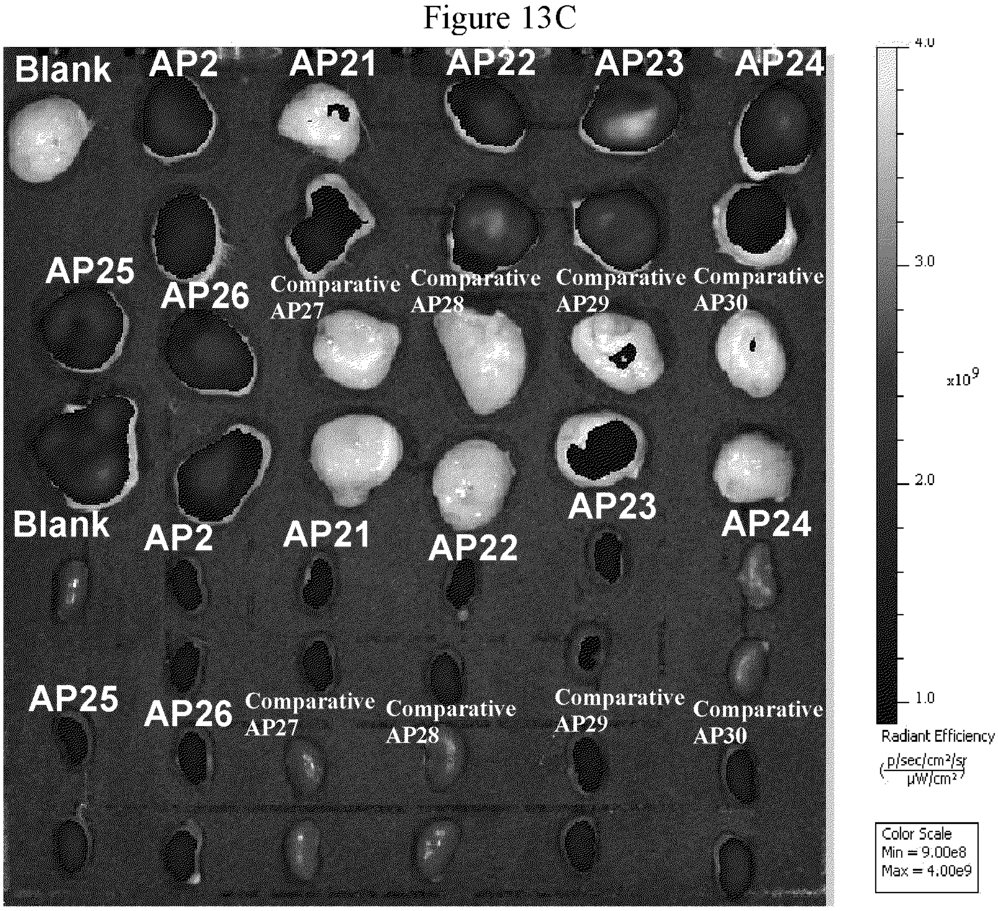


Figure 13D

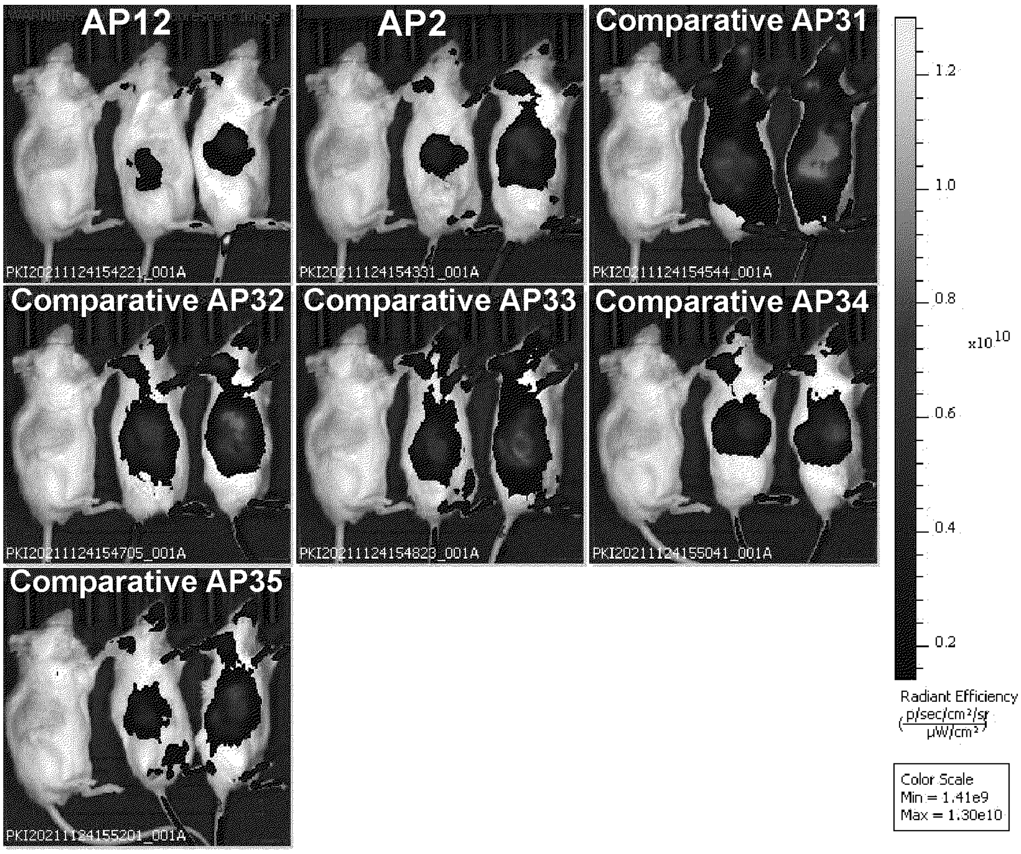


Figure 14A

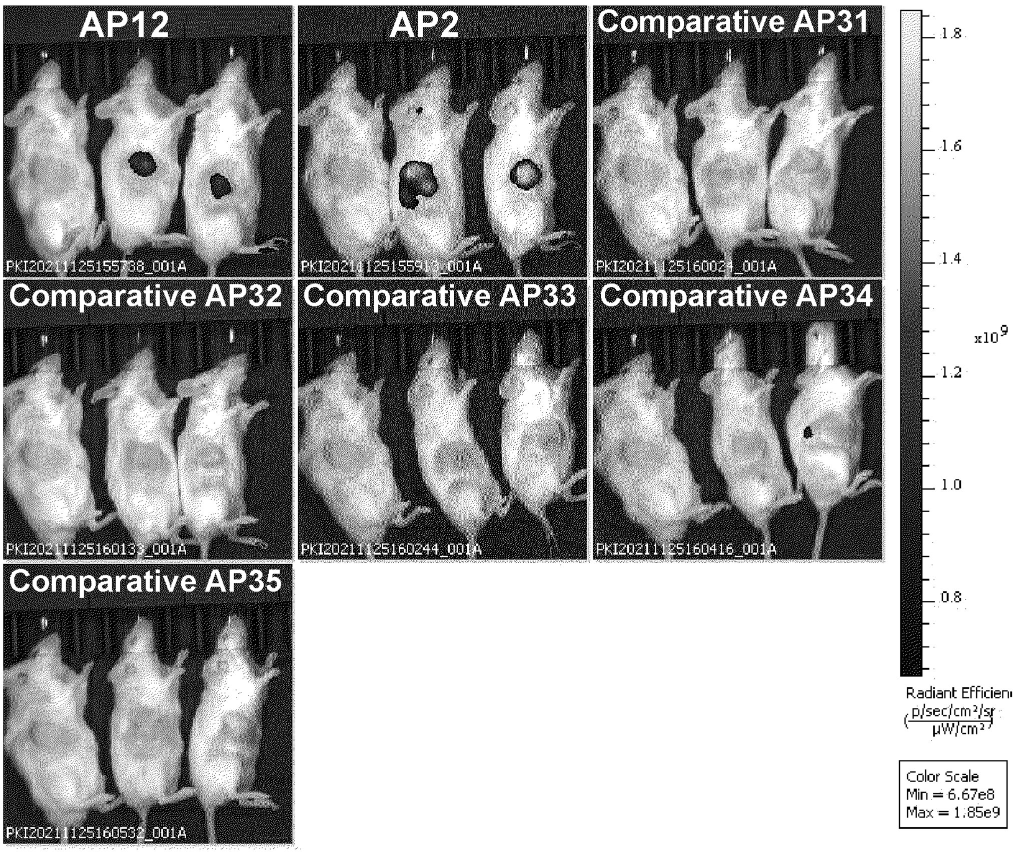


Figure 14B

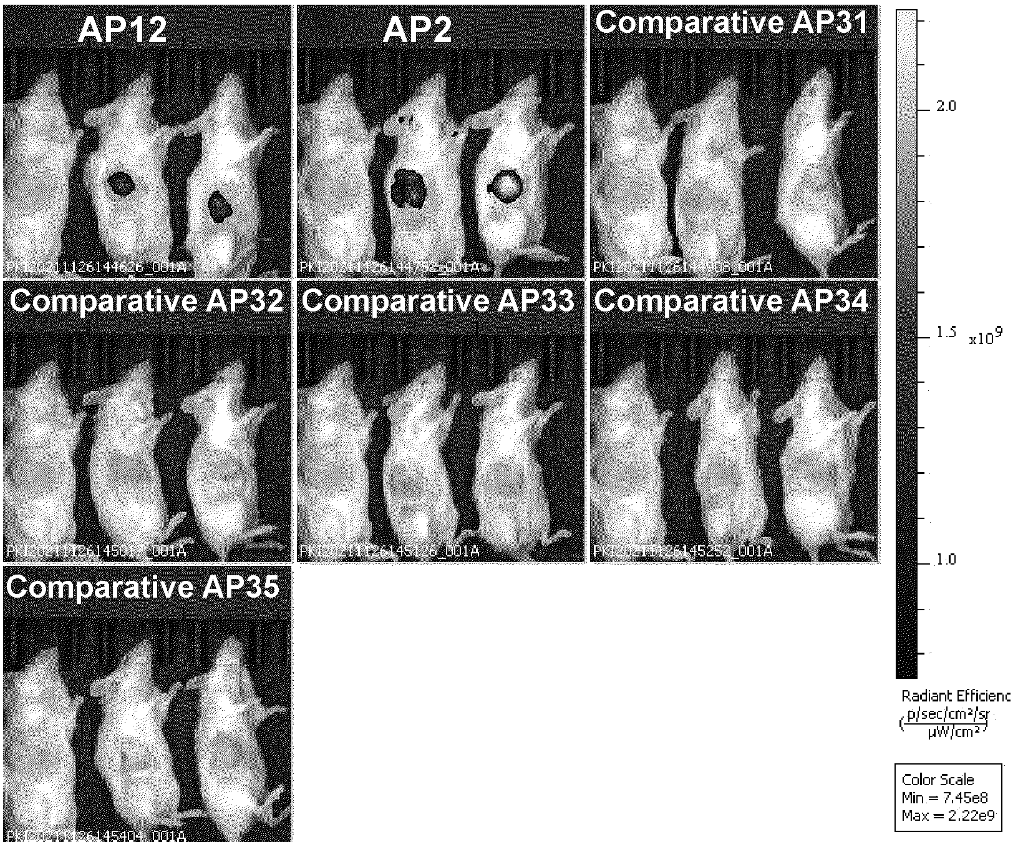


Figure 14C

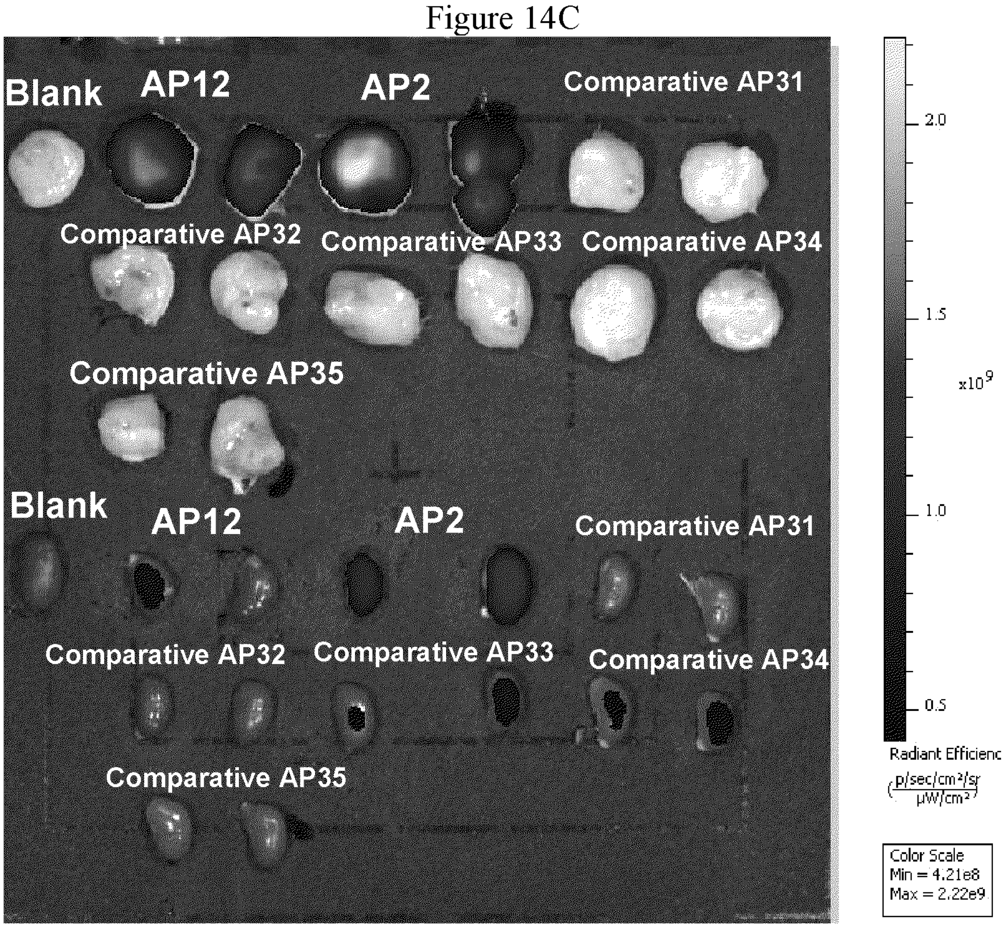


Figure 14D

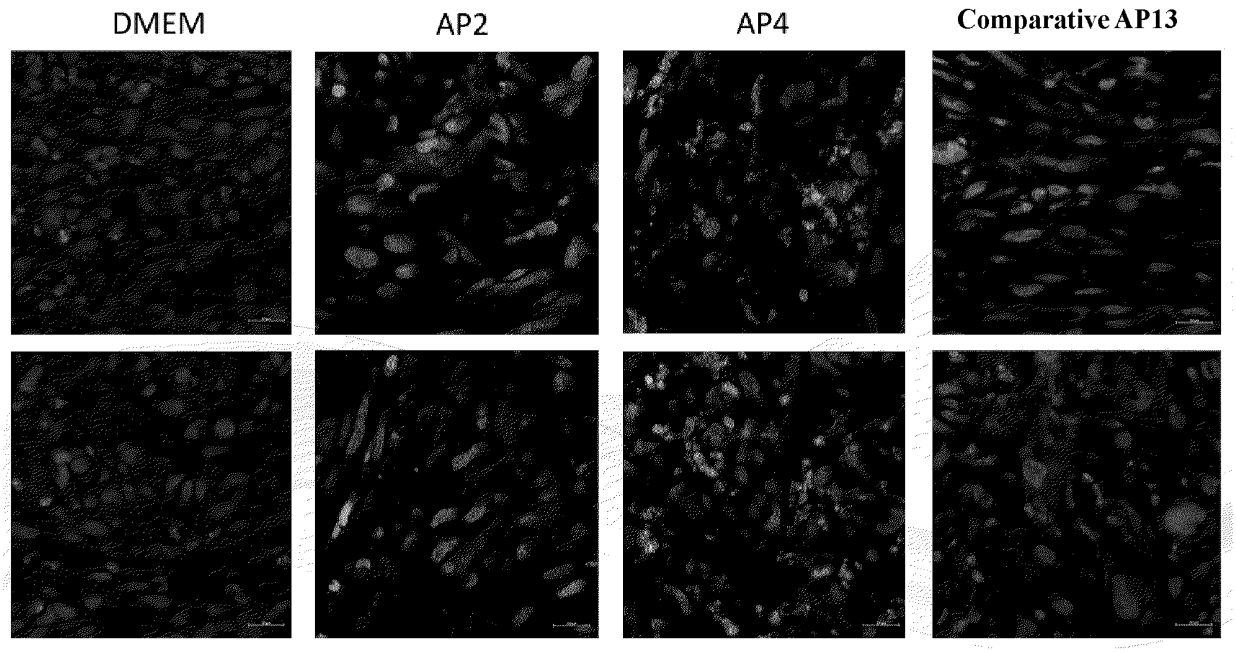


Figure 15

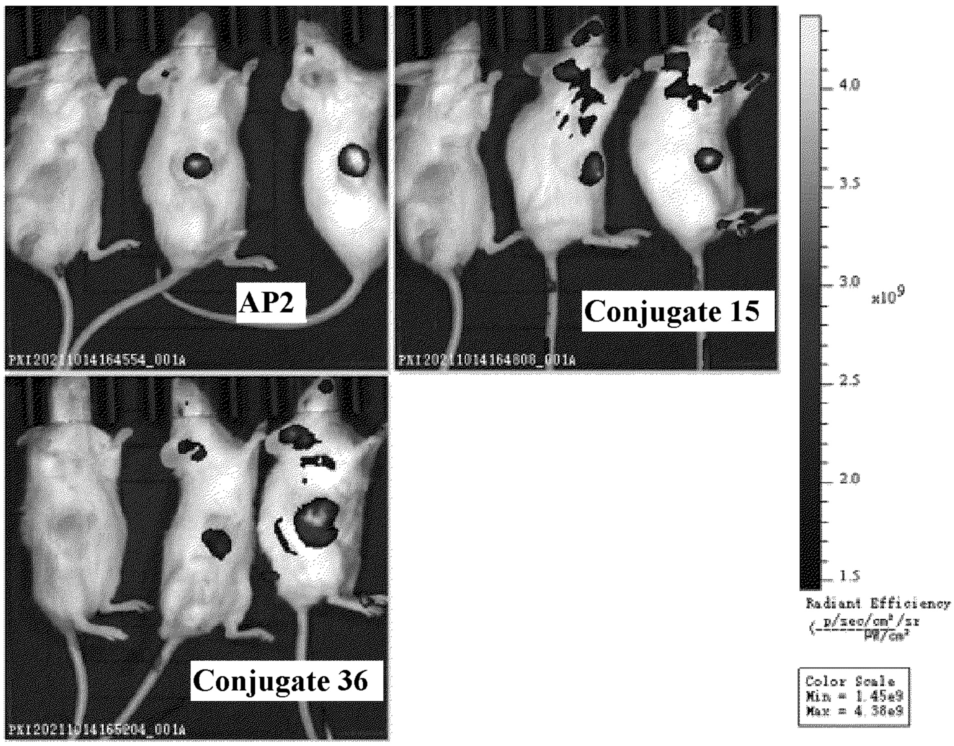


Figure 16A

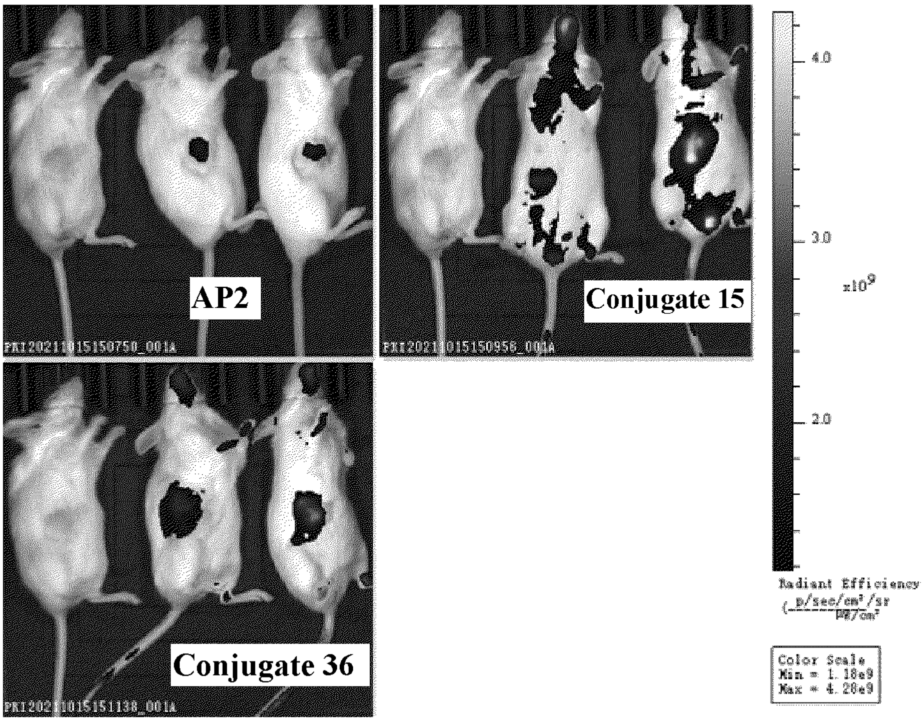


Figure 16B

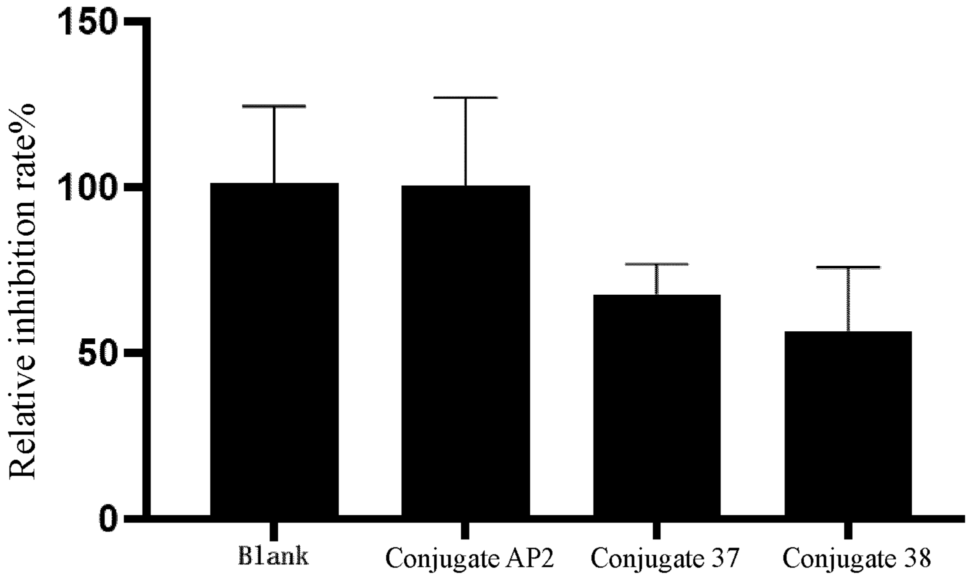


Figure 17

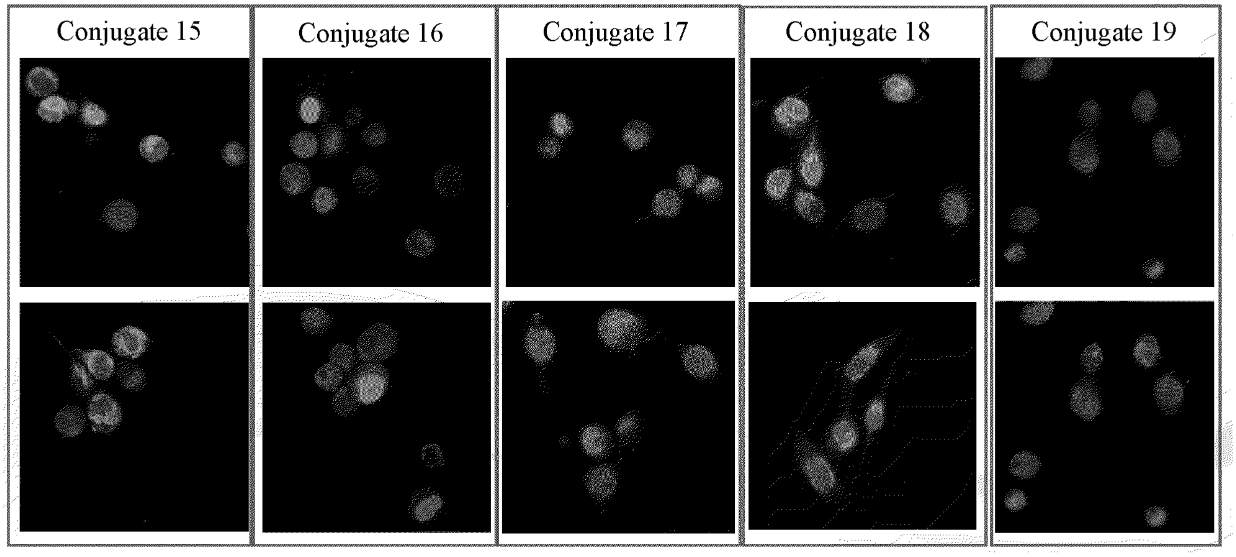


Figure 18

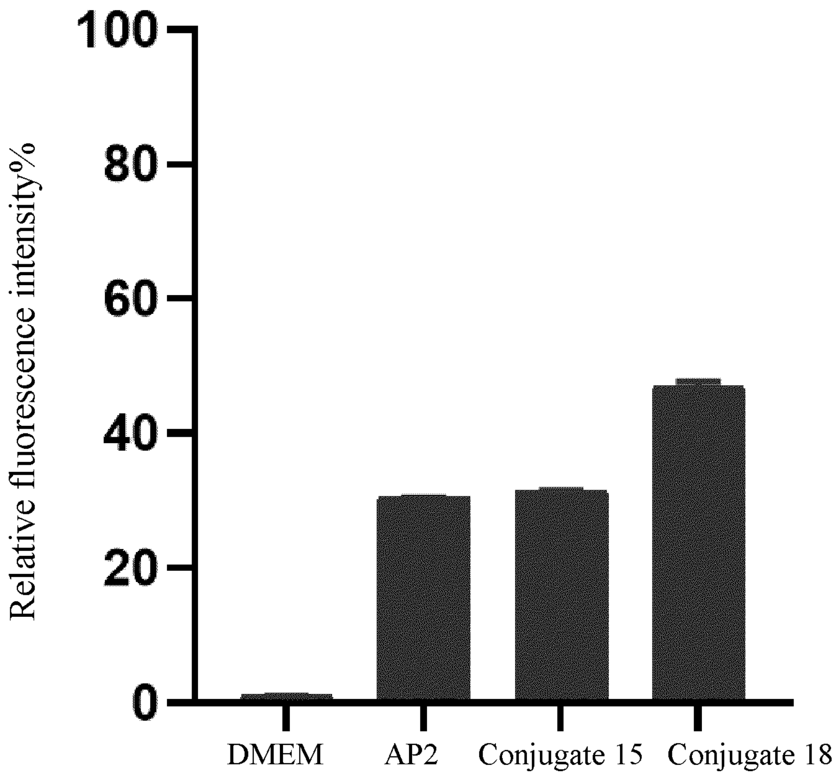


Figure 19

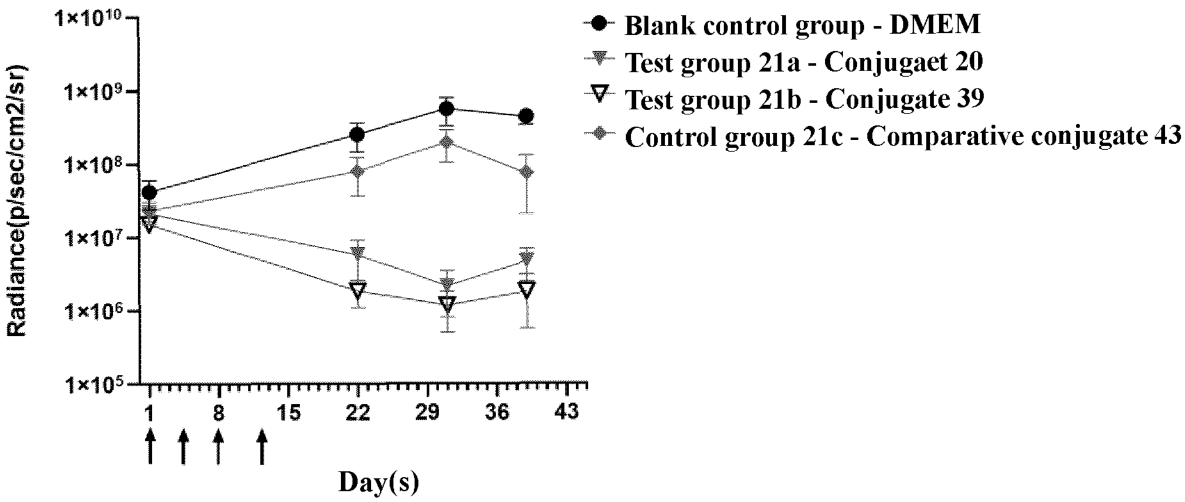


Figure 20

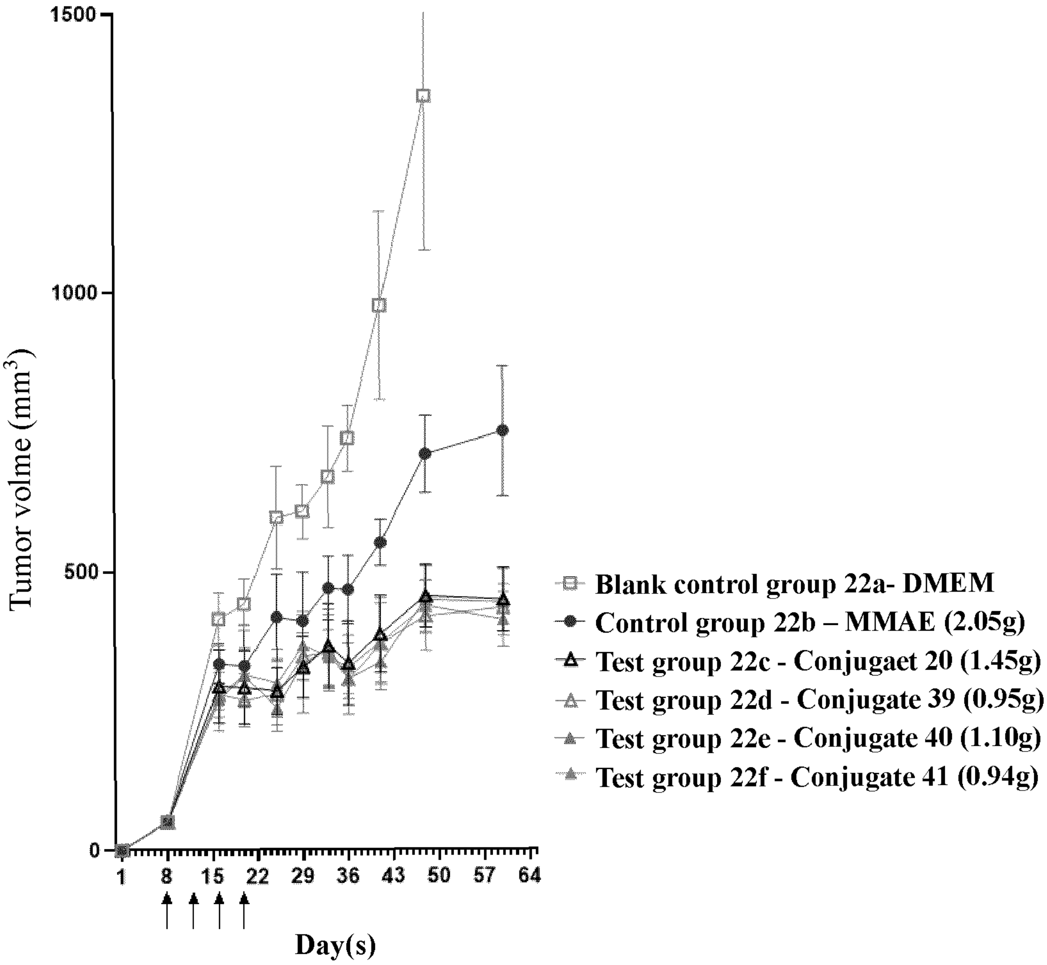


Figure 21

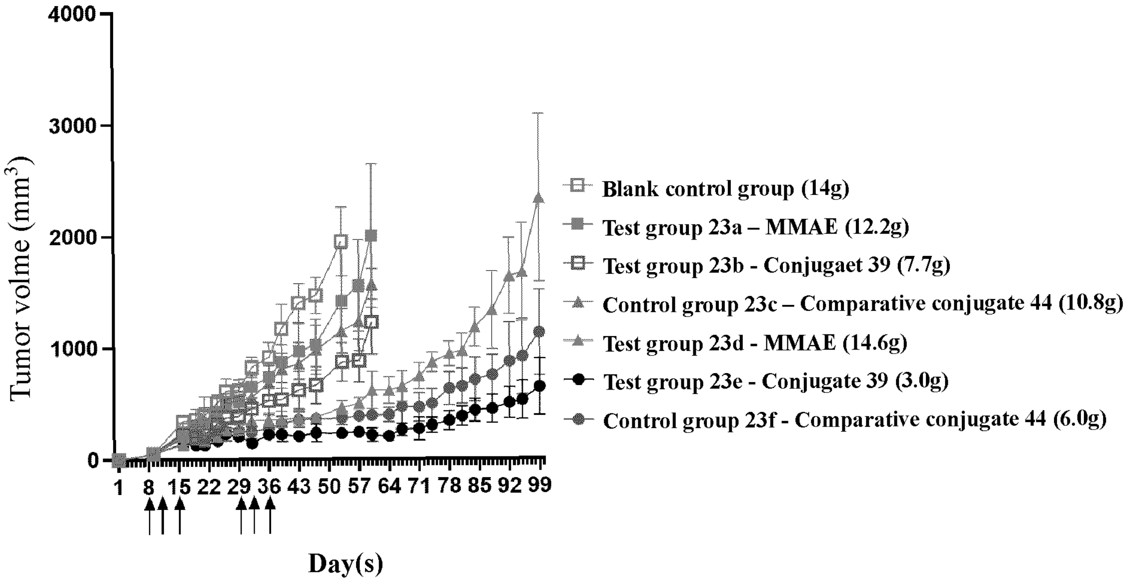


Figure 22

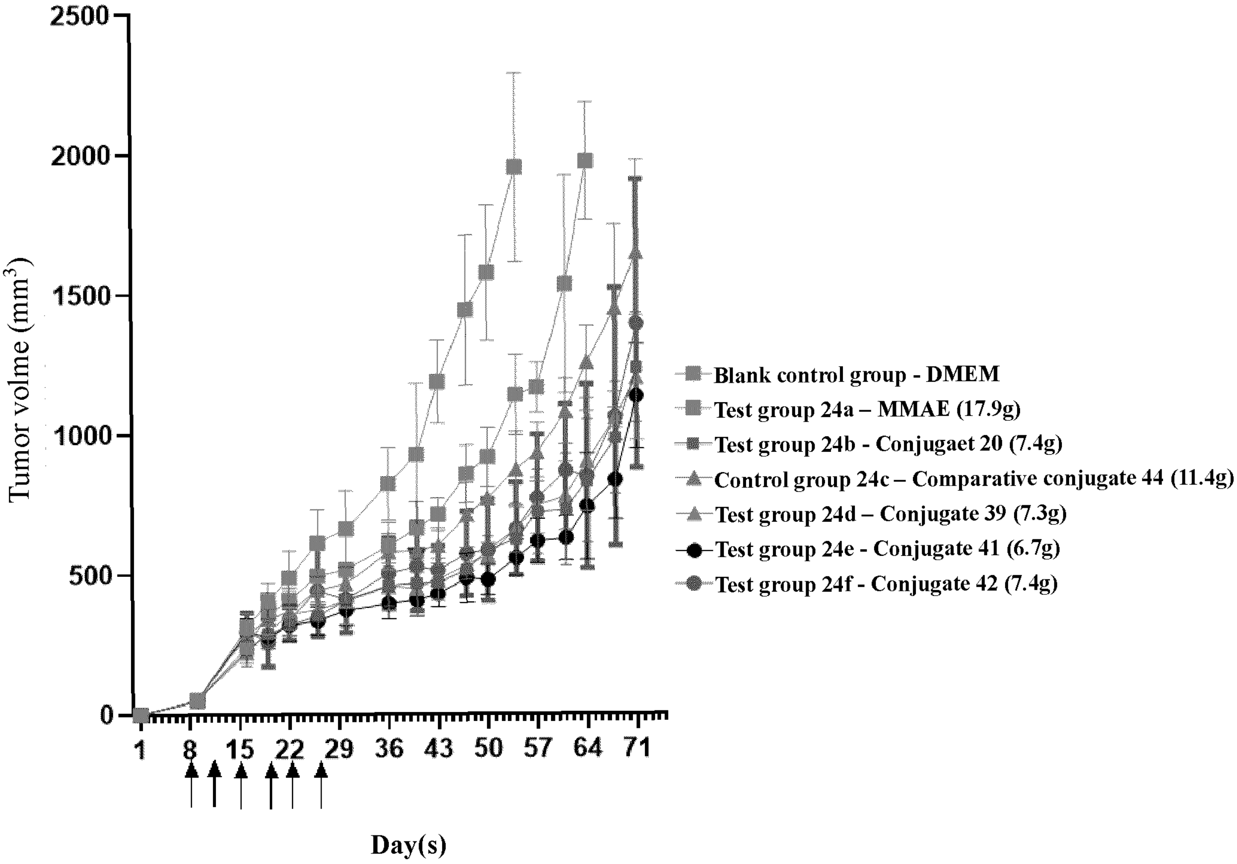


Figure 23

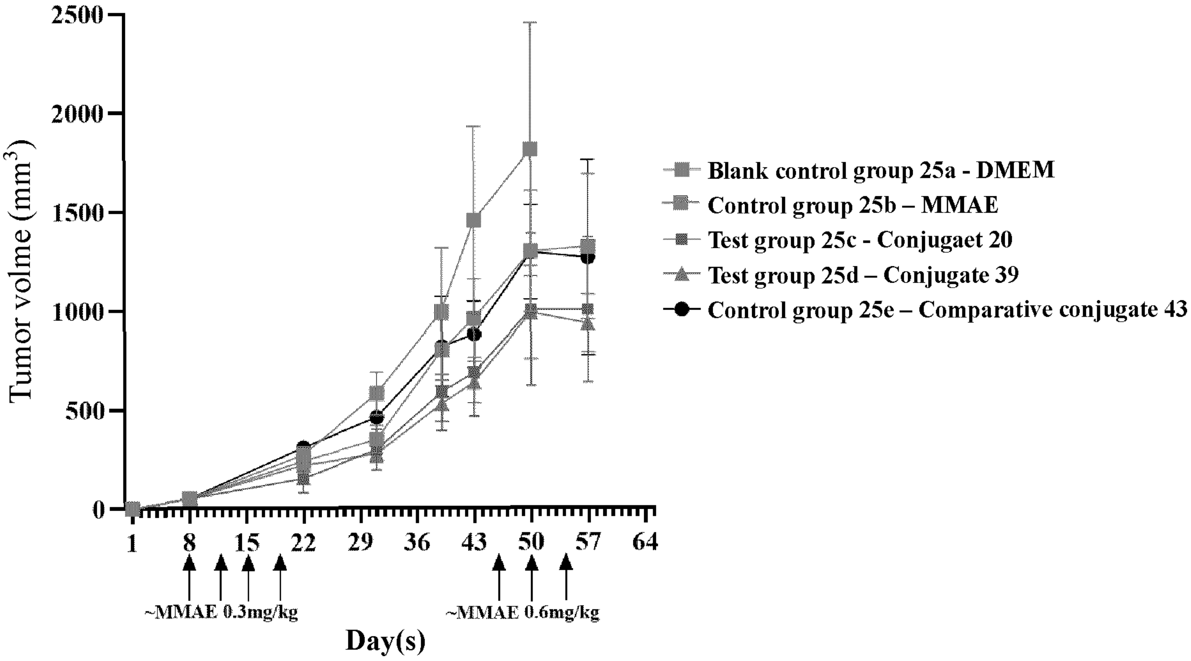

2500
2000
1500
1000
500
0
Tumor volme (mm3)
1
8
15
22
29
36
43
50
57
64
~MMAE 0.3mg/kg
~MMAE 0.6mg/kg
Day(s)
Blank control group 25a - DMEM
Control group 25b – MMAE
Test group 25c - Conjugaet 20
Test group 25d – Conjugate 39
Control group 25e – Comparative conjugate 43


Figure 24

## INTERNATIONAL SEARCH REPORT

International application No.
**PCT/CN2023/088466**

**A. CLASSIFICATION OF SUBJECT MATTER**

A61K48/00(2006.01)i；A61P35/00(2006.01)i；C12N15/113(2010.01)i

According to International Patent Classification (IPC) or to both national classification and IPC

**B. FIELDS SEARCHED**

Minimum documentation searched (classification system followed by classification symbols)

A61K，A61P，C12N

Documentation searched other than minimum documentation to the extent that such documents are included in the fields searched

Electronic data base consulted during the international search (name of data base and, where practicable, search terms used)

CNTXT, CNABS, VEN, ENTXT, 万方, WANFANG, CNKI, PubMed, GenBank, ISI web of knowledge: 适配体, siRNA, 核苷酸, 修饰, aptamer, nucleotide, modification, SEQ ID NO: 1-3, 15-72

**C. DOCUMENTS CONSIDERED TO BE RELEVANT**

| Category* | Citation of document, with indication, where appropriate, of the relevant passages | Relevant to claim No. |
|---|---|---|
| A | CN 110945132 A (SUZHOU RIBO LIFE SCIENCE CO., LTD.) 31 March 2020 (2020-03-31) entire document | 1-53 |
| A | CN 110997919 A (SUZHOU RIBO LIFE SCIENCE CO., LTD.) 10 April 2020 (2020-04-10) entire document | 1-53 |
| A | CN 102533770 A (GUANGZHOU RIBOBIO CO., LTD.) 04 July 2012 (2012-07-04) entire document | 1-53 |
| A | CN 108289906 A (PUBLIC UNIVERSITY CORPORATION NAGOYA CITY UNIVERSITY et al.) 17 July 2018 (2018-07-17) entire document | 1-53 |
| A | WO 2017035340 A1 (ALNYLAM PHARMACEUTICALS, INC.) 02 March 2017 (2017-03-02) entire document | 1-53 |
| A | WO 2021204216 A1 (BEIJING RIBOCURE PHARMACEUTICALS CO., LTD.) 14 October 2021 (2021-10-14) entire document | 1-53 |

☐ Further documents are listed in the continuation of Box C. ☑ See patent family annex.

* Special categories of cited documents:
“A” document defining the general state of the art which is not considered to be of particular relevance
“D” document cited by the applicant in the international application
“E” earlier application or patent but published on or after the international filing date
“L” document which may throw doubts on priority claim(s) or which is cited to establish the publication date of another citation or other special reason (as specified)
“O” document referring to an oral disclosure, use, exhibition or other means
“P” document published prior to the international filing date but later than the priority date claimed
“T” later document published after the international filing date or priority date and not in conflict with the application but cited to understand the principle or theory underlying the invention
“X” document of particular relevance; the claimed invention cannot be considered novel or cannot be considered to involve an inventive step when the document is taken alone
“Y” document of particular relevance; the claimed invention cannot be considered to involve an inventive step when the document is combined with one or more other such documents, such combination being obvious to a person skilled in the art
“&” document member of the same patent family

| Date of the actual completion of the international search | Date of mailing of the international search report |
|---|---|
| **12 July 2023** | **18 July 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2023/088466**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**

International application No.
**PCT/CN2023/088466**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **52**
because they relate to subject matter not required to be searched by this Authority, namely:

Claim 52 relates to a method for diagnosis and/or treatment of a living human or animal body, which falls within the cases set out in PCT Rule 39.1(IV) for which no international search is required. In the present search report, a search is performed on the basis of "the use in preparation of a pharmaceutical composition".

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2023/088466**

| Patent document cited in search report | Publication date (day/month/year) | Patent family member(s) | Publication date (day/month/year) |
|---|---|---|---|
| CN 110945132 A | 31 March 2020 | AU 2018377716 A1 | 09 April 2020 |
| | | JP 2021503930 A | 15 February 2021 |
| | | RU 2020118025 A | 04 January 2022 |
| | | RU 2020118025 A3 | 09 March 2022 |
| | | CA 3083970 A1 | 06 June 2019 |
| | | US 2022395526 A1 | 15 December 2022 |
| | | TW 201928057 A | 16 July 2019 |
| | | KR 20200095483 A | 10 August 2020 |
| | | WO 2019105437 A1 | 06 June 2019 |
| | | EP 3719125 A1 | 07 October 2020 |
| | | EP 3719125 A4 | 08 September 2021 |
| CN 110997919 A | 10 April 2020 | WO 2019105418 A1 | 06 June 2019 |
| | | KR 20200091414 A | 30 July 2020 |
| | | EP 3719128 A1 | 07 October 2020 |
| | | EP 3719128 A4 | 27 October 2021 |
| | | CA 3083968 A1 | 06 June 2019 |
| | | US 2023132756 A1 | 04 May 2023 |
| | | TW 201925469 A | 01 July 2019 |
| | | TWI 791696 B | 11 February 2023 |
| | | US 2022049249 A1 | 17 February 2022 |
| | | US 11492620 B2 | 08 November 2022 |
| | | JP 2021504415 A | 15 February 2021 |
| | | AU 2018374219 A1 | 21 May 2020 |
| | | AU 2018374219 B2 | 15 December 2022 |
| | | AU 2018374219 C1 | 11 May 2023 |
| CN 102533770 A | 04 July 2012 | None | |
| CN 108289906 A | 17 July 2018 | US 2018334674 A1 | 22 November 2018 |
| | | US 11066665 B2 | 20 July 2021 |
| | | EP 3378482 A1 | 26 September 2018 |
| | | EP 3378482 A4 | 31 July 2019 |
| | | JP 6198201 B1 | 20 September 2017 |
| | | JPWO 2017086467 A1 | 16 November 2017 |
| | | WO 2017086467 A1 | 26 May 2017 |
| WO 2017035340 A1 | 02 March 2017 | KR 20180054640 A | 24 May 2018 |
| | | AU 2016310494 A1 | 08 March 2018 |
| | | AU 2016310494 B2 | 09 June 2022 |
| | | US 2021163951 A1 | 03 June 2021 |
| | | IL 293355 A | 01 July 2022 |
| | | HK 1257523 A1 | 25 October 2019 |
| | | EP 3340994 A1 | 04 July 2018 |
| | | JP 2018525416 A | 06 September 2018 |
| | | JP 6941598 B2 | 29 September 2021 |
| | | JP 2022000433 A | 04 January 2022 |
| | | MX 2018002158 A | 06 July 2018 |
| | | EA 201890571 A1 | 31 October 2018 |
| | | US 2020140871 A1 | 07 May 2020 |
| | | US 10851377 B2 | 01 December 2020 |
| | | US 2018187198 A1 | 05 July 2018 |
| | | US 2023104125 A1 | 06 April 2023 |
| | | CA 2996701 A1 | 02 March 2017 |

Form PCT/ISA/210 (patent family annex) (July 2022)

**INTERNATIONAL SEARCH REPORT**
**Information on patent family members**

International application No.
**PCT/CN2023/088466**

| Patent document cited in search report | Publication date (day/month/year) | Patent family member(s) | Publication date (day/month/year) |
|---|---|---|---|
| | | IL 257486 A | 28 June 2018 |
| | | IL 257486 B | 01 July 2022 |
| | | AU 2022228147 A1 | 17 November 2022 |
| | | WO 2017035340 A9 | 27 April 2017 |
| WO 2021204216 A1 | 14 October 2021 | None | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 103380113 A


### Non-patent literature cited in the description


- **MCKERTISH CM** ; **KAYSER V**. Advances and Limitations of Antibody Drug Conjugates for Cancer. *Biomedicines*, 23 July 2021, vol. 9 (8), 872
- Oligonucleotide Synthesis: Methods and Applications. *Methods in Molecular Biology*, vol. 288, 17, 31
- **ØSTERGAARD, MICHAEL E. et al.** Efficient synthesis and biological evaluation of 5'-GalNAc conjugated antisense oligonucleotides. *Bioconjugate chemistry*, 2015, vol. 26 (8), 1451-1455
- Molecular Cloning. Cold Spring Harbor LBboratory Press, 1989
- Engineering of Targeted Nanoparticles for Cancer Therapy Using Internalizing Aptamers Isolated by Cell-Uptake Selection. *ACS Nano*, 24 January 2012, vol. 6 (1)